(19) Europäisches Patentamt
European Patent Office
Office européen des brevets

(11) **EP 1 882 748 A2**

(12) **EUROPEAN PATENT APPLICATION**

(43) Date of publication:
**30.01.2008 Bulletin 2008/05**

(51) Int Cl.:
***C12Q 1/68*** *(2006.01)* ***C07H 21/00*** *(2006.01)*
***C07H 19/00*** *(2006.01)*

(21) Application number: **07119296.7**

(22) Date of filing: **11.09.2003**

(84) Designated Contracting States:
**AT BE BG CH CY CZ DE DK EE ES FI FR GB GR HU IE IT LI LU MC NL PT RO SE SI SK TR**

(30) Priority: **11.09.2002 US 410061 P**

(62) Document number(s) of the earlier application(s) in accordance with Art. 76 EPC:
**03794824.7 / 1 546 404**

(71) Applicant: **Exiqon A/S**
**2950 Vedbaek (DK)**

(72) Inventors:
- **Ramsing, Niels Birger**
  **8240 Risskov (DK)**
- **Nielsen, Alex Toftgaard**
  **2980 Kokkedal (DK)**
- **Kochkin, Alexei A.**
  **3000 Helsingør (DK)**
- **Tolstrup, Niels**
  **2930 Klampenborg (DK)**
- **Pfundheller, Henrik M.**
  **2970 Hørsholm (DK)**
- **Lomholt, Christian**
  **1963 Frederiksberg C (DK)**

(74) Representative: **Inspicos A/S**
**Bøge Allé 5**
**P.O. Box 45**
**2970 Hørsholm (DK)**

Remarks:
This application was filed on 25-10-2007 as a divisional application to the application mentioned under INID code 62.

# (54) A population of nucleic acids including a subpopulation of LNA oligomers

(57) The present application discloses LNA-2,6-diaminopurine (LNA-D) and LNA-2-aminopurine (LNA-2AP) of the formulae

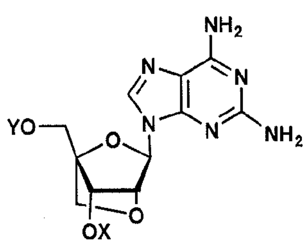

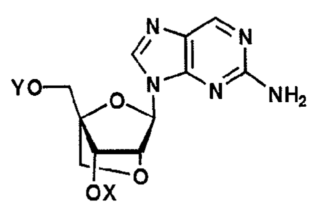

and corresponding methods for the preparation thereof, as well as LNA oligomers comprising such LNA-2,6-diaminopurine (LNA-D) and/or LNA-2-aminopurine (LNA-2AP) units. The application also discloses a pair of substantially complementary oligonucleotides, each comprising, in pairwise opposing positions, one or more SBC nucleotides or units, wherein at least one of the oligonucleotides is an LNA oligomer having SBC LNA units.

EP 1 882 748 A2

Figure 4

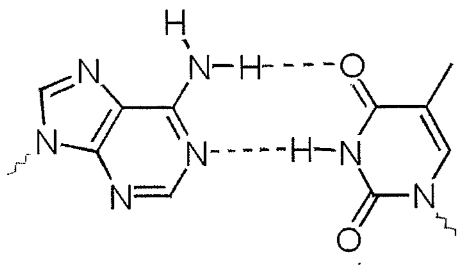

A - T

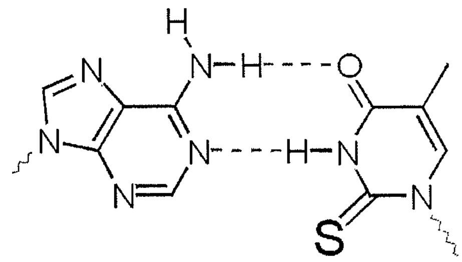

A - $^{2S}$T

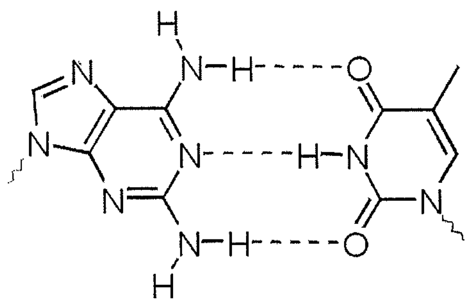

D - T

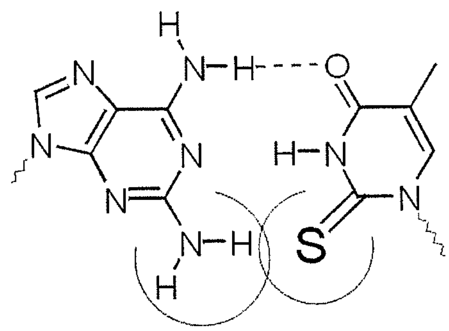

D - $^{2S}$T

SEQUENCE LISTING

<110>  Exiqon A/S

<120>  A poplulation of nucleic acids including a subpopulation of LNA oligomers

<130>  15603EP01

<160>  26

<170>  PatentIn version 3.2

<210>  1
<211>  45
<212>  DNA
<213>  unidentified

<400>  1
ttaccagtac cttttcaaat cgattctcaa ttcaaattca tcaaa    45

<210>  2
<211>  45
<212>  DNA
<213>  Artificial

<220>
<223>  DNA sequence from random synthetic library

<400>  2
ttacaagtac cttttcaaaa cgattctcaa ttcacattca tcaaa    45

<210>  3
<211>  45
<212>  DNA
<213>  artificial

<220>
<223>  DNA sequence from random synthetic library

<400>  3
ttaccggtac cttttcaaat ggattctcaa ttcaatttca tcaaa    45

<210>  4
<211>  45
<212>  DNA
<213>  artificial

<220>
<223>  DNA sequence from random synthetic library

<400>  4
ttaccaatac cttttcaaat ccattctcaa ttcaaactca tcaaa    45

<210>  5

<211> 45
<212> DNA
<213> artificial

<220>
<223> DNA sequence from random synthetic library

<400> 5
ttaccaggac cttttcaaat cgcttctcaa ttcaaataca tcaaa 45

<210> 6
<211> 45
<212> DNA
<213> artificial

<220>
<223> DNA sequence from random synthetic library

<400> 6
ttaccagtgc cttttcaaat cgactctcaa ttcaaattta tcaaa 45

<210> 7
<211> 45
<212> DNA
<213> artificial

<220>
<223> DNA sequence from random synthetic library

<400> 7
ttaccagtaa cttttcaaat cgatgctcaa ttcaaattct tcaaa 45

<210> 8
<211> 45
<212> DNA
<213> artificial

<220>
<223> DNA sequence from random synthetic library

<400> 8
ttaccagtac gttttcaaat cgattttcaa ttcaaattca acaaa 45

<210> 9
<211> 542
<212> DNA
<213> C. elegans

<400> 9
ctccaggaga gaagggagat ggcggtatgc caggaatgcc cggacttcca ggaccatccg 60

gtcgtgatgg atacccagga gaaaagggag accgaggaga tactggaaat gctggaccac 120

gtggaccacc tggagaggct ggatccccag gaaacccagg aatcggaagc attggaccaa 180

aaggagatcc tggagatcta ggttctgtcg gaccaccagg tccaccggga ccacgtgagt 240

tcaccggatc cggctcaatt gtcggacctc gcggaaaccc tggagaaaag ggagacaagg 300

gagagccagg agagggaggt caacgcggtt acccaggaaa tggaggactc tcaggacagc 360

caggactccc aggaatgaag ggagaaaagg gattgtctgg accagctgga ccaagaggaa 420

aggaaggtcg cccaggaaac gctggaccac caggattcaa gggagatcgt ggtcttgacg 480

gacttggcgg aatcccagga cttccaggcc aaaagggaga agctggatac ccaggaagag 540

at 542

<210> 10
<211> 545
<212> DNA
<213> C. elegans

<400> 10
ctccaggaga gaagggagat ggcggtatgc caggaatgcc cggacttcca ggaccatccg 60

gtcgtgatgg atacccagga gaaaagggag accgaggaga tactggaaat gctggaccac 120

gtggaccacc tggagaggct ggatccccag gaaacccagg aatcggaagc attggaccaa 180

aaggagatcc tggagacatt ggtgcgatgg gaccggccgg tccgccaggc ccaatcgcct 240

ccaccatgtc caagggaacc attatcggtc ctaagggaga cctaggagag aagggagaga 300

agggagagcc aggagaggga ggtcaacgcg gttacccagg aaatggagga ctctcaggac 360

agccaggact cccaggaatg aagggagaaa agggattgtc tggaccagct ggaccaagag 420

gaaaggaagg tcgcccagga aacgctggac caccaggatt caagggagat cgtggtcttg 480

acggacttgg cggaatccca ggacttccag gccaaaaggg agaagctgga tacccaggaa 540

gagat 545

<210> 11
<211> 45
<212> DNA
<213> artificial

<220>
<223> DNA sequence from random synthetic library

<400> 11
ttaccagtac cttttcaaat cgattctcaa ttcaaattca tcaaa 45

<210> 12
<211> 22
<212> DNA
<213> artificial

<220>
<223>  T10 linker followed by 5 non-bases and a capture sequence

<220>
<221>  misc_feature
<222>  (11)..(15)
<223>  Phosphate and sugar moieties without attached bases

<220>
<221>  misc_feature
<222>  (16)..(22)
<223>  Any nucleotide or ribonucleotide

<400>  12
tttttttttt nnnnnnnnnn nn    22

<210>  13
<211>  20
<212>  DNA
<213>  Haemophilus influenza

<400>  13
ggtgcaccgg gtgcaggtaa    20

<210>  14
<211>  20
<212>  DNA
<213>  Haemophilus influenza

<400>  14
cctaagattt tatctaactc    20

<210>  15
<211>  20
<212>  DNA
<213>  Haemophilus influenza

<400>  15
atggcaactc aagaagaaaa    20

<210>  16
<211>  20
<212>  DNA
<213>  Haemophilus influenza

<400>  16
ttaccaaaca tcacgcctat    20

<210>  17
<211>  12
<212>  DNA
<213>  artificial

<220>
<223>  DNA sequence from random synthetic library

<400>  17
tgtgggatgt tg                                                          12

<210>  18
<211>  12
<212>  DNA
<213>  artificial

<220>
<223>  DNA sequence from random synthetic library

<220>
<221>  misc_feature
<222>  (7)..(7)
<223>  2,6-diaminopurine

<400>  18
tgtgggntgt tg                                                          12

<210>  19
<211>  12
<212>  DNA
<213>  artificial

<220>
<223>  DNA sequence from random synthetic library

<400>  19
caacatccca ca                                                          12

<210>  20
<211>  12
<212>  DNA
<213>  artificial

<220>
<223>  DNA sequence from random synthetic library

<400>  20
caacaaccca ca                                                          12

<210>  21
<211>  12
<212>  DNA
<213>  artificial

<220>
<223>  DNA sequence from random synthetic library

<400>  21
cactggactc tt                                                          12

<210> 22
<211> 12
<212> DNA
<213> artificial

<220>
<223> DNA sequence from random synthetic library

<220>
<221> misc_feature
<222> (7)..(7)
<223> 2,6-diaminopurine

<400> 22
cactggnctc tt 12

<210> 23
<211> 12
<212> DNA
<213> artificial

<220>
<223> DNA sequence from random synthetic library

<400> 23
aagagtccag tg 12

<210> 24
<211> 12
<212> DNA
<213> artificial

<220>
<223> DNA sequence from random synthetic library

<400> 24
aagaggccag tg 12

<210> 25
<211> 12
<212> DNA
<213> Artificial

<220>
<223> DNA sequence from random synthetic library

<400> 25
aagaccgtgt gc 12

<210> 26
<211> 12
<212> DNA

```
<213>  Artificial

<220>
<223>  DNA sequence from random synthetic library

<400>  26
aagactgtgt gc                                                         12
```

## Description

### FIELD OF THE INVENTION

The present invention relates to oligonucleotides having duplex stabilizing characteristics and/or modified base-pairing characteristics, populations of such oligonucleotides with desirable properties and methods for the use of such oligonucleotides and populations of oligonucleotides.

### BACKGROUND OF THE INVENTION

Oligonucleotides are widely used as research reagents. They are useful for understanding the function of many other biological molecules as well as in the preparation of other molecules. For example, the use of oligonucleotides as primers in PCR reactions has given rise to an expanding commercial industry. PCR has become a mainstay of commercial and research laboratories, and applications of PCR have multiplied. Oligonucleotides, comprised of both natural and synthetic monomers, are employed as primers in such PCR technology.

Oligonucleotides are also used in other laboratory procedures. Several of these uses are described in common laboratory manuals such as Molecular Cloning, A Laboratory Manual, Second Ed., J. Sambrook, et al., Eds., Cold Spring Harbor Laboratory Press, 1989; and Current Protocols In Molecular Biology, F. M. Ausubel, et al., Eds., Current Publications, 1993. Such uses include the (i) synthesis of labeled oligonucleotide probes for visualization after in situ Hybridization, (ii) synthesis of microarray capture probes, (iii) generation of capture probes for nucleic acid sample preparations, (iv) screening expression libraries with oligomeric compounds, (v) DNA sequencing, (vi) *in vitro* amplification of DNA by the polymerase chain reaction, (vii) use of fluorescently labeled oligonuclotides for real time vizualisation of PCR amplification efficiency (e.g. double dye probes, molecular beacons, and scorpions) and (viii) in site-directed mutagenesis of cloned DNA. See Book 2 of Molecular Cloning, A Laboratory Manual, supra. See also "DNA-protein interactions and The Polymerase Chain Reaction" in Vol. 2 of Current Protocols In Molecular Biology, supra. Oligonucleotides have even been used as building blocks in nanotechnology applications to make molecular structures with a defined geometry (cubes, cylinders etc.).

Of particular interest to the present invention is the use of oligonucleotides as capture probes in DNA microarrays. With the advent of microarrays for profiling the expression of thousands of genes, such as GeneChip™ arrays (Affymetrix, Inc., Santa Clara, CA), correlations between expressed genes and cellular phenotypes may be identified at a fraction of the cost and labor necessary for traditional methods, such as Northern- or dot-blot analysis. Microarrays permit the development of multiple parallel assays for identifying and validating biomarkers of disease and drug targets which can be used in diagnosis and treatment. Gene expression profiles can also be used to estimate and predict metabolic and toxicological consequences of exposure to an agent (e.g. such as a drug, a potential toxin or carcinogen, etc.) or a condition (e.g. temperature, pH, etc).

However, several basic limitations restrict widespread use of DNA array technology in research as well as in *in vitro* molecular diagnostics. Microarrays experiments often yield redundant data, only a fraction of which has value for the experimenter. Additionally, because of the highly parallel format of microarray-based assays, conditions may not be optimal for individual capture probes. Many genes and pathways are still unknown and our understanding of nucleic acid hybridization is still limited. The contemporary array designs thus keep changing as the knowledge of application relevant targets increases and as we improve our understanding of the thermodynamics and kinetics governing nucleic acid hybridization. Most arrays are therefore only produced in small quantities and are consequently expensive yet disposable research tools. Furthermore, results obtained with early arrays are difficult to compare with results obtained from later arrays that use different capture probes.

Several research teams have attempted to generate universal arrays of short DNA probes that can be used for many different purposes by including all possible sequences of a given length on the same chip. Such penta- or hexamer DNA arrays have been used in attempts to sequence a target by hybridization (1-4). Unfortunately short DNA probes only form duplexes with a very low thermal stability ($T_m$) which necessitates the use of extreme assay conditions (4.5 M NaCl, -20 to 50˚C).

Arrays with very short capture probes are also limited by the low capture efficiency of such capture probes, and the tendency of target nucleic acids to form stable intra-molecular structures, which may further decrease the accessibility of the target to the probes. Using longer capture probes in universal microarrays increases the required complexity exponentially as the complete set of oligonucleotides with n-bases is $4^n$. Furthermore, the use of longer capture probes reduces the ability to discriminate between perfect and imperfect duplexes, especially if the mismatch is terminally located.

Thus, improved technologies are needed to produce useful universal arrays that may be used for nucleic acid classification, identification and quantification.

LNA (Locked Nucleoside Analogues) is nucleic acid analogue that displays unprecedented hybridization affinity

towards complementary DNA and RNA and at the same time show equal or superior abilities to discriminate match sequences from mismatch sequences as compared to native nucleic acids. LNA has been used in a variety of nucleic acid assays including genotyping assays, expression microarrays, poly-T sample prep, as antisense molecule, as decoy molecule and in LNAzymes (Petersen and Wengel, TIBTECH, 2003, 21, 74-81).The present work demonstrates how the unique helix stabilizing properties of LNA strongly increase the stability of short LNA-DNA duplexes so that the improved stringency of hybridization and capture efficiency may dramatically improve the performance of a universal LNA heptamer chip. Further inventions presented in this proposal such as modified nucleobases (e.g. SBC-LNA units) may further enhance the performance of a universal chip, or they may be used for different applications.

Finally, we present alternative approaches to the interpretation of hybridization data from arrays with short (and frequently occurring) capture probe sequences. The novel approach may greatly increase the value and versatility of universal microarray data.

Conventional microarray approaches have attempted to establish whether a particular target sequence is present in a sample by detecting a duplex formed with a corresponding complementary probe sequence. The novel approach presented in this patent application does not attempt to establish the presence or absence of any particular sequence segment corresponding to any particular capture probe. Instead the aim is to quantify the reproducible binding of a complex target to numerous short capture probes. The resulting hybridization pattern (="signature") can be used to classify the sample based on comparison with similar hybridization patterns of known standard sequences. Indeed we do not believe it feasible to establish conclusively whether a corresponding target sequence to any particular short capture probe sequence is present in or absent from a given sample. The corresponding target sequence in the sample may be inaccessible due to secondary structures in the sample sequence or it may appear as if the sequence is present only due to an overabundance of a similar sequence the binding of which may even involve non-Watson-Crick basepairing. The observed hybridization pattern is therefore NOT used to establish the presence or absence of particular signature sequences in a sample. Instead it is classified by numeric comparison with similar hybridization patterns.

US 2002/0197630 discloses methods, devices, libraries, kits and systems for detecting nucleic acids.

WO 03/020739 A2 discloses LNA oligomers having LNA units with universal nucleobases.

SUMMARY OF THE INVENTION

In general, the invention features populations of high affinity nucleic acids that have duplex stabilizing properties and thus are useful for a variety of nucleic acid amplification and hybridization methods. Some of these oligonucleotides contain novel nucleotides created by combining specialized synthetic nucleobases with an LNA backbone, thus creating high affinity oligonucleotides with specialized properties such as retained or increased sequence discrimination for the complementary strand or reduced ability to form intramolecular double-stranded structures. The invention also provides improved methods for identifying target nucleic acids in a sample and for classifying a nucleic acid sample by comparing its pattern of hybridization to an array to the corresponding pattern of hybridization of one or more standards to the array.

The invention also features populations of nucleic acids (oligonucleotides/LNA oligomers) with a variety of modified nucleobases that exhibit substantially constant $T_m$ values upon hybridization with a complementary oligonucleotide, irrespective of the nucleobases present on the complementary oligonucleotide. Other desirable modified nucleobases have decreased ability to form intramolecular double-stranded structures or to form duplexes with oligonucleotides containing one or more modified nucleobases. The invention also provides arrays of nucleic acids containing these modified nucleobases that have a decreased variance in melting temperature and/or an increased capture efficiency compared to naturally-occuring nucleic acids. These arrays as well as the oligonucleotides in solution can be used in a variety of applications for the detection, characterization, identification, and/or amplification of one or more target nucleic acids. These oligonucleotides can also be used for solution assays, such as homogeneous assays.

In particular, the present invention provides a population of nucleic acids, said population comprising a first population of nucleic acids of the same length, said length being in the range of 5-15 nucleotides or units, said first population representing at least 1% of the possible different nucleic acid sequences for nucleic acids of said length, at least one nucleic acid in the first population being an LNA oligomer. The population is preferably bonded, e.g. covalently bonded, to a solid support.

In one aspect, the invention provides the population wherein the variance in the melting temperature of the first population is at least 10%, at least 20%, at least 30%, at least 40%, at least 50%, at least 60%, or at least 70% less than the variance in the melting temperature of the corresponding control population of nucleic acids.

In a further aspect, the invention provides the population of nucleic acids, wherein at least one LNA oligomer of the first population has a melting temperature that is at least 5, at least 8˚C, at least 10˚C, at least 12˚C, at least 15˚C, at least 20˚C, at least 25˚C, at least 30˚C, at least 35˚C, or at least 40˚C higher than that of the corresponding control nucleic.

In a still further aspect, the invention provides the population of nucleic acids, wherein the first population has at least one LNA oligomer with a capture efficiency that is at least 50%, at least 100%, at least 150%, at least 200%, at

least 500%, at least 800%, at least 1000%, or 12000% greater than that of the corresponding control nucleic acid at the temperature equal to the melting temperature of the LNA oligomer of the first population.

In particular, the present invention features a Universal LNA Array (an array comprising LNA oligomers) which is a truly generic research and diagnostic tool that generates a unique signature for any complex nucleic acid sample. The novel approach presented in this patent application does not attempt to establish the presence or absence of any particular sequence segment corresponding to any particular capture probe. Instead the aim is to quantify the reproducible binding of a complex target to numerous short capture probes. The resulting hybridization pattern (="signature") can be used to classify the sample based on comparison with similar hybridization patterns of known standard sequences. The same array can therefore be used in a wide variety of applications ranging from detection of microbial pathogens in food samples and classification of hospital infections, to cancer diagnostics based on altered mRNA expression patterns in an affected tissue.

A particular array is composed of LNA enhanced heptamer probes that are capable of generating a unique spot pattern (=signature) for any single-stranded DNA or RNA molecule or mixture of molecules such as cDNA or mRNA from tumor cells. Different signatures can be classified by comparison with a large set of standard signatures. As each signature contains thousands of data points, it is not only possible to identify any given sequence due to its unique spot pattern, but also to analyze the complex spot pattern of samples containing mixtures of sequences to determine the relative abundance of different standards in the mixture.

A particular advantage of the presented approach in an identification context is its extreme flexibility and ability to identify novel organisms and the ability to determine the relative abundance of known organisms in mixed samples. Using selective primers any organism or virus can be detected with the same chip. If knowledge of the strain is desired then a highly variable marker gene can be used, and if a generic identification is adequate, then conserved 16S rDNA primers can be used. It is also possible to determine if the signature matches any known signature or if the organism is unknown.

In the Examples section herein, we have demonstrated the ability of a small scale version of the universal LNA array containing only 280 heptamer LNA enhanced capture probes to:

1. Identify five different pathogenic *Haemophilus* strains. The identification is based on partial amplification of two common household genes whose sequence similarity is subsequently quantified with the universal LNA array.

2. Determine the abundance of two different splice variants of the gene LET2 from C. *elegans.* Different mixtures containing known amounts of the two genes were investigated with the universal LNA array to quantify the concentration of each gene in each sample.

3. Classify complex mRNA samples from Yeast according the different different gene expression pattern before and after heat shock treatment.

In particular, the invention also provides an array including a solid support and a population of nucleic acids bonded to said solid support, said population comprising a first population of nucleic acids of the same length, said length being in the range of 5-15 nucleotides or units, said first population representing at least 1% of the possible different nucleic acid sequences for nucleic acids of said length, at least 50% of the nucleic acids in the first population being LNA oligomers, and the variance in the melting temperature of the first population is at least 50% less than the variance in the melting temperature of the corresponding control population of nucleic acids.

BRIEF DESCRIPTION OF THE DRAWINGS

Figure 1 is a graphical representation of the effect of systematic LNA T and A/T substitutions on the melting temperature of all 262,144 possible 9-mer oligonucleotides. Bottom line: DNA, Middle line: LNA-T substituted, Top line: LNA-A/T substituted.

Figure 2 illustrates the average melting temperature of LNA and DNA duplexes of different lengths. The black diamonds show the increasing stability of oligonucleotide DNA duplexes as predicted by a thermodynamic nearest neighbour model. Similar calculations for LNA enhanced capture probes containing increasing amounts of LNA are shown by other symbols of increasing intensity as indicated in the legend. The arrows point to the equivalent stability of a 7-mer LNA probes with 4 or 5 LNA nucleotides and an 11-mer DNA probe.

Figure 3 illustrates various types of LNA units.

Figure 4 illustrates the chemical structures of Selective Binding Complementary (SBC) bases.

Figure 5 is a schematic illustration of three methods for synthesizing 2-thio-T-LNA.

Figure 6 shows the different synthesis strategies for converting the LNA pyrimidine derivative VIII to the 2-thio-LNA pyrimidine derivative IV.

Figure 7 shows a synthesis strategy for synthesis of the 2-thio-LNA pyrimidine derivative IV via coupling of the coupling sugar I with a 5-modified 2-thio-pyrimidine nucleobase.

Figure 8 shows a synthesis strategy for synthesis of the 2-thio-LNA pyrimidine derivative IV via conversion of the coupling sugar I to a 1-amino-sugar derivative V that can be reacted with the isothiocyanate derivative VI followed by ring closure to give IV.

Figure 9 shows the base-pairing between modified bases and naturally-occuring nucleotides. These modified nucleobases may be incorporated as part of an LNA, DNA, or RNA unit and used in any of the oligomers of the invention.

Figure 10 shows the structure of desirable adenosine analogs. These modified nucleobases may be incorporated as part of an LNA, DNA, or RNA unit and used in any of the oligomers of the invention. Key: X = N or $CHR_1$= $C_{1-4}$ alkyl, $C_{1-4}$ alkoxy, $C_{1-4}$ alkylthio, F, or $NHR_3$ where $R_3$ is H, or $C_{1-4}$ alkyl; $R_2$ = H, $C_{1-6}$ alkyl, $C_{1-6}$ alkenyl, or $C_{1-6}$ alkynyl.

Figure 11 shows the structure of desirable thymine analogs (WO 97/12896). These modified nucleobases may be incorporated as part of an LNA, DNA, or RNA unit and used in any of the oligomers of the invention. Key: X = N or CH; $R_4$ = H, or $C_{1-4}$ alkyl; $R_5$ = H, $C_{1-4}$ alkyl, $C_{1-4}$ alkoxy, $C_{1-4}$ alkylthio, or F.

Figure 12 shows the structure of desirable guanine analogs (WO 97/12896). These modified nucleobases may be incorporated as part of an LNA, DNA, or RNA unit and used in any of the oligomers of the invention. Key: $R_1$ = H or $C_{1-4}$ alkyl.

Figure 13 shows the synthesis of the 3’,5’-di-O-benzylated LNA 2-thio-thymine nucleobase protected compounds 4 via coupling of 1 with 2-thio-thymine followed by ringclosure.

Figure 14 is a schematic illustration of the use of an exemplary synthesis for LNA-furanoPyr-SBC-C.

Figure 15 illustrates the synthesis of LNA-I. Keys: (a) hypoxantine, BSA, TMSOTf, 1,2-dichloromethane; 93%; (b) NaOH, THF, EtOH, $H_2O$; 69%; (c) NaOBz, DMSO; 76%; (d) NaOH, THF, MeOH, $H_2O$; 85%; (e) DMT-Cl, pyridine; 92%; (f) Pd/C, $HCO_2NH_4$ 77%; (g) 2-cyanoethyl-*N*,*N*-diisopropyl-phosphoramidochloridite, DIPEA, DMF; 75%.

Figure 16 illustrates the synthesis of LNA-D. Keys: (a) 2-chloro-6-aminopurine, BSA, TMSOTf, 1,2-dichloromethane; 90 %; (b) NaOH, 1,4-dioxane, $H_2O$; 87%; (c) NaOBz, DMF; (d) $NaN_3$, DMSO; (e) NaOH, EtOH; 79% (three steps); (f) 10% Pd/C, $HCO_2NH_4$, MeOH, $H_2O$ 84%; (g) 1. BzCl, pyridine; 2. NaOH, EtOH, pyridine; 62%; (h) DMT-Cl, pyridine; 80%; (i) 2-cyanoethyl-*N*,*N*-diisopropylphosphoramidochloridite, DIPEA, DMF; 74%.

Figure 17 iIllustrates the synthesis of LNA-2AP. Keys: (a) $TIPDSCl_2$, DMF, Imidazole; 63%; (b) $Pac_2O$, pyridine; 95%; (c) $Et_3N.3HF$, THF; 97%; (d) DMT-Cl, pyridine; 81%; (e) 2-cyanoethyl-tetraisopropylphosphordiamidite; DCI, EtOAc, THF; 56%.

Figure 18 illustrates the synthesis of LNA-2AP. Keys: (a) NaOH, 1,4-dioxane, $H_2O$ 72%; (b) 20% $Pd(OH)_{2/}C$, $HCO_2NH_4$ MeOH, $H_2O$ 89%; (c) *N*,*N*-dimethylformamide dimethyl acetal, DMF; (d) DMT-Cl, pyridine; 87% (two steps); (e) 2-cyanoethyl-*N*,*N*-diisopropylphosphoramidochloridite, DIPEA, DMF; 64%.

Figure 19 illustrates the synthesis of $^{2S}$U-LNA. Keys: (i) NaOBz, DMSO, 140 ˚C, 84%; (ii) NaOH, THF/MeOH, 98%; (iii) $Pd(OH)_2/C$, $HCO_2NH_4$, MeOH, reflux, 92%; (iv) $Ac_2O$, Pyridine, 99%; (v) AcOH, $Ac_2O$, $H_2SO_4$, 99%; (vi) 2-thiouracil, *N,O,*-bis-trimethylsilylacetamide, $SnCl_4$, MeCN; (vii) 1M HCl, MeOH, 38% (two steps); (viii) 1,3 dichloro-1,1,3,3-tetraisopropyl-disiloxane, Pyridine, 36%; (ix) NaH, THF, 54%; (x) TolCl, (Et)N(iPr)$_2$, Pyridine; (xi) $Et_3N·HF$, AcOH, THF, 85% (two steps); (xii) DMT-Cl, Pyridine, 79%; (xiii) 2-cyanoethyl-*N,N,N’,N’*-tetraisopropyl phosphoradiamidite, 4,5-dicyanoimidazole, $CH_2Cl_2$, EtOAc, 91%.

Figure 20 is a figure generated by Mathematica™ modeling of binding of *Pseudomonas fluorescens* 16S rRNA to a universal heptamer array containing all 16384 possible 7-mers. The figure illustrates all possible 7 mers organized in 128x128 array. The spots are heptanucleotides whose corresponding sequence is present in the 16S rRNA of *Pseudomonas fluorescens*. The occational bright spots correspond to sequences that are present more than just once.

Figure 21 illustrates the inherent problems in a simultaneous use of multiple probes. Fig. 21A illustrates common problems when several probes are applied simultaneously. Both probe 1 and 2 show a large discrimination between match and mismatch, but unfortunately there is no overlap between the two $\Delta T_m$ so the probes can not be used together. Probe 1 and probe 3 can be used together, but the very small $\Delta_{Tm}$ observed for probe 3 makes it highly unlikely that this will be a usefull probe. Fig. 2B illustrates the optimal design of probes that may be used simultaneously. Dashed horizontal lines indicate the necessary experimental temperature.

Figure 22 is a graph comparing the $\Delta T_m$ of an LNA enhanced probe with the $\Delta T_m$ of the equivalent DNA probe. The curves show the first derivative of four melting profiles. Gray curves are for the DNA probe and black curves are for the LNA probe. The peaks correspond to the measured $T_m$ values. As illustrated, the $\Delta T_m$ has been increased by 700% just by inclusion of LNA in the probe.

Figure 23 is a schematic illustration of the use of a nucleic acid of the invention to capture a double-stranded DNA molecule.

Figure 24 is a bar graph demonstrating that LNA enables the design of compatible probes. The nucleotides of Allele 1 and 2 in the mismatch position are G and A, respectively, which means that it is the difficult G:T mismatch that has to be discriminated. The gray letters in the sequence of the probes show the LNA substitutions.

Figure 25 is a picture of gels showing the comparison of LNA containing primers and DNA primers in multiplex PCR amplification. The template was human chromosomal DNA. The degree of multiplexity was six. The black dots indicate DNA amplified due to lack of specificity of DNA based primers. A single LNA molecule was placed at the penultimate 3'-position of the primers.

Figure 26 is a graph showing the accuracy of the predicted $T_m$ for LNA substituted oligonucleotides. Neural networks trained with the nearest neighbour information, length and DNA/LNA neighbour effect were efficient for predicting $T_m$. The standard error of prediction obtained when comparing actual measured $T_m$ values and predicted $T_m$ values is 5 °C.

Figure 27 shows the $T_m$ and $\Delta T_m$ values obtained by on-chip melting of target DNA in mcroarray hybridizations. Probes with different LNA substitutions were analyzed for their ability to resolve a single centrally positioned mismatch (T-G and A-C). For each design variant of the 12-mer probes, the $T_m$ of perfect match and single mismatch were measured. Each triplet of bars contains the $T_m$ of match (left bar), $T_m$ of mismatch (central bar), and the $\Delta T_m$ (right bar). In the sequences below the columns, the positions of LNA substitutions are indicated with grey hatched capital letters for the different capture probes.

Figure 28 shows the layout of a test array with short LNA enhanced capture probes designed to test different LNA substitution patterns and flanking universal nucleobases such as 5-nitroindole. Upper case letters in the sequences denote LNA units; lower case letters DNA units. z=5-nitroindole, i=DNA-inosine, I= LNA-inosine and X is a degenerate position with a mixture of all LNA nucleotides. The lower right panel is a picture of the hybridization pattern of a test sample (synthetic 45 mer) bound to an array of the invention.

Figure 29 depicts the simplest possible assumption (i.e. that the hybridization pattern of a sample is a simple linear combination of the hybridization patterns of its constituent components). If this is, the case then it is straightforward to compute the relative abundance of each component by simple linear deconvolution of the hybridization pattern of using a least squares approach.

Figure 30. Prototype of a self-contained micro-fluidic array system being developed by Exiqon for pre-spotted arrays such as the universal LNA array. The hybridization chamber is covered with a foil after spotting to form a protected hybridization channel with a total volume of less than 10 μl. The slide also contains an inlet that fit standard micropipettes and an integrated waste chamber. The slide has the same footprint as conventional microscope slides (75 x 25 x 1 $mm^3$) and is compatible with standard array scanners.

Figure 31 contains representative data to illustrate calibration of the scoring matrix for the optimization algorithm in Fig. 7. Each box of sequences contain six different substitution patterns for a given capture probe. Based on the hybridization pattern to the left, the sequences outlined in bold were selected as the best substitution pattern for each sequence. The only exception is aatcgat which contains a six base-pair inverse repeat so it does not capture any target regardless of substitution pattern.

Figure 32 illustrates simulated hybridization pattern on a heptamer chip (=signature) for the sequence EMBRYO_9_AMP at 12˚C. The simulation was calculated by Mathematica using a simplified thermodynamic model.

Figure 33 illustrates simulated hybridization pattern on a heptamer chip (=signature) for the sequence LARVAE_10_AMP at 12˚C. The simulation was calculated by Mathematica using a simplified thermodynamic model.

Figure 34 illustrates simulated hybridization pattern on a heptamer chip (=signature) for the sequence LARVAE_10_MUT at 12˚C. The simulation was calculated by Mathematica using a simplified thermodynamic model.

Figure 35 illustrates simulated hybridization pattern on a heptamer chip (=signature) for the mixed signal generated by mixing (30% EMBRYO_9_AMP sequence, 60% sequence LARVAE_10_AMP, 10% LARVAE_10_MUT) at 12˚C. The simulation pattern was calculated by Mathematica using a simplified thermodynamic model. It was subsequently subjected to different types of noise (se test example 8b) and re-analyzed to determine the extend of noise addition, which would obscure the recovery of the mixing rations between the different replicants.

Figure 36 illustrates the layout of the test chip "OCFA-beta". All four replicates of the 384 capture probes are included. The sequence of each capture probe is listed in Example 8b. The Dark squares correspond to Cy3 or Cy5 labelled control probes. ("landing lights").

Figure 37. Comparison of 94 LNA capture probes (outlined in light gray) and 94 DNA capture probes (outlined in dark gray. The two sets of probes have identical nucleobase sequences, but the LNA capture probe set contain LNA substitutions in the sugar moiety. Hybridization has been carried out under low stringency deliberately to favourize the DNA probes.

Figure 38. Thermal melting curves showing reversible binding targets to short heptamer LNA capture probes, but not to heptamer DNA probes. Temperature is shown on the left scale (thick line). It was kept constant at 15 ˚C for the first 60 min followed by a linear temperature increase to 45 ˚C at 1˚C/min and a subsequent cool down to 15 ˚C at the same rate. After 120 min the temperature was again keep constant at 15 ˚C.

Figure 39. "Bar-Code" depiction of universal LNA Array signatures of two different household genes for five different *Haemophilus* strain. The lower half of the figure (row 1 -30, see text) depicts the measured pattern after hybridization with a partial amplification of the adenylate kinase (adk) gene as target. The upper half of the figure (row 31 -60, see text) depicts the measured hybridization pattern with a partial amplification of the recA gene as target

Figure 40. Similarity matrix. The signatures of two different household genes for five different *Haemophilus* strain are compared pairwise. High similarity is indicated by light colors (white = identity. The dark squares arise when the signature of one gene is compared with the signature of another gene (i.e. comparing apples and oranges). The white diagonal arises when a signature is compared with itself.

Figure 41. Similarity tree for universal LNA Array signatures based on the similarity matrix shown in the preceding figure. The tree topography for the two household genes is expectedly similar. The derived similarity tree based on quantified differences in hybridization patterns corresponds to phylogenetic trees for the genes and strains that were investigated. Representative hybridization patterns for the two genes recA and adk are shown.

Figure 42. Analysis of universal LNA array signatures of known mixtures of two similar target genes. Partial amplificates of two different splice variants of the LET2 gene of *C. elegans* were mixed in different ratios and the produced hybridization patterns analyzed to quantify the abundance of each target. A reasonable correlation between expected concentrations (according to the known composition of the gene mixtures) and detected concentration based on deconvolution of the universal LNA array signatures is found using a simple linear model.

Figure 43. Experimental procedure to investigate changes in gene expression patterns in yeast after heat shock. Replicates of each treatment were investigated by hybridization at two different temperatures.

Figure 44. Universal LNA array signatures of Yeast mRNA. (A) Hybridization pattern of mRNA from yeast after heat shock. Please note the performance difference between DNA and LNA heptamers and the high degree of reproducibility for the four different replicates of the 384 probe set. (B) "Bar-Code" depiction of universal LNA Array signatures of complex mRNA pools Lower half (row 1-24) is signatures with heat shock, the upper half (row 25 - 48) is signatures without heat shock. As the applied target mixture in this experiment is much more complex than the simple target mixtures applied in the previous example, we get a reproducible "barcode" with less contrast between "positive" and "negative" capture probes. Indeed most capture probes contribute to the complex signature of such a sample.

Figure 45. Similarity tree for the signatures obtained of mRNA from yeast with and without heat shock. Light gray = samples without heat shock. Dark gray = samples with heat shock. "A" signatures were signatures recorded at low stringency (5x SSCT at 4 °C) and "B" signatures were recorded at high stringency (1x SSCT at 25 °C). Despite the strong difference in stringency, the four signatures of mRNA samples after heat shock can be correctly identified as different from similar signatures of mRNA samples without heat shock.

## DETAILED DESCRIPTION OF THE INVENTION

A general method for equalizing the melting temperatures of oligonucleotides of the same length has been developed. Decreasing the variation in melting temperatures ($T_m$) of a population of nucleic acids allows the nucleic acids to hybridize to target molecules under similar binding conditions, thereby simplifying the simultaneous hybridization of multiple nucleic acids. Similar melting temperatures also allow the same hybridization conditions to be used for multiple experiments, which is particularly useful for assays involving hybridization to nucleic acids of varying "AT" content. For example, current methods often require less stringent conditions for hybridization of nucleic acids with high "AT" content compared to nucleic acids with low "AT" content. Due to this variation in hybridization stringency, current methods may require significant trial and error to optimize the hybridization conditions for each experiment.

To overcome limitations in current nucleic acid hybridization and/or amplification techniques, populations of nucleic acid probes or primers with minimal variation in melting temperature have been developed. For example, the unique properties of LNA increase binding affinity of nucleic acids for DNA and RNA. The stability of duplexes can generally be ranked as follows: DNA: DNA < DNA: RNA < RNA: RNA ≤ LNA: DNA < LNA: RNA < LNA: LNA. The DNA: DNA duplex is thus the least stable and the LNA: LNA duplex the most stable. The affinity of the LNA units A and T corresponds approximately to the affinity of DNA G and C to their complementary nucleobases. General substitution of one or more A and T nucleotides with LNA A and LNA T in DNA oligonucleotides is therefore a simple way of equalizing differences in $T_m$. Furthermore, the mean melting temperature is increased significantly, which is often important for shorter oligonucleotides (see Figure 2).

Predictions of melting temperature of all possible 9-mer oligonucleotides have shown that the mean temperature increases from 39.7°C to 59.3°C by substituting all DNA A and T nucleotides with LNA A and T nucleotides (Figure 1). The variance in $T_m$ of all 9-mers furthermore decreases from 59.6°C for DNA oligonucleotides to only 4.7°C for the LNA substituted oligonucleotides as reflected in the standard deviations listed in Table 1A and the sorted listing of Tm estimates for all possible 9-mers shown in Figure 1. The estimated Tm values are based on the latest LNA $T_m$ prediction algorithms such as those disclosed herein, which have a variance of 6-7°C. Table 1B shows the estimated melting temperature range, variance, and standard deviation for oligonucleotides of various lengths.

Table 1A. Overview of the effect of global LNA T and A/T substitutions on the $T_m$ properties of all possible 9-mer oligonucleotides.

| 9-mer oligonucleotide | Mean $T_m$ | Range of predicted $T_m$ values | Standard deviation $T_m$ |
|---|---|---|---|
| DNA | 39.7 | 54.4 | 7.7 |
| LNA-T substituted | 51.1 | 47.8 | 6.2 |
| LNA-A/T substituted | 59.3 | 16.9 | 2.2 |

Table 1B. Summary of estimated melting temperatures for oligonucleotides of various lengths based on averages for 10,000 randomly chosen sequences of each length.

| Length of oligonucleotide | Average Variance for all sequences of this length | Average Standard Deviation for all seq. | Range of predicted $T_m$ for all sequences. |
|---|---|---|---|
| 5 | 90.2 | 9.5 | 49.3 |
| 6 | 79.7 | 8.9 | 52.8 |
| 7 | 72.2 | 8.5 | 52.5 |
| 8 | 65.7 | 8.1 | 54.5 |
| 9 | 58.3 | 7.6 | 50.2 |
| 10 | 55.5 | 7.4 | 51.3 |
| 11 | 52.2 | 7.2 | 51.7 |
| 12 | 46.9 | 6.8 | 46.3 |
| 13 | 44.8 | 6.7 | 46.3 |
| 14 | 40.7 | 6.4 | 43.9 |
| 15 | 39.0 | 6.2 | 45.3 |

Examples 6 and 7 also provide algorithms for optimizing the substitution patterns of the nucleic acids to minimize self-complementarity that may otherwise inhibit the binding of the nucleic acids to target molecules.

In various embodiments of the nucleic acids and arrays of the invention, LNA A and LNA T substitutions are made to equalize the melting temperatures of the nucleic acids. In other embodiments, LNA A and LNA C substitutions are made to minimize self-complementarity and to increase specificity. LNA C and LNA T substitutions also minimize self-complementarity. The above populations of nucleic acids are useful, e.g., as probes for microarrays or multiplex analysis or as PCR primers (e.g. random or degenerate primers, primers for sequencing, or primers for mutation detection). Nucleic acids with minimal variance in melting temperature are generally useful for any method involving nucleic acid hybridization. Oligonucleotide microarrays of the invention (e.g. arrays of random nucleic acids) generated on a chip by photochemistry also have improved product performance and lower fabrication times.

Thus, the present invention i.a. provides a population of nucleic acids, said population comprising a first population of nucleic acids of the same length, said length being in the range of 5-15 nucleotides or units, said first population representing at least 1% of the possible different nucleic acid sequences for nucleic acids of said length, at least one nucleic acid in the first population being an LNA oligomer.

As mentioned above, the present invention provides "a population of nucleic acids". By "a population of nucleic acids" is meant more than one nucleic acid. The populations of nucleic acids of the invention may contain any number of unique molecules. For example, the population may contain as few as 10, $10^2$, $10^3$, $10^4$, or $10^5$ unique molecules or as many as $10^7$, $10^8$, $10^9$ or more unique molecules. In some embodiments, at least 1, at least 5, at least 10, at least 50, at least 100 or more of the polynucleotide sequences are non-naturally-occurring sequences. Desirably, at least 20%, at least 40%, or at least 60% of the unique polynucleotide sequences are non-naturally-occurring sequences.

The population comprises a first population of nucleic acids of the same length. It should be understood that the population may comprise the nucleic acid of the first population only, or the first population may be a subpopulation in relation to the population of nucleic acids. In the latter embodiment, the population of nucleic acids further includes one or more nucleic acids and/or a second nucleic acid population of a different length (e.g. shorter or longer nucleic acids) than that of the first population of nucleic acids. In some embodiments, longer nucleic acids contain one or more nucleotides with universal nucleobases. For example, nucleotides with universal nucleobases can be used in order to increase the thermal stability of nucleic acids that would otherwise have a thermal stability lower than some or all of the nucleic acids in the first population.

The nucleic acids in the first population are however of the same length, i.e. the nucleic acids in the first population contain 5, 6, 7, 8, 9, 10, 11, 12, 13, 14, or 15 nucleotides or units. In particular, the length is 5-15 nucleotides or units, such as 5-10 nucleotides or units, e.g. 5, 6, 7, 8, 9, or 10 nucleotides or units. The term "nucleotides or units" is used in order to cover "normal" nucleotides based on deoxyribose and ribose sugars as well as LNA units.

The first population of nucleic acids comprises at least 1% of the possible different nucleic acid sequences for nucleic acids of said length. By the term "possible different nucleic acid sequences for nucleic acids of said length" is meant the number of different nucleic acid sequences assuming that each unit of a nucleic acid can be represented by four different nucleotides (A, T(U), C, G). Thus, the term relates to the formula $4^n$ where n represents the number of

units (the length) of the nucleic acid. The possible different nucleic acid sequences for the nucleic acids of 5-15 will therefore be: 1024, 4096, 16,384, 65,536, ..., 1,073,741,824. Thus, at least 1% of the possible different nucleic acid sequences for a 7-mer corresponds to 1% of 16,384, i.e. at lest 164 different nucleic acids.

In various embodiments, the first population has at least 10, at least 100, or at least 1,000, or at least 5,000, or at least 10,000 different nucleic acids. In special embodiments, the first population comprises at least 100,000 or even at least 1,000,000 different nucleic acids.

In further embodiments, the first population includes at least 5%, at least 10%, at least 20%, at least 25%, at least 30%, at least 40%, at least 50%, at least 60%, at least 70%, at least 80%, at least 90%, or even 100% of the possible different nucleic acid sequences for nucleic acids of that length.

As it will become apparent from the following, only a minor fraction of the possible different nucleic acids of a particular length is necessary in order to capture nucleic acids of, e.g., biological samples comprising a plurality of target nucleic acids. Thus, in one particular embodiment, the first population comprises 1-9% such as 1-5% of the possible different nucleic acid sequences for nucleic acids of said length, in particular for a length of 5-10 nucleotides or units.

The population of nucleic acids is preferably bonded, e.g. covalently bonded, to a solid support. By "solid support" is meant any rigid or semi-rigid material to which a nucleic acid binds or is directly or indirectly attached. The support can be any porous or non-porous water insoluble material, including without limitation, membranes, filters, chips, slides, wafers, fibers, magnetic or nonmagnetic beads, gels, tubing, strips, plates, rods, polymers, particles, microparticles, capillaries, and the like. The support can have a variety of surface forms, such as wells, trenches, pins, channels and pores. As it will be explained further below, the populations of nucleic acids can, e.g., be covalently bonded to the solid support by photoactivated coupling or the population can be synthesized directly on the solid support by using the solid support as a carrier. By "bonding" is meant attachment via hydrogen bonds, via electrostatic forces, via hydrophobic interactions, or via covalent bonds, or combinations of these..

When bound, the individual nucleic acids of the population can be bound covalently, either directly or via a spacer. By "spacer" is meant a distance-making group and is used for joining two or more different moieties of the types defined above, e.g. a nucleic acid and a solid support material. Spacers are selected on the basis of a variety of characteristics including their hydrophobicity, hydrophilicity, molecular flexibility and length (e.g. Hermanson et. al., "Immobilized Affinity Ligand Techniques," Academic Press, San Diego, California (1992). Generally, the length of the spacers is less than or about 400 Å, in some applications desirably less than 100 Å. The spacer, thus, comprises a chain of carbon atoms optionally interrupted or terminated with one or more heteroatoms, such as oxygen atoms, nitrogen atoms, and/or sulphur atoms. Thus, the spacer may comprise one or more amide, ester, amino, ether, and/or thioether functionalities, and optionally aromatic or mono/polyunsaturated hydrocarbons, polyoxyethylene such as polyethylene glycol, oligo/polyamides such as poly-$\alpha$-alanine, polyglycine, polylysine, peptides, oligosaccharides, or oligo/polyphosphates. Moreover the spacer may consist of combined units thereof. The length of the spacer may vary, taking into consideration the desired or necessary positioning and spatial orientation of the nucleic acid. In particular embodiments, the spacer includes a chemically cleavable group. Examples of such chemically cleavable groups include disulphide groups cleavable under reductive conditions, peptide fragments cleavable by peptidases and ketals and acetals cleaved by acid.

Desirably, the nucleic acids of the population are bonded to the solid support in a predefined arrangement, e.g. in an array. By an "array" is meant a fixed pattern of at least two different immobilized nucleic acids on a solid support. Desirably, the array includes at least $10^2$, such as at least $10^3$, e.g. at least $10^4$ different nucleic acids. In some important embodiments, the array includes 100-5000 different nucleic acids.

This being said, the invention also provides an array comprising a population of nucleic acids as defined herein.

As mentioned above, at least one nucleic acid in the first population is an LNA oligomer, i.e. a nucleic acid having one or more LNA units. In more preferred embodiments, at least 10%, at least 20%, at least 25%, at least 30%, at least 40%, at least 50%, at least 60%, at least 70%, at least 80%, at least 90%, or 100% of the nucleic acid in the first population are LNA oligomers. In some embodiment, e.g. where the all A and T nucleobases of a population of nucleic acids are represented by LNA A and LNA T, respectively, 90%-100% of the nucleic acids of the first population are LNA oligomers.

LNA oligomers have improved characteristics over nucleic acids with respect to hybridization and specificity and selectivity as it will be known to the person skilled in the art, and the present inventors have found that these properties are particularly useful in connection with the populations and arrays defined herein.

When used herein, the term "LNA" (Locked Nucleoside Analogues) refers to nucleoside analogues (e.g. bicyclic nucleoside analogues, e.g., as disclosed in WO 99/14226) either incorporated in an oligonucleotide or as a discrete chemical species (e.g. a LNA nucleoside and a LNA nucleotide). The term "monomeric LNA" explicitly refers to a discrete chemical species and may, e.g., refer to the monomers LNA A, LNA T, LNA C, LNA G, LNA U, or any other LNA monomers.

By "LNA unit" is meant an LNA monomer (e.g. an LNA nucleoside or LNA nucleotide) incorporated in an oligomer (e.g. an oligonucleotide or nucleic acid). LNA units as disclosed in WO 99/14226 are in general desirable modified nucleotides for incorporation into the nucleotides of the populations of the invention. Additionally, such nucleic

acids may be modified at either the 3' and/or 5' end by any type of modification known in the art. For example, either or both ends may be capped with a protecting group, attached to a flexible linking group, attached to a reactive group to aid in attachment to the solid surface, etc. Desirable LNA units and their method of synthesis also are disclosed in WO 00/56746, WO 00/56748, WO 00/66604, Morita et al., Bioorg. Med. Chem. Lett. 12(1):73-76, 2002; Hakansson et al., Bioorg. Med. Chem. Lett. 11(7):935-938, 2001; Koshkin et al., J. Org. Chem. 66(25):8504-8512, 2001; Kvaerno et al., J. Org. Chem. 66(16):5498-5503, 2001; Hakansson et al., J. Org. Chem. 65(17):5161-5166, 2000; Kvaerno et al., J. Org. Chem. 65(17):5167-5176, 2000; Pfundheller et al., Nucleosides Nucleotides 18(9):2017-2030, 1999; and Kumar et al., Bioorg. Med. Chem. Lett. 8(16):2219-2222, 1998.

By "LNA oligomer" is meant an oligonucleotide (nucleic acid) comprising at least one LNA unit of the general Formula A, described *infra,* having the below described illustrative examples of substituents:

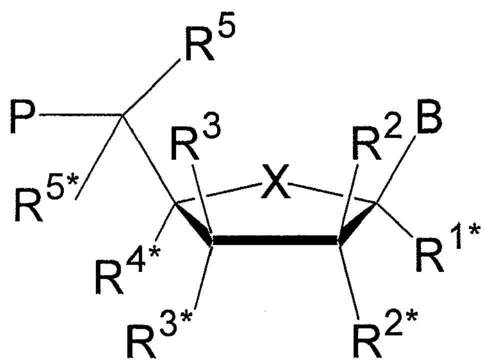

**A**

wherein X is selected from -O-, -S-, -N($R^N$)-, -C($R^6R^{6*}$)-, -O-C($R^7R^{7*}$)-, -C($R^6R^{6*}$)-O-, -S-C($R^7R^{7*}$)-, -C($R^6R^{6*}$)-S-, -N($R^{N*}$)-C($R^7R^{7*}$)-, -C($R^6R^{6*}$)-N($R^{N*}$)-, and -C($R^6R^{6*}$)-C($R^7R^{7*}$);

B is selected from hydrogen, hydroxy, optionally substituted $C_{1-4}$-alkoxy, optionally substituted $C_{1-4}$-alkyl, optionally substituted $C_{1-4}$-acyloxy, nucleobases (including modified nucleobases, e.g., SBC nucleobases and universal nucleobases), and photochemically active groups;

P designates the radical position for an internucleoside linkage to a succeeding monomer, or a 5'-terminal group, such internucleoside linkage or 5'-terminal group optionally including the substituent $R^5$. One of the substituents $R^2$, $R^{2*}$, $R^3$, and $R^{3*}$ is a group $P^*$ which designates an internucleoside linkage to a preceding monomer, or a 2'/3'-terminal group. The substituents of $R^{1*}$, $R^{4*}$, $R^5$, $R^{5*}$, $R^6$ $R^{6*}$, $R^7$, $R^{7*}$, $R^N$ and the ones of $R^2$, $R^{2*}$, $R^3$, and $R^{3*}$ not designating 9 P* each designates a biradical comprising about 1-8 groups/atoms selected from -C($R^aR^b$)-, - C($R^a$)=C($R^a$)-, -C($R^a$)=N-, -C($R^a$)-O-, -O-, -Si($R^a$)$_2$-, -C($R^a$)-S, -S-, -$SO_2$-, -C($R^a$)-N($R^b$)-, - N($R^a$)-, and >C=Q, wherein Q is selected from -O-, -S-, and -N($R^a$)-, and $R^a$ and $R^b$ each is independently selected from hydrogen, optionally substituted $C_{1-12}$-alkyl, optionally substituted $C_{2-12}$-alkenyl, optionally substituted $C_{2-12}$-alkynyl, hydroxy, $C_{1-12}$-alkoxy, $C_{2-12}$-alkenyloxy, carboxy, $C_{1-12}$-alkoxycarbonyl, $C_{1-12}$-alkylcarbonyl, formyl, aryl, aryloxy-carbonyl, aryloxy, arylcarbonyl, heteroaryl, hetero-aryloxy-carbonyl, heteroaryloxy, heteroarylcarbonyl, amino, mono- and di($C_{1-6}$-alkyl)amino, carbamoyl, mono- and di($C_{1-6}$-alkyl)-amino-carbonyl, amino-$C_{1-6}$-alkyl-aminocarbonyl, mono- and di($C_{1-6}$-alkyl)amino-$C_{1-6}$-alkyl-aminocarbonyl, $C_{1-6}$-alkyl-carbonylamino, carbamido, $C_{1-6}$-alkanoyloxy, sulphono, $C_{1-6}$-alkylsulphonyloxy, nitro, azido, sulphanyl, $C_{1-6}$-alkylthio, halogen, photochemically active groups, where aryl and heteroaryl may be optionally substituted, and where two geminal substituents $R^a$ and $R^b$ together may designate optionally substituted methylene (=$CH_2$), and wherein two non-geminal or geminal substituents selected from $R^a$, $R^b$, and any of the substituents $R^{1*}$, $R^2$, $R^{2*}$, $R^3$, $R^{3*}$, $R^{4*}$, $R^5$, $R^{5*}$, $R^6$ and $R^{6*}$, $R^7$, and $R^{7*}$ which are present and not involved in P, $P^*$ or the biradical(s) together may form an associated biradical selected from biradicals of the same kind as defined before; the pair(s) of non-geminal substituents thereby forming a mono- or bicyclic entity together with (i) the atoms to which said non-geminal substituents are bound and (ii) any intervening atoms;

each of the substituents $R^{1*}$, $R^2$, $R^{2*}$, $R^3$, $R^{4*}$, $R^5$, $R^{5*}$, $R^6$ and $R^{6*}$, $R^7$, and $R^{7*}$ which are present and not involved in P, $P^*$ or the biradical(s), is independently selected from hydrogen, optionally substituted $C_{1-12}$-alkyl, optionally substituted $C_{2-12}$-alkenyl, optionally substituted $C_{2-12}$-alkynyl, hydroxy, $C_{1-12}$-alkoxy, $C_{2-12}$-alkenyloxy, carboxy, $C_{1-12}$-alkoxycarbonyl, $C_{1-12}$-alkylcarbonyl, formyl, aryl, aryloxy-carbonyl, aryloxy, arylcarbonyl, heteroaryl, hetero-aryloxy-carbonyl, heteroaryloxy, heteroarylcarbonyl, amino, mono- and di($C_{1-6}$-alkyl)amino, carbamoyl, mono- and di($C_{1-6}$-alkyl)-amino-carbonyl, amino-$C_{1-6}$-alkyl-aminocarbonyl, mono- and di($C_{1-6}$-alkyl)amino-$C_{1-6}$-alkyl-aminocarbonyl, $C_{1-6}$-alkyl-carbonylamino, carbamido, $C_{1-6}$-alkanoyloxy, sulphono, $C_{1-6}$-alkylsulphonyloxy, nitro, azido, sulphanyl, $C_{1-6}$-alkylthio, halogen, pho-

tochemically active groups, where aryl and heteroaryl may be optionally substituted, and where two geminal substituents together may designate oxo, thioxo, imino, or optionally substituted methylene, or together may form a spiro biradical consisting of a 1-5 carbon atom(s) alkylene chain which is optionally interrupted and/or terminated by one or more heteroatoms/groups selected from -O-, -S-, and -($NR^N$)- where $R^N$ is selected from hydrogen and $C_{1-4}$-alkyl, and where two adjacent (non-geminal) substituents may designate an additional bond resulting in a double bond; and $R^{N*}$, when present and not involved in a biradical, is selected from hydrogen and $C_{1-4}$-alkyl;

and basic salts and acid addition salts thereof.

By "photochemically active groups" is meant compounds which are able to undergo chemical reactions upon irradiation with light. Illustrative examples of functional groups are quinones, especially 6-methyl-1,4-naphtoquinone, anthraquinone, naphtoquinone, and 1,4-dimethyl-anthraquinone, diazirines, aromatic azides, benzophenones, psoralens, diazo compounds, and diazirino compounds.

It should be understood that the above-mentioned specific examples under photochemically active groups correspond to the "active/functional" part of the groups in question. For the person skilled in the art it is furthermore clear that photochemically active groups are typically represented in the form M-K- where M is the "active/functional" part of the group in question and where K is a spacer (see the definition further above) through which the "active/functional" part is attached to the 5- or 6-membered ring.

Exemplary 5', 3', and/or 2' terminal groups (representing the group P and/or the one of the substituents $R^2$, $R^{2*}$, $R^3$, and $R^{3*}$ being a group P*) include -H, -OH, halo (e.g. chloro, fluoro, iodo, or bromo), optionally substituted aryl, (e.g. phenyl or benzyl), alkyl (e.g, methyl or ethyl), alkoxy (e.g. methoxy), acyl (e.g. acetyl or benzoyl), aroyl, aralkyl, hydroxy, hydroxyalkyl, alkoxy, aryloxy, aralkoxy, nitro, cyano, carboxy, alkoxycarbonyl, aryloxycarbonyl, aralkoxycarbonyl, acylamino, aroylamine, alkylsulfonyl, arylsulfonyl, heteroarylsulfonyl, alkylsulfinyl, arylsulfinyl, heteroarylsulfinyl, alkylthio, arylthio, heteroarylthio, aralkylthio, heteroaralkylthio,amidino, amino, carbamoyl, sulfamoyl, alkene, alkyne, protecting groups (e.g. silyl, 4,4'-dimethoxytrityl, monomethoxytrityl, or trityl(triphenylmethyl)), linkers (e.g. a linker containing an amine, ethylene glycol, quinone such as anthraquinone), detectable labels (e.g. radiolabels or fluorescent labels), and biotin.

In the present context, the term "nucleobase" covers "naturally-occuring" as well as "modified" nucleobases. The term "nucleobase" includes not only the known purine and pyrimidine heterocycles, but also heterocyclic analogues and tautomers thereof such as xanthine, diaminopurine, 8-oxo-$N^6$-methyladenine, 7-deazaxanthine, 7-deazaguanine, $N^4$,$N^4$-ethanocytosin, $N^6$,$N^6$-ethano-2,6-diaminopurine, 5-methylcytosine (mC), 5-($C^3$-$C^6$)-alkynyl-cytosine, 5-fluorouracil, 5-bromouracil, pseudoisocytosine, 2-hydroxy-5-methyl-4-triazolopyridine, isocytosine, isoguanine, hypoxanthine and the nucleobases described in: Benner et al., U.S. Pat No. 5,432,272; in Susan M. Freier and Karl-Heinz Altmann, Nucleic Acids Research, 1997, vol. 25, pp 4429-4443; in U.S. Pat. No. 3,687,808 (Merigan, et al.); in Chapter 15 by Sanghvi, in Antisense Research and Application, Ed. S. T. Crooke and B. Lebleu, CRC Press, 1993,; in Englisch et al., Angewandte Chemie, International Edition, 1991, 30, 613-722 (see especially pages 622 and 623); in the Concise Encyclopedia of Polymer Science and Engineering, J. I. Kroschwitz Ed., John Wiley & Sons, 1990, pages 858-859; and in Cook, Anti-Cancer Drug Design 1991, 6, 585-607, each of which are hereby incorporated by reference in their entirety).

By the term "naturally occcuring nucleobase" is meant the nucleobases adenine (A), guanine (G), cytosine (C), thymine (**T**) and uracil (U) and taotomers hereof. With reference to the present disclosure (in particular Tables 8, 9 and 10), it should be noted that the nucleobase 5-methyl-cytosine ($^{Me}$C) can be used interchangeably with the nucleobase cytosine (C). Thus, the nucleobase ($^{Me}$C) can for the embodiments disclosed herein be viewed as a naturally-occurring nucleobase.

By the term "modified nucleobases" is meant all non-naturally-occurring nucleobases as described above.

By the term "SBC nucleobases" is meant "Selective Binding Complementary" nucleobases, i.e. modified nucleobases that can make stable hydrogen bonds to their complementary nucleobases, but are unable to make stable hydrogen bonds to other SBC nucleobases. As an example, the SBC nucleobase A', can make a stable hydrogen bonded pair with its complementary unmodified nucleobase, T. Likewise, the SBC nucleobase T' can make a stable hydrogen bonded pair with its complementary unmodified nucleobase, A. However, the SBC nucleobases A' and T' will form an unstable hydrogen bonded pair as compared to the basepairs A'-T and A-T'. Likewise, a SBC nucleobase of C is designated C' and can make a stable hydrogen bonded pair with its complementary unmodified nucleobase G, and a SBC nucleobase of G is designated G' and can make a stable hydrogen bonded pair with its complementary unmodified nucleobase C, yet C' and G' will form an unstable hydrogen bonded pair as compared to the basepairs C'-G and C-G'. A stable hydrogen bonded pair is obtained when 2 or more hydrogen bonds are formed e.g. the pair between A' and T, A and T', C and G', and C' and G. An unstable hydrogen bonded pair is obtained when 1 or no hydrogen bonds is formed e.g. the pair between A' and T', and C' and G'.

Especially interesting SBC nucleobases are 2,6-diaminopurine (A', also called D) together with 2-thio-uracil (U', also called $^{2S}$U)(2-thio-4-oxo-pyrimidine) and 2-thio-thymine (T', also called $^{2S}$T)(2-thio-4-oxo-5-methyl-pyrimidine). Figure 4 illustrates that the pairs A-$^{2S}$T and D-T have 2 or more than 2 hydrogen bonds whereas the D-$^{2S}$T pair forms a single (unstable) hydrogen bond. Likewise the SBC nucleobases pyrrolo-[2,3-d]pyrimidine-2(3H)-one (C', also called

PyrroloPyr) and hypoxanthine (G', also called I)(6-oxo-purine) are shown in Figure 9 where the pairs PyrroloPyr-G and C-I have 2 hydrogen bonds each whereas the PyrroloPyr-I pair forms a single hydrogen bond.

By "SBC LNA oligomer" is meant a "LNA oligomer" containing at least one "LNA unit" where the nucleobase is a "SBC nucleobase". By "LNA unit with an SBC nucleobase" is meant a "SBC LNA monomer". Generally speaking SBC LNA oligomers include oligomers that besides the SBC LNA monomer(s) contain other modified or naturally-occuring nucleotides or nucleosides. By "SBC monomer" is meant a non-LNA monomer with a SBC nucleobase. By "isosequential oligonucleotide" is meant an oligonucleotide with the same sequence in a Watson-Crick sense as the corresponding modified oligonucleotide e.g. the sequences agTtcATg is equal to agTscD$^{2S}$Ug where s is equal to the SBC DNA monomer 2-thio-t or 2-thio-u, D is equal to the SBC LNA monomer LNA-D and $^{2S}$U is equal to the SBC LNA monomer LNA $^{2S}$U.

By the term "universal nucleobase" is meant a modified nucleobase that when incorporated into oligonucleotides will exhibit a $T_m$ difference equal to 15, 12, 10, 8, 6, 4, or 2°C or less upon hybridizing to the four complementary oligonucleotide variants containing the naturally-occurring nucleobases (e.g. adenine, guanine, cytosine, uracil, and thymine) that are identical except for the nucleotide corresponding to the universal nucleobase. Thus, they are not nucleobases in the most classical sense but serve as nucleobases. Especially mentioned as universal nucleobases are 3-nitropyrrole, optionally substituted indoles (e.g. 5-nitroindole), hypoxanthine, pyrene, isocarbostyril and derivatives thereof and 8-aza-7-deazaadenine glycosylated at the N8 position. Other desirable universal nucleobases include, pyrrole, diazole or triazole derivatives, including those universal nucleobases known in the art. Further examples of universal nucleobases can be found in WO 03/020739 A2.

Other desirable universal nucleobases contain one or more carbon alicyclic or carbocyclic aryl units, i.e. non-aromatic or aromatic cyclic units that contain only carbon atoms as ring members. Universal nucleobases that contain carbocyclic aryl groups are generally desirable, particularly a moiety that contains multiple linked aromatic groups, particularly groups that contain fused rings. That is, optionally substituted polynuclear aromatic groups are especially desirable such as optionally substituted naphthyl, optionally substituted anthracenyl, optionally substituted phenanthrenyl, optionally substituted pyrenyl, optionally substituted chrysenyl, optionally substituted benzanthracenyl, optionally substituted dibenzanthracenyl, optionally substituted benzopyrenyl, with substituted or unsubstituted pyrenyl being particularly desirable.

Desirable universal nucleobases of the present invention when incorporated into an oligonucleotide containing all LNA units or a mixture of LNA and DNA or RNA units will exhibit substantially constant $T_m$ values upon hybridization with a complementary oligonucleotide, irrespective of the nucleobases present on the complementary oligonucleotide.

Unless indicated otherwise, an alicyclic group as referred to herein is inclusive of groups having all carbon ring members as well as groups having one or more hetero atom (e.g. N, O, S or Se) ring members. The disclosure of the group as a "carbon or hetero alicyclic group" further indicates that the alicyclic group may contain all carbon ring members (i.e. a carbon alicyclic) or may contain one or more hetero atom ring members (i.e. a hetero alicyclic). Alicyclic groups are understood not to be aromatic, and typically are fully saturated within the ring (i.e. no endocyclic multiple bonds). Desirably, the alicyclic ring is a hetero alicyclic, i.e. the alicyclic group has one or more hetero atoms ring members, typically one or two hetero atom ring members such as O, N, S or Se, with oxygen being often desirable. The one or more cyclic linkages of an alicyclic group may be comprised completely of carbon atoms, or generally more desirable, one or more hetero atoms such as O, S, N or Se, desirably oxygen for at least some embodiments. The cyclic linkage will typically contain one or two or three heteroatoms, more typically one or two hetero atoms in a single cyclic linkage.

By "nucleic acid", "oligonucleotide," and "oligomer," is meant a successive chain of monomers (i.e. nucleotides or units) connected via internucleoside linkages. An internucleoside linkage between two successive monomers in the oligo consist of 2 to 4, desirably 3, groups/atoms selected from $-CH_2-$, -O-, -S-, $-NR^H-$, >C=O, $>C=NR^H$, >C=S, $-Si(R'')_2-$, -SO-, $-S(O)_2-$, $-P(O)_2-$, $-PO(BH_3)-$, -P(O,S)-, $-P(S)_2-$, -PO(R")-, $-PO(OCH_3)-$, and $-PO(NHR^H)-$, where $R^H$ is selected from hydrogen and $C_{1-4}$-alkyl, and R" is selected from $C_{1-6}$-alkyl and phenyl. Illustrative examples of such linkages are $-CH_2-CH_2-CH_2-$, $-CH_2-CO-CH_2-$, $-CH_2-CHOH-CH_2-$, $-O-CH_2-O-$, $-O-CH_2-CH_2-$, $-O-CH_2-CH=$ (including $R^5$ when used as a linkage to a succeeding monomer), $-CH_2-CH_2-O-$, $-NR^H-CH_2-CH_2-$, $-CH_2-CH_2-NR^H-$, $-CH_2-NR^H-CH_2-$, $-O-CH_2-CH_2-NR^H-$, $-NR^H-CO-O-$, $-NR^H-CO-NR^H-$, $-NR^H-CS-NR^H-$, $-NR^H-C(=NR^H)-NR^H-$, $-NR^H-CO-CH_2-NR^H-$, - O-CO-O-, $-O-CO-CH_2-O-$, $-O-CH_2-CO-O-$, $-CH_2-CO-NR^H-$, $-O-CO-NR^H-$, $-NR^H-CO-CH_2-$, $-O-CH_2-CO-NR^H-$, $-O-CH_2-CH_2-NR^H-$, -CH=N-O-, $-CH_2-NR^H-O-$, $-CH_2-O-N=$ (including $R^5$ when used as a linkage to a succeeding monomer), $-CH_2-O-NR^H-$, $-CO-NR^H-CH_2-$, $-CH_2-NR^H-O-$, $-CH_2-NR^H-CO-$, $-O-NR^H-CH_2-$, $-O-NR^H-$, $-O-CH_2-S-$, $-S-CH_2-O-$, $-CH_2-CH_2-S-$, $-O-CH_2-CH_2-S-$, $-S-CH_2-CH=$ (including $R^5$ when used as a linkage to a succeeding monomer), $-S-CH_2-CH_2-$, $-S-CH_2-CH_2-O-$, $-S-CH_2-CH_2-S-$, $-CH_2-S-CH_2-$, $-CH_2-SO-CH_2-$, $-CH_2-SO_2-CH_2-$, -O-SO-O-, $-O-S(O)_2-O-$, $-O-S(O)_2-CH_2-$, $-O-S(O)_2-NR^H-$, $-NR^H-S(O)_2-CH_2-$, $-O-S(O)_2-CH_2-$, $-O-P(O)_2-O-$, -O-P(O,S)-O-, $-O-P(S)_2-O-$, $-S-P(O)_2-O-$, -S-P(O,S)-O-, $-S-P(S)_2-O-$, $-O-P(O)_2-S-$, -O-P(O,S)-S-, $-OP(S)_2-S-$, $-S-P(O)_2-S-$, -S-P(O,S)-S-, $-S-P(S)_2-S-$, -O-PO(R")-O-, $-O-PO(OCH_3)-O-$, $-O-PO-(OCH_2CH_3)-O-$, $-O-PO(OCH_2CH_2S-R)-O-$, $-O-PO(BH_3)-O-$, $-O-PO(NHR^N)-O-$, $-O-P(O)_2-NR^H-$, - $NR^H-P(O)_2-O-$, $-O-P(O,NR^H)-O-$, $-CH_2-P(O)_2-O-$, $-O-P(O)_2-CH_2-$, and $-O-Si(R'')_2-O-$; among which $-CH_2-CO-NR^H-$, $-CH_2-NR^H-O-$, $-S-CH_2-O-$, $-O-P(O)_2-O-$, -O-P(O,S)-O-, $-O-P(S)_2-O-$, - $NR^H-P(O)_2-O-$, $-O-P(O,NR^H)-O-$,

-O-PO(R")-O-, -O-PO($CH_3$)-O-, and -O-PO($NHR^N$)-O-, where $R^H$ is selected form hydrogen and $C_{1-4}$-alkyl, and R" is selected from $C_{1-6}$-alkyl and phenyl, are especially desirable. Further illustrative examples are given in Mesmaeker et. al., Current Opinion in Structural Biology 1995, 5, 343-355 and Susan M. Freier and Karl-Heinz Altmann, Nucleic Acids Research, 1997, vol 25, pp 4429-4443. The left-hand side of the internucleoside linkage is bound to the 5-membered ring as substituent P* at the 3'-position, whereas the right-hand side is bound to the 5'-position of a preceding monomer.

Particular internucleoside linkages of the oligomers may be natural phosphorodiester linkages, or other linkages such as -O-$P(O)_2$-O-, -O-P(O,S)-O-, -O-$P(S)_2$-O-, -$NR^H$-$P(O)_2$-O-, - O-P(O,$NR^H$)-O-, -O-PO(R")-O-, -O-PO($CH_3$)-O-, and -O-PO($NHR^N$)-O-, where $R^H$ is selected from hydrogen and $C_{1-4}$-alkyl, and R" is selected from $C_{1-6}$-alkyl and phenyl.

By "succeeding monomer" is meant the neighbouring monomer in the 5'-terminal direction, and by "preceding monomer" is meant the neighbouring monomer in the 3'-terminal direction.

Some interesting LNA units are exemplified in the formulae Ia and Ib below.

In formula Ia the configuration of the furanose is denoted β-D, and in formula Ib the configuration is denoted α-L. Configurations which are composed of mixtures of the two, e.g. β-D and α-L, are also included.

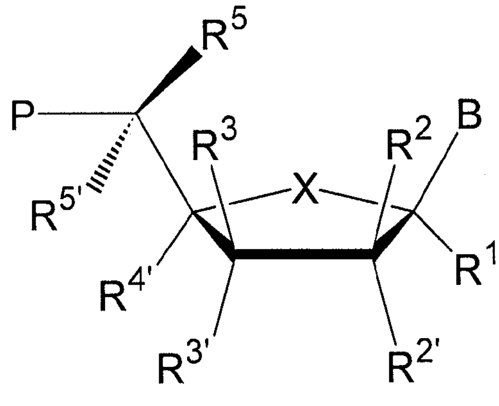

**Ia**

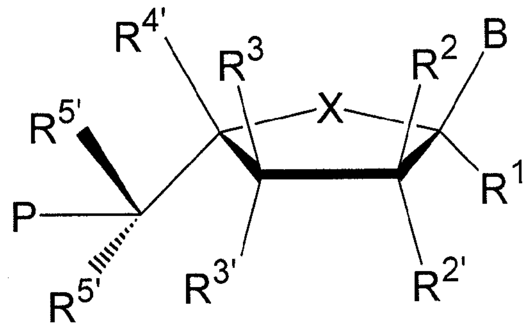

**Ib**

In Ia and Ib, X is selected from oxygen, sulfur and carbon (-$CH_2$-); B is a nucleobase, such as a naturally-occurring nucleobase or a modified nucleobase (particularly a SBC nucleobase) e.g. pyrene and pyridyloxazole derivatives, pyrenyl, pyrenylmethylglycerol moieties, all of which may be optionally substituted. Other desirable universal nucleobases include, pyrrole, diazole or triazole moieties, all of which may be optionally substituted, and other groups e.g. modified adenine, cytosine, 5-methylcytosine, isocytosine, pseudoisocytosine, guanine, thymine, uracil, 5-bromouracil, 5-propynyluracil, 5-propyny-6-fluoroluracil, 5-methylthiazoleuracil, 6-aminopurine, 2-aminopurine, hypoxanthine, diaminopurine, 7-propyne-7-deazaadenine, 7-propyne-7-deazaguanine. $R^1$, $R^2$ or $R^{2'}$, $R^3$ or $R^{3'}$, $R^5$ and $R^{5'}$ are hydrogen, methyl, ethyl, propyl, propynyl, aminoalkyl, methoxy, propoxy, methoxy-ethoxy, fluoro, or chloro.

P designates the radical position for an internucleoside linkage to a succeeding monomer, or a 5'-terminal group, $R^3$ or $R^{3'}$ is an internucleoside linkage to a preceding monomer, or a 3'-terminal group. The internucleotide linkage may be a phosphate, phosphorothioate, phosphorodithioate, phosphoramidate, phosphoroselenoate, phosphorodiselenoate, alkylphosphotriester, or methyl phosphonate. The internucleotide linkage may also contain non-phosphorous linkers, hydroxylamine derivatives (e.g. -$CH_2$-$NCH_3$-O-$CH_2$-), hydrazine derivatives, e.g. -$CH_2$-$NCH_3$-$NCH_3$-$CH_2$, amid derivatives, e.g. -$CH_2$- CO-NH-$CH_2$-, $CH_2$-NH-CO-$CH_2$-.

In Ia, $R^{4'}$ and $R^{2'}$ together designate -$CH_2$-O-, -$CH_2$-S-, -$CH_2$-NH-,-$CH_2$-NMe-, -$CH_2$-$CH_2$-O-, - $CH_2$-$CH_2$-S-, -$CH_2$-$CH_2$-NH-, or -$CH_2$-$CH_2$-NMe- where the oxygen, sulfur or nitrogen, respectively, is attached to the 2'-position ($R^2$/$R^{2'}$ position).

In Formula Ib, $R^{4'}$ and $R^2$ together designate -$CH_2$-O-, -$CH_2$-S-, -$CH_2$-NH-, -$CH_2$-NMe-, -$CH_2$-$CH_2$-O-, -$CH_2$-$CH_2$-S-, -$CH_2$-$CH_2$-NH-, or -$CH_2$-$CH_2$-NMe- where the oxygen, sulphur or nitrogen, respectively, is attached to the 2-position ($R^2$/$R^{2'}$ position).

In one embodiment, LNA units are those in which X is oxygen (Formula Ia and Ib); B is a universal nucleobase such as pyrene or a SBC base such as 2,6-diaminopurine, etc.; $R^1$, $R^2$ or $R^{2'}$, $R^3$ or $R^{3'}$, $R^5$ and $R^{5'}$ are hydrogen; P is a phosphate, phosphorothioate, phosphorodithioate, phosphoramidate, and methyl phosphornates; $R^3$ or $R^{3'}$ is an internucleoside linkage to a preceding monomer, or a 3'-terminal group. In Formula Ia, $R^4$ and $R^{2'}$ together designate -$CH_2$-O-, -$CH_2$-S-, -$CH_2$-NH-, -$CH_2$-NMe-, -$CH_2$-$CH_2$-O-, -$CH_2$-$CH_2$-S-, -$CH_2$-$CH_2$-NH-, or -$CH_2$-$CH_2$-NMe- where the oxygen, sulphur or nitrogen, respectively, is attached to the 2'-position, and in Formula Ib, $R^{4'}$ and $R^2$ together designate -$CH_2$-O-, -$CH_2$-S-, -$CH_2$-NH-,-$CH_2$-NMe-, -$CH_2$-$CH_2$-O-, -$CH_2$-$CH_2$-S-, -$CH_2$-$CH_2$-NH-, or -$CH_2$-$CH_2$-NMe- where the

oxygen, sulphur or nitrogen, respectively, is attached to the 2'-position in the $R^2$ configuration.

In another embodiment, LNA units are as above where B is a nucleobase, e.g. a naturally occurring nucleobase.

Particularly interesting LNA units have the configuration and substitution pattern shown immediately below and are particularly applicable.

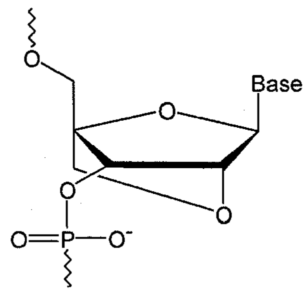

Furthermore, ENA's (2'O,4'C-ethylene-bridged nucleic acids) may also be utilised:

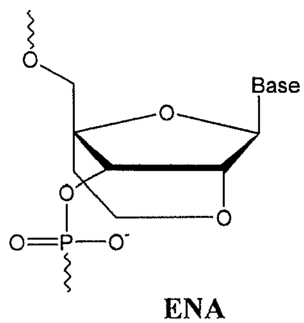

**ENA**

Examples of useful LNA monomers for incorporation into an LNA oligomer include those of the following formula IIa

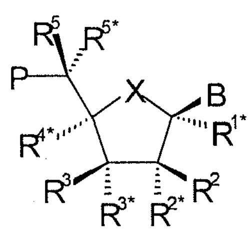

IIa

wherein X oxygen, sulfur, nitrogen, substituted nitrogen, carbon and substituted carbon, and desirably is oxygen; B is a modified nucleobase as discussed above e.g. an optionally substituted carbocyclic aryl such as optionally substituted pyrene or optionally substituted pyrenylmethylglycerol, or an optionally substituted heteroalicylic or optionally substituted heteroaromatic such as optionally substituted pyridyloxazole. Other desirable universal nucleobases include, pyrrole, diazole or triazole moieties, all of which may be optionally substituted; $R^{1*}$, $R^2$, $R^3$, $R^5$ and $R^{5*}$ are hydrogen; P designates the radical position for an internucleoside linkage to a succeeding monomer, or a 5'-terminal group, $R^{3*}$ is an internucleoside linkage to a preceding monomer, or a 3'-terminal group; and $R^{2*}$ and $R^{4*}$ together designate $-O-CH_2-$ or $-CH_2-CH_2-O-$ where the oxygen is attached in the 2'-position, or a linkage of $-(CH_2)_n-$ where n is 2, 3 or 4, desirably 2, or a linkage of $-S-CH_2-$ or $-NH-CH_2-$.

Desirable LNA monomers and oligomers share some chemical properties of DNA and RNA; they are water

soluble, can be separated by agarose gel electrophoresis, and can be ethanol precipitated.

Desirable LNA monomers and oligonucleotide units include nucleoside units having a 2'-4' cyclic linkage, as described in the International Patent Application WO 99/14226 and WO 00/56746, WO 00/56748, and WO 00/66604.

In one embodiment, desirable LNA monomers for use in oligonucleotides of the invention are 2'-deoxyribonucleotides, ribonucleotides, and analogues thereof that are modified at the 2'-position in the ribose, such as 2'-O-methyl, 2'-fluoro, 2'-trifluoromethyl, 2'-O-(2-methoxyethyl), 2'-O-aminopropyl, 2'-O-dimethylamino-oxyethyl, 2' -O-fluoroethyl or 2'-O-propenyl, and analogues wherein the modification involves both the 2'and 3' position, desirably such analogues wherein the modifications links the 2'- and 3'-position in the ribose, such as those described in Nielsen et al., J. Chem. Soc., Perkin Trans. 1, 1997, 3423-33, and in WO 99/14226, and analogues wherein the modification involves both the 2'- and 4'-position, desirably such analogues wherein the modifications links the 2'- and 4'-position in the ribose, such as analogues having a $-CH_2-O-$, $-CH_2-S-$ or a $-CH_2-NH-$ or a $-CH_2-NMe$-bridge (see Singh et al. J. Org. Chem. 1998, 6, 6078-9). Although LNA monomers having the β-D-ribo configuration are often the most applicable, other configurations also are suitable for purposes of the invention. Of particular use are α-L-ribo, the β-D-xylo and the α-L-xylo configurations (see Beier et al., Science, 1999, 283, 699 and Eschenmoser, Science, 1999, 284, 2118), in particular those having a 2'-4' $-CH_2-S-$, $-CH_2-NH-$, $-CH_2-O-$ or $-CH_2-NMe$-bridge.

Further examples of LNA units are shown in Figure 3. In Figure 3, the groups X and B are defined as above. P designates the radical position for an internucleoside linkage to a succeeding monomer, nucleoside such as an L-nucleoside, or a 5'-terminal group, such internucleoside linkage or 5'-terminal group optionally including the substituent $R^5$. One of the substituents $R^2$, $R^{2*}$, $R^3$, and $R^{3*}$ is a group $P^*$ which designates an internucleoside linkage to a preceding monomer, or a 2'/3'-terminal group. Y and Z represent the biradical defined above for the formula A.

The nucleoside can be comprised of a β-D, a β-L or an α-L nucleoside. Desirable nucleosides may be linked as dimers wherein at least one of the nucleosides is a β-L or α-L.

In the above embodiments, B may also designate the pyrimidine bases cytosine, 5-methyl-cytosine, thymine, uracil, or 5-fluorouridine (5-FUdR) other 5-halo compounds, or the purine bases adenosine, guanosine or inosine.

As discussed above, a variety of LNA units may be employed in the monomers and oligomers of the invention including bicyclic and tricyclic DNA or RNA having a 2'-4' or 2'-3' sugar linkages, in particular 2'-O,4'-C-methylene-β-D-ribofuranosyl moiety, known to adopt a locked C3'-endo RNA-like furanose conformation. Other nucleic acid units that may be included in an oligonucleotide of the invention may comprise 2'-deoxy-2'-fluoro ribonucleotides; 2'-O-methyl ribonucleotides; 2'-O-methoxyethyl ribonucleotides; peptide nucleic acids; 5-propynyl pyrimidine ribonucleotides; 7-deazapurine ribonucleotides; 2,6-diaminopurine ribonucleotides; and 2-thio-pyrimidine ribonucleotides, and nucleotides with other sugar groups (e.g. xylose).

It is understood that references herein to a nucleic acid unit, nucleic acid residue, LNA unit, or similar term are inclusive of both individual nucleoside units and nucleotide units and nucleoside units and nucleotide units within an oligonucleotide.

In the currently most preferred embodiment, the LNA units of the LNA oligomer(s) have the formula

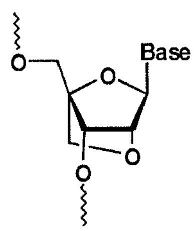

wherein "Base" designates a nucleobase. In one important embodiment, the nucleobase is a naturally-occurring nucleobase. In another important embodiment, the nucleobase is an SBC nucleobase. Further embodiment, which may be combined with the above, are those where the 2',4'-methylene(oxy) bridge is replaced by a 2',4'-methylene(thio), 2',4'-methylene(amino), or 2',4'-methylene(methylamino) bridge.

*Populations of Nucleic Acids with Decreased Variance in Melting Temperature, Increased Thermal Stability and/or Increased Capture Efficiency*

In one aspect, the invention features the population of nucleic acids wherein the variance in the melting temperature of the first population is at least 10%, at least 20%, at least 30%, at least 40%, at least 50%, at least 60%, or 70% less than the variance in the melting temperature of the corresponding control population of nucleic acids.

In desirable embodiments, the standard deviation in melting temperature for the nucleic acids of the first

population is less than 10, less than 9.5, less than 9, less than 8.5, less than 8, less than 7.5, less than 7, less than 6.5, or less than 6. In certain embodiment, the range in melting temperatures for nucleic acids in the first population is less than 70˚C, less than 60˚C, less than 50˚C, less than 40˚C, less than 30˚C, or 20˚C. Desirably, the variance in the melting temperature of the first population is less than 59˚C, less than 50˚C, less than 40˚C, less than 30˚C, less than 25˚C, less than 20˚C, less than 15˚C, less than 10˚C, or less than 5˚C.

In another aspect, the invention provides the population of nucleic acids that includes a first population of nucleic acid wherein each nucleic acid includes one or more universal nucleobases. In desirable embodiments, the LNA has at least one LNA A or LNA T. In some embodiments, the population of nucleic acids also includes one or more nucleic acids of a different length.

In a further aspect, the invention features the population of nucleic acids, wherein at least one LNA oligomer of the first population has a melting temperature that is at least 5, at least 8˚C, at least 10˚C, at least 12˚C, at least 15˚C, at least 20˚C, at least 25˚C, at least 30˚C, at least 35˚C, or at least 40˚C higher than that of the corresponding control nucleic acid. Desirably, at least 10%, at least 20%, at least 25%, at least 30%, at least 40%, at least 50%, at least 60%, at least 70%, at least 80%, at least 90%, or even 100% of the nucleic acid in the first population are LNA oligomers with a melting temperature that is at least 5, at least 8˚C, at least 10˚C, at least 12˚C, at least 15˚C, at least 20˚C, at least 25˚C, at least 30˚C, at least 35˚C, or at least 40˚C higher than that of the corresponding control nucleic acid. In some embodiments, the first population only has nucleic acids with naturally-occurring nucleobases.

In another aspect, the invention features the population of nucleic acids, wherein the first population has at least one LNA oligomer with a capture efficiency that is at least 50%, at least 100%, at least 150%, at least 200%, at least 500%, at least 800%, at least 1000%, or 12000% greater than that of the corresponding control nucleic acid at the temperature equal to the melting temperature of the nucleic acid of the first population.

Desirably, at least 10%, at least 20%, at least 25%, at least 30%, at least 40%, at least 50%, at least 60%, at least 70%, at least 80%, at least 90%, or even 100% of the nucleic acid in the first population are LNA oligomers with a capture efficiency that is at least 50%, at least 100%, at least 150%, at least 200%, at least 500%, at least 800%, at least 1000%, or 12000% greater than that of the corresponding control nucleic acid at the temperature equal to the melting temperature of the nucleic acid of the first population.

In a further related aspect, the invention features the population of nucleic acids, wherein at least 10%, at least 20%, at least 25%, at least 30%, at least 40%, at least 50%, at least 60%, at least 70%, at least 80%, at least 90%, or even 100% of the nucleic acid in the first population are LNA oligomers with a melting temperature that is at least 5, at least 8˚C, at least 10˚C, at least 12˚C, at least 15˚C, at least 20˚C, at least 25˚C, at least 30˚C, at least 35˚C, or at least 40˚C higher than that of the corresponding control nucleic acid and with a capture efficiency at least 50%, at least 100%, at least 150%, at least 200%, at least 500%, at least 800%, at least 1000%, or 12000% greater than that of the corresponding control nucleic acid at the temperature equal to the melting temperature of the nucleic acid of the first population.

In other embodiments, the first population includes at least 1%, at least 5%, at least 10%, at least 20%, at least 25%, at least 30%, at least 40%, at least 50%, at least 60%, at least 70%, at least 80%, at least 90%, or even 100% of the nucleic acid sequences expressed by a particular cell or tissue at a given point in time (e.g. an expression array with sequences corresponding to the sequences of mRNA molecules expressed by a particular cell type or a cell under a particular set of conditions).

The term "$T_m$" means the "melting temperature". The melting temperature is the temperature at which 50% of a population of double-stranded nucleic acid molecules becomes dissociated into single strands. The equation for calculating the $T_m$ of nucleic acids is well-known in the art. The $T_m$ of a hybrid nucleic acid is often estimated using a formula adopted from hybridization assays in 1 M salt, and commonly used for calculating $T_m$ for PCR primers: $T_m$ =[(number of A+T) x 2˚C + (number of G+C) x 4˚C]. C. R. Newton et al. PCR, 2nd Ed., Springer-Verlag (New York: 1997), p. 24. This formula was found to be inaccurate for primers longer that 20 nucleotides. Other more sophisticated computations exist in the art which take structural as well as sequence characteristics into account for the calculation of $T_m$ A calculated $T_m$ is merely an estimate; the optimum temperature is commonly determined empirically.

A modified nucleobase that gives rise to a $T_m$ differential of a specified amount (e.g. less than 15, less than 12 ˚C, less than 10 ˚C, less than 8 ˚C, less than 6 ˚C, less than 4 ˚C, less than 2 ˚C, or less than 1˚C) means that the modified nucleobase exhibits the specified $T_m$ differential when incorporated into a specified 9-mer oligonucleotide with respect to the four complementary variants, as defined immediately below.

Unless otherwise indicated, a $T_m$ differential provided by a particular modified nucleobase is calculated by the following protocol (steps a) through d)):

a) incorporating the modified nucleobase of interest into the following oligonucleotide 5'-d(GTGA**M**ATGC), wherein **M** is the modified nucleobase;

b) mixing 1.5 x $10^{-6}$M of the oligonucleotide having incorporated therein the modified nucleobase with each of 1.5x$10^{-6}$M of the four oligonucleotides having the sequence 3'-d(CACT**Y**TACG), wherein **Y** is A, C, G, T, respectively,

in a buffer of 10mM sodium phosphate, 100 mM sodium chloride, 0.1 mM EDTA, pH 7.0;

c) allowing the oligonucleotides to hybridize; and

d) detecting the $T_m$ for each of the four hybridized nucleotides by heating the hybridized nucleotides and observing the temperature at which the maximum of the first derivative of the melting curve recorded at a wavelength of 260 nm is obtained.

Unless otherwise indicated, a $T_m$ differential for a particular modified nucleobase is determined by subtracting the highest $T_m$ value determined in steps a) through d) immediately above from the lowest $T_m$ value determined by steps a) through d) immediately above.

By "variance in $T_m$ is meant the variance in the values of the melting temperatures for a population of nucleic acids. The $T_m$ for each nucleic acid is determined by experimentally measuring or computationally predicting the temperature at which 50% of a population double-stranded molecules with the sequence of the nucleic acid becomes dissociated into single strands. For a nucleic acid with only A, T, C, G, and/or U nucleobases, the $T_m$ is the temperature at which 50% of a population of 100% complementary double-stranded molecules with the sequence of the nucleic acid becomes dissociated into single strands. For determining the $T_m$ variance when a nucleic acid has one or more nucleobases other than A, T, C, G, or U, the $T_m$ of this "modified" nucleic acid is approximated by determining the $T_m$ for each possible double-stranded molecule in which one strand is the modified nucleic acid and the other strand has either A, T, C, or G in each position corresponding to a nucleobase other than A, T, C, G, or U in the modified nucleic acid. For example, if the modified nucleic acid has the sequence XMX in which X is 0, 1, or more A, T, C, G, or U nucleobases and M is any other nucleobase (i.e. not A, T, C, G or U), the $T_m$ is calculated for each possible double-stranded molecule in which one strand is XMX and the other strand is X'YX' in which X' is the nucleobase complementary to the corresponding X nucleobase and Y is either A, T, C, or G. The average is then calculated for the $T_m$ values for each possible double-stranded molecule (i.e., four possible duplexes per modified nucleobase in the modified nucleic acid) and used as the approximate $T_m$ value for the modified nucleic acid.

By the terms "corresponding control nucleic acid" and "control nucleic acid" are meant a β-D-2-deoxyribose nucleic acid (DNA) having the same nucleobase sequence and the same length as the nucleic acid in question, e.g. an LNA oligomer, however with the proviso that the nucleobases can only be A, T, C and G. Thus, if a unit of the nucleic acid in question has a U (urasil) nucleobase, the nucleobase in the corresponding unit in the control nucleic acid is T, and if a unit of the nucleic acid in question has a nucleobase not being A, T, C, G or U, the melting temperature and capture efficiency of the corresponding control nucleic acid is calculated as the average melting temperature and average capture efficiency for the nucleic acids that have A, T, C, and G in each position corresponding to a non-naturally-occurring nucleobase (non-"A, T, C, G or U") in the nucleic acid in the first population.

By the term "corresponding control population of nucleic acids" is meant a population of "control nucleic acids" corresponding to the population of nucleic acids.

By "capture efficiency" is meant the amount of target nucleic acid(s) bound to a particular nucleic acid or a population of nucleic acids. Standard methods can be used for calculating the capture efficiency by measuring the amount of bound target nucleic acid(s) and/or measuring the amount of unbound target nucleic acid(s). The capture efficiency of a nucleic acid or nucleic acid population of the invention is typically compared to the capture efficiency of a control nucleic acid or control nucleic acid population under the same incubation conditions (e.g. using same buffer and temperature).

*Particular Populations of Nucleic Acids*

In some embodiments, the nucleic acids of the first population only have naturally-occurring nucleobases.

In some embodiments, the at least one LNA oligomer of the first population has at least one LNA unit selected from LNA C, LNA G, LNA U, LNA A and LNA T.

In desirable embodiments, the at least one LNA oligomer has at least one LNA unit selected from LNA A and LNA T. In more particular embodiments, each LNA oligomer has at least one LNA unit selected from LNA A and LNA T. Desirably, all of the adenine and thymine-containing nucleotides in the LNA oligomers are LNA A and LNA T, respectively.

In other embodiments (which may be combined with the beforementioned embodiments), an LNA oligomer with an increased capture efficiency or melting temperature compared to a control nucleic acid has at least one LNA unit selected from LNA T and LNA C. In some embodiments, all of the thymidine and cytosine-containing nucleotides in the LNA oligomers are LNA T and LNA C, respectively.

In some embodiments, a nucleic acid with an increased specificity or decreased self-complementarity compared to a control nucleic acid has at least one LNA A or LNA C. In some embodiments, all of the adenine and cytosine-containing nucleotides in the LNA are LNA A and LNA C, respectively.

In some embodiments, the first population only has nucleic acids and LNA oligomers with naturally-occurring nucleobases, i.e. nucleobases selected from A, T, G, C and U.

In another embodiment, the LNA oligomers contain at least one LNA unit, such as an LNA unit with a modified nucleobase. Modified nucleobases desirably base-pair with adenine, guanine, cytosine, uracil, or thymine. In some embodiments, one or more LNA units with naturally-occurring nucleobases are incorporated into the oligonucleotide at a distance from the LNA unit having a modified nucleobase of 1 to 6 (e.g. 1 to 4) nucleobases. In certain embodiments, at least two LNA units with naturally-occurring nucleobases are flanking an LNA unit having a modified nucleobase. Desirably, at least two LNA units independently are positioned at a distance from the LNA unit having the modified nucleobase of 1 to 6 (e.g. 1 to 4 nucleobases).

By proper selection of the nucleic acids, in particular the position of LNA units in the LNA oligomers, and by possible modification of the nucleobases, the formation of certain secondary structures can be suppressed. Thus, other desirable nucleic acids have an LNA oligomer substitution pattern (i.e. the positioning of LNA units in the LNA oligomer) that results in negligible formation of secondary structure by the nucleic acids with itself. In one such embodiment, the nucleic acids do not form hairpins, dimer duplexes or other secondary structures that would otherwise inhibit or prevent their binding to a target nucleic acid. Preferably, the position of the LNA units in each LNA oligomer has been chosen by an algorithm substantially as described in Example 6 to reduce their propensity to form hairpins dimer duplexes or other secondary structures.

Desirably, opposing nucleotides in a palindrome pair or opposing nucleotides in inverted repeats or in reverse complements are not both LNA units.

In various embodiments, the nucleic acids in the first population form less than 3, 2, or 1 intramolecular base-pairs or base-pairs between two identical molecules.

For example, 5-mers, 6-mers, or 7-mers in a population of nucleic acids of the invention have one or more of the following substitution patterns: XxXXXxX or XxXXxX or XXXXX, in which "X" denotes an LNA unit and "x" denotes a DNA or RNA unit.

In some embodiments, one or more nucleic acids in the first population are LNA/DNA, LNA/RNA, or LNA/DNA/RNA chimeras.

In a further important embodiment of the invention, the first population comprises nucleic acids wherein at least one nucleotide or unit includes an SBC monomer. The SBC nucleobase is preferably selected from the group consisting of 2,6-diaminopurine, 2-thio-thymine and 2-thio-uracil. More preferred, at least one LNA oligomer has at least one LNA unit with a nucleobase selected from the group consisting of 2,6,-diaminopurine, 2-thio-thymine and 2-thio-uracil, i.e. a SBC LNA unit.

Other examples of SBC nucleobases to incorporate in the nucleic acids, in particular the LNA oligomers, are illustrated in Figures 10-12.

In another embodiment, which may be combined with the former, the first population comprises nucleic acids wherein at least one nucleotide or unit includes a universal nucleobase. In particular, one or more nucleic acids of the first population may have a nucleotide or unit that includes a universal nucleobase located at the 5' or 3' terminus of the nucleic acid. In a variant hereof, one or more nucleic acids of the first population have one or more (e.g. 2, 3, 4, 5, or more) nucleotides or units that include a universal nucleobases located at the 5' and 3' termini of the nucleic acid. In a special embodiment, all of the nucleic acids in the first population have the same number of universal nucleobases.

In a further embodiment hereof, all nucleic acids of the first population has at least one nucleotide or unit that includes a universal nucleobase.

Said universal nucleobases are desirably selected from the group consisting of hypoxanthine, pyrene, 3-nitropyrrole and 5-nitroindole.

In a further desirable embodiment, the LNA oligomer or oligomers of the first population has at least one LNA unit with a nucleobase selected from 2,6-diaminopurine, 2-aminopurine, 2-thio-thymine, 2-thio-uracil, and hypoxanthine.

*Methods for Detecting Target Nucleic Acids*

In one aspect, the invention features a method for detecting the presence of one or more, e.g. two or more, target nucleic acids in a sample, said method comprising (a) incubating said sample comprising said one or more target nucleic acids with the population of nucleic acids defined herein, under conditions that allow at least one of said target nucleic acids to hybridize to at least one of the nucleic acids in said population of nucleic acids.

The sequences are typically chosen to be as diverse as possible and not to match any particular target sequence. Hybridization is typically subsequently detected between at least 2, at least 3, at least 5, at least 10, at least 15, at least 20, at least 30, at least 40, at least 50, at least 75, or at least 100 target nucleic acids and the population of nucleic acids.

The method preferably comprises the further step of (b) detecting the hybridization. Thus in a related aspect, the invention provides a method for detecting the presence of one or more target nucleic acids in a sample, wherein the

method involves (a) incubating a nucleic acid sample with a population of nucleic acids of the invention under conditions that allow at least one of the target nucleic acids to hybridize to at least one of the nucleic acids in the population and (b) detecting the hybridization.

In desirable embodiments of the above detection methods, at least 5, at least 10, at least 15, at least 20, at least 30, at least 40, at least 50, at least 80, at least 100, at least 150, at least 200, or more target nucleic acids hybridize to the nucleic acids of the first population. Desirably, the method is repeated under one or more different incubation conditions. In particular embodiments, the method is repeated at 1, 3, 5, 8, 10, 15, 20, 30, 40 or more different temperatures, cation concentrations (e.g. concentrations of monovalent cations such as $Na^+$ and $K^+$ or divalent cations such as $Mg^{2+}$ and $Ca^{2+}$), denaturants (e.g. hydrogen bond donors or acceptors that interfere with the hydrogen bonds keeping the base-pairs together such as formamide or urea). Desirably, the method also includes identifying the target nucleic acid hybridized to the nucleic acids of the population and/or determining the amount of the target nucleic acid hybridized to the nucleic acids of the population. In particular embodiments, the target nucleic acids are labeled with a fluorescent group. In desirable embodiments, the determination of the amount of bound target nucleic acid involves one or more of the following: (i) adjusting for the varying intensity of the excitation light source used for detection of the hybridization, (ii) adjusting for photobleaching of the fluorescent group, and/or (iii) comparing the fluorescent intensity of the target nucleic acid(s) hybridized to the population of nucleic acids to the fluorescent intensity of a different sample of nucleic acids hybridized to the nucleic acids of the population (e.g. a different sample hybridized to the same population on the same or a different solid support such as the same chip or a different chip). Desirably, this comparison in fluorescent intensity involves adjusting for a difference in the amount of the population used for hybridization to each sample and/or adjusting for a difference in the buffer (e.g. a difference in $Mg^{2+}$ concentration) used for hybridization to each sample.

Desirably, the target nucleic acids are cDNA molecules reverse transcribed from a patient sample. In particular embodiments, the sample has nucleic acids amplified using one or more primers specific for an exon of a nucleic acid of interest, and the method involves determining the presence or absence of a splice variant including the exon in the sample. In some embodiments, the sample has nucleic acids amplified using one or more primers specific for a polymorphism in a nucleic acid of interest, and the method involves determining the presence or absence of the polymorphism in the sample. In still other embodiments, the sample has nucleic acids amplified using one or more primers specific for a nucleic acid of a pathogen of interest, and the method involves determining the presence or absence of the nucleic acid of the pathogen in the sample.

In an important embodiment, the one or more target nucleic acids include a nucleic acid of a pathogen (e.g. a nucleic acid in a sample such as a blood or urine sample from a mammal).

In a desirable embodiment, the population of nucleic acids is covalently bonded to a solid support by reaction of a nucleoside phosphoramidite with an activated solid support, and subsequent reaction of a nucleoside phosphoramidite with an activated nucleotide or nucleic acid bound to the solid support. In some embodiments, the solid support or the growing nucleic acid bound to the solid support is activated by illumination, a photogenerated acid, or electric current.

Oligonucleotides of the invention are particularly useful for detection and analysis of mutations including SNPS. In particular, for at least some applications, it may be desirable to employ an oligonucleotide as a "mutation resistant probe", i.e. a probe which does not detect a certain single base variation (complementary to the LNA unit with modified nucleobase) but maintains specific base pairing for other units of the probe. Hence, such a probe of the invention can detect a range of related mutations.

*Complex of Target Nucleic Acids and Nucleic Acid Probes*

In one aspect, the invention features a complex of one or more target nucleic acids and the population of nucleic acids defined herein, wherein one or more target nucleic acids are hybridized to a population of nucleic acids. Desirably, at least 2, at least 3, at least 4, at least 5, at least 6, at least 7, at least 10, at least 15, at least 20, at least 30, or at least 40 different target nucleic acids are hybridized. In some embodiments, the target nucleic acids are cDNA molecules reverse transcribed from a patient sample.

*Methods for Classifying Nucleic Acids Samples*

In one aspect, the invention features a method for classifying a test nucleic acid sample including target nucleic acids. This method involves (a) incubating a test nucleic acid sample with the population of nucleic acids defined herein under conditions that allow at least one of the nucleic acids in the test sample to hybridize to at least one nucleic acid in said population, (b) detecting the hybridization pattern of the test nucleic acid sample, and (c) comparing the hybridization pattern to the hybridization pattern of a first nucleic acid standard. In one embodiment, the comparison indicates whether or not the test sample has the same classification as the first standard. Desirably, the method also includes comparing the hybridization pattern of the test nucleic acid sample to the hybridization pattern of a second standard. In various embodiments, the hybridization pattern of the test nucleic acid sample is compared to at least 3, at least 4, at

least 5, at least 8, at least 10, at least 15, at least 20, at least 30, at least 40, or more standards.

Desirably, the method also includes identifying the target nucleic acid hybridized to the population and/or determining the amount of the target nucleic acid hybridized to the population. In particular embodiments, the target nucleic acids are labeled with a fluorescent group. In desirable embodiments, the determination of the amount of bound target nucleic acid involves one or more of the following: (i) adjusting for the varying intensity of the excitation light source used for detection of the hybridization, (ii) adjusting for photobleaching of the fluorescent group, and/or (iii) comparing the fluorescent intensity of the target nucleic acid(s) hybridized to the population of nucleic acids to the fluorescent intensity of a different sample of nucleic acids hybridized to the nucleic acids of the population (e.g. a different sample hybridized to same population on the same or a different solid support such as the same chip or a different chip). Desirably, this comparison in fluorescent intensity involves adjusting for a difference in the amount of the population used for hybridization to each sample and/or adjusting for a difference in the buffer (e.g. a difference in $Mg^{2+}$ concentration) used for hybridization to each sample.

In another aspect, the invention features a method for classifying a test nucleic acid sample including target nucleic acids. This method involves (a) incubating a test nucleic acid sample with a population of nucleic acids under conditions that allow at least one of the nucleic acids in the test sample to hybridize to at least one nucleic acid in the population, (b) detecting the hybridization pattern of the test nucleic acid sample, and (c) comparing the hybridization pattern to the hybridization pattern of a first nucleic acid standard, whereby the comparison indicates whether or not the test sample has the same classification as the first standard. The comparison of hybridization patterns involves one or more of the following: (i) adjusting for the varying intensity of the excitation light source used for detection of the hybridization, (ii) adjusting for photobleaching of the fluorescent group, and/or (iii) comparing the fluorescent intensity of the target nucleic acid(s) hybridized to the population of nucleic acids to the fluorescent intensity of a different sample of nucleic acids hybridized to the nucleic acids of the population (e.g. a different sample hybridized to the same population on the same or a different solid support such as the same chip or a different chip). Desirably, this comparison in fluorescent intensity involves adjusting for a difference in the amount of the population used for hybridization to each sample and/or adjusting for a difference in the buffer (e.g. a difference in $Mg^{2+}$ concentration) used for hybridization to each sample. Desirably, the method also includes comparing the hybridization pattern of the test nucleic acid sample to the hybridization pattern of a second standard. In various embodiments, the hybridization pattern of the test nucleic acid sample is compared to at least 3, at least 4, at least 5, at least 8, at least 10, at least 15, at least 20, at least 30, at least 40, or more standards. Desirably, the method also includes identifying the target nucleic acid hybridized to the population and/or determining the amount of the target nucleic acid hybridized to the population. In particular embodiments, the target nucleic acids are labeled with a fluorescent group. Desirably, the first population includes at least 1%, at least 5%, at least 10%, at least 20%, at least 25%, at least 30%, at least 40%, at least 50%, at least 60%, at least 70%, at least 80%, at least 90%, or 100% of the possible different nucleic acid sequences for nucleic acids of that length. In other embodiments, the first population is capable of binding at least 1%, at least 5%, at least 10%, at least 20%, at least 25%, at least 30%, at least 40%, at least 50%, at least 60%, at least 70%, at least 80%, at least 90%, or 100% of the nucleic acid sequences expressed by a particular cell or tissue (e.g. an expression array with sequences corresponding to the sequences of mRNA molecules expressed by a particular cell type or a cell under a particular set of conditions).

In desirable embodiments of any of the above detection methods, at least 5, at least 10, at least 15, at least 20, at least 30, at least 40, at least 50, at least 80, at least 100, at least 150, at least 200, or more target nucleic acids hybridize to the population of nucleic acids. Desirably, the method is repeated under one or more different incubation conditions. In particular embodiments, the method is repeated at 1, 3, 5, 8, 10, 15, 20, 30, 40 or more different temperatures, cation concentrations (e.g. concentration of monovalent cations such as $Na^+$ and $K^+$ or divalent cations such as $Mg^{2+}$ and Ca $^2$), denaturants (e.g. hydrogen bond donors or acceptors that interfere with the hydrogen bonds keeping the base-pairs together such as formamide or urea).

Desirably, the target nucleic acids are cDNA molecules reverse transcribed from a patient sample. In particular embodiments, the sample has nucleic acids amplified using one or more primers specific for an exon of a nucleic acid of interest, and the method involves determining the presence or absence of a splice variant including the exon in the sample. In some embodiments, the sample has nucleic acids amplified using one or more primers specific for a polymorphism in a nucleic acid of interest, and the method involves determining the presence or absence of the polymorphism in the sample. In still other embodiments, the sample has nucleic acids amplified using one or more primers specific for a nucleic acid of a pathogen of interest, and the method involves determining the presence or absence of the nucleic acid of the pathogen in the sample.

Desirably, the comparison of the hybridization pattern of a patient nucleic acid sample to that of one or more standards is used to determine whether or not a patient has a particular disease, disorder, condition, or infection or an increased risk for a particular disease, disorder, condition, or infection. In some embodiments, the comparison is used to determine what pathogen has infected a patient and to select a therapeutic for the treatment of the patient. Desirably, the comparison is used to select a therapeutic for the treatment or prevention of a disease or disorder in the patient. In

yet other embodiments, the comparison is used to include or exclude the patient from a group in a clinical trial.

In a desirable embodiment, the population of nucleic acids is covalently bonded to a solid support by reaction of a nucleoside phosphoramidite with an activated solid support, and subsequent reaction of a nucleoside phosphoramidite with an activated nucleotide or nucleic acid bound to the solid support. In some embodiments, the solid support or the growing nucleic acid bound to the solid support is activated by illumination, a photogenerated acid, or electric current.

The use of a variety of different monomers in the nucleic acids of the invention offers a means to "fine tune" the chemical, physical, biological, pharmacokinetic, and pharmacological properties of the nucleic acids thereby facilitating improvement in their safety and efficacy profiles when used as a therapeutic drug.

*Databases with Hybridization Patterns of Nucleic Acids Samples and/or Standards*

The invention also features a variety of databases. These databases are useful for storing the information obtained in any of the methods of the invention. These databases may also be used in the diagnosis of disease or an increased risk for a disease or in the selection of a desirable therapeutic for a particular patient or class of patients.

Accordingly, in one such aspect, the invention provides an electronic database including at least 1, at least 10, at least $10^2$, at least $10^3$, at least 5 x $10^3$, at least $10^4$, at least $10^5$, at least $10^6$, at least $10^7$, at least $10^8$, or at least $10^9$ records of a nucleic acid of interest or a population of nucleic acids of interest (e.g. one or more nucleic acids in a standard or in a test nucleic acid sample) correlated to records of its hybridization pattern to a population of nucleic acids of the invention under one or more incubation conditions (e.g. one or more temperatures, denaturant concentrations, or salt concentrations).

In another aspect, the invention features the computer including the database of the above aspect and a user interface (i) capable of displaying a hybridization pattern for a nucleic acid of interest or a population of nucleic acids of interest whose record is stored in the computer or (ii) capable of displaying a nucleic acid of interest (e.g. displaying the polynucleotide sequence or another identifying characteristic of the nucleic acid of interest) or a population of nucleic acids of interest that produces a hybridization pattern whose record is stored in the computer.

*Novel Monomers and Oligomers and Methods for Synthesizing Them*

Some of the nucleobases mentioned above are believed to give rise to novel LNA monomers and LNA oligomers. Thus, the present invention also provides the following novel LNA monomers, namely:

an LNA monomer being LNA-hypoxanthine (LNA-I) of the formula

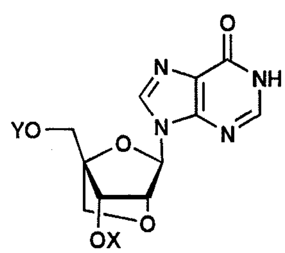

wherein X is a phosphoamidite group and Y is an oligonucleotide compatible hydroxyl-protection group such as DMT;

an LNA monomer being LNA-2,6-diaminopurine (LNA-D) of the formula

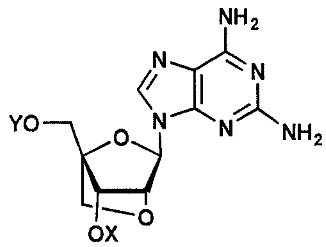

wherein X is a phosphoamidite group and Y is an oligonucleotide compatible hydroxyl-protection group such as DMT;

an LNA monomer being LNA-2-aminopurine (LNA-2AP) of the formula

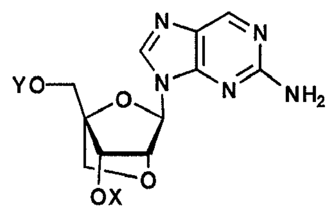

wherein X is a phosphoamidite group and Y is an oligonucleotide compatible hydroxyl-protection group such as DMT;

an LNA monomer being LNA-2-thiothymine (LNA-$^{2S}$T) of the formula

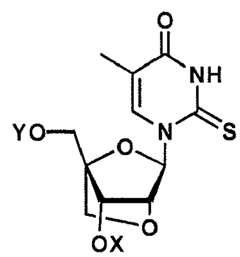

wherein X is a phosphoamidite group and Y is an oligonucleotide compatible hydroxyl-protection group such as DMT; and

an LNA monomer being LNA-2-thiouracil (LNA-$^{2S}$U) of the formula

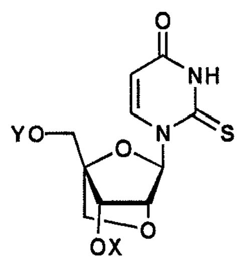

wherein X is a phosphoamidite group and Y is an oligonucleotide compatible hydroxyl-protection group such as DMT.

The present invention also provides:

a method of synthesizing the LNA-hypoxanthine (LNA-I) monomer, essentially comprising the steps described below or specifically in Example 13 herein;

a method of synthesizing the LNA-2,6-diaminopurine (LNA-D) monomer, essentially comprising the steps described below or in Example 13 herein;

a method of synthesizing the LNA-2-aminopurine (LNA-2AP) monomer, essentially comprising the steps described below or in Example 13 herein;

a method of synthesizing the LNA-2-thiothymine (LNA-$^{2S}$T) monomer, essentially comprising the steps described

below or in Example 11 or 12 herein; and

a method of synthesizing the LNA-2-thiouracil (LNA-$^{2S}$U) monomer, essentially comprising the steps described below or in Example 11 or 12 herein.

One method involves synthesizing a 2-thio-uridine nucleoside or nucleotide of formula **IV** using a compound of formula **VIII, IX, X, XI,** or **XII** as shown in Figure 6.

In a particular embodiment, nucleobase thiolation is performed on the O2 position of compound **XI** to form compound **IV**. In another embodiment, sulphurization on both 02 and 04 in compound **VIII** generates a 2,4-dithio-uridine nucleoside or nucleotide of formula **X** which is converted into compound **IV**. In yet another embodiment, a cyclic ether of formula **XI** is transferred into compound **IV** or a 2-O-alkyl-uridine nucleoside or nucleotide of formula **XII** through reaction with the 5' position. In other embodiments, a 2-O-alkyl-uridine nucleoside or nucleotide of formula **XII** is generated by direct alkylation of a uridine nucleoside or nucleotide of formula **VIII.**

In desirable embodiments, $R^4$ and $R^2$ in formula **IV** are each independently alkyl (e.g. methyl or ethyl), acyl (e.g. acetyl or benzoyl), or any appropriate protecting group such as silyl, 4,4'-dimethoxytrityl, monomethoxytrityl, or trityl(triphenylmethyl). $R^{5''}$ is any appropriate protecting group such as silyl, 4,4'-dimethoxytrityl, monomethoxytrityl, trityl (triphenylmethyl), acetyl, benzoyl, or benzyl. In desirable embodiments, $R^5$ is hydrogen, alkyl (e.g. methyl or ethyl), 1-propynyl, thiazol-2-yl, pyridine-2-yl, thien-2-yl, imidazol-2-yl, (4/5-methyl)-thiazol-2-yl, 3-(iodoacetamido)propyl, 4-[*N*,*N*-bis(3-aminopropyl)amino]butyl), or halo (e.g. chloro, bromo, iodo, fluoro).

The group -$OR^3$ in the formulas **IV, VIII, IX, X, XI**, and **XII** is selected from the group consisting of H, -OH, P $(O(CH_2)_2CN)N(iPr)_2$, $P(O(CH_2)_2CN)N(iPr)_2$, phosphate, phosphorothioate, phosphorodithioate, phosphoramidate, phosphoroselenoate, phosphorodiselenoate, alkylphosphotriester, methyl phosphonate, halo (e.g. chloro, fluoro, iodo, or bromo), optionally substituted aryl, (e.g. phenyl or benzyl), alkyl (e.g, methyl or ethyl), alkoxy (e.g. methoxy), acyl (e.g. acetyl or benzoyl), aroyl, aralkyl, hydroxy, hydroxyalkyl, alkoxy, aryloxy, aralkoxy, nitro, cyano, carboxy, alkoxycarbonyl, aryloxycarbonyl, aralkoxycarbonyl, acylamino, aroylamine, alkylsulfonyl, arylsulfonyl, heteroarylsulfonyl, alkylsulfinyl, arylsulfinyl, heteroarylsulfinyl, alkylthio, arylthio, heteroarylthio, aralkylthio, heteroaralkylthio,amidino, amino, carbamoyl, sulfamoyl, alkene, alkyne, protecting groups (e.g. silyl, 4,4'-dimethoxytrityl, monomethoxytrityl, or trityl(triphenylmethyl)), linkers (e.g. a linker containing an amine, ethylene glycol, quinone such as anthraquinone), detectable labels (e.g. radiolabels or fluorescent labels), and biotin.

The group -$OR^{5'}$ in the formulas **IV,** and **VIII, IX, X,** and **XII** is selected from the group consisting of H, -OH, P $(O(CH_2)_2CN)N(iPr)_2$, $P(O(CH_2)_2CN)N(iPr)_2$, phosphate, phosphorothioate, phosphorodithioate, phosphoramidate, phosphoroselenoate, phosphorodiselenoate, alkylphosphotriester, methyl phosphonate, halo (e.g. chloro, fluoro, iodo, or bromo), optionally substituted aryl, (e.g. phenyl or benzyl), alkyl (e.g, methyl or ethyl), alkoxy (e.g. methoxy), acyl (e.g. acetyl or benzoyl), aroyl, aralkyl, hydroxy, hydroxyalkyl, alkoxy, aryloxy, aralkoxy, nitro, cyano, carboxy, alkoxycarbonyl, aryloxycarbonyl, aralkoxycarbonyl, acylamino, aroylamine, alkylsulfonyl, arylsulfonyl, heteroarylsulfonyl, alkylsulfinyl, arylsulfinyl, heteroarylsulfinyl, alkylthio, arylthio, heteroarylthio, aralkylthio, heteroaralkylthio,amidino, amino, carbamoyl, sulfamoyl, alkene, alkyne, protecting groups (e.g. silyl, 4,4'-dimethoxytrityl, monomethoxytrityl, or trityl(triphenylmethyl)), linkers (e.g. a linker containing an amine, ethylene glycol, quinone such as anthraquinone), detectable labels (e.g. radiolabels or fluorescent labels), and biotin.

In yet another aspect, the invention features a method of synthesizing a compound. This method involves synthesizing a 2-thiopyrimidine nucleoside or nucleotide of formula **IV** using a compound of formula **III** or compounds of the formula **I, II,** and **III** as shown in Figure 7.

In some embodiments, Lewis acid-catalyzed condensation of a substituted sugar of formula **I** and a substituted 2-thio-uracil of formula **II** results in a substituted 2-thio-uridine nucleoside or nucleotide of the formula **III**. In some embodiments, a compound of formula III is converted into a LNA 2-thiouridine nucleoside or nucleotide of formula **IV.**

In desirable embodiments $R^{4'}$ and $R^{5'}$ are, e.g., methanesulfonyloxy, p-toluenesulfonyloxy, or any appropriate protecting group such as silyl, 4,4'-dimethoxytrityl, monomethoxytrityl, trityl(triphenylmethyl), acetyl, benzoyl, or benzyl, $R^{1'}$ is, e.g., acetyl, benzoyl, alkoxy (e.g. methoxy). $R^{2'}$ is, e.g.,acetyl or benzoyl, and $R^{3'}$ is any appropriate protecting group such as silyl, 4,4'-dimethoxytrityl, monomethoxytrityl, trityl(triphenylmethyl), acetyl, or benzoyl. In desirable embodiments, $R^5$ is hydrogen, alkyl (e.g. methyl or ethyl), 1-propynyl, thiazol-2-yl, pyridine-2-yl, thien-2-yl, imidazol-2-yl, (4/5-methyl)-thiazol-2-yl, 3-(iodoacetamido)propyl, 4-[*N*,*N*-bis(3-aminopropyl)amino]butyl), or halo (e.g. chloro, bromo, iodo, fluoro).

The group -$OR^{3'}$ in the formulas **I, III,** and **IV** is selected from the group consisting of H, - OH, $P(O(CH_2)_2CN)$ $N(iPr)_2$, phosphate, phosphorothioate, phosphorodithioate, phosphoramidate, phosphoroselenoate, phosphorodiselenoate, alkylphosphotriester, methyl phosphonate, halo (e.g. chloro, fluoro, iodo, or bromo), optionally substituted aryl, (e.g. phenyl or benzyl), alkyl (*e.g*, methyl or ethyl), alkoxy (*e.g.* methoxy), acyl (*e.g.* acetyl or benzoyl), aroyl, aralkyl, hydroxy, hydroxyalkyl, alkoxy, aryloxy, aralkoxy, nitro, cyano, carboxy, alkoxycarbonyl, aryloxycarbonyl, aralkoxycarbonyl, acylamino, aroylamine, alkylsulfonyl, arylsulfonyl, heteroarylsulfonyl, alkylsulfinyl, arylsulfinyl, heteroarylsulfinyl,

alkylthio, arylthio, heteroarylthio, aralkylthio, heteroaralkylthio, amidino, amino, carbamoyl, sulfamoyl, alkene, alkyne, protecting groups (e.g. silyl, 4,4'-dimethoxytrityl, monomethoxytrityl, or trityl(triphenylmethyl)), linkers (e.g. a linker containing an amine, ethylene glycol, quinone such as anthraquinone), detectable labels (e.g. radiolabels or fluorescent labels), and biotin.

The group $R^{5'}$ in the formulas **I, III,** and **IV** is selected from the group consisting of H, -OH, $P(O(CH_2)_2CN)N(iPr)_2$, phosphate, phosphorothioate, phosphorodithioate, phosphoramidate, phosphoroselenoate, phosphorodiselenoate, alkylphosphotriester, methyl phosphonate, halo (e.g. chloro, fluoro, iodo, or bromo), optionally substituted aryl, (e.g. phenyl or benzyl), alkyl (e.g, methyl or ethyl), alkoxy (e.g. methoxy), acyl (e.g. acetyl or benzoyl), aroyl, aralkyl, hydroxy, hydroxyalkyl, alkoxy, aryloxy, aralkoxy, nitro, cyano, carboxy, alkoxycarbonyl, aryloxycarbonyl, aralkoxycarbonyl, acylamino, aroylamine, alkylsulfonyl, arylsulfonyl, heteroarylsulfonyl, alkylsulfinyl, arylsulfinyl, heteroarylsulfinyl, alkylthio, arylthio, heteroarylthio, aralkylthio, heteroaralkylthio,amidino, amino, carbamoyl, sulfamoyl, alkene, alkyne, protecting groups (e.g. silyl, 4,4'-dimethoxytrityl, monomethoxytrityl, or trityl(triphenylmethyl)), linkers (e.g. a linker containing an amine, ethylene glycol, quinone such as anthraquinone), detectable labels (e.g. radiolabels or fluorescent labels), and biotin.

Another method involves synthesizing a 2-thiopyrimidine nucleoside or nucleotide of formula **IV** using a compound of formula **VII**, compounds of the formula **V**, **VI**, and **VII**, or compounds of the formula **I**, **V**, **VI**, and **VII** as shown in Figure 8.

In some embodiments, a 2-thio-uridine nucleoside or nucleotide of the formula **IV** is synthesized through ring-synthesis of the nucleobase by reaction of an amino sugar of the formula **V** and a substituted isothiocyanate of the formula **VI.**

In desirable embodiments, $R^{4'}$ and $R^{5'}$ are each idenpendently, e.g., methanesulfonyloxy, p-toluenesulfonyloxy, or any appropriate protecting group such as silyl, 4,4'-dimethoxytrityl, monomethoxytrityl, trityl(triphenylmethyl), acetyl, benzoyl, or benzyl. $R^{1'}$ is, e.g., acetyl or benzoyl or alkoxy (e.g. methoxy), and $R^{2'}$ is, e.g., acetyl or benzoyl, $R^{3'}$ is any appropriate protecting group such as silyl, 4,4'-dimethoxytrityl, monomethoxytrityl, trityl(triphenylmethyl), acetyl, or benzoyl. $R^5$ are $R^6$ each idenpendently, e.g., hydrogen or alkyl (e.g. methyl or ethyl). $R^6$ can also be, e.g., an appropriate protecting group such as silyl, 4,4'-dimethoxytrityl, monomethoxytrityl, or trityl(triphenylmethyl). In desirable embodiments, $R^5$ is hydrogen or methyl, and $R^6$ is methyl or ethyl.

The group $-OR^{3'}$ in the formulas **I, V, VII,** and **IV** is selected from the group consisting of H, -OH, $P(O(CH_2)_2CN)N(iPr)_2$, phosphate, phosphorothioate, phosphorodithioate, phosphoramidate, phosphoroselenoate, phosphorodiselenoate, alkylphosphotriester, methyl phosphonate, halo (e.g. chloro, fluoro, iodo, or bromo), optionally substituted aryl, (e.g. phenyl or benzyl), alkyl (*e.g*, methyl or ethyl), alkoxy (e.g. methoxy), acyl (*e.g.* acetyl or benzoyl), aroyl, aralkyl, hydroxy, hydroxyalkyl, alkoxy, aryloxy, aralkoxy, nitro, cyano, carboxy, alkoxycarbonyl, aryloxycarbonyl, aralkoxycarbonyl, acylamino, aroylamine, alkylsulfonyl, arylsulfonyl, heteroarylsulfonyl, alkylsulfinyl, arylsulfinyl, heteroarylsulfinyl, alkylthio, arylthio, heteroarylthio, aralkylthio, heteroaralkylthio,amidino, amino, carbamoyl, sulfamoyl, alkene, alkyne, protecting groups (e.g. silyl, 4,4'-dimethoxytrityl, monomethoxytrityl, or trityl(triphenylmethyl)), linkers (e.g. a linker containing an amine, ethylene glycol, quinone such as anthraquinone), detectable labels (e.g. radiolabels or fluorescent labels), and biotin.

$R^{5'}$ in the formulas **I**, **V**, **VII**, and **IV** is selected from the group consisting of H, -OH, $P(O(CH_2)_2CN)N(iPr)_2$, phosphate, phosphorothioate, phosphorodithioate, phosphoramidate, phosphoroselenoate, phosphorodiselenoate, alkylphosphotriester, methyl phosphonate, halo (e.g. chloro, fluoro, iodo, or bromo), optionally substituted aryl, (e.g. phenyl or benzyl), alkyl (*e.g*, methyl or ethyl), alkoxy (e.g. methoxy), acyl (*e.g.* acetyl or benzoyl), aroyl, aralkyl, hydroxy, hydroxyalkyl, alkoxy, aryloxy, aralkoxy, nitro, cyano, carboxy, alkoxycarbonyl, aryloxycarbonyl, aralkoxycarbonyl, acylamino, aroylamine, alkylsulfonyl, arylsulfonyl, heteroarylsulfonyl, alkylsulfinyl, arylsulfinyl, heteroarylsulfinyl, alkylthio, arylthio, heteroarylthio, aralkylthio, heteroaralkylthio,amidino, amino, carbamoyl, sulfamoyl, alkene, alkyne, protecting groups (e.g. silyl, 4,4'-dimethoxytrityl, monomethoxytrityl, or trityl(triphenylmethyl)), linkers (e.g. a linker containing an amine, ethylene glycol, quinone such as anthraquinone), detectable labels (e.g. radiolabels or fluorescent labels), and biotin.

In a related aspect, the invention features a compound of the formula **IV** as described in the above aspect or a nucleic acid that includes one or more compounds of the formula **IV**.

Another method involves synthesizing a 2-thiopyrimidine nucleoside as shown in Figure 13. In desirable embodiments, the method further comprises removing the benzyl groups of one or both compounds of the formula **4** and reacting the 5'-hydroxy group with DMTCl and reacting the 3'-hydroxy group with a phosphodiamidite (e.g. 2-cyanoethyl tetraisopropylphosphorodiamidite) to produce the corresponding nucleoside phosphoramidite.

In some embodiments, a glycosyl-donor is coupled to a nucleobase as shown in pathway A. In other embodiments, ring synthesis of the nucleobase is performed as show in pathway B. In still other embodiments, LNA-T diol is modified as shown in pathway C.

In desirable embodiments, R is hydrogen, methyl, 1-propynyl, thiazol-2-yl, pyridine-2-yl, thien-2-yl, imidazol-2-yl, (4/5-methyl)-thiazol-2-yl, 3-(iodoacetamido)propyl, 4-[*N*,*N*-bis(3-aminopropyl)amino]butyl, or halo (e.g. chloro, bro-

mo, iodo, fluoro). Desirably, $R_1$, $R_2$, and $R_3$ are each any appropriate protecting group such as acetyl, benzyl, silyl, 4,4'-dimethoxytrityl, monomethoxytrityl, or trityl(triphenylmethyl).

In a related aspect, the invention features a 2-thiopyrimidine nucleoside or nucleotide as described in the above aspect or a nucleic acid that includes one or more 2-thiopyrimidine nucleosides or nucleotides as described in the above aspect.

Still another method involves synthesizing a 2-thiopyrimidine nucleoside or nucleotide of formula **4** using a compound of formula **3**, compounds of the formula **2** and **3**, or compounds of the formula **1**, **2**, **3**, and **4** as shown in Figure 13. This method can also be performed using any other appropriate protecting groups instead of Bn (benzyl), Ac (acetyl), or Ms (methansulfonyl).

In desirable embodiments, the method further comprises reacting one or both compounds of the formula **4** with a phosphodiamidite (e.g. 2-cyanoethyl tetraisopropylphosphorodiamidite) to produce the corresponding nucleoside phosphoramidite.

In a related aspect, the invention features a compound of the formula **4** as described in the above aspect or a nucleic acid that includes one or more compounds of the formula **4**.

A further method involves synthesizing a nucleoside or nucleotide of formula **10** or **11** using a compound of any one of the formula **6-9**, compounds of the formula **5** and any one of the formulas **6-9**, or compounds of the formula **4**, **5**, and any one of the formulas **6-9** as shown in Figure 15. This method may also be performed using any other appropriate protecting groups instead of DMT, Bn, Ac, or Ms.

In some embodiments, a compound of formula **4** is used as a glycosyl donor in a coupling reaction with silylated hypoxantine to form a compound of the formula **5**. In certain embodiments, a compound of the formula **5** is used in a ring-closing reaction to forma compound of the formula **6**. Desirably, deprotection of the 5'-hydroxy group of compound **6** is performed by displacing the 5'-O-mesyl group with sodium benzoate to produce a compound of the formula **7** that is converted into a compound of the formula **8** after saponification of the 5'-benzoate. In some embodiments, compound **8** is converted to a DMT-protected compound **9** prior to debenzylation of the 3'-O-hydroxy group. In desirable embodiments, a phosphoramidite of the formula **11** is generated by phosphitylation of a nucleoside of the formula **10**.

In desirable embodiments, the $R_1$ is H or $P(O(CH_2)_2CN)N(iPr)_2$. In other embodiments, the group $R_1$ or $-OR_1$ is selected from the group consisting of-OH, $P(O(CH_2)_2CN)N(iPr)_2$, phosphate, phosphorothioate, phosphorodithioate, phosphoramidate, phosphoroselenoate, phosphorodiselenoate, alkylphosphotriester, methyl phosphonate, halo (e.g. chloro, fluoro, iodo, or bromo), optionally substituted aryl, (e.g. phenyl or benzyl), alkyl (*e*.g, methyl or ethyl), alkoxy (e.g. methoxy), acyl (*e.g*. acetyl or benzoyl), aroyl, aralkyl, hydroxy, hydroxyalkyl, alkoxy, aryloxy, aralkoxy, nitro, cyano, carboxy, alkoxycarbonyl, aryloxycarbonyl, aralkoxycarbonyl, acylamino, aroylamine, alkylsulfonyl, arylsulfonyl, heteroarylsulfonyl, alkylsulfinyl, arylsulfinyl, heteroarylsulfinyl, alkylthio, arylthio, heteroarylthio, aralkylthio, heteroaralkylthio, amidino, amino, carbamoyl, sulfamoyl, alkene, alkyne, protecting groups (e.g. silyl, 4,4'-dimethoxytrityl, monomethoxytrityl, or trityl(triphenylmethyl)), linkers (e.g. a linker containing an amine, ethylene glycol, quinone such as anthraquinone), detectable labels (e.g. radiolabels or fluorescent labels), and biotin.

In a related aspect, the invention features a compound of the formula **11** as described in the above aspect or a nucleic acid that includes one or more compounds of the formula **11**.

A still further method involves synthesizing a nucleoside or nucleotide of formula **20** or **21** as shown in Figure 16, in which compound 4 is the same sugar shown in the above aspect. This method can also be performed using any other appropriate protecting groups instead of DMT, Bn, Bz (benzoyl), Ac, or Ms. Additionally, the method can be performed with any other halogen (e.g. fluoro or bromo) instead of chloro.

In desirable embodiments to promote the ring-closing reaction, a solution of compound **14** in aqueous 1,4-dioxane is treated with sodium hydroxide to give a bicyclic compound **15**. In some embodiments, sodium benzoate is used for displacement of 5'-mesylate of compound **15** to give compound **16**. In some embodiments, compound **17** is formed by reaction of compound **16** with sodium azide. In some embodiments, compound **18** is produced by saponification of the 5'-benzoate of compound **17.** In certain embodiments, hydrogenation of compound **18** produces compound **19**. In certain embodiments, the peracelation method is used to benzolylate the 2- and 6-amino groups of compound **19**, yielding **20**, which is desirably converted into the phosphoramidite compound **21**.

In a related aspect, the invention features a derivative of a compound of the formula 20 or **21** as described in the above aspect in which 3' -OH or $-OP(O(CH_2)_2CN)N(iPr)_2$ group is replaced by any other group is selected from the group consisting of phosphorothioate, phosphorodithioate, phosphoramidate, phosphoroselenoate, phosphorodiselenoate, alkylphosphotriester, methyl phosphonate, halo (e.g. chloro, fluoro, iodo, or bromo), optionally substituted aryl, (e.g. phenyl or benzyl), alkyl (*e.g*, methyl or ethyl), alkoxy (e.g. methoxy), acyl (*e.g.* acetyl or benzoyl), aroyl, aralkyl, hydroxy, hydroxyalkyl, alkoxy, aryloxy, aralkoxy, nitro, cyano, carboxy, alkoxycarbonyl, aryloxycarbonyl, aralkoxycarbonyl, acylamino, aroylamine, alkylsulfonyl, arylsulfonyl, heteroarylsulfonyl, alkylsulfinyl, arylsulfinyl, heteroarylsulfinyl, alkylthio, arylthio, heteroarylthio, aralkylthio, heteroaralkylthio,amidino, amino, carbamoyl, sulfamoyl, alkene, alkyne, protecting groups (e.g. silyl, 4,4'-dimethoxytrityl, monomethoxytrityl, or trityl(triphenylmethyl)), linkers (e.g. a linker containing an amine, ethylene glycol, quinone such as anthraquinone), detectable labels (e.g. radiolabels or fluorescent

labels), and biotin.

In yet another aspect, the invention features a method of synthesizing a compound. This method involves synthesizing a nucleoside or nucleotide of formula 20 or 21 as shown in Figure 17. This method can also be performed using any other appropriate protecting groups instead of DMT.

In some embodiments, compound **17** is formed by reaction of compound **7** withl,3-dichloro-1,1,3,3-tetraisopropyldisiloxane. Desirably, compound **18** is formed by reaction of compound **17** with phenoxyacetic anhydride. In some embodiments, compound **19** is generated by reaction of compound **18** with acid. Desirably, compound **20** is produced by reacting compound **19** with DMT-Cl. In desirably embodiments, compound **20** is reacted with 2-cyanoethyl tetraisopropylphosphorodiamidite to give the phosphoramidite **21.**

In desirable embodiments, the R is H or $P(O(CH_2)_2CN)N(iPr)_2$. In other embodiments, the R or -OR is any of the groups listed for $R^3$ or $R^{3'}$ in formula Ia or formula Ib or listed for $R^3$ or $R^{3*}$ in formula IIa, Scheme A, or Scheme B, or the group

-OR or R is selected from the group consisting of-OH, $P(O(CH_2)_2CN)N(iPr)_2$, phosphate, phosphorothioate, phosphorodithioate, phosphoramidate, phosphoroselenoate, phosphorodiselenoate, alkylphosphotriester, methyl phosphonate, halo (e.g. chloro, fluoro, iodo, or bromo), optionally substituted aryl, (e.g. phenyl or benzyl), alkyl (*e.g*, methyl or ethyl), alkoxy (e.g. methoxy), acyl (*e.g.* acetyl or benzoyl), aroyl, aralkyl, hydroxy, hydroxyalkyl, alkoxy, aryloxy, aralkoxy, nitro, cyano, carboxy, alkoxycarbonyl, aryloxycarbonyl, aralkoxycarbonyl, acylamino, aroylamine, alkylsulfonyl, arylsulfonyl, heteroarylsulfonyl, alkylsulfinyl, arylsulfinyl, heteroarylsulfinyl, alkylthio, arylthio, heteroarylthio, aralkylthio, heteroaralkylthio,amidino, amino, carbamoyl, sulfamoyl, alkene, alkyne, protecting groups (e.g. silyl, 4,4'-dimethoxytrityl, monomethoxytrityl, or trityl(triphenylmethyl)), linkers (e.g. a linker containing an amine, ethylene glycol, quinone such as anthraquinone), detectable labels (e.g. radiolabels or fluorescent labels), and biotin.

In a related aspect, the invention features a compound of the formula **20** or **21** as described in the above aspect or a nucleic acid that includes one or more compounds of the formula **20** or **21**.

A still further method involves synthesizing a nucleoside or nucleotide of formula **24** or **25** as shown in Figure 18. This method can also be performed using any other appropriate protecting groups instead of Bz, Bn, and DMT. Additionally, the method can be performed with any other halogen (e.g. fluoro or bromo) instead of chloro.

In some embodiments, the compound **16** is formed from compounds **4**, **14**, and **15** as illustrated in an aspect above. Desirably, the 5'-O-benzoyl group of compound **16** is hydrolyzed by aqueous sodium hydroxyde to give compound **22**. Compound **23** is desirably produced by incubation of compound **22** in the presence of paladium hydroxide and ammonium formate. Desirably, the 2-amine of compound **23** is selectively protected with an amidine group after treatment with *N,N*-dimethylformamide dimethyl acetal to yield compound **24.** In some embodiments, the diol **24** is 5'-*O*-DMT protected and 3'-*O*-phosphitylated produce the phosphoramidite LNA-2AP compound **25.**

In some embodiments, compound **25** has one of the following groups instead of the $P(O(CH_2)_2CN)N(iPr)_2$ group: any of the groups listed for $R^3$ or $R^{3'}$ in formula Ia or formula Ib or listed for $R^3$ or $R^{3*}$ in formula IIa, Scheme A, or Scheme B, or a group selected from the group consisting of-OH, phosphate, phosphorothioate, phosphorodithioate, phosphoramidate, phosphoroselenoate, phosphorodiselenoate, alkylphosphotriester, methyl phosphonate, halo (e.g. chloro, fluoro, iodo, or bromo), optionally substituted aryl, (e.g. phenyl or benzyl), alkyl (*e.g*, methyl or ethyl), alkoxy (e.g. methoxy), acyl (*e.g.* acetyl or benzoyl), aroyl, aralkyl, hydroxy, hydroxyalkyl, alkoxy, aryloxy, aralkoxy, nitro, cyano, carboxy, alkoxycarbonyl, aryloxycarbonyl, aralkoxycarbonyl, acylamino, aroylamine, alkylsulfonyl, arylsulfonyl, heteroarylsulfonyl, alkylsulfinyl, arylsulfinyl, heteroarylsulfinyl, alkylthio, arylthio, heteroarylthio, aralkylthio, heteroaralkylthio, amidino, amino, carbamoyl, sulfamoyl, alkene, alkyne, protecting groups (e.g. silyl, 4,4'-dimethoxytrityl, monomethoxytrityl, or trityl(triphenylmethyl)), linkers (e.g. a linker containing an amine, ethylene glycol, quinone such as anthraquinone), detectable labels (e.g. radiolabels or fluorescent labels), and biotin.

In a related aspect, the invention features a compound of the formula **24** or **25** as described in the above aspect or a nucleic acid that includes one or more compounds of the formula **24** or **25**.

In another aspect, the invention features a compound of the formula **6pC**or the product of a compound of the formula **6pC** treated with ammonia as described in Example 14 or a nucleic acid that includes one or more of these compounds. In a related aspect, the invention features a method of synthesizing a compound by performing one or more of the steps listed in Example 14.

These LNA monomers are particularly useful for the preparation of LNA oligomers in general, and in particular for the preparation of the populations of the present invention.

Thus, the invention also relates to the LNA oligomers having included therein at least one LNA unit corresponding to the monomers **IV, 4, 10, 11, 21, 25, 30, 31, 44, 45**.

In particular, the present invention also provides the following LNA oligomers:

an LNA oligomer comprising an LNA-hypoxanthine (LNA-I) unit as shown in formula 1 below

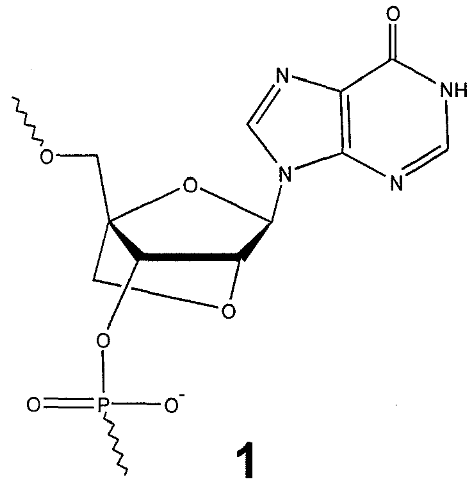

1 ;

an LNA oligomer comprising an LNA-2,6-diaminopurine (LNA-D) unit as shown in formula 2 below

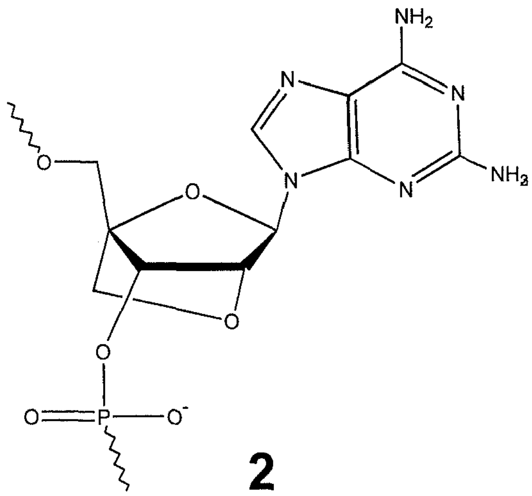

2 ;

an LNA oligomer comprising an LNA-2-aminopurine (LNA-2AP) unit as shown in formula 3 below

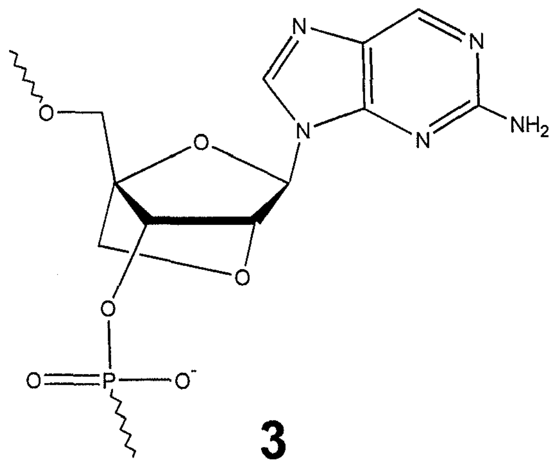

3 ;

an LNA oligomer comprising an LNA-2-thiothymine (LNA-$^{2S}$T) unit as shown in formula 4 below

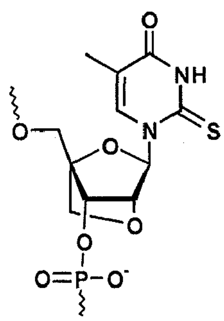

4;

and

an LNA oligomer comprising an LNA-2-thiouracil (LNA-$^{2S}$U) unit as shown in formula 5 below

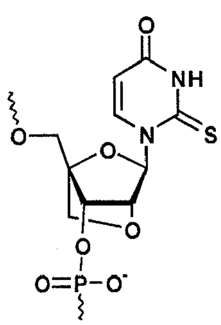

5.

All of the above oligomers are useful within the populations defined herein. Thus in a particular embodiment, the LNA oligomers of the population defined above comprises one or more of the LNA units of formulae 1-5 above.

This being said, it is envisaged that the novel LNA oligomers, in particular the LNA oligomers comprising one or more of the LNA units of the formulae 1-5 above, are also useful in may other applications either as individual LNA oligomers, in combination with other types of nucleic acids and oligonucleotides, as pluralities of LNA oligomers, as DNA/LNA, RNA/LNA chimera, etc.

*Novel SBC LNA oligomer pairs*

In view of the description of SBC LNA oligomers, the present invention also provides a pair of substantially complementary oligonucleotides, each comprising, in pairwise opposing positions, one or more SBC nucleotides or units, wherein at least one of the oligonucleotides is an LNA oligomer having SBC LNA units. Such pairs of oligonucleotides typically have 5-50, such as 1-15, nucleotides or unit. The incorporation of one or more pairs of complementary SBC nucleotides or units causes a reduction of the number of Watson-Crick hydrogen bonds compared to the isosequential pair of oligonucleotides.

In one embodiment, the SBC pair is an A':T' pair. In particular, the SBC pair is an A':T pair and where the SBC nucleobase T' has the structure as shown in formula (i) and where the SBC nucleobase A' has the structure as shown in formula (ii)

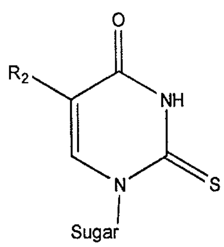

(i)

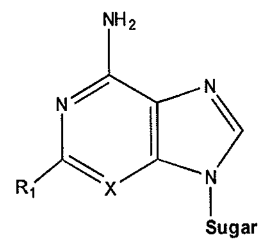

(ii)

wherein X = N or CH; $R_1$= $C_{1-4}$ alkyl, $C_{1-4}$ alkoxy, $C_{1-4}$ alkylthio, F, or $NHR_3$ where $R_3$ is H, or $C_{1-4}$ alkyl; and $R_2$ = H, $C_{1-6}$ alkyl, $C_{1-6}$ alkenyl, or $C_{1-6}$ alkynyl. In particular, X = N or CH; $R_1$ = $NHR_3$ where $R_3$ is H, or $C_{1-4}$ alkyl, and $R_2$ = H, $C_{1-6}$ alkyl, $C_{1-6}$ alkenyl, or $C_{1-6}$ alkynyl, e.g. X = N or CH; $R_1$ = $NH_2$, and $R_2$ = H, $C_{1-6}$ alkyl, $C_{1-6}$ alkenyl, or $C_{1-6}$ alkynyl, more particularly X = N; $R_1$ = $NH_2$, and $R_2$ = H, $C_{1-6}$ alkyl, $C_{1-6}$ alkenyl, or $C_{1-6}$ alkynyl, still more particularly X = N; $R_1$ = $NH_2$, and $R_2$ = H or $CH_3$, even more particularly X = N; $R_1$ = $NH_2$, and $R_2$ = H or X = N; $R_1$ = $NH_2$, and $R_2$ = $CH_3$.

In a further embodiment both sugars are of the LNA type, i.e. both oligonucleotides of the pair are LNA oligomers.

In another embodiment, the SBC pair is a G':C' pair. In particular, the SBC pair is a G:C pair and where the SBC nucleobase C' has the structure as shown in formula (iii) and where the SBC nucleobase G' has the structure as shown in formula (iv)

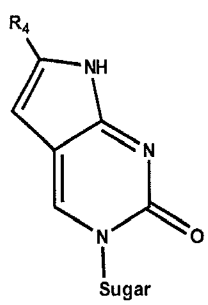

(iii)

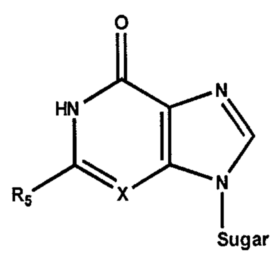

(iv)

wherein X = N or CH; $R_4$ = H, or $C_{1-4}$ alkyl; $R_5$ = H, $C_{1-4}$ alkyl, $C_{1-4}$ alkoxy, $C_{1-4}$ alkylthio, or F. In particular, X = N and $R_4$ = $R_5$ = H.

In one embodiment thereof, both sugars are of the LNA type, e.g. both oligonucleotides of the pair are LNA oligomers.

In still another embodiment, the SBC pair is a G':C' pair where the SBC nucleobase C' has the structure as shown in formula (v) and where the SBC nucleobase G' has the structure as shown in formula (vi)

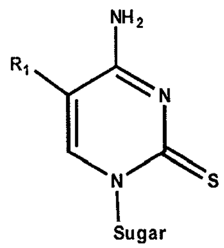

(v)

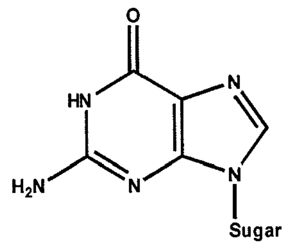

(vi)

wherein $R_1$ = H, or $C_{1-4}$ alkyl. In particular, $R_1$ = H.

In one embodiment thereof, both sugars are of the LNA type, i.e. both of the oligonucleotides of the pair are LNA oligomers.

In yet another embodiment, the above described SBC pairs are used in single-stranded oligonucleotides in order to reduce the number of intramolecular Watson-Crick hydrogen bonds. Such oligonucleotides typically have 5-50, such as 1-15, nucleotides or units. The incorporation of one or more pairs of complementary SBC nucleotides or units causes a reduction of the number of intramolecular Watson-Crick hydrogen bonds compared to the isosequential oligonucleotide.

The above defined pairs of SBC oligomers are particularly useful in connection with the populations defined herein.

*Methods for the Synthesis of Oligonucleotides and Nucleic Acids*

Nucleic acids and LNA oligomers are readily synthesized by standard phosphoramidite chemistry. The flexibility of the phosphoramidite synthesis approach further facilitates the easy production of LNA oligomers carrying all types of standard linkers and fluorophores.

Synthesis of LNA oligomers involves one or more of any of the nucleosides or nucleotides of the invention with (i) any other nucleoside or nucleotide of the invention, (ii) any other nucleoside or nucleotide of formula Ia, formula Ib, formula IIa, Scheme A, or Scheme B, and/or (iii) any naturally-occurring nucleoside or nucleotide. Desirably, the method involves reacting one or more nucleoside phosphoramidites of any of the above aspects with a nucleotide or nucleic acid.

Suitable oligonucleotides may also contain natural DNA or RNA units (e.g. nucleotides) with naturally-occurring nucleobases, as well as LNA units that contain naturally-occurring nucleobases. Furthermore, the oligonucleotides of the invention may also contain modified DNA or RNA, such as 2'-O-methyl RNA, with natural or modified nucleobases (e.g. SBC nucleobases or pyrene). Desirable oligonucleotides contain at least one of and desirably both of 1) one or more DNA or RNA units (e.g. nucleotides) with naturally-occurring nucleobases, and 2) one or more LNA units with naturally-occurring nucleobases, in addition to LNA units with a modified nucleobase. In other embodiments, the nucleic acid does not contain a modified nucleobase.

As discussed above, particularly desirable oligonucleotides contain a non-modified DNA or RNA unit at the 3' terminus and a modified DNA or RNA unit at one position upstream from (generally referred to hereing as the -1 or penultimate position) the 3' terminal non-modified nucleic acid unit. In some embodiments, the modified nucleobase is at the 3' terminal position of a nucleic acid primer, such as a primer for the detection of a single nucleotide polymorphism. Other particularly desirable nucleic acids have an LNA unit with or without a modified nucleobase in the 5' and/or 3' terminal position.

Also desirable are oligonucleotides that do not have an extended stretches of modified DNA or RNA units, e.g. greater than about 4, 5 or 6 consecutive modified DNA or RNA units. That is, desirably one or more non-modified DNA or RNA will be present after a consecutive stretch of about 3, 4 or 5 modified nucleic acids.

Generally desirable are oligonucleotides that contain a mixture of LNA units that have non-modified or naturally-occurring nucleobases (i.e., adenine, guanine, cytosine, 5-methyl-cytosine, uracil, or thymine) and LNA units that have modified nucleobases as disclosed herein.

Particularly desirable oligonucleotides of the invention include those where an LNA unit with a modified nucleobase is interposed between two LNA units each having non-modified or naturally-occurring nucleobases (adenine, guanine, cytosine, 5-methyl-cytosine, uracil, or thymine. The LNA "flanking" units with naturally-occurring nucleobase moieties may be directly adjacent to the LNA with modified nucleobase moiety, or desirably is within 2, 3, 4 or 5 nucleic acid units of the LNA unit with modified nucleobase. Nucleic acid units that may be spaced between an LNA unit with a modified nucleobase and an LNA unit with natural nucleobasis suitably are DNA and/or RNA and/or alkyl-modified

RNA/DNA units, typically with naturally-occurring nucleobases, although the DNA and or RNA units also may contain modified nucleobases.

In the practice of the present invention, target genes may be suitably single-stranded or double-stranded DNA or RNA; however, single-stranded DNA or RNA targets are desirable. It is understood that the target to which the nucleic acids of the invention are directed includes allelic forms of the targeted gene and the corresponding mRNAs including splice variants. There is substantial guidance in the literature for selecting particular sequences for nucleic acids with LNA or other high affinity nucleotides given a knowledge of the sequence of the target polynucleotide, e.g., Peyman and Ulmann, Chemical Reviews, 90:543-584, 1990; Crooke, Ann. Rev. Pharmacol. Toxicol., 32:329-376 (1992); and Zamecnik and Stephenson, Proc. Natl. Acad. Sci., 75:280-284 (1974).

By "selecting" is meant substantially partitioning a molecule from other molecules in a population. Desirably, the partitioning provides at least a 2-fold, desirably, a 30-fold, more desirably, a 100-fold, and most desirably, a 1,000-fold enrichment of a desired molecule relative to undesired molecules in a population following the selection step. The selection step may be repeated a number of times, and different types of selection steps may be combined in a given approach. The population desirably contains at least $10^9$ molecules, more desirably at least $10^{11}$, at least $10^{13}$, or at least $10^{14}$ molecules and, most desirably, at least $10^{15}$ molecules.

The chimeric oligomers of the present invention are highly suitable for a variety of diagnostic purposes such as for the isolation, purification, amplification, detection, identification, quantification, or capture of nucleic acids such as DNA, mRNA or non-protein coding cellular RNAs, such as tRNA, rRNA, snRNA and scRNA, or synthetic nucleic acids, *in vivo* or *in vitro.*

The oligomer can comprise a photochemically active group that facilitates the direct or indirect detection of the oligomer or the immobilization of the oligomer onto a solid support. Such group are typically attached to the oligo when it is intended as a probe for *in situ* hybridization, in Southern hybridization, Dot blot hybridization, reverse Dot blot hybridization, or in Northern hybridization.

When the photochemically active group includes a spacer, the spacer may suitably comprise a chemically cleavable group.

*Methods for Synthesis of Nucleic Acids on a Solid Support*

In another aspect, the invention provides a method for the synthesis of a population of nucleic acids (e.g. a population of nucleic acids of the invention) on a solid support. This method involves the reaction of a plurality of nucleoside phosphoramidites with an activated solid support (e.g. a solid support with an activated linker) and the subsequent reaction of a plurality of nucleoside phosphoramidites with activated nucleotides or nucleic acids bound to the solid support. At least 1, at least 5, at least 10%, at least 20%, at least 25%, at least 30%, at least 40%, at least 50%, at least 60%, at least 70%, at least 80%, at least 90%, or even 100% of the nucleic acid in the first population are non-naturally occurring nucleic acids with a melting temperature that is at least 5, at least 8˚C, at least 10˚C, at least 12˚C, at least 15˚C, at least 20˚C, at least 25˚C, at least 30˚C, at least 35˚C, or at least 40˚C higher than that of the corresponding control nucleic acid with 2'-deoxynucleotides and/or with a capture efficiency at least 50%, at least 100%, at least 150%, at least 200%, at least 500%, at least 800%, at least 1000%, or 12000% greater than that of the corresponding control nucleic acid at the temperature equal to the melting temperature of the nucleic acid of the first population. For example, the control nucleic acid may have β-D-2-deoxyribose instead of one or more bicyclic or sugar groups of a LNA unit or other modified or non-naturally-occurring units in a nucleic acid of the first population. In some embodiments, the first population and the control population only have naturally-occurring nucleobases. If a nucleic acid in the first population has one or more non-naturally-occurring nucleobases, the melting temperature and capture efficiency of the corresponding control nucleic acid is calculated as the average melting temperature and average capture efficiency for all of the nucleic acids that have either A, T, C, or G in each position corresponding to a non-naturally-occurring nucleobase in the nucleic acid in the first population.

In some embodiments of any of the above aspects, the solid support or the growing nucleic acid bound to the solid support is activated by illumination, a photogenerated acid, or electric current. In desirable embodiments, one or more spots or regions (e.g. a region with an area of less than 1 $cm^2$, less than 0.1 $cm^2$, less than 0.01 $cm^2$, less than 1 $mm^2$, or less than 0.1 $mm^2$ that desirably contains one particular nucleic acid monomer or oligomer) on the solid support are irradiated to produce a photogenerated acid that removes the 5'-OH protecting group of one or more nucleic acid monomers or oligomers to which a nucleotide is subsequently added. In other embodiments, an electric current is applied to one or more spots or regions (e.g. a region with an area of less than 1 $cm^2$, less than 0.1 $cm^2$, less than 0.01 $cm^2$, less than 1 $mm^2$, or less than 0.1 $mm^2$ that desirably contains one particular nucleic acid monomer or oligomer) on the solid support to remove an electrochemically sensitive protecting group of one or more nucleic acid monomers or oligomers to which a nucleotide is subsequently added. In still other embodiments, one or more spots or regions (e.g. a region with an area of less than 1 $cm^2$, less than 0.1 $cm^2$, less than 0.01 $cm^2$, less than 1 $mm^2$, or less than 0.1 $mm^2$ that desirably contains one particular nucleic acid monomer or oligomer) on the solid support are irradiated to remove

a photosensitive protecting group of one or more nucleic acid monomers or oligomers to which a nucleotide is subsequently added. In various embodiments, the solid support (e.g. chip, coverslip, microscope glass slide, quartz, or silicon) is less than 1, less than 0.5, less than 0.1. or less than 0.05 mm thick.

*Methods for the Synthesis of Longer Nucleic Acids*

In another aspect, the invention relates to a method of reacting a population of nucleic acids of the invention with one or more nucleic acids. This method involves incubating an immobilized population of nucleic acids of the invention with a solution that includes one or more probes (e.g. at least 2, at least 3, at least 4, at least 5, at least 10, at least 15, at least 20, at least 30, at least 40, at least 50, at least 60, at least 80, at least 100, or at least 150 different nucleic acids) and one or more target nucleic acids (e.g. at least 2, at least 3, at least 4, at least 5, at least 10, at least 15, at least 20, at least 30, at least 40, at least 50, at least 60, at least 80, at least 100, or at least 150 different target nucleic acids). The incubation is performed in the presence of a ligase under conditions that allow the ligase to covalently react one or more immobilized nucleic acids with one or more nucleic acid probes in solution that hybridize to the same target nucleic acid. Desirably, at least 2, at least 5, at least 10, at least 15, at least 20, at least 30, at least 40, at least 50, at least 80, or at least 100 pairs of immobilized nucleic acids and nucleic acid probes are ligated. In various embodiments, the incubation occurs between 15 and 45˚C, such as between 20 and 40˚C or between 25 and 35˚C

*Methods for the Immobilization of Nucleic Acids with Secondary Structure or Double-stranded Nucleic Acids*

In one aspect, the invention relates to a method for immobilizing a double-stranded nucleic acid or a nucleic acid with secondary structure (e.g. a RNA or DNA hairpin) by contacting the nucleic acid with an immobilized LNA containing SBC nucleotides or an immobilized population of nucleic acids of the invention under conditions that allow the nucleic acid to bind the immobilized LNA or the immobilized population of nucleic acids (se Figure 23). In desirable embodiments, the LNA has at least one 2,6,-diaminopurine, 2-thio-thymine or, 2-thio-uracil. Desirably, the LNA has a nucleotide with a 2'O, 4'C -methylene linkage between the 2' and 4' position of a sugar moiety. In desirable embodiments, the method is used in a heterogeneous assay.

*Desirable Embodiments of Any of the Aspects of the Invention*

In other embodiments of any of various aspects of the invention, a nucleic acid probe or primer specifically hybridizes to a target nucleic acid but does not substantially hybridize to non-target molecules which include other nucleic acids in a cell or biological sample having a sequence that is less than 99, 95, 90, 80, or 70% identical or complementary to that of the target nucleic acid. Desirably, the amount of the these non-target molecules hybridized to, or associated with, the nucleic acid probe or primer, as measured using standard assays, is 2-fold, desirably 5-fold, more desirably 10-fold, and most desirably 50-fold lower than the amount of the target nucleic acid hybridized to, or associated with, the nucleic acid probe or primer. In other embodiments, the amount of a target nucleic acid hybridized to, or associated with, the nucleic acid probe or primer, as measured using standard assays, is 2-fold, desirably 5-fold, more desirably 10-fold, and most desirably 50-fold greater than the amount of a control nucleic acid hybridized to, or associated with, the nucleic acid probe or primer. In certain embodiments, the nucleic acid probe or primer RNA is substantially complementary (e.g. at least 80, at least 90, at least 95, at least 98, or 100% complementary) to a target nucleic acid or a group of target nucleic acids from a cell. In other embodiments, the probe or primer is homologous to multiple RNA or DNA molecules, such as RNA or DNA molecules from the same gene family. In other embodiments, the probe or primer is homologous to a large number of RNA or DNA molecules. In desirable embodiments, the probe or primer binds to nucleic acids which have polynucleotide sequences that differ in sequence at a position that corresponds to the position of a universal nucleobase in the probe or primer. Examples of control nucleic acids include nucleic acids with a random sequence or nucleic acids known to have little, if any, affinity for the nucleic acid probe or primer. In some embodiments, the target nucleic acid is an RNA, DNA, or cDNA molecule.

Desirably, the association constant ($K_a$) of the nucleic acid towards a complementary target molecule is higher than the association constant of the complementary strands of the double-stranded target molecule. In some desirable embodiments, the melting temperature of a duplex between the nucleic acid and a complementary target molecule is higher than the melting temperature of the complementary strands of the double-stranded target molecule.

In some embodiments, the LNA-pyrene is in a position corresponding to the position of a non-base (e.g. a unit without a nucleobase) in another nucleic acid, such as a target nucleic acid. Incorporation of pyrene in a DNA strand that is hybridized against the four naturally-occurring nucleobases decreases the $T_m$ by -4.5˚C to -6.8˚C; however, incorporation of pyrene in a DNA strand in a position opposite a non-base only decreases the $T_m$ by -2.3˚C to -4.6˚C, most likely due to the better accomodation of the pyrene in the B-type duplex (Matray and Kool, J. Am. Chem. Soc. 120, 6191, 1998). Thus, incorporation on LNA-pyrene into a nucleic acid in a position opposite a non-base (e.g. a unit without

a nucleobase or a unit with a small group such as a noncyclic group instead of a nucleobase) in a target nucleic acid may also minimize any potential decrease in $T_m$ due to the pyrene substitution

In other embodiments of any of various aspects of the invention, a nucleic acid probe or primer specifically hybridizes to a target nucleic acid but does not substantially hybridize to non-target molecules, which include other nucleic acids in a cell or biological sample having a sequence that is less than 99, 95, 90, 80, or 70% identical or complementary to that of the target nucleic acid. Desirably, the amount of the these non-target molecules hybridized to, or associated with, the nucleic acid probe or primer, as measured using standard assays, is 2-fold, desirably 5-fold, more desirably 10-fold, and most desirably 50-fold lower than the amount of the target nucleic acid hybridized to, or associated with, the nucleic acid probe or primer. In other embodiments, the amount of a target nucleic acid hybridized to, or associated with, the nucleic acid probe or primer, as measured using standard assays, is 2-fold, desirably 5-fold, more desirably 10-fold, and most desirably 50-fold greater than the amount of a control nucleic acid hybridized to, or associated with, the nucleic acid probe or primer. Desirably, the probe or primer only hybridizes to one target nucleic acid from a sample under denaturing, high stringency hybridization conditions. In certain embodiments, the nucleic acid probe or primer RNA is substantially complementary (e.g. at least 80, at least 90, at least 95, at least 98, or 100% complementary) to only one target nucleic acid from a cell. In other embodiments, the probe or primer is homologous to multiple RNA or DNA molecules, such as RNA or DNA molecules from the same gene family. In other embodiments, the probe or primer is homologous to a large number of RNA or DNA molecules. Examples of control nucleic acids include nucleic acids with a random sequence or nucleic acids known to have little, if any, affinity for the nucleic acid probe or primer.

In various embodiments, the number of molecules in the population of nucleic acids is at least 2, at least 4, at least 5, at least 6, at least 7, at least 8, or at least 10-fold greater than the number of molecules in the test nucleic acid sample. In some embodiments, a LNA is a triplex-forming oligonucleotide.

*Advantages*

The present invention has a variety of advantages related to nucleic acid analysis methods. The ability to equalize melting temperatures of a series of mucleotides is generally applicable and desirable in all situations where more than one sequence is used simultaneously (e.g. DNA arrays with more than one capture probe, PCR and especially multiplex PCR, homogeneous assays such as Taqman and Molecular beacon). Sample preparation of specific sequences (e.g. DNA or RNA extraction using capture probes on filters or magnetic beads) is another area where melting temperature equalization of specific probe sequences is useful. Even very short sequences such as 5-mers are capable of efficiently hybridizing to and retaining target molecules. In some embodiments, spotted universal arrays with 5-mers, 6-mers, or 7-mers are used to minimize complexity (e.g. 1,096 - 16,384 capture probes), while providing sufficient effectiveness and stability. Efficient capture of target molecules has even been detected with probes with a very high AT content of greater than 80%.

Additionally, the temperature-, cation concentration-, or denaturant concentration-dependent hybridization pattern of a test nucleic acid to a universal array (e.g. an array with all possible heptamers) can be used to rapidly classify the composition of the test sample according to a set of standards by, e.g., linear deconvolution of the hybridization pattern (e.g. solving 327680 equations with 200 unknowns). Use of photo-activated LNA amidites for on chip synthesis of the DNA arrays increase the number of different capture probes that can conveniently be placed on an array from less than 100,000 (e.g. an universal 5-mer, 6-mer, 7-mer, or 8-mer array) to more than 100.000 (e.g. a 9-mer, 10-mer, or 11-mer, or 12-mer array). The increased number of available capture probes and/or the increased length of capture probes may in some applications enable detection and classification of samples after hybridization at a single temperature, cation concentration, or denaturant concentration. Because of the low variance in melting temperatures for the nucleic acid array of the present invention, more stringent hybridizations and shorter, less expensive capture probes may be used.

For example, the invention provides high affinity nucleotides (e.g. LNA and other high affinity nucleotides with a modified nucleobase and/or backbone) that can be used, e.g., in universal arrays capable of producing a unique signature for any complex DNA or RNA sample that can be compared to signatures of known standards. If desired, universal nucleobases can be added as part of flanking regions in capture probes (e.g. probes of a universal array) to stabilize hybridization with high affinity nucleotides in the capture probes. Replacement of one or more DNA-t nucleotides with LNA-T and/or replacement of one or more DNA-a nucleotides with LNA-A reduces the variability of melting temperatures for capture probes of similar length but different GC and AT content by desirably at least 10, at least 20, at least 30, at least 40 or at least 50%. This principle applies to both universal arrays and to specialized arrays (e.g. expression arrays). Additionally, replacement of one or more DNA-t nucleotides with LNA-T and/or replacement of one or more DNA-c with LNA-C increases the stability of a large number of capture probes, while desirably avoiding self-complementary sequences with LNA: LNA base-pairs within a capture probe that would otherwise reduce or eliminate the binding of target molecules to the probe. Although a general T and C substitution may not reduce the variability of melting temperatures of the probes, this substitution increases the melting temperature and binding efficiency of many

capture probes that contain these two nucleotides.

The invention also provides a general substitution algorithm for enhancement of the hybridization signal of a test nucleic acid sample by inclusion of high affinity monomers (e.g. LNA and other high affinity nucleotides with a modified nucleobase and/or backbone) in the array. This method increases the stability and binding affinity of capture probes while avoiding substitutions in positions that may form self-complementary base-pairs which may otherwise inhibit binding to a target molecule. The substitution algorithm is broadly useful for universal arrays and specialized arrays, as well as for PCR primers and FISH probes.

Thus, the populations of the invention may also be used as as PCR primers or FISH probes.

The invention also features a deconvolution algorithm that allows analysis of "biosignatures" = hybridization patterns obtained at one or more different stringencies e.g. by varying temperature, ionic strength, or denaturant concentration. Comparison of the biosignature of a complex sample with biosignatures of individual components, which may themselves be mixtures of sequences such as a cDNA, generates a set of linear equations that can be resolved to determine the abundance of each individual standard. This is demonstrated in the experimental data, where biosignatures based on a limited number of universal capture probes are used to: i) detect and classify pathogenic microorganisms, ii) determine the abundance of different splicevariants in controlled mixtures and iii) changes in expression pattern in yeast cells after heat shock.

Other features and advantages of the invention will be apparent from the following detailed description.

An additional object of the present invention is to provide oligonucleotides which combine an increased ability to discriminate between complementary and mismatched targets with the ability to act as substrates for nucleic acid active enzymes such as for example DNA and RNA polymerases, ligases, phosphatases. Such oligonucleotides may be used for instance as primers for sequencing nucleic acids and as primers in any of the several well known amplification reactions, such as the PCR reaction.

Introduction of LNA monomers with naturally-occurring nucleobases into either DNA, RNA, or pure LNA oligonucleotides can result in extremely high thermal stability of duplexes with complimentary DNA or RNA, while at the same time obeying the Watson-Crick base pairing rules. In general, the thermal stability of heteroduplexes is increased 3-8˚C per LNA monomer in the duplex. Oligonucleotides containing LNA can be designed to be substrates for polymerases (e.g. Taq polymerase), and PCR based on LNA primers is more discriminatory towards single nucleobase mutations in the template DNA compared to normal DNA-primers (e.g. allele specific PCR). Furthermore, very short LNA oligomers (e.g. 5-mers or 8-mers) which have high $T_m$'s when compared to similar DNA oligomers can be used as highly specific catching probes with outstanding discriminatory power towards single nucleobase mutations (e.g. SNP detection).

LNA oligonucleotides are capable of hybridizing with double-stranded DNA target molecules as well as RNA secondary structures by strand invasion as well as of specifically blocking a wide selection of enzymatic reactions such as digestion of double-stranded DNA by restriction endonucleases; and digestion of DNA and RNA with deoxyribonucleases and ribonucleases, respectively.

In a further aspect, oligonucleotides of the invention may be used to construct new affinity pairs which exhibit enhanced specificity towards each other. The affinity constants can easily be adjusted over a wide range and a vast number of affinity pairs can be designed and synthesized. One part of the affinity pair can be attached to the molecule of interest (e.g. proteins, amplicons, enzymes, polysaccharides, antibodies, haptens, peptides, etc.) by standard methods, while the other part of the affinity pair can be attached to e.g. a solid support such as beads, membranes, microtiter plates, sticks, tubes, etc. The solid support may be chosen from a wide range of polymer materials such as for instance polypropylene, polystyrene, polycarbonate or polyethylene. The affinity pairs may be used in selective isolation, purification, capture and detection of a diversity of the target molecules.

Oligonucleotides of the invention may also be employed as probes in the purification, isolation and detection of for instance pathogenic organisms such as viral, bacteria and fungi etc. Oligonucleotides of the invention may also be used as generic tools for the purification, isolation, amplification and detection of nucleic acids from groups of related species such as for instance rRNA from gram-positive or gram negative bacteria, fungi, mammalian cells etc.

Oligonucleotides of the invention may also be employed as an aptamer in molecular diagnostics, e.g. in RNA mediated catalytic processes, in specific binding of antibiotics, drugs, amino acids, peptides, structural proteins, protein receptors, protein enzymes, saccharides, polysaccharides, biological cofactors, nucleic acids, or triphosphates or in the separation of enantiomers from racemic mixtures by stereospecific binding.

Oligonucleotides of the invention may also be used for labeling of cells, e.g. in methods wherein the label allows the cells to be separated from unlabelled cells.

Oligonucleotides may also be conjugated to a compound selected from proteins, amplicons, enzymes, polysaccharides, antibodies, haptens, and peptides.

Kits are also provided containing one or more oligonucleotides of the invention for the isolation, purification, amplification, detection, identification, quantification, or capture of natural or synthetic nucleic acids. The kit typically will contain a reaction body, e.g. a slide or biochip. One or more oligonucleotides of the invention may be suitably immobilized on such a reaction body.

The invention also provides methods for using kits of the invention for carrying out a variety of bioassays. Any type of assay wherein one component is immobilized may be carried out using the substrate platforms of the invention. Bioassays utilizing an immobilized component are well known in the art. Examples of assays utilizing an immobilized component include for example, immunoassays, analysis of protein-protein interactions, analysis of protein-nucleic acid interactions, analysis of nucleic acid-nucleic acid interactions, receptor binding assays, enzyme assays, phosphorylation assays, diagnostic assays for determination of disease state, genetic profiling for drug compatibility analysis, and SNP detection (US 6,316,198; 6,303,315).

Identification of a nucleic acid sequence capable of binding to a biomolecule of interest can be achieved by immobilizing a library of nucleic acids onto the substrate surface so that each unique nucleic acid was located at a defined position to form an array. The array would then be exposed to the biomolecule under conditions which favored binding of the biomolecule to the nucleic acids. Non-specifically binding biomolecules could be washed away using mild to stringent buffer conditions depending on the level of specificity of binding desired. The nucleic acid array would then be analyzed to determine which nucleic acid sequences bound to the biomolecule. Desirably the biomolecules would carry a fluorescent tag for use in detection of the location of the bound nucleic acids.

Oligonucleotides of the invention can be employed in a wide range of applications, particularly those in those applications involving a hybridization reaction. Oligonucleotides may also be used in DNA sequencing aiming at improved throughput in large-scale, shotgun genome sequencing projects, improved throughput in capillary DNA sequencing (e.g. ABI prism 3700) as well as at an improved method for 1) sequencing large, tandemly repeated genomic regions, 2) closing gaps in genome sequencing projects and 3) sequencing of GC-rich templates. In DNA sequencing, oligonucleotide sequencing primers are combined with LNA enhancer elements for the read-through of GC-rich and/or tandemly repeated genomic regions, which often present many challenges for genome sequencing projects. LNA may increase the specificity of certain sequencing primers and thus facilitate selection of a particular version of a repeated sequence and possibly also use strand invasion to open up recalcitrant GC rich sequences.

The incorporation of one or more universal nucleosides into the oligomer makes bonding to unknown nucleobases possible and allows the oligonucleotide to match ambiguous or unknown nucleic acid sequences.

As discussed above, oligonucleotides of the invention may be used for therapeutic applications, e.g. as an antisense, antigene or ribozyme or double-stranded nucleic acid therapeutic agents. In these therapeutic methods, one or more oligonucleotides of the invention is/are administered as desired to a patient suffering from or susceptible the targeted disease or disorder, e.g. a viral infection.

In an exemplary *in vitro* method for measuring the ability of a nucleic acid of the invention to silence a target gene, cells are cultured in standard medium supplemented with 1% fetal calf serum as previously described (Lykkesfeld et al., Int. J. Cancer 61:529-534, 1995). At the start of the experiment cells are approximately 40% confluent. The serum containing medium is removed and replaced with serum-free medium. Transfection is performed using, e.g., Lipofectin (GibcoBRL cat. No 18292-011) diluted 40X in medium without serum and combined with the oligo to a concentration of 750 nM oligo, 0.8 ug/ml Lipofectin. Then, the medium is removed from the cells and replaced with the medium containing oligo-Lipofectin complex. The cells are incubated at 37°C for 6 hours, rinsed once with medium without serum and incubated for a further 18 hours in DME/F12 with 1% FCS at 37°C. Standard methods are used for measuring the level of mRNA or protein encoded by the target gene to measure the level of gene silencing.

Oligonucleotides of the invention may also be used in high specificity oligo arrays, e.g., wherein a multitude of different oligomers are affixed to a solid surface in a predetermined pattern (Nature Genetics, suppl. vol. 21, Jan 1999, 1-60 and WO 96/31557). The usefulness of such an array, which can be used for simultaneously analyzing a large number of target nucleic acids, depends to a large extent on the specificity of the individual oligomers bound to the surface. The target nucleic acids may carry a detectable label or be detected by incubation with suitable detection probes which may also be an oligonucleotide of the invention.

Assays using an immobilized array of nucleic acid sequences may be used for determining the sequence of an unknown nucleic acid; single nucleotide polymorphism (SNP) analysis; analysis of gene expression patterns from a particular species, tissue, cell type and; gene identification.

The oligonucleotides used in the methods of the present invention may be used without any prior analysis of the structure assumed by a target nucleic acid. For any given case, it can be determined empirically using appropriately selected reference target molecules whether a chosen probe or array of probes can distinguish between genetic variants sufficiently for the needs of a particular assay. Once a probe or array of probes is selected, the analysis of which probes bind to a target, and how efficiently these probes bind (i.e. how much of probe/target complex can be detected) allows a hybridization signature of the conformation of the target to be created. It is contemplated that the signature may be stored, represented or analyzed by any of the methods commonly used for the presentation of mathematical and physical information, including but not limited to line, pie, or area graphs or 3-dimensional topographic representations. The data may also be used as a numerical matrix, or any other format that may be analyzed visually, mathematically or by computer-assisted algorithms, such as for example EURAYdesign™ software and/or neural networks.

The resulting signatures of the nucleic acid structures serve as sequence-specific identifiers of the particular

molecule, without requiring the determination of the actual nucleotide sequence. If desired, a specific sequence may be identified by comparison of their signature to a reference signature using any appropriate algorithm.

It is also contemplated that information on the structures assumed by a target nucleic acid may be used in the design of the probes, such that regions that are known or suspected to be involved in folding may be chosen as hybridization sites. Such an approach will reduce the number of probes that are likely to be needed to distinguish between targets of interest.

There are many methods used to obtain structural information involving nucleic acids, including the use of chemicals that are sensitive to the nucleic acid structure, such as phenanthroline/copper, EDTA-$Fe^{2+}$, cisplatin, ethylnitrosourea, dimethyl pyrocarbonate, hydrazine, dimethyl sulfate, and bisulfite. Enzymatic probing using structure-specific nucleases from a variety of sources, such as the Cleavase™ enzymes (Third Wave Technologies, Inc., Madison, Wis.), Taq DNA polymerase, E. coli DNA polymerase I, and eukaryotic structure-specific endonucleases (e.g. human, murine and Xenopus XPG enzymes, yeast RAD2 enzymes), murine FEN-1 endonucleases (Harrington and Lieber, Genes and Develop., 3:1344 [1994]) and calf thymus 5' to 3' exonuclease (Murante et al., J. Biol. Chem., 269:1191 [1994]). In addition, enzymes having 3' nuclease activity such as members of the family of DNA repair endonucleases (e.g. the RrpI enzyme from Drosophila melanogaster, the yeast RAD1/RAD10 complex and E. coli Exo III), are also suitable for examining the structures of nucleic acids.

If the analysis of structure as a step in probe selection is to be used for a segment of nucleic acid for which no information is available concerning regions likely to form secondary structures, the sites of structure-induced modification or cleavage must be identified. It is most convenient if the modification or cleavage can be done under partially reactive conditions (i.e., such that in the population of molecules in a test sample, each individual will receive only one or a few cuts or modifications). When the sample is analyzed as a whole, each reactive site should be represented, and all the sites may be thus identified. Using a Cleavase Fragment Length Polymorphism™ cleavage reaction as an example, when the partial cleavage products of an end labeled nucleic acid fragment are resolved by size (e.g. by electrophoresis), the result is a ladder of bands indicating the site of each cleavage, measured from the labeled end. A similar analysis can be done for chemical modifications that block DNA synthesis; extension of a primer on molecules that have been partially modified will yield a nested set of termination products. Determining the sites of cleavage/modification may be done with some degree of accuracy by comparing the products to size markers (e.g. commercially available fragments of DNA for size comparison) but a more accurate measure is to create a DNA sequencing ladder for the same segment of nucleic acid to resolve alongside the test sample. This allows rapid identification of the precise site of cleavage or modification.

The oligonucleotides may interact with the target in any number of ways. For example, in another embodiment, the oligonucleotides may contact more than one region of the target nucleic acid. When the target nucleic acid is folded as described, two or more of the regions that remain single-stranded may be sufficiently proximal to allow contact with a single oligonucleotide. The capture oligonucleotide in such a configuration is referred to herein as a "bridge" or "bridging" oligonucleotide, to reflect the fact that it may interact with distal regions within the target nucleic acid. The use of the terms "bridge" and "bridging" is not intended to limit these distal interactions to any particular type of interaction. It is contemplated that these interactions may include non-standard nucleic acid interactions known in the art, such as G-T base pairs, Hoogsteen interactions, triplex structures, quadraplex aggregates, and the multibase hydrogen bonding such as is observed within nucleic acid tertiary structures, such as those found in tRNAs. The terms are also not intended to indicate any particular spatial orientation of the regions of interaction on the target strand, i.e., it is not intended that the order of the contact regions in a bridge oligonucleotide be required to be in the same sequential order as the corresponding contact regions in the target strand. The order may be inverted or otherwise shuffled.

Monomers are referred to as being "complementary" if they contain nucleobases that can form hydrogen bonds according to Watson-Crick base-pairing rules (e.g. G with C, A with T, or A with U) or other hydrogen bonding motifs such as for example diaminopurine with T, inosine with C, and pseudoisocytosine with G.

By "substantially complementarity" is meant having a sequence that is at least 60, at least 70, at least 80, at least 90, at least 95, or 100% complementary to that of another sequence. Sequence complementarity is typically measured using sequence analysis software with the default parameters specified therein (e.g. Sequence Analysis Software Package of the Genetics Computer Group, University of Wisconsin Biotechnology Center, 1710 University Avenue, Madison, WI 53705). This software program matches similar sequences by assigning degrees of homology to various substitutions, deletions, and other modifications.

The term "homology" refers to a degree of complementarity. There can be partial homology or complete homology (i.e. identity). A partially complementary sequence that at least partially inhibits a completely complementary sequence from hybridizing to a target nucleic acid is referred to using the functional term "substantially homologous."

When used in reference to a double-stranded nucleic acid sequence such as a cDNA or genomic clone, the term "substantially homologous" refers to a probe that can hybridize to a strand of the double-stranded nucleic acid sequence under conditions of low stringency, e.g. using a hybridization buffer comprising 20% formamide in 0.8M saline/0.08M sodium citrate (SSC) buffer at a temperature of 37°C and remaining bound when subject to washing once with

that SSC buffer at 37°C.

When used in reference to a single-stranded nucleic acid sequence, the term "substantially homologous" refers to a probe that can hybridize to (i.e., is the complement of) the single-stranded nucleic acid template sequence under conditions of low stringency, e.g. using a hybridization buffer comprising 20% formamide in 0.8M saline/0.08M sodium citrate (SSC) buffer at a temperature of 37°C and remaining bound when subject to washing once with that SSC buffer at 37°C.

By "corresponding unmodified reference nucleobase" is meant a nucleobase that is not part of an LNA unit and is in the same orientation as the nucleobase in an LNA unit.

By "mutation" is meant an alteration in a naturally-occurring or reference nucleic acid sequence, such as an insertion, deletion, frameshift mutation, silent mutation, nonsense mutation, or missense mutation. Desirably, the amino acid sequence encoded by the nucleic acid sequence has at least one amino acid alteration from a naturally-occurring sequence.

By "target nucleic acid" or "nucleic acid target" is meant a particular nucleic acid sequence of interest. Thus, the "target" can exist in the presence of other nucleic acid molecules or within a larger nucleic acid molecule.

By "double-stranded nucleic acid" is meant a nucleic acid containing a region of two or more nucleotides that are in a double-stranded conformation. In various embodiments, the double-stranded nucleic acids consists entirely of LNA units or a mixture of LNA units, ribonucleotides, and/or deoxynucleotides. The double-stranded nucleic acid may be a single molecule with a region of self-complimentarity such that nucleotides in one segment of the molecule base pair with nucleotides in another segment of the molecule. Alternatively, the double-stranded nucleic acid may include two different strands that have a region of complimentarity to each other. Desirably, the regions of complimentarity are at least 70, at least 80, at least 90, at least 95, at least 98, or 100% complimentary. Desirably, the region of the double-stranded nucleic acid that is present in a double-stranded conformation includes at least 5, at least 10, at least 20, at least 30, at least 50, at least 75, at least 100, at least 200, at least 500, at least 1000, at least 2000 or at least 5000 nucleotides or includes all of the nucleotides in the double-stranded nucleic acid. Desirable double-stranded nucleic acid molecules have a strand or region that is at least 70, at least 80, at least 90, at least 95, at least 98, or 100% identical to a coding region or a regulatory sequence (e.g. a transcription factor binding site, a promoter, or a 5' or 3' untranslated region) of a nucleic acid of interest. In some embodiments, the double-stranded nucleic acid is less than 200, less than 150, less than 100, less than 75, less than 50, or less than 25 nucleotides in length. In other embodiments, the double-stranded nucleic acid is less than 50,000; less than 10,000; less than 5,000; or less than 2,000 nucleotides in length. In certain embodiments, the double-stranded nucleic acid is at least 200, at least 300, at least 500, at least 1000, or at least 5000 nucleotides in length. In some embodiments, the number of nucleotides in the double-stranded nucleic acid is contained in one of the following ranges: 5-15 nucleotides, 16-20 nucleotides, 21-25 nucleotides, 26-35 nucleotides, 36-45 nucleotides, 46-60 nucleotides, 61-80 nucleotides, 81-100 nucleotides, 101-150 nucleotides, or 151-200 nucleotides, inclusive. In addition, the double-stranded nucleic acid may contain a sequence that is less than a full-length sequence or may contain a full-length sequence.

By "infection" is meant the invasion of a host animal by a pathogen (e.g. a bacteria, yeast, or virus). For example, the infection may include the excessive growth of a pathogen that is normally present in or on the body of an animal or growth of a pathogen that is not normally present in or on the animal. More generally, aninfection can be any situation in which the presence of a pathogen population(s) is damaging to a host. Thus, an animal is "suffering" from an infection when an excessive amount of a pathogen population is present in or on the animal's body, or when the presence of a pathogen population(s) is damaging the cells or other tissue of the animal. In one embodiment, the number of a particular genus or species of paghogen is at least 2, at least 4, at least 6, or at least 8 times the number normally found in the animal.

At bacterial infection may be due to gram positive and/or gram negative bacteria. In desirable embodiments, the bacterial infection is due to one or more of the following bacteria: *Chlamydophila pneumoniae, C. psittaci, C. abortus, Chlamydia trachomatis, Simkania negevensis, Parachlamydia acanthamoebae, Pseudomonas aeruginosa, P. alcaligenes, P. chlororaphis, P. fluorescens, P. luteola, P. mendocina, P. monteilii, P. oryzihabitans, P. pertocinogena, P. pseudalcaligenes, P. putida, P. stutzeri, Burkholderia cepacia, Aeromonas hydrophilia, Escherichia coli, Citrobacter freundii, Salmonella typhimurium, S. typhi, S. paratyphi, S. enteritidis, Shigella dysenteriae, S. flexneri, S. sonnei, Enterobacter cloacae, E. aerogenes, Klebsiella pneumoniae, K. oxytoca, Serratia marcescens, Francisella tularensis, Morganella morganii, Proteus mirabilis, Proteus vulgaris, Providencia alcalifaciens, P. rettgeri, P. stuartii, Acinetobacter calcoaceticus, A. haemolyticus, Yersinia enterocolitica, Y. pestis, Y. pseudotuberculosis, Y. intermedia, Bordetella pertussis, B. parapertussis, B. bronchiseptica, Haemophilus influenzae, H. parainfluenzae, H. haemolyticus, H. parahaemolyticus, H. ducreyi, Pasteurella multocida, P. haemolytica, Branhamella catarrhalis, Helicobacter pylori, Campylobacter fetus, C. jejuni, C. coli, Borrelia burgdorferi, V. cholerae, V. parahaemolyticus, Legionella pneumophila, Listeria monocytogenes, Neisseria gonorrhea, N. meningitidis, Kingella dentrificans, K. kingae, K. oralis, Moraxella catarrhalis, M. atlantae, M. lacunata, M. nonliquefaciens, M. osloensis, M. phenylpyruvica, Gardnerella vaginalis, Bacteroides fragilis, Bacteroides distasonis, Bacteroides 3452A homology group, Bacteroides vulgatus, B. ovalus, B. thetaiotaomicron, B. uniformis, B. eggerthii, B. splanchnicus, Clostridium difficile, Mycobacterium tuberculosis, M. avium, M. intra-*

*cellulare, M. leprae, C. diphtheriae, C. ulcerans, C. accolens, C. afermentans, C. amycolatum, C. argentorense, C. auris, C. bovis, C. confusum, C. coyleae, C. durum, C. falsenii, C. glucuronolyticum, C. imitans, C. jeikeium, C. kutscheri, C. kroppenstedtii, C. lipophilum, C. macginleyi, C. matruchoti, C. mucifaciens, C. pilosum, C. propinquum, C. renale, C. riegelii, C. sanguinis, C. singulare, C. striatum, C. sundsvallense, C. thomssenii, C. urealyticum, C. xerosis , Streptococcus pneumoniae, S. agalactiae, S. pyogenes, Enterococcus avium, E. casseliflavus, E. cecorum, E. dispar, E. durans, E. faecalis, E. faecium, E. flavescens, E. gallinarum, E. hirae, E. malodoratus, E. mundtii, E. pseudoavium, E. raffinosus, E. solitarius, Staphylococcus aureus, S. epidermidis, S. saprophyticus, S. intermedius, S. hyicus, S. haemolyticus, S. hominis,* and/or *S. saccharolyticus.* Desirably, a nucleic acid is administered in an amount sufficient to prevent, stabilize, or inhibit the growth of a pathogenic bacteria or to kill the bacteria.

In various embodiments, the viral infection relevant to the methods of the invention is an infection by one or more of the following viruses: West Nile virus (e.g. Samuel, "Host genetic variability and West Nile virus susceptibility," Proc. Natl. Acad. Sci. USA August 21, 2002; Beasley, Virology 296:17-23, 2002), Hepatitis, picornarirus, polio, HIV, coxsacchie, herpes (e.g. zoster, simplex, EBV, or CMV), adenovirus, retrovius, falvi, pox, rhabdovirus, picorna virus (e.g. coxsachie, entero, hoof and mouth, polio, or rhinovirus), St. Louis encephalitis, Epstein-Barr, myxovirus, JC, coxsakievirus B, togavirus, measles, paramyxovirus, echovirus, bunyavirus, cytomegalovirus, varicella-zoster, mumps, equine encephalitis, lymphocytic choriomeningitis, rabies, simian virus 40, polyoma virus, parvovirus, papilloma virus, primate adenovirus, and/or BK.

By "mutation" is meant an alteration in a naturally-occurring or reference nucleic acid sequence, such as an insertion, deletion, frameshift mutation, silent mutation, nonsense mutation, or missense mutation. Desirably, the amino acid sequence encoded by the nucleic acid sequence has at least one amino acid alteration from a naturally-occurring sequence.

## EXAMPLES

### Example 1: Methods for Minimizing the Variance in Melting Temperatures in Nucleic Acid Populations of the Invention

Any simultaneous use of more than one primer or probe is made difficult because the involved primers or probes must work under the same conditions. An indication of whether or not two or more primers or probes will work under the same conditions is the relative $T_{ms}$ at which the hybridized oligonucleotides dissociate. In cases where probes are applied for specific detection of mutations or homologous sequences, the $\Delta T_m$ is of importance. $\Delta T_m$ expresses the difference between $T_m$ of the match and the $T_m$ of the mismatch hybridizations. Generally, the larger $\Delta T_m$ obtained, the more specific detection of the sequence of interest. In addition, a large $\Delta T_m$ facilitates more probes to be used simultaneously and in this way a higher degree of multiplexity can be applied (Figure 21).

High affinity nucleotide analogs such a LNA can be also be used universally to equalize the melting properties of oligonucleotides with different AT and CG content. The increased affinity of LNA adenosine and LNA thymidine corresponds approximately to the normal affinity of DNA guanine and DNA cytosine. An overall substitution of all DNA-A and DNA-T with LNA-A and LNA-T results in melting properties that are nearly sequence independent but only depend on the length of the oligonucleotide. This may be important for design of oligonucleotide probes used in large multiplex analysis and likewise for applications using random oligonucleotides, where differences in stability often lead to strong biases. The effect of LNA A and T substitutions has been evaluated by predicting the $T_m$ value of all possible 9-mer oligonucleotides with different universal substitutions. The distribution of the 262,000 $T_m$-values is shown in Figure 1, where a very homogenous $T_m$ value is observed for the universally LNA A and T substituted oligonucleotides. The standard deviation of the melting temperature for all 9-mers drops from 7.7˚C for pure DNA to only 2.2˚C for LNA A and T substituted oligonucleotides. This equalizing effect may also be utilized for photomediated on-chip synthesis of oligonucleotides.

Furthermore, the novel LNA SBC monomers LNA-D (LNA 2,6-diaminopurine / LNA 2-amino-A) and LNA 2-thio-U or LNA 2-thio-T, see Figure 4 and Table 9, can be used to further equalize $T_m$ as shown in Table 4. Thus, the exchange of one LNA-A monomer with one LNA-D monomer (entry 11) increases the $T_m$ from 61.6˚C (entry 8) to 67.8˚C (entry 11) compared to the same oligonucleotide where A has been replaced with G which has a $T_m$ of 70.9˚C (entry 10). Likewise, the replacement of LNA-T with LNA 2-thio-U, see Table 9, increases the $T_m$ of the corresponding duplexes with DNA. The mismatch discrimination abilities of LNA oligonucleotides modified with LNA-D and LNA 2-thio-U/T are retained as shown in Table 5.

Figure 21A demonstrates a few common problems one may experience when several probes are applied simultaneously in traditional methods. As can be seen in Figure 21A, despite a considerable $\Delta T_m$, probes 1 and 2 are not compatible due to a significant difference in $T_m$ (melting temperature of match hybridization). This is in contrast to probes 1 and 3 which do have a similar $T_m$ but can not be operated together since the $\Delta T_m$ of probe 3 is too small to offer a proper discrimination between homologous and non-homologous sequences. Figure 21B demonstrates three probes designed correctly to be operated in multiplex setting. All probes have similar $T_m$ values and a significant $\Delta T_m$,

which makes them highly suited for operation under the same conditions, in this case approximately 56˚C.

It is often difficult to design probes and primers with the same range of melting temperature due to the variance in A/T and G/C content of the probing sites. Highly A/T rich regions typically give lower $T_m$ values. Furthermore, if single mismatches are to be resolved, G/T mismatches are known to contribute little to $\Delta T_m$. As discussed above, the use of LNA is a desirable way to solve problems related to multiplex use of primers and probes. LNA offers the possibility to adjust $T_m$ and increase the $\Delta T_m$ at the same time. LNA increases $T_m$ with 4-8˚C/substitution and increases $\Delta T_m$ in many cases with several 100% (Table 2 and Figure 22).

Table 2. Demonstration of LNA controlled increase of $T_m$ and $\Delta T_m$.

| $T_m$ of LNA:DNA Duplexes | Perfect match 3'-ACGACCAC-5' | Single mismatch 3'-ACG**G**CCAC-5' | $\Delta T_m$ |
|---|---|---|---|
| LNA 8-mer 5'-TGCTGGTG-3' | 71˚C | 45˚C | 26˚C |
| DNA 8-mer 5'-TGCTGGTG-3' | 35˚C | 25˚C | 10˚C |

As LNA can be mixed with DNA during standard oligonucleotide synthesis, LNA can be placed at optimal positions in probes in order to adjust $T_m$ (Figure 27). Furthermore, LNA placed at even few correct positions may significantly enhance $\Delta T_m$ as demonstrated in Figure 27.

Figures 24 and 27 demonstrate how LNA can be used to optimize and trim capture probes to work together in a multiplex hybridisation experiment. The probes are designed to detect a single nucleotide polymorphism (SNP) in the ApoB gene. As can be seen, the two DNA probes cannot work together primarily because the $\Delta T_m$ is too small for the probe detecting allele 2. This is probably due to the fact that it is a G:T mismatch. However, by incorporation of LNA the $\Delta T_m$ of the probes were enhanced by 38% and 300%, respectively. As a result, the probes can now be operated together at 40˚C.

The specificity of PCR may also be enhanced by the use of LNA in the primers, and this facilitates a higher degree of multiplexity in the PCR as shown on Figure 25. By incorporation of LNA into the primers, the $T_m$ of the primers can be adjusted to work at the same temperature (see temperature gradient). It can also be seen from the gel in Figure 25 also shows that amplification is more specific when LNA is included in the primers. This is due to the LNA increased $\Delta T_m$, which relates to higher specificity. Once $\Delta T_m$ of the primers is high, more primers can potentially be brought to work together.

*Prediction of $T_m$*

LNA can be used for enhancing any experiment that is based on hybridization. The series of algorithms described herein have been developed to predict the optimal use of LNA. Melting properties of 129 different LNA substituted capture probes hybridized to their corresponding DNA targets were measured in solution using UV-spectrophotometry. The data set was divided into a training set with 90 oligonucleotides and a test set with 39 oligonucleotides. The training set was used for training of both linear regression models and neural networks. As seen in Figure 26, neural networks trained with nearest neighbour information, length, and DNA/LNA neighbour effect are efficient for prediction of $T_m$ with the given set of data.

*Applications of the Normalization of Thermal Stability by LNA A and T Nucleotide Substitutions*

All assays in which DNA/RNA hybridization is conducted may benefit from the use of LNA in terms of increased specificity and quality. Exemplary uses include sequencing, primer extension assays, PCR amplification, such as multiplex PCR, allele specific PR amplification, molecular beacons, (e.g. nucleic acids be multiplexed with one colour based on multiple $T_m$'S), Taq-man probes, *in situ* hybridisation probes (e.g. chromosomal and bacterial 16S rRNA probes), capture probes to the mRNA poly-A tail, capture probes for microarray detection of SNPs, capture probes for expression microarrays (sensitivity increased 5-8 times), and capture probes for assessment of alternative mRNA splicing.

Example 2: Methods for Analyzing Test Nucleic Acid Samples using Arrays of the Invention

An elegant solution to the limitations of many current nucleic acid hybridization methods is to put a large number or all of the possible capture sequences on one chip and use the same generic chip for multiple experiments. Thus, a "universal array" consisting of a subpopulation or the complete population of all possible oligonucleotides of a given length may be used as a general purpose tool to obtain hybridization patterns under different incubation conditions (also called "DNA signatures" or "genatures"). For example, the hybridization pattern can be obtained at different temperatures, cation concentrations (e.g. concentrations of monovalent cations such as $Na^+$ and $K^+$ or divalent cations such as $Mg^{2+}$ and $Ca^{2+}$), or denaturant concentrations (e.g. hydrogen bond donors or acceptors that interfere with the hydrogen bonds

keeping the base-pairs together such as formamide or urea). The temporal concentration gradients can be applied, e.g., to capture probes spotted in a channel on a microfluidic device. Obtaining hybridization patterns under multiple incubation conditions can be used to increase the amount of information obtained from hybridization to short capture probes (e.g. probes with less than 8, 7, 6, or 5 nucleotides) to the amount of information obtained from hybridization to long capture probes (e.g. probes with at least 9, 10, 11, 12, or more nucleotides) at one incubation condition.

These detailed hybridization patterns may be classified or analyzed by comparison to a set of standard signatures (e.g. 1, 2, 3, 4, 5, 8, 10, or more standard hybridization patterns), Figure 29. As each signature may contain many thousand data points (e.g. = 16.384 different heptamers * 20 temperatures = 327.680 data points), deconvolution of a complex sample into a large number of constituents is possible due to a highly over determined equation system. Furthermore, a sample signature can be compared to the most similar combination of standards to evaluate the quality of the fit to determine if a linear combination of the known standards adequately describes the sample. This comparison is particularly useful for medical applications in which it is desirable to rapidly analyze a large number of samples and/or to identify samples that cannot be resolved reliably with a particular set of standards.

The universal array and subsequent analysis procedure may be used as a low-cost generic nucleic acid characterization tool for a variety of applications such as the classification of tumors depending on cDNA libraries, detection of single nucleotide polymorphisms (SNP), detection of alternative slice sites, detection of microbial pathogens or contaminants, characterization of complex microbial communities in food process technologies (e.g. quality control, spoilage, or pathogen detection), and bioremediation.

At least at low temperatures and low denaturant concentrations, a large portion of the nucleic acids in a test sample may bind a capture probe that has a sequence that is less than 100% complementary to the sequence of the target nucleic acid. For example, the target nucleic acid may have nucleotides near either terminus that are not complementary to the corresponding region of a bound capture probe. Conversely, regions within a target nucleic acid that are perfectly complementary to a capture probe sequences may not be accessible due to secondary structure of the nucleic acid. However, these effects are expected to be reproducible and thus present in both the sample signature and the signatures of the standards, thereby minimizing or preventing any potential complications due to these effects.

The dramatic increase in stability of LNA oligomers (e.g. increased $T_m$) and the improved stringency of hybridization (e.g. increased $\Delta T_m$ between probes bound to complementary nucleic acids and probes bound to noncomplementary nucleic acids) improve the performance of a microarray (e.g. a universal array or an array with probes of naturally-occurring sequences) dramatically.

The thermal stability of a large set of oligonucleotide duplexes (> 1000) has been determined by UV spectroscopy to create and evaluate a thermodynamic nearest neighbour model (Tm-predict, accessible at http://lna-tm.com) that can predict the thermal stability of LNA substituted oligonucleotide duplexes (Figure 26). This model has been used for calculating the expected melting temperature for oligonucleotides of different length and LNA substitution pattern (Figure 2).

While the predicted average stability of DNA heptamers is only 22˚C, the stability of partially substituted LNA heptamers is increased above 50˚C in 1 M NaCl which is required for efficient capture of target nucleic acids. By comparison, to obtain a similar stability using DNA requires the use of 11-mer oligonucleotides, which would need the synthesis of 411 = 4,194,304 different oligonucleotides for a universal DNA array. In contrast, the use of LNA-enhanced heptamers requires only 47 = 16,384 different sequences (Figure 2), thus reducing the complexity for a universal array by several orders of magnitude. Our feasibility study and extensive in silico simulations indicate that an optimized selection algorithm may reduce the complexity even further (to about 1200 different heptamer probes spotted in triplicates) without significant loss of information.

As different target nucleic acids typically have different thermal stabilities (e.g. different stabilities due to different lengths or different levels of complimentarily to a capture probe), the amount of the target molecule that is bound to each capture probe is desirably measured at different temperatures, cation concentrations, or denaturant concentrations. Consecutive pictures of the array may be acquired after incrementally increasing the temperature of the array. If a full heptamer array of 128 x 128 capture probes is observed at 2˚C intervals from 30 to 70˚C, then 128 x128 x20 = 327,680 data points, which constitute the "biosignature" of the sample, are obtained. This biosignature may be used, e.g., for classifyhing the sample according to a set of standards. If the sample contains a mixture of different sequences and the signature of each of the sequences is known (i.e., the signatures are included in the standards), then the amount of each sequence in the sample can be accurately determined (Figure 29). The basic assumption for this determination is that the biosignature of the complex sample is a linear combination of the signatures of the individual components, as illustrated in the following equation.

$$I_i = \sum_{j=1,n} a_j * I_{j,i}$$

For example, even with 200 different standards, the composition of the test sample can be determined by solving 327,680 equations with only 200 unknowns, as illustrated below.

$$I_1 = a_1*I_{1,1}+a_2*I_{1,2}+\ldots+a_{200}*I_{1,200}$$

$$I_2 = a_1*I_{2,1}+a_2*I_{2,2}+\ldots+a_{200}*I_{2,200}$$

$$I_3 = a_1*I_{3,1}+a_2*I_{3,2}+\ldots+a_{200}*I_{3,200}$$

...

$$I_i = a_1*I_{i,1}+a_2*I_{i,2}+\ldots+a_{200}*I_{i,200}$$

...

$$I_{320000} = a_1*I_{320000,1}+a_2*I_{320000,2}+\ldots+a_{200}*I_{320000,200}$$

An equation system that is so overdetermined is quite tolerant to background noise, despite the large number of unknowns. Such an overdetermined linear equation system can be solved by standard methods as implemented in any mathematical data analysis packages, such as Mathematica 4.0 (Wolfram Research). Furthermore, it is possible to back calculate to compare the theoretical biosignature of the sample with the experimental biosignature to estimate the accuracy of the analysis (Figure 20 and 32-35).

The best estimate for $a_{pi}$ and $a_{ni}$ coefficients is determined by finding the coefficients $a_{pi}$ and $a_{ni}$ so that the linear combination of the standard signatures best resemble the complex sample signature by a standard least-squares criteria. A log transformation of the experimental intensities is desirably performed prior to analysis to ensure that a 2-fold higher signal has the same impact as a 2-fold lower signal, i.e., the best fit minimizes the relative and not the absolute differences. The method is desirably calibrated with a set of standard signatures and trained/tested with a set of known samples to determine acceptance and rejection criteria. Theoretically, a biosignature of 16,384 probes (7-mers) observed at 20 different temperatures can be deconvoluted into relative contributions of more than 300,000 different standards. In desirable embodiments, 10-100 standards are used.

In desirable embodiments, there is an excess (e.g. at least a 3, 5, 8, or 10-fold excess) of capture probes compared to target molecules such that each standard in the sample is bound independently. To ensure that this desirable condition is met, the test nucleic acid sample may be diluted prior to analysis. Under the opposite condition in which there is a vast excess of target molecules and few capture probes, a competitive pattern may arise, which can also be deconvoluted. For example, the algorithms described herein or pattern recognition algorithms from image analysis can be used for this deconvolution.

An exemplary application of this classification method is diagnosis of early tumors based on mRNA expression patterns. For example, a patient sample is compared to signatures of 20 malignant tumors and 20 benign tumors to determine which standard the signature of the patient sample most closely resembles. In particular, a biopsy from a patient with bladder cancer can be classified by comparison to cDNA libraries from benign and malignant tumors. cDNA libraries of 20 patients with benign tumors can be used for generating positive standards $P_1$ - $P_{20}$, and cDNA libraries

of 20 patients with malignant tumors can be used for generating negative standards $N_1$ - $N_{20}$ for comparison to the unknown sample cDNA library. A value of over 10 for the quantity $\Sigma a_{Pi} / \Sigma a_{Ni}$ indicates that the sample is from a benign tumor, while a value of less than 0.1 for the quantity $\Sigma a_{Pi} / \Sigma a_{Ni}$ indicates that the sample is from a malignant tumor. For cases in which, $0.1 < \Sigma aPi / \Sigma aNi < 10$, or $\Sigma$ IExperiment≠ ΣIPredict, additional tests may optionally be performed to confirm the classification.

For the above comparison, a theoretical hybridization pattern as a linear combination of standard patterns is calculated based on the estimated abundance. The deviation from known standard patterns is quantified. Quality control may be used to identify unusual samples or errors. This method leads to a quantified and documented accuracy of diagnosis and ability to characterize deviations. To selectively retrieve unknown and/or deviating gene sequences, the unique sequences (e.g. heptamers) that were absent in standards can be used as PCR primers.

An exemplary application of these methods includes comparing hybridization patterns of cDNA from a patient sample to classify early-tumors or detect an infection or a diseased state. The microarrays of the invention may also be used as a general tool to analyze the PCR products generated by amplification of a test sample with PCR primers for one or more nucleic acids of interest. For example, PCR primers can be used to amplify nucleic acids with a particular SNP, and then the PCR products can be identified and/or quantified using a microarray of the invention. For identification of splice variants, PCR primers to specific exons can be used to amplify nucleic acids that are then applied to a microarray for detection and/or quantification as described herein. To detect microbial pathogens, species-specific PCR primers can be used to amplify nucleic acids in a sample for subsequent analysis using a microarray. For example, the hybridization pattern of the PCR products to the array can be used to distinguish between different bacteria, viruses, or yeast and even between different strains of the same pathogenic species. In particular embodiments, the array is used for determining whether a patient sample contains a bacteria strain that is known to be resistant or susceptible to particular antibiotics or contains a virus or yeast strain known to be resistant or susceptible to certain drugs. Changes in product composition or raw material origin can also be detected using a microarray. The arrays can also be used to determine the composition of mRNA cocktails by linear deconvolution of biosignatures.

Exemplary environmental microbiology applications of these arrays include identification of major rRNA types in contaminated soil samples and classification of microbial isolates with a high resolution signature (e.g. signatures of rRNA amplification products). These rRNA amplificates are formed from rRNA by rtPCR or from the rDNA gene by conventional PCR. Numerous general and selective primers for different groups of organisms have been published. Most frequently an almost full length amplificate of the 16S rDNA gene is used (e.g. the primers 26F and 1492R). For purifying rRNA from a soil sample, standard methods such as one or more commercial extraction kits from companies such as QIAGEN ("Rneasy", Q-biogene "RNA PLUS," or "Total RNA safe" can be used.

*Exemplary Methods for Identifying Unknown Sequences in a Test Sample*

Oligonucleotides in the sample but not in a standard (i.e., corresponding spot absent in one or more standards) can be identified by their signal intensity. These previously unknown oligonucleotides can be used as PCR primers after extending the sequence at the 5' end with degenerate positions to extract novel sequences from the sample. For example, if two sequences corresponding to unexpected spots reside in the same molecule within a distance that is amplifyable by PCR, primers based on these two sequences can be used to amplify the novel moleucle. For two unexpected sequences A and B, PCR amplification can be performed with primes of sequence A and B' and with primers of sequence A' and B, in which A' and B' are the reverse complement of A and B, respectively.

Alternatively, a capture probe that hybridizes to a novel molecule can be used to purify the novel molecule from the test sample. For example, the capture probe can be immobilized on a magnetic bead and used to select the novel molecule. If desired, the selected molecule can be amplified using the capture probe as a primer and using a degenerate primer as an optional second primer.

<u>Example 3: Exemplary microarrays</u>

Arrays comprising the population of nucleic acids can be generated by standard methods for either synthesis of nucleic acid probes that are then bonded to a solid support or synthesis of the nucleic acid probes on a solid support (e.g. by sequential addition of nucleotides to a reactive group on the solid support). In desirable methods for on-chip synthesis of the capture probes, photogenerated acids are produced in light-irradiate sites of the chip and used to deprotect the 5'-OH group of nucleic acid monomers and oligomers (e.g. to remove an acid-labile protecting group such as 5'-*O*-DMT) to which a nucleotide is to be added (Gao et al., Nucleic Acid Research 29:4744-4750, 2001). Standard methods can also be used to label the nucleic acids in a test sample with, e.g., a fluorescent label, incubate the labeled nucleic acid sample with the array, and remove any unbound or weakly bound test nucleic acids from the array. Exemplary methods are described, for example, in US 6,410,229; 6,406,844; 6,403,957; 6,403,320; 6,403,317; 6,346,413; 6,344,316; 6,329,143; 6,310,189; 6,309,831; 6,309,823; 6,261,776; 6,239,273; 6,238,862; 6,156,501; 5,945,334;

5,919,523; 5,889,165; 5,885,837; 5,744,305; 5,445,934; 5,800,9927; and 5,874,219.

In an exemplary method for synthesis of an array, capture probes were immobilized using AQ technology with a HEG5 linker (US 6,033,784) onto an Immobilizer™ slide. An exemplary chip consists of 288 spots in four replicates (i.e., 1152 spots) with a pitch of 250 μm, and an exemplary hybridization buffer is 5xSSCT (i.e., 750 mM NaCl, 75 mM Sodium Citrate, pH 7.2, 0.05% Tween) and 10 mM $MgCl_2$. An exemplary target is a 45-mer oligonucleotide with Cy5 at the 5' end and with a final concentration in the hybridization solution of 1 μM. (Figure 28)

Hybridization was performed with 200 μL hybridization solution in a hybridization chamber created by attaching a CoverWell™ gasket to the Immobilizer™ slide. The incubation was conducted overnight at 4°C. After hybridization, the hybridization solution was removed, and the chamber was flushed with 3 x 1.0 mL hybridization buffer described above without any target nucleic acid. A coverWell™ chamber was then filled with 200 μL hybridization solution without target. The slide was observed with a Zeiss Axioplan 2 epifluorescence microscope with a 5x Fluar objective and a Cy5 filterset from OMEGA. The temperature of the microscope stage was controlled with a Peltier element. Thirty-five images at each temperature were acquired automatically with a Photometrics camera, automated shutter, and motorized microscope stage. The images were acquired, stitched together, calibrated and stored in stack by the software package "MetaVue". An example of a hybridization pattern generated with such an array is included in Figure 28.

Arrays can be generated using capture probes of any desired length (e.g. arrays of pentamers, hexamers, or heptamers.) In various embodiments, 1, 2, 3, 4, 5, 6, 7, 8, or more nucleotides of the probes are LNA nucleotides. Desirably, at least 1, 2, 3, 5, 7, 9, or all of the A and T nucleotides in the probes are LNA A and LNA T nucleotides. LNA nucleotides can be placed in any position of the capture probe, such as at the 5' terminus, between the 5' and 3' termini, or at the 3' terminus. LNA nucleotides may be consecutive or may be separated by one or more other nucleotides. The microarrays can be used to analyze target nucleic acids of any "AT" or "GC" content, and are especially useful for analyzing nucleic acids with high "AT" content because of the increased affinity of the microarrays of the present invention for such nucleic acids compared to traditional microarrays. The arrays can also be used to detect any type of nucleotide mutation (e.g. an insertion, deletion, frameshift mutation, silent mutation, nonsense mutation, or missense mutation) in any position of the target nucleic acid (e.g. an internal mutation or a mutation at a terminus of the nucleic acid). Desirably, the array has at least 100, 200, 300, 400, 500, 600, 800, 1000, 2000, 5000, 8000, 10000, 15000, 20000, or more different probes. If desired, nucleotides with a universal nucleobase can be included in the capture probes to increase the $T_m$ of the capture probes (e.g. capture probes of less than 7, 6, 5, or 4 nucleotides). In desirable embodiments, 1, 2, 3, 4, 5, or more nucleotides with a universal nucleobase are located at the 5' and/or 3' termini of the capture probes.

Example 4: Exemplary Methods for the Prediction of Melting Temperatures for Nucleic Acid Populations of the Invention

LNA units have different melting properties than DNA and RNA nucleotides. Until recently, thermodynamical models for melting temperature prediction have existed for DNA and RNA only, but not for LNA. Now a $T_m$ prediction model for LNA/DNA mixed oligonucleotides has been developed. The $T_m$ prediction tool is available on-line at the Exiqon website (www.LNA-Tm.com and http://www.exiqon.com/Poster/Tmpred-ET-view.pdf).

Numerous applications in molecular biology are based on the ability of DNA and RNA to hybridize in a temperature dependent manner (e.g. the microarray techniques, PCR reactions and blotting techniques). The melting properties of nucleic acid duplexes, in particular the melting temperature $T_m$, are crucial for optimal design of such experiments. $T_m$ is usually computed using a two-state thermodynamical model (Breslauer, Meth. Enzymol., 259:221-242, 1995). Several different groups have estimated model parameters for nearest neighbours in the sequence based on experimental data (for a review see SantaLucia, Proc. Natl. Acad. Sci., 95:1460-1465, 1998).

The model described herein predicts the $T_m$ of duplexes of mixed LNA/DNA oligonucleotides hybridized to their complementary DNA strands. DNA monomers are denoted with lowercase letters, and LNA monomers are denoted with uppercase letters, e.g. there are eight types of monomers in the mixed strand: a, c, g, t, A, C, G and T. The model is based on the formula (SantaLucia, 1998, *supra;* Allawi et al., Biochemistry 36:10581-10594, 1997).

$$T_m = \frac{\Delta H}{\Delta S + R \cdot \ln(C - C_m / 2) + 0.368(L-1)\ln[\mathrm{Na}^+]},$$

in which the salt concentration [Na+] enters as an entropic correction together with the oligonucleotide concentrations. $R$ is the gas constant, $C$ and $C_m$ are the concentrations of the two strands where $C \geq C_m$, and $L$ is the length of the strands. For self-complementary sequences, $C$-$C_m$ /2 is replaced by the total strand concentration $C_T$ and a symmetry correction of -1.4 cal/k·mol is added to $\Delta S$ (SantaLucia, 1998, *supra*).

The LNA model differs from SantaLucia's DNA model in the way the changes in enthalpy $\Delta H$ and entropy $\Delta S$

are calculated. As in SantaLucia's model, they depend on nearest neighbour sequence information and special contributions for the terminal base-pairs in the two ends of the duplex. However, with eight types of monomers (LNA and DNA) the increased number of nearest neighbour combinations requires more model parameters to be determined and hence more data.

*Parameter Reduction*

Usually $\overline{\Delta}H$ and $\Delta S$ are calculated as a sum of contributions from all nearest neighbour pairs in the sequence. The inclusion of LNA doubles the number of monomer types and quadruples the number of possible nearest neighbour pairs. Parameter reduction strategies are used for matching the model complexity to limited data sets. A strategy for reducing model complexity is to sum $\Delta H$ from single base-pair contributions, which do not take the influence of adjacent nucleotides into account. However, nearest neighbour contributions are added as a correction term to the single base-pair contributions.

Another strategy is to use hierarchically reduced monomer alphabets. Here, similar monomers are identified with the same letter. A four-letter alphabet, {w,s,W,S}, defines classes according to binding strength: w={a,t}, s={c,g}, W={A,T} and S={C,G}. The smallest alphabet, {D,L}, simply identifies the monomer type: DNA or LNA. As an example, the sequence GcTAAcTt can be written as SsWWWsWw or as LDLLLDLD.

The principle is to split $\Delta H$ and $\Delta S$ into contributions that depend on different levels of detail of the sequence. The fine levels of detail require many parameters to be determined, while the coarse levels need fewer parameters. The more detailed contributions can then be treated as minor corrections, thus effectively reducing the total number of model parameters.

*Training*

Model parameters were determined using data from melting experiments on hundreds of oligonucleotides. The oligonucleotides were random sequences with lengths between 8 and 20 and a percentage of LNA between 20 and 70. Melting curves were obtained using a Perkin-Elmer UV λ-40 spectrophotometer, but only the $T_m$ values were used for modeling. Model parameters were adjusted using a gradient descent algorithm that minimizes the error function

$$E = \sum_{\substack{\text{data} \\ \text{set}}} \frac{1}{N}\left(T_m^{pred} - T_m^{\exp}\right)^2,$$

i.e., the distance between predicted and experimental $T_m$ values. Many different models were trained in this way and their performance was evaluated on test sets distinct from the training data. Seven reliable models were chosen and combined to form the committee model implemented at the Exiqon website (www.LNA-Tm.com.)

*Machine Learning and Thermodynamics*

The aim of this work has been to estimate $T_m$ values as accurately as possible. To this end, a machine learning approach has been adopted in which the prediction of the physical $\Delta H$ and $\Delta S$ quantities is less important. The parameters of this model may be inaccurate as thermodynamic quantities. First, the gradient descent algorithm produces a broad ensemble of models in which the $\Delta H$ and $\Delta S$ parameters can vary substantially, while maintaining an accurracy in the predicted $T_m$. Second, the thermodynamic meaning of $\Delta H$ and $\Delta S$ is based on a two-state assumption, which may not be realistic in every case. Even short oligonucleotides can form different secondary structures or melt through multiple-state transitions (Tøstesen et al., J. Phys. Chem. B. 105:1618-1630, 2001). Third, the use of an optical instrument instead of a calorimetric instrument (DSC) introduces an error in the measured $\Delta H$ and $\Delta S$. Nevertheless, the uncertain thermodynamic interpretation of the $\Delta H$ and $\Delta S$ model parameters does not imply that the $T_m$ prediction model is unreliable.

*Results*

The $T_m$ prediction model has been tested on two data sets that were not used during the training process. One set consisted of pure DNA oligonucleotides without LNA monomers and had a standard deviation of the residuals (SEP) of 1.57 degrees. The other set consisted of mixed oligonucleotides with both LNA and DNA and had a SEP of 5.25 degrees. The difference in prediction accuracy between the two types of oligonucleotides suggests that $T_m$ prediction of mixed strands is a more complex task than $T_m$ prediction of pure DNA. This is possibly due to irregularities in the

duplex helical structure induced by the LNA monomers (Nielsen et al., Bioconjug. Chem. 11:228-238, 2000). The obtained prediction accuracy is in both cases adequate for most biological applications. In conclusion, the reduced nearest neighbour model implemented at the Exiqon website (www.LNA-Tm.com) can predict $T_m$ surprisingly well for both types of oligonucleotides (Figure 26). This indicates that the parameter reduction strategy is applicable for other types of modified oligonucleotides.

Example 5: Exemplary Methods for Optional Calibration and Optimization of Hybridization Patterns

The following example includes exemplary techniques for (i) compensating for uneven illumination, (ii) compensating for photobleaching during measurements, (iii) obtaining a relative signal, and (iv) scaling the temperature-, cation-, or denaturant -dependent hybridization patterns prior to deconvolution to a set of standard signatures. These calibration procedures enable a successful comparison of a complex sample signature to a set of standard signatures (e.g. the deconvolution of temperature-, cation-, or denaturant-dependent hybridization patterns). Calibration is desirable for comparing hybridization patterns of different DNA arrays, whereas calibration is less important for comparing signals obtained from the same array. The following uses of relative signals and corrections for photobleaching may also be applied to the analysis of a variety of arrays, with or without nucleic acid probes of the invention.

*Correction for uneven illumination*

The viewing field in a Zeiss microscope is typically not evenly illuminated despite efforts to adjust the mercury arc excitation light source. To adjust for the varying intensity of the excitation light source, the following procedure is applied. An image of a defocused slide with an even distribution of the same fluorophore as the label used on the target DNA (e.g. a solution of Cy5-labelled oligonucleotide permanently mounted on a slide) is obtained. This image is called the "intensity image." The pixel with the lowest intensity within the "intensity image" is referred to as $I_{min}$. All subsequent images in the genature that need to be calibrated are corrected by dividing the intensity of each pixel with the intensity of the corresponding pixel of the "intensity image" and multiplying by $I_{min}$, as follows.

$$I_{calibrated} = I_{original} * I_{min} / I_{intensity\ image}$$

*Correction for fading*

As several images are acquired to obtain a temperature-, cation-, or denaturant dependent hybridization pattern, the following procedure can be used to compensate for the photobleaching of the fluorophores that necessarily occurs. This procedure involves determining the average intensity of the "landing lights" (i.e., a set of oligonucleotides labeled with the same fluorophore that is put on the array for orientation purposes). The intensity of each pixel in the n'th image is corrected by multiplying this intensity by the average intensity of all "landing lights" in the first picture and dividing the average intensity of the landing lights in the n'th image, as follows.

$$I_{corrected} = I_{image\ n} * \mathrm{Mean}(I_{landing\ lights,\ first\ image}) / \mathrm{Mean}(I_{landing\ lights,\ image\ n})$$

*Evaluation of spot intensities*

The combined intensity of each capture probe on the array is determined by a set of image analysis algorithms designed to find and quantify the intensity of each spot on a volume base. This step can be performed by commercial applications such as "Array Vision."

*Correction for uneven spotting*

To correct for differences in the amount of capture probe that has been spotted in individual spots on different arrays, the absolute intensity signal is converted to a relative signal. This conversion can be performed in several different ways. In one method, SYBR green II staining of the bound capture probe is performed before or after hybridization. SYBR green II binds strongly to both single and double-stranded DNA and fluoresces strongly, when bound but not

when in solution. SYBR green can be introduced initially and an image of the amount of bound capture probe can be acquired. The SYBR green is subsequently washed away before hybridization. It can also be applied after hybridization. At the end of hybridization, the last remaining target nucleic acid can be washed away with low salt buffer. Afterwards, the SYBR green can be introduced to quantify the amount of capture probe. Alternatively, capture probes labeled with a different fluorophore than the target nucleic acids can be used. If desired, hybridization conditions can be modified to minimize any interference in hybridization due to the fluorophore. In another procedure, labeled DNA random monomers of the same length as the capture probes are added after the hybridization experiment. These random monomers can easily be made using a mixture of all four amidites during synthesis, labeled with a different dye, and added at the end of the experiment, e.g., when the temperature has returned to room temperature. These aforementioned correction methods can be generally used for any microarray, include the arrays of the present invention.

*Correct for differences in sample conditions*

A distinct advantage of acquiring several images of the DNA array at increasing temperatures or denaturant concentrations is the ability to compensate for small impurities in the sample preparation. For example, some samples may contain small amounts of cations, notably $Mg^{2+}$, that may change the melting behavior of the capture probes. To correct for this effect, the sample can be spiked with a few labeled oligonucleotides with known sequence and melting behavior. If the observed temperature or denaturant hybridization pattern of these spiked sequences differ from the established standards, then the thermal hybridization pattern of the entire array can be scaled to the established standard by simply correcting the temperature to a salt corrected temperature or correcting the denaturant concentration to a salt corrected denaturant concentration that makes the data for the spiked oligonucleotides fit the standard curve. The chip typically contains so many different spots (e.g. a chip with 16,384 heptamers) that using a few spots (e.g. 10 - 20 spots) for calibration does not noticeably diminish the information content. The spiked oligonucleotides desirably have the same length as the capture probes and have a different AT/GC content. These oligonucleotides are also labeled with the same fluorophore as the target nucleic acids because using a different fluorophore may increase the duration of the experiment and the amount of photobleaching due to double exposure of the fluorophores. If desired, small permutations in the salt concentration can be tested to evaluate the sensitivity of this approach.

*Chip design for testing different substitution patterns and flanking regions (Figure 28)*

Desirably, all capture probes are synthesized with AQ2 modification (US 6,033,784). An exemplary linker that should not cause unspecific target binding is five hexa-ethylene-glycol (HEG5). The length of the linker is sufficient to allow capture of mRNA with a reasonable length (e.g. 800 nucleotides). The capture probes may be spotted with a Packard spotter on immobilizer™ slides or on native slides. Examples of LNA substitution patterns for heptamers include (a) xxxxxxx, (b) xXxXxXx, (c) XxxXxxX, (d) XxXxXxX, (e) XxXXXxX, and (f) XXXXXXX., in which upper case letters denote LNA nucleotides and lower case letters denote DNA nucleotides. Examples of LNA substitution patterns for hexamers include (a) xxxxxx, (b) xXxxXx, (c) XxXxXx, (d) XxXXxX, (e) XXXXXX, and (f) XXXXX. Different flanking regions of inosine, 5 nitro-indole, and/or random bases may be used, e.g., (a) none, xxxxxxx; (b) one inosine, ixxxxxxxi; (c) two inosines, iixxxxxxxii; (d) one random, nxxxxxxxn; and (e) one 5-nitro-indole, zxxxxxxxz.

Exemplary target sequences with different AT-GC contents include two targets with 6 AT and 1 GC base pairs (86% AT), and one target with 5 AT and 2 GC base pairs (71% AT) from HSP 78. For ACT 1, one target with 5 AT and 2 GC base pairs (71% AT) and two targets with 4 AT and 3 GC base pairs (57% AT) are additional examples. One target with 4 AT and 3 GC base pairs (57% AT) and two targets with 3 AT and 4 GC base pairs (43% AT) from SSA 4 can be used. These three target nucleic acids correspond to sequence stretches from three different mRNAs that are available in pure form from our research laboratories. The target sites in each gene were selected so that they are not likely to participate in a strong secondary structure in cDNA generated from mRNA. This evaluation was done with using publicly available "mfold" (by M. Zucker, such as the European mfold server version 0.01) by looking for regions with high ss-counts. These regions were subsequently evaluated in folding patterns for the respective sequences (about 25 different structures for each sequence all with ΔG within 5% of best fold).

Three different frame-shifted sequences for each target sequence enables one to look at non-central mismatch discrimination with the same labeled test sequence: (i) abcdefg, (ii) bcdefgh, and (iii) cdefghi. Exemplary capture probes with flanking regions of universal LNA bases include inosine LNA: IxxXxXxXI, IXxXXXxXI, and IXXXXXXI; and 2-aminopurine-LNA: ÅXxXxXxXÅ, ÅXxXXXxXÅ, and ÅXXXXXXÅ. If desired, to evaluate the ability of particular probes to invade strong secondary structures in mRNA, double helix structures in cDNA molecules (e.g. the eight base-paired helix in ACT1 at position 108-115 and 144-151, and the base-paired helix in SSA4 at position 503-512 and 550-559) may be targeted with various LNA substituted capture probes.

In an exemplary microarray used for optional optimization of assay conditions, the number of capture probes on the slide is (6*6+5*5) x 3 = 183 with additional oligomers containing universal LNA bases: (3 *2) x 3 = 18, additional

oligomers containing 5-nitro-indole: 6 x 3 = 18, and additional LNA probes for invasion of secondary structure: 5 x 2 = 10, resulting in a total of 229 probes. Each probe is spotted, e.g., in four replicates (i.e., 1008 spots total) in a grid layout of 4 blocks of 229 different oligomers spotted and 23 "landing lights" as 18 rows and 14 columns. The area of each replicate block is 18 x 14 spots = 3.6 mm x 2.8 mm. Desirably, at least 23 slides are used for evaluation. The test slide is evaluated with labeled synthetic target sequences of 3 x (3+9+3) = 45 nucleotides in length that are labeled with Cy5 in the 5' end. The synthetic target sequence is composed of three parts (each 15 nucleotides) corresponding to a non-structured domain of each of the three evaluated genes. The base sequence of the resulting combined target sequence is constructed such that it does not form significant or any secondary structures.

If desired, to test the effect of mismatches, eight different versions of a target sequence are used. The mismatches were chosen in this example so that all possible mutations are as evenly represented in the resulting 21 mismatch experiments as possible. These probes include Wild Type (5'-Cy5-ttaccagtacctttt-caaatcgattctcaa-ttcaaattcatcaaa) (SEQ ID NO: 1), M1 (5'-Cy5-ttacaagtacctttt-caaaacgattctcaa-ttcacattcatcaaa) (SEQ ID NO: 2), M2 (5'-Cy5-ttaccggtacctttt-caaatggattctcaa-ttcaatttcatcaaa) (SEQ ID NO: 3), M3 (5'-Cy5-ttaccaatacctttt-caaatccattctcaa-ttcaaactcatcaaa) (SEQ ID NO: 4), M4 (5'-Cy5-ttaccaggacctttt-caaatcgcttctcaa-ttcaaatacatcaaa) (SEQ ID NO: 5), M5 (5'-Cy5-ttaccagtgcctttt-caaatcgactctcaa-ttcaaatttatcaaa) (SEQ ID NO: 6), M6 (5'-Cy5-ttaccagtaactttt-caaatcgatgctcaa-ttcaaattcttcaaa) (SEQ ID NO: 7), and M7 (5'-Cy5-ttaccagtacgtttt-caaatcgattttcaa-ttcaaattcaacaaa) (SEQ ID NO: 8).The resulting mismatch occurrence table is shown below (Table 3).

Table 3. Mismatch table

| Mismatch table | Target A | Target C | Target G | Target T |
|---|---|---|---|---|
| Capture probe A | 3 | 2 | 2 | - |
| Capture probe C | 1 | 1 | - | 0 |
| Capture probe G | 2 | - | 2 | 2 |
| Capture probe T | - | 2 | 2 | 2 |
| A total of 21 mismatches | | | | |

The test slide may also be evaluated with different mixtures of mRNA, such as ACT1; HSP78; SSA4; 33% ACT and 33% HSP and 33% SSA4; 10% ACT and 25% HSP and 65% SSA4; 85% ACT and 12% HSP and 3% SSA4; and 5% ACT and 85% HSP and 10% SSA4. Hybridization with synthetic DNA targets (e.g. 1 wild-type and 7 mutant sequences) as described above uses 16 slides, and hybridization with mRNA mixtures (3 standards and 4 mixtures) uses 7 slides.

*Exemplary Computer*

Any of the methods described herein may be implemented using virtually any computer system. A computer system **2** includes internal and external components. The internal components include a processor **4** coupled to a memory **6**. The external components include a mass-storage device **8**, *e.g.* a hard disk drive, user input devices **10**, *e.g.*, a keyboard and a mouse, a display **12**, *e.g.* a monitor, and usually, a network link **14** capable of connecting the computer system to other computers to allow sharing of data and processing tasks. Programs are loaded into the memory **6** of this system **2** during operation. These programs include an operating system **16**, e.g. Microsoft Windows, which manages the computer system, software **18** that encodes common languages and functions to assist programs that implement the methods of this invention, and software **20** that encodes the methods of the invention in a procedural language or symbolic package. Languages that can be used to program the methods include, without limitation, Visual $C/C^{++}$ from Microsoft. In preferred applications, the methods of the invention are programmed in mathematical software packages that allow symbolic entry of equations and high-level specification of processing, including algorithms used in the execution of the programs, thereby freeing a user of the need to program procedurally individual equations or algorithms. An exemplary mathematical software package useful for this purpose is Matlab from Mathworks (Natick, MA). Using the Matlab software, one can also apply the Parallel Virtual Machine (PVM) module and Message Passing Interface (MPI), which supports processing on multiple processors. This implementation of PVM and MPI with the methods herein is accomplished using methods known in the art. Alternatively, the software or a portion thereof is encoded in dedicated circuitry by methods known in the art.

Example 6: Optional Algorithm to Optimize the Substitution Pattern of Nucleic Acids of the Invention

High affinity nucleotides such as LNA and other nucleotides that are conformationally restricted to prefer the C3'-endo conformation or nucleotides with a modified backbone and/or nucleobase stabilize a double helix configuration.

As these effects are generally additive, the most stable duplex between a high affinity capture oligonucleotide and an unmodified target oligonucleotide should generally arise when all nucleotides in the capture probe or primer are replaced by their high affinity analogue. The most stable duplex should thus be formed between a fully modified LNA capture probe and the corresponding DNA/RNA target molecule. Such a fully modified capture probe should be more efficient in capturing target molecules, and the resulting duplex is more thermally stable.

However, many high affinity nucleotides (e.g. as LNA) have an even higher affinity for other high affinity nucleotides (e.g. as LNA) than for DNA/RNA. A fully modified capture probe may thus form duplexes with itself, or if it is long enough, internal hairpins that are even more stable than duplexes with the desired target molecule. Probes with even a small inverse repeat segment where all constituent positions are substituted with high affinity nucleotides may bind to itself and be unable to bind the target. Thus, a sequence dependent substitution pattern is desirably used to avoid substitutions in positions that may form self-complementary nucleobase-pairs.

For example, a computer algorithm can be used to automatically determine the optimal substitution pattern for any given capture probe sequence according to the following two criteria. First, the difference between the stability of (i) the duplex formed between the capture probe and the target molecule and (ii) the best possible duplex between two capture probes should be above a certain threshold. If this is not possible, then the substitution pattern with the largest possible difference is chosen. Second, the capture probe should contain as many substitutions as possible in order to bind as much target as possible at any given temperature and to increase the thermal stability of the formed duplex. Alternatively, the second criterion is substituted with the following alternative criterion to obtain capture probes with similar thermal stability. The number and position of capture probe substitutions should be adjusted so that all the duplexes between capture probes and targets have a similar thermal stability (i.e., $T_m$ equalization).

For short capture probes such as those used in an universal microarray, incomplete matches between target and capture probe are likely to be a reproducible feature of the recorded biosignatures. For these short probes, the second criterion for increasing thermal stability is more desirable that the alternative second criterion for $T_m$ equalization. For long capture probes and PCR primers, the second alternative criterion is desirably used since $T_m$ equalization is desirable for these probes and primers.

An exemplary algorithm works as follows. For each nucleotide sequence in an universal array of length n (e.g. for each of the 16,384 possible oligonucleotide sequences in a 7-mer universal array), all possible substitution patterns, i.e., $2^n$ different sequences are evaluated (e.g. for each 7-mer sequence, the $2^7$ = 128 different possible substitution patterns are evaluated giving 16,384 x 128 = 2,097,152 evaluations for the complete set). Each evaluation consist of estimating the energetic stability of the duplex between the substituted capture sequence and a perfect match unmodified target ("target duplex") and the energetic stability of the most stable duplex that can be formed between two substituted capture probes themselves ("self duplex").

The energetic stability estimate for a duplex may be calculated, e.g., using a Smith-Waterman algorithm with the following scoring matrix.

Gap initiation penalty: -8

Gap continuation penalty: -50

| | a | c | g | t | A | C | G | T |
|---|---|---|---|---|---|---|---|---|
| a | -2 | | | | | | | |
| c | -2 | -2 | | | | | | |
| g | -2 | 3 | -2 | | | | | |
| t | 2 | -2 | 1 | -2 | | | | |
| A | -3 | -3 | -3 | 4 | -3 | | | |
| C | -3 | -3 | 6 | -3 | -3 | -3 | | |
| G | -3 | 6 | -3 | 2 | -3 | 9 | -3 | |
| T | 4 | -3 | 2 | -3 | 6 | -3 | 3 | -3 |

This scoring matrix was partly based on the best parameter fit to a large (over 1000) number of melting curves of different DNA and LNA containing duplexes and partly by visual scoring of test capture probe efficiency. If desired, this scoring matrix may be optimized by optimizing the parameter fit as well as increasing or optimizing the dataset used to obtain these parameters.

As an example of these calculations, the heptamer sequence ATGCAGA in which each position can be either an LNA or a DNA nucleotide is used. The target duplex formed between a fully modified capture probes with this sequence and its unmodified target receive a score of 34 as illustrated below.

```
Capture sequence:  A-T-G-C-A-G-A
                   | | | | | | |
Target sequence:   t-a-c-g-t-c-t
Score:             4+4+6+6+4+6+4 = 34
```

The most stable self duplex that can be formed between two modified capture probes has an almost equivalent energetic stability with a score of 30 as illustrated below.

```
Capture sequence:  A-T-G-C-A-G-A
                      | | | |
Target sequence: A-G-A-C-G-T-A
Score:                +6+9+9+6    = 30
```

Thus, the capture probe efficiency of a fully modified probe is likely reduced by its propensity to form a stable duplex with itself. In contrast, by choosing a slightly different substitution pattern, ATGcaGA in which capital letters represent LNA nucleotides, the stability of the target duplex is reduced slightly from 34 to 29.

```
Capture sequence:  A-T-G-c-a-G-A
                   | | | | | | |
Target sequence:   t-a-c-g-t-c-t
Score:             4+4+6+3+2+6+4 = 29
```

However, the most stable self complementary duplex that can be formed is reduced much more from 30 to 20, as illustrated below.

```
Capture sequence:  A-T-G-c-a-G-A
                      | | | |
Target sequence: A-G-a-c-G-T-A
Score:                +4+6+6+4    = 20
```

The difference between the stability of the desired target duplex and the undesired self duplex can be further increased by using the capture sequence AtgcaGA where the target duplex has a score of 24.

```
Capture sequence:  A-t-g-c-a-G-A
                   | | | | | | |
Target sequence:   t-a-c-g-t-c-t
Score:             4+2+3+3+2+6+4 = 24
```

whereas the score of the self duplex is only 10, as shown below.

```
Capture sequence:   A-t-g-c-a-G-A
                        | | | |
Target sequence:  A-G-a-c-g-t-A
Score:                  +2+3+3+2     = 10
```

The additional destabilization of the self duplex is generally not required if the difference in stability between the target duplex and self duplex is above a threshold of 25% of the target duplex stability, as illustrated below.

Discrimination for ATGCAGA = (34-30)/34 = 12% < threshold (25%)

Discrimination for ATGcaGA = (29-20)/29 = 31% ≥ threshold (25%)

Discrimination for ATGCAGA = (24-10)/24 = 58% ≥ threshold (25%)

Thus, ATCcaGA is the substitution pattern with the highest degree of substitution for which the stability of the target duplex is adequately more stable than the stability of the best self duplex (e.g. above 25%).

This algorithm can be used to determine desirable substitution patterns for any size capture probe or any given probe sequence. The following simple design rules may also be applied for probe design, especially for short probes. The best self alignment for the corresponding DNA capture probe in the sequence is determined using a simple Smith-Waterman scoring matrix of:

| | a | c | g | t |
|---|---|---|---|---|
| a | -2 | | | |
| c | -2 | -2 | | |
| g | -2 | 3 | -2 | |
| t | 2 | -2 | 1 | -2 |

Additionally, all possible positions in the sequence are substituted, with the exception of desirably avoiding the substitution of both nucleobases of a self-complementary base-pair. The most stable self duplex thus does not contain any LNA:LNA base-pairs but only LNA: DNA basepairs.

<u>Example 7: Exemplary Methods for Optimization of Nucleic Acids and MicroArrays of the Invention</u>

*Experimental protocol to optimize substitution pattern for short capture probes*

a) Immobilization of capture probes by AQ technology with a HEG5 linker (see patent US 6,033,784) onto an Immobilizer™ slide

b) The current OCFA chip consists of 288 spots in four replicates (i.e. 1152 spots) with a pitch of 250 μm. (Layout shown in Figure 28)

c) Hybridization buffer was 5xSSCT (=750 mM NaCl, 75 mM Sodium Citrate, pH 7.2, 0.05% Tween) and 10 mM $MgCl_2$

d) Target was a 45-mer oligonucleotide with Cy5 at the 5' end. Final concentration in the hybridization solution was 1 $\mu$M

e) Hybridization was with 200 $\mu$L hybridization solution in a hybridization chamber created by attaching a CoverWell™ gasket to the Immobilizer™ slide. The incubation was overnight at 4˚C.

f) After hybridization, the hybridization solution was removed and the chamber flushed with 3 x 1.0 mL hybridization buffer (see above) without any taget DNA.

g) CoverWell™ chamber was then filled with 200 $\mu$L hybridization solution without target.

h) The slide was observed with a Zeiss Axioplan 2 epifluorescence microscope. With a 5x Fluar objective and a Cy5 filterset from OMEGA.

i) The temperature of the microscope stage was controlled with a Peltier element

j) 35 images at each temperature were acquired automatically with a Photometrics camera, automated shutter, and motorized microscope stage.

k) The images were acquired, stitched together, calibrated and stored in stack by the software package "MetaVue"

l) Quantification of spot intensities and evaluation of optimal substitution pattern

*Results of optimization*

Representative experimental data to calibrate scoring matrix for optimization algorithm described in example 7 is shown in Figure 31.

Example 8: Exemplary Methods for Deconvoluting Hybridization Patterns of the Invention

The following algorithm can be used to deconvolute hybridization patterns using Mathematica software (see below). The algorithm involves reading two sequence files from an ASCII input file, such as the sequences of PCR amplificates of two splice variants. The sequences are parsed to obtain an ideal biosignature for each sequence. The observed biosignature depends on the presence or absence of both heptamers as well as their associated hexamers with a single terminal mismatch. The thermal stability and thermal transition depend on the length and the number of GC nucleobases in each capture probe. The two standard biosignatures are combined to obtain a theoretical signature of a mixed sample. The standard signatures and compared to the signature of the mixed sample after addition of white noise to each signature. The deconvolution then determines how much of each of the constituent standards is in the sample before noise addition.

*Heptamer Signature chip simulation*

*Background*

Two splice variants for the LET2 gene. They are about 500 nt long and very similar sequences.

- Sequence "Embryo_9_AMP" contain Exon 7, 8, 9, 11 and 12. It is 542 bp long and expressed in the embryo of C. *elegans.* The sequence (SEQ ID NO: 9) is:

CTCCAGGAGAGAAGGGAGATGGCGGTATGCCAGGAATGCCCGGACTTCCAGGACCATCCGGTCGTGATGGATAC
CCAGGAGAAAAGGGAGACCGAGGAGATACTGGAAATGCTGGACCACGTGGACCACCTGGAGAGGCTGGATCCCC
AGGAAACCCAGGAATCGGAAGCATTGGACCAAAAGGAGATCCTGGAGA*TCTAGGTTCTGTCGGACCACCAGGTC*
*CACCGGGACCACGTGAGTTCACCGGATCCGGCTCAATTGTCGGACCTCGCGGAAACCCTGGAGAAAAGGGAGAC*
AAGGGAGAGCCAGGAGAGGGAGGTCAACGCGGTTACCCAGGAAATGGAGGACTCTCAGGACAGCCAGGACTCCC
AGGAATGAAGGGAGAAAAGGGATTGTCTGGACCAGCTGGACCAAGAGGAAAGGAAGGTCGCCCAGGAAACGCTG
GACCACCAGGATTCAAGGGAGATCGTGGTCTTGACGGACTTGGCGGAATCCCAGGACTTCCAGGCCAAAAGGGA
GAAGCTGGATACCCAGGAAGAGAT

The sequence of exon 9 which is not found in the Larval splice variant is indicated by underline and italics

- Sequence "Larvae_10_AMP" contains Exon 7, 8, 10, 11 and 12. It is 545 bp long and expressed in the larvae of C. *elegans.* The sequence (SEQ ID NO: 10) is:

CTCCAGGAGAGAAGGGAGATGGCGGTATGCCAGGAATGCCCGGACTTCCAGGACCATCCGGTCGTGATGGATAC
CCAGGAGAAAAGGGAGACCGAGGAGATACTGGAAATGCTGGACCACGTGGACCACCTGGAGAGGCTGGATCCCC
AGGAAACCCAGGAATCGGAAGCATTGGACCAAAAGGAGATCCTGGAGA*CATTGGTGCGATGGGACCGGCCGGTC*
*CGCCAGGCCCAATCGCCTCCACCATGTCCAAGGGAACCATTATCGGTCCTAAGGGAGACCTAGGAGAGAAGGGA*
*GAG*AAGGGAGAGCCAGGAGAGGGAGGTCAACGCGGTTACCCAGGAAATGGAGGACTCTCAGGACAGCCAGGACT
CCCAGGAATGAAGGGAGAAAAGGGATTGTCTGGACCAGCTGGACCAAGAGGAAAGGAAGGTCGCCCAGGAAACG
CTGGACCACCAGGATTCAAGGGAGATCGTGGTCTTGACGGACTTGGCGGAATCCCAGGACTTCCAGGCCAAAAG
GGAGAAGCTGGATACCCAGGAAGAGAT

The sequence of exon 10 which is not found in the Embryonal splice variant is indicated by underline and italics

- Sequence "Larvae_10_MUT" contain Exon 7, 8, 10, 11 and 12. It is identical to "Larvae_10_AMP" except for 3 nt TCC -> AGG which deletes a BamHI restriction site.

The sequences are identical except a 105 bp (19 % of the total length) difference. We first simulate the biosignatures of each splice variant on a random 7-mer chip (i.e., the hybridization pattern at 2 degree intervals from 12°C to 50°C). We then assume that the combined signature of a sample with 30% Embryo_9, 60% Larvae_10 and 10 % Larvae_10_MUT is a linear combination of the three standard signatures. To evaluate the noise sensitivity, we then add different amounts of noise up to both standard signatures and mix signature. Finally, we compare the signal including noise to the standards (with a similar noise level) and deconvolve it to determine the abundance of each standard in the sample.

The following includes program lines that are interpreted by Mathematica by a solid box e.g.

```
Do[
   If[StringTake[inData[[i]], 1] == ">" ,
      nrofSequence = nrofSequence + 1;
     sequenceName[[nrofSequence]] = StringDrop[inData[[i]], 1];
```

Results of the calculations produced by Mathematica are indicated by a dashed box e.g.

DECONVOLUTION RESULTS

Amount of EMBRYO_9_AMP: 0.306417 Error: 0.0064168 = 2.13893%

(continued)

| | |
|---|---|
| Amount of LARVAE_10_AMP: | 0.327182 Error: 0.272818 = 45.4696% |
| Amount of LARVAE_10_MUT: | 0.297622 Error: 0.197622 = 197.6220 |

Time used for calculation: 2.724 Seconds

*Calculate biosignatures for splice variants of LET2 gene*

*Read Data and reformat*

```
Off[General: : "spell 1"];
filename = "C:/Mathematica/Indata/sekvensdata.txt";
inData = ReadList[filename, Word, RecordLists -> True];
inData = Flatten[inData];
nrofSequence = 0;
sequences = Table["dummy", {i, 20}];
sequenceName = Table["dummy", {i, 20}];
Do[
  If[ StringTake[inData[[i]], 1] == ">" ,
     nrofSequence = nrofSequence + 1;
     sequenceName[[nrofSequence]] = StringDrop[inData[[i]], 1];
     sequences[[nrofSequence]] = "";
    ,
   sequences[[nrofSequence]] =
    StringJoin[sequences[[nrofSequence]], inData[[i]]]]
   , {i, 1, Length[inData]}];
sequenceName = Take[sequenceName, nrofSequence];
sequences = Take[sequences, nrofSequence];
Do[Print[sequenceName[[i]]], {i, 1, nrofSequence}];
```

*Calculate Hepta word matrix*

```
nrofSequence = Length[sequences];
heptaUsage = Table[0, {i, 1, nrofSequence}, {j, 1, 4^7}];
Do[nrofHepta = Floor[StringLength[sequences[[i]]] - 6];
  Do[
   test = StringTake[sequences[[i]], {k, k + 6}];
   position = 1;
   Do[
    If[StringTake[test, {m}] == "C", position = position + 1*4^(m - 1)];
    If[StringTake[test, {m}] == "G", position = position + 2*4^(m - 1)];
    If[StringTake[test, {m}] == "T", position = position + 3*4^(m - 1)],
    {m, 1, 7}];
   heptaUsage[[i, position]] = heptaUsage[[i, position]] + 1;
   {k, 1, nrofHepta}];
  , {i, 1, nrofSequence}];
title = StringJoin["Sequence ", sequenceName[[nrofSequence]],
   " position of heptaners"];
ListDensityPlot[0.3*Partition[heptaUsage[[nrofSequence]], 128],
ColorFunctionScaling -> False, PlotLabel -> title,
TextStyle -> {FontFamily -> "Times", FontSize -> 12},
ImageSize -> {427, 450}];
```

*Calculate Hexa word matrix = single terminal mismatch*

```
nrofSequence = Length[sequences];
hexaUsage = Table[0, {i, 1, nrofSequence}, {j, 1, 4^7}];
Do[nrofHexa = Floor[StringLength[sequences[[i]]] - 5];
  Do[
   test = StringTake[sequences[[i]], {k, k + 5}];
   position = 1;
   Do[
    If[StringTake[test, {m}] == "C", position = position + 1*4^(m - 1)];
    If[StringTake[test, {m}] == "G", position = position + 2*4^(m - 1)];
    If[StringTake[test, {m}] == "T", position = position + 3*4^(m - 1)],
    {m, 1,6}];
    hexaUsage[[i, position]] = heptaUsage[[i, position]] + 1;
    hexaUsage[[i, position + 4^6]] = heptaUsage[[i, position + 4^6]] + 1;
    hexaUsage[[i, position + 2*4^6]] =
       heptaUsage[[i, position + 2*4^6]] + 1;
    hexaUsage[[i, position + 3*4^6]] =
      heptaUsage[[i, position + 3*4^6]] + 1;
   position = 1;
   Do[
    If[StringTake[test, {m}] == "C", position = position + 1*4^m];
    If[StringTake[test, {m}] == "G", position = position + 2*4^m];
    If[StringTake[test, {m}] == "T", position = position + 3*4^m],
    {m, 1,6}];
    hexaUsage[[i, position]] = heptaUsage[[i, position]] + 1;
   hexaUsage[[i, position + 1]] = heptaUsage[[i, position + 1]] + 1;
   hexaUsage[[i, position + 2]] = heptaUsage[[i, position + 2]] + 1;
   hexaUsage[[i, position + 3]] = heptaUsage[[i, position + 3]] + 1;
   , {k, 1, nrofHexa}];
  , {i, i, 1, nrofSequence}];
title = StringJoin["Sequence ", sequenceName[[nrofSequence]],
   " position of hexamers"];
ListDensityPlot[0.3*Partition[hexaUsage[[nrofSequence]], 128],
 ColorFunctionScaling -> False, PlotLabel -> title,
TextStyle -> {FontFamily -> "Times", FontSize -> 12},
 ImageSize -> {427, 450}];
```

*Melting simulation with perfect matches and single terminal mismatch*

```
meltDataAll = Table[0, {i, 1, nrofSequence}, {j, 1, 16384}, {k, 1, 20}];
gcData = Table[0, {k, 1, 16384}];
Do[nrGC = 0;
  test = i;
  Do[
   pos = 7 - j;
   type = IntegerPart[test/4^pos];
   If[type == 1, nrGC = nrGC + 1];
   If[type == 2, nrGC = nrGC + 1];
   test = test - type*4^pos;
   , {j, 1, 7}];
  gcData[[i]] = nrGC;
  , {i, 1, 16384}];
Do[
  Do[
   Do[
    t = k*2 + 10;
    Tm7 = 7*gcData[[j]] + 3*(7 - gcData[[j]]);
    delta 7 =
     If[t < Tm7, 100 - 50*Exp[(t - Tm7)/(3 - gcData[[j]]/4)],
      50*Exp[(Tm7 - t)/(3 - gcData[[j]]/4)]];
   Tm6 = 6*gcData[[j]] + 3*(6 - gcData[[j]]);
```

```
   delta6 =
    If[t < Tm6, 100 - 50*Exp[(t - Tm6)/(6 - gcData[[j]]/3)],
      50*Exp[(Tm6 - t)/(6 - gcData[[j]]/3)]];
   meltDataAll[[i, j, k]] =
     heptaUsage[[i, j]]*delta7 + 0.8*hexaUsage[[i, j]]*delta6
   , {k, 1, 20}]
  , {j, 1, 16384}]
 ,{i, 1, nrofSequence}];
test = Table[0, {j, 1, 4^7}];
Do[
Do[
 test[[j]] = meltDataAll[[i, j, 1]]/100;
 , {j, 1, 4^7}];
title = StringJoin["Sequence ", sequenceName[[i]], " at 12 degC"];
ListDensityPlot[Partition[test, 128], ColorFunctionScaling -> False,
 PlotLabel -> title, TextStyle -> {FontFamily -> "Times", FontSize -> 12},
 ImageSize -> {427, 450}]
,{i, 1, nrofSequence}];
```

Figures 32-34 illustrate sequence EMBRYO_9_AMP at 12˚C, sequence LARVAE_10_AMP at 12˚C, and sequence LARVAE_10_MUT at 12˚C.

*Simulate sample signature by mixing three standard signatures*

A linear combination of the three signatures are derived with
30% EMBRYO_9_AMP
60% LARVAE_10_AMP
10% LARVAE_10_MUT

This signature is then analyzed by deconvolution to determine the content of each sequence in the sample

*Generate mixed signature (Initialization)*

```
(* PARAMETERS *)
Print["PARAMETERS"];
a = 0.3;
b = 0.6;
c=(1-a-b);
Print["Amount of ", sequenceName[[1]], ": ", a];
Print["Amount of ", sequenceName[[2]], ": ", b];
Print["Amount of ", sequenceName[[3]], ": ", c];
timeStart = TimeUsed[];
std1 = Table[0, {j, 1, 20*(4^7)}];
std1 = Flatten[meltDataAll[[1]]];
test = Table[meltDataAll[[1, j, 1]], {j, 1, 4^7}];
totalsignal = Sum[test[[i]], {i, 1, 4^7}];
nrofspots = 4^7 - Count[test, 0];
averagesignal = totalsignal/nrofspots;
maxsignal = Max[test];
minsignal = Min[test];
Print["Standard 1: ", "Number of spots: ", N[nrofspots, 0], " = ",
 N[100*nrofspots/4^7, 2], "% of chip"];
Print[" ", "Average signal: ", averagesignal, " Range: ",
 minsignal, " - ", maxsignal];
std2 = Table[0, {j, 1, 20*(4^7)}];
std2 = Flatten[meltDataAll[[2]]];
test = Table[meltDataAll[[2, j, 1]], {j, 1, 4^7}];
totalsignal = Sum[test[[i]], {i, 1, 4^7}];
nrofspots = 4^7 - Count[test, 0];
averagesignal = totalsignal/nrofspots;
```

```
maxsignal = Max[test];
minsignal = Min[test];
Print["Standard 1: ", "Number of spots: ", N[nrofspots, 0], " = ",
  N[100*nrofspots/4^7, 2], "% of chip"];
Print[" ", "Average signal: ", averagesignal, " Range: ",
  minsignal, " - ", maxsignal];
std3 = Table[0, {j, 1, 20*(4^7)}];
std3 = Flatten[meltDataAll[[3]]];
test = Table[meltDataAll[[3, j, 1]], {j, 1, 4^7}];
totalsignal = Sum[test[[i]], {i, 1, 4^7}];
nrofspots = 4^7 - Count[test, 0];
averagesignal = totalsignal/nrofspots;
maxsignal = Max[test];
minsignal = Min[test];
Print["Standard 1: ", "Number of spots: ", N[nrofspots, 0], " = ",
 N[100*nrofspots/4^7, 2], "% of chip"];
Print["     ", "Average signal:    ", averagesignal, " Range: ",
 minsignal, " - ", maxsignal];
Print[" "];
mixData = Table[a*std1[[j]] + b*std2[[j]] + c*std3[[j]], {j, 1, 20*(4^7)}];
test = Table[mixData[[1 + (j - 1)*20]]/100, {j, 1, 4^7}];
title = StringJoin["Mixed signal (x=", ToString[a], " y=", ToString[b], " z=",
   ToString[c], ") at 12 degC"];
ListDensityPlot[Partition[test, 128], ColorFunctionScaling -> False,
PlotLabel -> title, TextStyle -> {FontFamily -> "Times", FontSize -> 12},
ImageSize -> {427, 450}];
```

PARAMETERS
Amount of EMBRYO_9_AMP: 0.3
Amount of LARVAE_10_AMP: 0.6
Amount of LARVAE_10_MUT: 0.1

| | | |
|---|---|---|
| Standard 1: | Number of spots: | 2335. = 14.2517% of chip |
| | Average signal: | 119.541 Range: 0 - 1517.17 |
| Standard 1: | Number of spots: | 2414. = 14.7339% of chip |
| | Average signal: | 118.461 Range: 0 - 1517.17 |
| Standard 1: | Number of spots: | 2398. = 14.6362% of chip |
| | Average signal: | 118.723 Range: 0 - 1517.17 |

Figure 35 illustrates mixed signal (x=0.3 y=0.6, z=0.1) at 12˚C.

*Deconvolve mixed signature*

```
timestart = TimeUsed[];
inData = Tabte[{std1[[j]], std2[[j]], std3[[j]], mixData[[j]]}, {j, 1,
    20*(4^7)}];
result = Fit[inData, {x, y, z}, {x, y, z}];
a1 = Coefficient[result, x];
b1 = Coefficient[result, y];
c1 = Coefficient[result, z];
Print["DECONVOLUTION RESULTS"];
Print["Amount of ", sequenceName[[1]], ": ", a1, " Error: ", a - a1, " = ",
   100*(a - a1)/a, "%"];
Print["Amount of ", sequenceName[[2]], ": ", b1, " Error: ", b - b1, " = ",
  100*(b - b1)/b, "%"];
Print["Amount of ", sequenceName[[3]], ": ", c1, " Error: ", c - c1, " = ",
  100*(c - c1)/C, "%"];
timeStop = TimeUsed[];
```

```
Print["Time used for calculation: ", timeStop - timeStart, " Seconds"];
Clear[inData];
```

DECONVOLUTION RESULTS

| | |
|---|---|
| Amount of EMBRYO_9_AMP: | 0.3 Error: 3.56937' $10^{-14}$ = 1.18979' $10^{-11}$% |
| Amount of LARVAE_10_AMP: | 0.6 Error: - 8.67084' $10^{-14}$ = -1.44514' $10^{-11}$% |
| Amount of LARVAE_10_MUT: | 0.1 Error: -5.27356' $10^{-15}$ = -5.27356' $10^{-12}$% |
| Time used for calculation: | 4.717 Seconds |

*Analysis of a calculated mixed signature with various amounts of noise*

Three types of noise are added to the standard signatures as well as the mixed signature prior to amnalysis

A worst case scenario is deliniated below where each parameter is 5 - 10 x the expected experimental value.

A) Fluorescent spots & dust. Slide dependent

- Present at particular positions for a given slide regardless of temperatures.
- Intensity up to 3x average intensity of all spots at 12˚C
- Affects 1% of all spots. These are randomly selected.

B) Spotvariation. Differences in the amount of capture probe on a slide depend on spotting & coating.

- Factor to be multiplied onto spot signal for any given temperature.
- Factor depend on spotposition not temperature
- A normal distribution with SD being +/- 20% of spot intensity

C) Measurement error. This error differe between measurements (i.e. temperatures)

- Absolute component. White noise with an amplitude of 10% of average spot intensity for all spots
- Relative component. A normal distribution with SD being +/-5% of spot intensity

```
«Statistics' Normal Distribution
(* PARAMETERS *)
dustarea = 0.01;
dustamplitude = 3*averagesignal;
spotvariation = 0.20;
measurementAbsolute = 0.10*averagesignal;
measurementRelative = 0.05;
Print["PARAMETERS"];
Print["A) FLUORESCENT PARTICLES (konstant position, absolute)"];
Print[" Coverage      ", 100*dustarea, "% of all spots"];
Print[" Amplitude up to ", dustamplitude/averagesignal,
 "x average spot intensity (Even dist)"];
Print["B) SPOT VARIATION (konstant position, relative to spot signal)"];
Print[" Relative amplitude ", 100*spotvariation,
 "% of spot signal (Normal dist +/- SD)"];
Print["C) MEASUREMENT ERROR (variable position, constant and relative \
component)"];
Print[" Absolute up to   ", measurementAbsolute, " = ",
100*measurementAbsolute/averagesignal, "% of average spot (Even dist)"];
Print[" Relative amplitude ", 100*measurementRelative,
 "% of spot signal (Normal dist +/- SD)"];
Print[" "];
Print["CALCULATE NOISE DATA"];
timeStart = TimeUsed[];
noise = Table[{If[Random[] < dustarea, Random[]*dustamplitude, 0],
    Random[NormalDistribution[1, spotvariation]]}, {j, 1, 4^7}];
std1N = Table[
```

```
   noise[[Ceiling[j/20], 1]] +
    std1[[j]]*noise[[Ceiling[j/20], 2]]*
     Random[NormalDistribution[1, measurementRelative]] +
    measurementAbsolute*Random[], {j, 1, 20*4^7}];
averagenoise =
 Sum[Abs[std1[[i]] - std1N[[i]]], {i, 1, Length[stdl]}]/Length[stdl];
Print["Average Noise of std1 =", averagenoise, " = ",
 100*averagenoise/averagesignal, " % of average spot = ",
 100*averagenoise*4^7/(averagesignal*nrofspots), "% of total signal"];
noise = Table[(If[Random[] < dustarea, Random[]*dustamplitude, 0],
    Random[NormalDistribution[1, spotvariation]]}, {j, 1, 4^7}];
std2N = Table[
  noise[[Ceiling[j/2o], 1]] +
    std2[[j]]*noise[[Ceiling[j/20], 2]]*
     Random[NormaIDistribution[1, measurementRelative]] +
    measurementAbsolute*Random[], {j, 1, 20*4^7}];
averagenoise =
 Sum[Abs[std2[[i]] - std2N[[i]]], {i, 1, Length[std1}]/Length[std1];
Print["Average Noise of std2 =", averagenoise, " = ",
 100*averagenoise/averagesignal, " % of average spot = ",
 100*averagenoise*4^7/(averagesignal*nrofspots), "% of total signal"];
noise = Table[(If[Random[] < dustarea, Random[]*dustamplitude, 0],
    Random[NormalDistribution[1, spotvariation]]}, {j, 1, 4^7}];
std3N = Table[
   noise[[Ceiling[j/20], 1]] +
    std3[[j]]*noise[[Ceiling[j/20], 2]]*
     Random[NormalDistribution[1, measurementRelative]] +
    measurementAbsolute*Random[], {j, 1, 20*4^7}];
averagenoise =
 Sum[Abs[std3[[i]] - std3N[[i]]], {i, 1, Length[std1}]/Length[std1];
Print["Average Noise of std3 =", averagenoise, " = ",
  100*averagenoise/averagesignal, " % of average spot = ",
  100*averagenoise*4^7/(averagesignal*nrofspots), "% of total signal"];
noise = Table[{If[Random[] < dustarea, Random[]*dustamplitude, 0],
    Random[NormalDistribution[1, spotvariation]]}, {j, 1, 4^7}];
mixDataN =
 Table[noise[[Ceiling[j/20], 1]] +
    mixData[[j]]*noise[[Ceiling[j/20], 2]]*
     Random[NormalDistribution[1, measurementRelative]] +
    measurementAbsolute*Random[], {j, 1, 20*4^7}];
averagenoise =
  Sum[Abs[mixData[[i]] - mixDataN[[i]]], {i, 1, Length[std1]}]/
   Length[std1];
Print["Average Noise of mix =", averagenoise, " = ",
  100*averagenoise/averagesignal, " % of average spot = ",
  100*averagenoise*4^ 7/(averagesignal*nrofspots), "% of total signal"];
timeStop = TimeUsed[];
Print["Time used for calculation: ", timeStop - timeStart, " Seconds"];
Print[" "];
timeStart = TimeUsed[];
inData = Table[{std1N[[j]], std2N[[j]], std3N[[j]], mixDataN[[j]]}, {j, 1,
    20*(4^7)}];
result = Fit[inData, {x, y, z}, {x, y, z}];
a1 = Coefficient[result, x];
b1 = Coefficient[result, y];
c1 = Coefficient[result, z];
Print["DECONVOLUTION RESULTS"];
Print["Amount of ", sequenceName[[1]], ": ", a1, " Error: ", Abs[a - a1],
  " = ", 100*Abs[a - a1]/a, "%"];
Print["Amount of ", sequenceName[[2]], ": ", b1, " Error: ", Abs[b - b1],
  " = ", 100*Abs[b - b1]/b, "%"];
Print["Amount of ", sequenceName[[3]], ": ", c1, " Error: ", Abs[c - c1],
  " = ", 100*Abs[c - c1]/c, "%"];
```

```
timeStop = TimeUsed[];
Print["Time used for calculation: ", timeStop - timeStart, " Seconds"];
Clear[inData];
```

CALCULATE NOISE DATA

Average Noise of std1 =8.66969 = 7.30242 % of average spot = 49.8928% of total signal
Average Noise of std2 =8.88071 = 7.48017 % of average spot = 51.1072% of total signal
Average Noise of std3 =8.76169 = 7.37991 % of average spot = 50.4222% of total signal
Average Noise of mix =8.78766 = 7.40179 % of average spot = 50.5717% of total signal
Time used for calculation: 111.09 Seconds

DECONVOLUTION RESULTS

| | |
|---|---|
| Amount of EMBRYO_9_AMP: | 0.306417 Error: 0.0064168 = 2.13893% |
| Amount of LARVAE_10_AMP: | 0.327182 Error: 0.272818 = 45.4696% |
| Amount of LARVAE_10_MUT: | 0.297622 Error: 0.197622 = 197.622% |

*Results of Noice evaluations*

| | |
|---|---|
| No noice: | Dust 0.0% 2x, Spotting +/- 0%, Measure +/-0% + 0 |
| Only dust: | Dust 0.1% 2x, Spotting +/- 0%, Measure +/-0% + 0 |
| Only spot: | Dust 0.0% 2x, Spotting +/- 5%, Measure +/-0% + 0 |
| Only measure: | Dust 0.1% 2x, Spotting +/- 0%, Measure +/-2% + 3 |
| Standard: | Dust 0.1% 2x, Spotting +/- 5%, Measure +/-2% + 3 |
| 2x Standard: | Dust 0.2% 2x, Spotting +/- 10%, Measure +/-4% + 6 |
| 5x Standard: | Dust 0.5% 2x, Spotting +/- 20%, Measure +/-5% + 10 |
| Worst case: | Dust 1.0% 3x, Spotting +/- 20%, Measure +/-5% + 10 => More than 50% of total signal is noice! |
| Standard: | Dust 0.1% 2x, Spotting +/- 5%, Measure +/-2% + 3 |

Example 8A: Reversible binding of targets to heptamer probes

Reversible binding of targets to a heptamer probe array was demonstrated using the setup described in Example 7, with the test array shown in Figure 28.

The hybridization solution contained (5xSSCT 750 mM NaCl, 75 mM Sodium Citrate, pH 7.2, 0.05% Tween) and 10 mM $MgCl_2$. The final target concentration in the hybridization solution was 0.01 μM. The target was a 45-mer oligonucleotide with a Cy3 fluorescent label at the 5' end. The target sequence is: 5'-Cy3-ttaccagtaccttttcaaatcgattctcaattcaaattcatcaaa-3' (SEQ ID NO: 11). A hybridization chamber was created by attaching a CoverWell™ gasket to the Immobilizer slide and filling it with 200 μL hybridization solution with target. The slide was immediately observed with a Zeiss Axioplan 2 epifluorescence microscope with a 5x Fluar objective and a Cy5 filterset from OMEGA. The temperature of the microscope stage was controlled with a Peltier element. Thirty-five images at each temperature were acquired automatically with a Photometrics camera, automated shutter, and motorized microscope stage. The images were acquired, stitched together, calibrated and stored in stack by the software package "MetaVue".

Reversibility of binding was tested with a synthetic oligonucleotide (45 mer) carrying a 5'-terminal Cy3 dye. Measurement was carried out in the presence of SYBR Green II.

The results depicted in Figure 38 demonstrate that on-line observation of hybridization was possible. The depicted fluorescent signals have been corrected for temperature dependent of quantum yield differences for the applied fluorophores (Cy3) by the method described in Example 5. Reversible binding of target to capture probes without the need for a washing step was observed. The binding was remarkably fast and initial annealing, thermal dissociation and subsequent re-annealing could be observed within less than two hours. The background signal generated by DNA capture probes was not influenced by the temperature increase and subsequent decrease. The low signal was hardly distinguishable from the background fluorescence due to unbound target and can most likely be ascribed to optical artifacts arising from residual crystals from the spotting buffer. The LNA heptamer probes on the other hand produced a clear signal that decreased at increasing temperatures but increased reproducibly after the subsequent cooling to the original temperature.

Example 8B: Array construction and handling

In the following examples 8C - 8F we present our results using a simplified version of the Universal LNA Array. This test version only contains 280 LNA enhanced capture probes and 92 DNA capture probes spotted 4 times on each slide. These were spotted on standard EURAY immobilizer slides. Only measurements made after hybridization at a single temperature were used for quantitative data analysis in the following examples.

It should be stressed that the future commercial version of the chip should include 1200 different capture probes spotted in triplicates. The data analysis could be further optimized by observation at eight consecutive temperatures in a specialized scanner. We have demonstrated the possibility of manufacturing such a scanner inexpensively using a commercial digital camera, LED light source, a Peltier element and customized filtersets.

*Synthesis of capture probes*

The capture probes were synthesized with a 5' anthraquinone (AQ) group for covalent photochemical attachment to the slide surface. Each capture probe also contained a $dT_{10}$-linker (i.e. ten DNA thymidine residues), followed by five non-bases ($nb_5$) which are phosphate and sugar moieties without any attached nucleobase. The non-base phosphoamidites were purchased from Glen Research Corporation, Sterling, VA, USA The sequence specific heptamer capture sequence was attached to the 3' end of the non-base linker. The complete sequence of the immobilized capture probes were thus: (SEQ ID NO: 12) 5'-AQ-t-t-t-t-t-t-t-t-t-t-nb-nb-nb-nb-nb-XXXXXXX-3', where XXXXXXX represent the exposed specific capture sequence. The presence of the non-base were intended to reduced any possible sequence bias due to the $dT_{10}$-linker. The chosen subset of all possible heptamer sequences were selected to be as diverse as possible and each contained 3 to 6 LNA nucleotides (average 4.6). The chosen LNA substitution patterns were sequence dependent for each heptamer in order to eliminate self complementarity (Example 6) and ensure similar melting behavior for all capture probes. 94 heptamer capture probes were synthesized in two versions with the same nucleobase sequence: 1) an LNA enhanced version with 3-6 LNA nucleotides and 2) a plain DNA version without LNA. Comparing the hybridization result of these two versions would enable us to quantify the effect of using LNA in short capture probes. For efficient orientation on the slide we also included a number of fluorescently labeled reference probes. The reference probes were synthesized with a 5' AQ group followed by a $dT_{10}$-linker and a 3' terminal fluorophore i.e. Cy3 or Cy5. All probes were purified using OASIS cartridges from Waters, USA according to the manufacturer's guidelines. The yield was determined by UV absorbance with a UV-spectrophotometer, NanoDrop ND-1000 (NanoDrop, USA). This instrument was also used to adjust capture probe concentration prior to spotting and to determine the target concentration in hybridization experiments.

*Array production*

All capture probes were spotted on EURAY Immobilizer polymer slides according to the standard protocol provided by Exiqon for use of these slides.

The 384 capture probes (280 LNA probes + 94 DNA capture probes + 10 labelled reference probes, "Landing lights") were spotted four times on each array with a pitch of 250 $\mu$m, and a spot volume of 300 pl. Standard Immobilizer spotting buffer was used and a capture probe concentration of 40 $\mu$M. The slides were hydrated overnight in a hydration chamber and UV illuminated (StrataLinker 2400, Stratagene, CA, USA, using UV light: 254 nm with an energy input of 2300 $\mu$J) to ensure covalent linkage of the capture probes to the polymer slide. The slides were briefly rinsed in 1x SSCT (150 mM NaCl, 15 mM Sodium Citrate, pH 7.2, 0.05% Tween) after illumination to remove unbound probe.

*Array layout*

The array layout for each of the four replicates areas containing 384 spots is shown in Figure 36. The sequence of each capture probe is listed in the table below. Each capture probe was covalently linked to the slide surface by through an AQ group

For the listed sequences: upper case letters denote LNA units and lower case letters DNA units. mC is a methyl-C LNA unit.

| Name | Sequence | Name | Sequence | Name | Sequence | Name | Sequence | Name | Sequence |
|---|---|---|---|---|---|---|---|---|---|
| MIAL-1c1 | AAmCTmCTg | LNA-53 | AGmCAmCGg | LNA-133 | GAATAAc | LNA-213 | gmCmCATmCa | DNA-41 | cggcggt |
| MIAL-1c2 | AmCTcTGa | LNA-54 | mCAGAGAa | LNA-134 | tcgAmCgmC | LNA-214 | mCmCgAtcc | DNA-42 | tctacag |
| MIAL-1c3 | mCTcTGac | LNA-55 | GAAmCmCTa | LNA-135 | mCmCAGTAg | LNA-215 | GTTGTTc | DNA-43 | cccctgc |
| MIAL-1c4 | tcTGAmCT | LNA-56 | cGGAAAc | LNA-136 | TAAATGc | LNA-216 | ctAGmCmCmC | DNA-44 | gctcagt |
| MIAL-1c5 | mCTGAmCTg | LNA-57 | mCAATmCmCt | LNA-137 | mCAmCmCmCTt | LNA-217 | GtTGTGa | DNA-45 | gtctatc |
| MIAL-1c6 | TGaCTGg | LNA-58 | GcTAmCAg | LNA-138 | AmCAAAGT | LNA-218 | mCmCmCgAAt | DNA-46 | tgaaaat |
| MIAL-1c7 | GAcTGga | LNA-59 | ATmCGmCmCc | LNA-139 | GmCTAmCmCc | LNA-219 | mCTAmCATt | DNA-47 | ccaacgc |
| MIAL-1g1 | AAmCTmCTc | LNA-60 | GTmCATmCc | LNA-140 | AAGAmCAc | LNA-220 | aTTATmCmC | DNA-48 | cccgcgc |
| MIAL-1g2 | AmCTmCTmCa | LNA-61 | GgmCAmCmCg | LNA-141 | mCmCmCTAAg | LNA-221 | gGcGGGg | DNA-49 | gggagat |
| MIAL-1g3 | cTmCTmCAc | LNA-62 | mCmCTcTGa | LNA-142 | TaTGGAt | LNA-222 | mCmCmCmCcac | DNA-50 | atcatag |
| MIAL-1g4 | TmCTmCAmCt | LNA-63 | AAATmCTg | LNA-143 | GcTAmCTa | LNA-223 | TamCmCgTc | DNA-51 | cgtgaag |
| MIAL-1g5 | mCTmCAmCTg | LNA-64 | TTmCmCAGa | LNA-144 | mCAmCmCTTa | LNA-224 | cTAAgmCmC | DNA-52 | aagcaaa |
| MIAL-1g6 | TmCamCTgg | LNA-65 | AmCTGagg | LNA-145 | tAAtAAG | LNA-225 | GTmCcTmCa | DNA-53 | agcacgg |
| MIAL-1g7 | mCAmCtggA | LNA-66 | mCgTmCcGc | LNA-146 | TcmCGmCmCa | LNA-226 | AGAGAmCg | DNA-54 | cagagaa |
| MIAL-2t1 | cTTgmCmCa | LNA-67 | mCmCamCmCmCt | LNA-147 | GAmCAGca | LNA-227 | cAmCmCTAt | DNA-55 | gaaccta |
| MIAL-2t2 | ttgmCmCAT | LNA-68 | TATTmCAg | LNA-148 | TmCmCcAtg | LNA-228 | AGAtTGt | DNA-56 | cggaaac |
| MIAL-2t3 | tgmCmCATc | LNA-69 | GcgTAAA | LNA-149 | mCaTmCAtc | LNA-229 | mCAmCmCmCTc | DNA-57 | caatcct |
| MIAL-2t4 | GmCmCATmCt | LNA-70 | GmCGAGgt | LNA-150 | mCmCagcTT | LNA-230 | GTcGTGc | DNA-58 | gctacag |
| MIAL-2t5 | mCmCATmCTt | LNA-71 | AATAGAg | LNA-151 | AGAGTtg | LNA-231 | AGTGAGa | DNA-59 | atcgccc |
| MIAL-2t6 | mCATmCTTc | LNA-72 | mCTmCAmCGa | LNA-152 | GAAAGAa | LNA-232 | ArnCAGGae | DNA-60 | gtcatcc |
| MIAL-2t7 | ATmCTTmCc | LNA-73 | ATTTAmCT | LNA-153 | cmCAmCmCGa | LNA-233 | TTGGAAt | DNA-01 | ggcaccg |
| MIAL-2c1 | mCtTGmCmCg | LNA-74 | TATmCmCAc | LNA-154 | GGggTGt | LNA-234 | GTTmCgTg | DNA-62 | cctctga |
| MIAL-2c2 | TTgmCmCgt | LNA-75 | mCTTGgmCt | LNA-155 | gcAgmCmCt | LNA-235 | tcTGAmCT | DNA-63 | aaatctg |
| MIAL-2c3 | TgmCmCgTc | LNA-76 | mCTmCTGTc | LNA-156 | mCGmCmCtAg | LNA-236 | AGTGcmCT | DNA-64 | ttccaga |
| MIAL-2c4 | gmCmCgTmCt | LNA-77 | GmCmCmCTgg | LNA-157 | GmCmCmCATa | LNA-237 | AmCgAmCAt | DNA-65 | actgagg |
| MIAL-2c5 | mCmCGTmCTt | LNA-78 | GGGGatg | LNA-158 | TmCTTmCAg | LNA-238 | mCgTATmCt | DNA-66 | cgtccgc |
| MIAL-2c6 | mCgTmCTTc | LNA-79 | ATTmCgmCt | LNA-159 | mCTmCAcgt | LNA-239 | GTGtAAc | DNA-67 | ccaccct |
| MIAL-2c7 | GTmCTTmCc | LNA-80 | AmCmCAGmCc | LNA-160 | AGGaTGc | LNA-240 | TAAmCTmCt | DNA-68 | tattcag |
| LNA-1 | TAgmCmCAt | LNA-81 | GccTGga | LNA-161 | tmCmCgAAA | LNA-241 | TAAGTmCg | DNA-69 | gcgtaaa |
| LNA-2 | TtmCmCmCmCa | LNA-82 | TAAgmCmCc | LNA-162 | mCGGgmCAg | LNA-242 | mCTTGaTt | DNA-70 | gcgaggt |
| LNA-3 | AAmCmCTAt | LNA-83 | mCcAtgmCc | LNA-163 | mCGmCmCmCtt | LNA-243 | TGTGaGg | DNA-71 | aatagag |
| LNA-4 | mCtATGAc | LNA-84 | amCmCmCGmCt | LNA-164 | mCAAATAg | LNA-244 | AATGaTG | DNA-72 | ctcacga |
| LNA-5 | mCAAtgmCT | LNA-85 | AmCTmCmCTg | LNA-165 | mCAtcgAG | LNA-245 | cGGcGGt | DNA-73 | atttact |
| LNA-6 | AAGGAmCt | LNA-86 | TAGtATmC | LNA-166 | aGTTmCTmC | LNA-246 | TmCTAmCAg | DNA-74 | tatccac |

(continued)

| Name | Sequence | Name | Sequence | Name | Sequence | Name | Sequence | Name | Sequence |
|---|---|---|---|---|---|---|---|---|---|
| LNA-7 | acGATGa | LNA-87 | ATmCTmCgc | LNA-167 | mCGmCmCcTa | LNA-247 | cmCmCmCTGc | DNA-75 | cttggct |
| LNA-8 | TTAmCgmCc | LNA-88 | TGTTTGc | LNA-168 | AmCmCATmCt | LNA-248 | GcTmCagt | DNA-76 | ctctgtc |
| LNA-9 | gmCmCATmCa | LNA-89 | TAmCmCcGa | LNA-169 | mCGmCcTmCt | LNA-249 | GTmCTAtc | DNA-77 | gccctgg |
| LNA-10 | mCmCgAtcc | LNA-90 | mCATmCTTg | LNA-170 | TGTcgTt | LNA-250 | TGAAAAt | DNA-78 | ggggatg |
| LNA-11 | GTTGTTc | LNA-91 | AAmCTmCAa | LNA-171 | cTGagAt | LNA-251 | mCmCaAmCGc | DNA-79 | attcgct |
| LNA-12 | ctAGmCmCmC | LNA-92 | mCmCGmCAtc | LNA-172 | TmCmCAAAg | LNA-252 | mcmccgcgc | DNA-80 | accagcc |
| LNA-13 | GtTGTGa | LNA-93 | mCacgTmCg | LNA-173 | tAAtAGg | DNA-1 | tagccat | DNA-81 | gcctgga |
| LNA-14 | mCmCmCgAAt | LNA-94 | AmCgAGac | LNA-174 | mCgTcgGg | DNA-2 | ttcccca | DNA-82 | taagccc |
| LNA-15 | mCTAmCATt | LNA-95 | TGcGTTmC | LNA-175 | mCmCTTmCAa | DNA-3 | aacctat | ONA-83 | ccatgcc |
| LNA-16 | aTTATmCmC | LNA-96 | AmCAGAAa | LNA-176 | mCGaGmCmCt | DNA-4 | ctatgac | DNA-84 | acccgct |
| LNA-17 | gGcGGGg | LNA-97 | AmCTTmCmCt | LNA-177 | AmCAmCAtc | DNA-5 | caatgct | DNA-85 | actcctg |
| LNA-18 | mCmCmCmCcac | LNA-98 | GAmCTAtg | LNA-178 | GAGAtTg | DNA-6 | aaggact | DNA-86 | tagtatc |
| LNA-19 | TamCmCgTc | LNA-99 | AGmCAGGg | LNA-179 | mCAAGGTc | DNA-7 | acgatga | DNA-87 | atctcgc |
| LNA-20 | cTAAgmCmC | LNA-100 | TTmCTAAc | LNA-180 | mCmCgcAgc | DNA-8 | ttacgcc | DNA-88 | tgtttgc |
| LNA-21 | GTmCcTmCa | LNA-101 | TATTGTc | LNA-181 | TTTmCAmCc | ONA-9 | gccatca | DNA-89 | tacccga |
| LNA-22 | AGAGAmCg | LNA-102 | mCgtAAmCT | LNA-182 | TGATGgg | DNA-10 | ccgatcc | DNA-90 | catcttg |
| LNA-23 | cAmCmCTAt | LNA-103 | ggmCTmCmCa | LNA-183 | GaAATAg | DNA-11 | gttgttc | DNA-91 | aactcaa |
| LNA-24 | AGAtTGt | LNA-104 | aGTTTTmC | LNA-184 | GTGTmCTa | DNA-12 | ctagccc | DNA-92 | ccgcatc |
| LNA-25 | mCAmCmCmCTc | LNA-105 | TgcgTmCa | LNA-185 | mCTTmCmCmCa | DNA-13 | gttgtga | DNA-93 | cacgtcg |
| LNA-26 | GTcGTGc | LNA-106 | gGGGGTt | LNA-186 | GgcTAAc | DNA-14 | cccgaat | DNA-94 | acgagac |
| LNA-27 | AGTGAGa | LNA-107 | cAGGAGa | LNA-187 | TmCmCATAa | DNA-15 | ctacatt | Reference | Cy3 |
| LNA-28 | AmCAGGac | LNA-108 | GGGAcAg | LNA-188 | AmCAmCAmCc | DNA-16 | attatcc | Reference | Cy3 |
| LNA-29 | TTGGAAt | LNA-109 | AGtTmCgc | LNA-189 | GcAAGcg | DNA-17 | ggcgggg | Reference | Cy3 |
| LNA-30 | GTTmCgTg | LNA-110 | AGGTGgg | LNA-190 | GaAtcTG | DNA-18 | cccccac | Reference | Cy3 |
| LNA-31 | tcTGAmCT | LNA-111 | AmCTmCTGc | LNA-191 | cGAGAta | DNA-19 | taccgtc | Reference | Cy5 |
| LNA-32 | AGTGcmCT | LNA-112 | GcAmCATa | LNA-192 | GGAcTGt | DNA-20 | ctaagcc | | |
| LNA-33 | AmCgAmCAt | LNA-113 | AcTGgAg | LNA-193 | mCGmCTGac | DNA-21 | gtcctca | | |
| LNA-34 | mCgTATmCt | LNA-114 | AGtcgAG | LNA-194 | TTmCAGca | DNA-22 | agagacg | | |
| LNA-35 | GTGtAAc | LNA-115 | TmCTmCTTg | LNA-195 | AGAmCGmCc | DNA-23 | cacctat | | |
| LNA-36 | TAAmCTmCt | LNA-116 | GGaGAmCc | LNA-196 | TAmCATmCa | DNA-24 | agattgt | | |
| LNA-37 | TAAGTmCg | LNA-117 | AGGTTGa | LNA-197 | TAmCTmCgc | DNA-25 | caccctc | | |
| LNA-38 | mCTTGaTt | LNA-118 | mCAGAAGc | LNA-198 | GGgTGAa | DNA-26 | gtcgtgc | | |
| LNA-39 | TGTGaGg | LNA-119 | GgcTTmCc | LNA-199 | TmCmCAGag | DNA-27 | agtgaga | | |

(continued)

| Name | Sequence | Name | Sequence | Name | Sequence | Name | Sequence | Name | Sequence |
|---|---|---|---|---|---|---|---|---|---|
| LNA-40 | AATGaTG | LNA-120 | cTTGcTg | LNA-200 | mCAAmCAGc | DNA-28 | acaggac | | |
| LNA-41 | cGGcGGt | LNA-121 | mCAmCmCAGt | LNA-201 | AmCAtAmCc | DNA-29 | ttggaat | | |
| LNA-42 | TmCTAmCAg | LNA-122 | TmCTmCTTc | LNA-202 | mCmCgTAAc | DNA-30 | gttcgtg | | |
| LNA-43 | cmCmCmCTGc | LNA-123 | gaTmCGmCmC | LNA-203 | gmCgGGGc | DNA-31 | tctgact | | |
| LNA-44 | GcTmCagt | LNA-124 | GcATTTA | LNA-204 | ATTAmCAg | DNA-32 | agtgcct | | |
| LNA-45 | GTmCTAtc | LNA-125 | AAAmCAAA | LNA-205 | TAgmCmCAt | DNA-33 | acgacat | | |
| LNA-46 | TGAAAAt | LNA-126 | mCAAGAAt | LNA-206 | TtmCmCmCmCa | DNA-34 | cgtatct | | |
| LNA-47 | mCmCaAmCGc | LNA-127 | GAGtcgA | LNA-207 | AAmCmCTAt | DNA-35 | gtgtaac | | |
| LNA-48 | mCmCcgcgc | LNA-128 | GTmCgTTa | LNA-208 | mCtATGAc | DNA-36 | taactct | | |
| LNA-49 | GGGagAt | LNA-129 | GTTmCmCAa | LNA-209 | mCAAtgmCT | DNA-37 | taagtcg | | |
| LNA-50 | ATmCAtAG | LNA-130 | cGTTGcc | LNA-210 | AAGGAmCt | DNA-38 | cttgatt | | |
| LNA-51 | cGTGAAG | LNA-131 | mCmCmCgTTt | LNA-211 | acGATGa | DNA-39 | tgtgagg | | |
| LNA-52 | AAGcAAA | LNA-132 | ATmCTAtc | LNA-212 | TTAmCgmCc | DNA-40 | aatgatg | | |

*Array hybridization*

Hybridizations with a final target concentration of 1 ng/μl were carried out in 13x SSC (1950 mM NaCl, 195 mM Tris HCl, pH 7.2) with 6.5 mM $MgCl_2$ and 0.1 % Tween overnight at 4 ˚C unless otherwise noticed. 20 μl of hybridization solution with target was applied to each microarray slide and covered with a 50 x 24 mm coverslip. The slide was the placed in a hydration chamber at 4 ˚C overnight. The slides were subsequently washed 5 min in 5x SSCT (750 mM NaCl, 75 mM Tris HCl, pH 7.2) with 2.5 mM $MgCl_2$ at 4 ˚C. Excess wash solution was removed by centrifugation at 2000 rpm for 2 min at 10 ˚C.

*Scanning and data analysis*

For the experiments described in example 8C to 8F the slides were scanned in an ArrayWorx scanner using appropriate filters (i.e. Cy3 or Cy5), scan times (1 to 4 sec) and maximum resolution (5 μm). Several individual pictures were stitched together to produce a composite image of the whole array. Subsequent image analysis was made with ArrayVision version 6.0 rev. 3. Spot intensities were quantified on a volume basis after subtraction of the surrounding background fluorescence (= sVOL). The measured intensity values were transferred to *Mathematica* version 4.0, Wolfram Research Inc, Urbana, Illinois, USA, for more complex analysis. Our custom-made programs for this purpose include scaling and initial data filtering using different types of median filters to eliminate erroneous noise due to random fluorescent particles, and small slide to slide variations.. The corrected intensity values were then depicted graphically as a "barcode" diagram (e.g. Figure 39 or Figure 44B). The barcode for each experiment is the measured intensity for each of the 280 different capture probes depicted as six horizontal rows of dots with a shading proportional to the measured intensity value (high intensity represented by a dark shading). The first four rows correspond to the intensity value measured for each of the four replicates of the 280 capture probes spotted on the array (see layout in Figure 36). The fifth row is the average intensity for the four replicates and the sixth row is the median intensity (i.e. average of the two remaining measurements after removing the highest and lowest value of the four). We have found that the resulting barcodes give excellent visual feedback about the relative similarity of hybridization patterns. Usually barcodes are shown next to each other to facilitate comparison. An example is shown in Figure 39 which contains barcodes from ten different hybridization experiments (i.e. 10 x 6 horizontal rows). It is evident that the hybridization pattern of the first five experiments (row 1 to 30) is markedly different from the hybridization pattern of the latter five experiments (row 31 to 60). The individual barcodes (i.e. graphics representation of the signatures) are compared pair-wise by computing the sum of squared differences in intensities between different measurements. The pair-wise distances form the basis of a similarity matrix where low values correspond to a large similarity between hybridization patterns. The similarity matrix was then depicted as a distance tree using the FITCH algorithm in the PHYLIP package. The distance tree was drawn with the program DRAWTREE also from the PHYLIP package

Example 8C: Binding of target to LNA and DNA heptamer capture probes

The simple test array described in Example 8B was used to demonstrate the superior performance of LNA enhanced heptamer capture probes compared to similar DNA capture probes. Splice variants of the LET2 gene from the nematode *Caenorhabditis elegans* were cloned from embryonic and larval mRNA after initial rt-PCR amplification. Random clones were sequences to identify a clone with each of the two splice variants. Clones with the following two sequences were obtained:

**Embryo_9** containing Exon 7, 8, 9, 11 and 12. The splice variant amplified by appropriate primers is 542 bp long and believed to be expressed in the embryo of *C. elegans.* The sequence (SEQ ID NO: 9) is:

CTCCAGGAGAGAAGGGAGATGGCGGTATGCCAGGAATGCCCGGACTTCCAGGACCATCCGGTCGTG
ATGGATACCCAGGAGAAAAGGGAGACCGAGGAGATACTGGAAATGCTGGACCACGTGGACCACCTG
GAGAGGCTGGATCCCCAGGAAACCCAGGAATCGGAAGCATTGGACCAAAAGGAGATCCTGGAGA*TCT*
*AGGTTCTGTCGGACCACCAGGTCCACCGGGACCACGTGAGTTCACCGGATCCGGCTCAATTGTCGGA*
*CCTCGCGGAAACCCTGGAGAAAAGGGAGAC*AAGGGAGAGCCAGGAGAGGGAGGTCAACGCGGTTAC
CCAGGAAATGGAGGACTCTCAGGACAGCCAGGACTCCCAGGAATGAAGGGAGAAAAGGGATTGTCT
GGACCAGCTGGACCAAGAGGAAAGGAAGGTCGCCCAGGAAACGCTGGACCACCAGGATTCAAGGGA
GATCGTGGTCTTGACGGACTTGGCGGAATCCCAGGACTTCCAGGCCAAAAGGGAGAAGCTGGATACC
CAGGAAGAGAT

The sequence of exon 9 which is not found in the larval splice variant is indicated by underline and italics **Larvae_10** containing Exon 7, 8, 10, 11 and 12. The splice variant amplified by appropriate primers is 545 bp long and believed to be expressed in the larvae of *C. elegans.* The sequence (SEQ ID NO: 10) is:

CTCCAGGAGAGAAGGGAGATGGCGGTATGCCAGGAATGCCCGGACTTCCAGGACCATCCGGTCGTG
ATGGATACCCAGGAGAAAAGGGAGACCGAGGAGATACTGGAAATGCTGGACCACGTGGACCACCTG
GAGAGGCTGGATCCCCAGGAAACCCAGGAATCGGAAGCATTGGACCAAAAGGAGATCCTGGAGA*CAT*
*TGGTGCGATGGGACCGGCCGGTCCGCCAGGCCCAATCGCCTCCACCATGTCCAAGGGAACCATTATC*
*GGTCCTAAGGGAGACCTAGGAGAGAAGGGAGAG*AAGGGAGAGCCAGGAGAGGGAGGTCAACGCGG
TTACCCAGGAAATGGAGGACTCTCAGGACAGCCAGGACTCCCAGGAATGAAGGGAGAAAAGGGATTG
TCTGGACCAGCTGGACCAAGAGGAAAGGAAGGTCGCCCAGGAAACGCTGGACCACCAGGATTCAAG

GGAGATCGTGGTCTTGACGGACTTGGCGGAATCCCAGGACTTCCAGGCCAAAAGGGAGAAGCTGGAT
ACCCAGGAAGAGAT

The sequence of exon 10 which is not found in the embryonic splice variant is indicated by underline and italics.

After an initial PCR amplification and purification of the cloned LET2 genes, primer extension with a Cy3 labelled primers were used to obtain single-stranded gene targets for each splice variants. The concentration of each splice variant was measured by UV absorbance with the Nanodrop UV spectrophotometer. The target concentration of each target was adjusted to a final concentration of 2 ng/μl for hybridization experiments performed as described in Example 8B above. One purpose of this study was to compare the capture efficency of LNA enhanced capture probes and DNA enhanced capture probes. Figure 37 clearly demonstrate the remarkable difference between the signal obtained with DNA capture probes (spots within the dark gray rectangle) and LNA enhanced probes with the same sequence (spots within the light gray rectangle).

The average number of probes giving positive signals in ten experiments with various mixtures of the two splice variants as targets were 11 out of 94 possible probes for the DNA heptamers (N=40), but 33 positive probes out of 94 possible for the LNA enhanced heptamers (N=40). The average probe signal was also more than 8x larger for LNA enhanced heptamers (mean signal 319934 for LNA heptamers, N=3760) than for DNA heptamers (mean signal 39903 for DNA heptamers, N=3760).

Example 8D: Abundance of different splice variants

Different mixtures containing known amounts of the two genes were investigated with the simple test array

described in Example 8B to demonstrate how an universal LNA array may be used to quantify the abundance of different genes in a sample. This demonstration is similar to the theoretical calculations in Example 8. However, the theoretical calculations shown in the example above are based on a complete heptamer chip containing all possible heptamers (i.e. 16384 probes) observed at 20 different temperatures (i.e. a total dataset of 327680 observations) for each standard and mixture of splice variants. The experimental data presented here are however, only based on four replicate observations of 280 probes at a single temperature. The number of data points acquired are thus only about 3% of the data being used for the theoretical calculations.

The splice variants used for target material are described in Example 8C above and were prepares as describe there, The two spice variants were about 540 nt long, Most of their sequence were identical except for about 20% as indicated by the underlined and italics sequence segment in Example 8C. Single-stranded labeled amplificate of each sequence was prepared as described above (Example 8C). The labeled target of the two splice variants was mixed in different ratio's so that the total target concentration was always 2 ng/μl in the hybridization mixtures. Four different slides with each of the two splice variants (2 ng/μl) were used as standards to determine the composition of twelve mixtures of the two slice variants. Each mixture was applied to a heptamer array as described in example 8B and 8C. The acquired hybridization pattern (signature) of the mixture was analyzed by comparing it to the 8 standard patterns by the method outlined in Example 2 and implemented in Example 8. Using a least squares criteria to determine the abundance of each standard in the mixture by solving 1120 equations with 8 unknowns gives the results shown in Figure 42. The expected concentration of each splice variant was based on the composition of the different mixtures, whereas the detected amount was the result of the LNA array analysis. No constraints were applied to the total concentration of target estimated from the analysis.

A remarkable correlation between the expected content of each target and the analysis result was observed for both targets (Figure 42) despite: a) the limited sequence difference between the two splice variants (< 20%); b) the analysis is only based on 280 randomly chosen LNA enhanced capture probes in four replicates observed at a single low stringency temperature.

Example 8E: Identification of five different pathogenic *Haemophilus* strains.

The simple test array described in Example 8B was further used to demonstrate a procedure for identification of five different strains of *Haemophilus* related to *Haemophilus influenza.* The identification was based on partial amplification of two common household genes whose sequence similarity was subsequently quantified based on the detected hybridization pattern (=signature) with the simple test array described in Example 8B.

*Haemophilus influenza* and several closely related species are Gram negative Gamma-Proteobacteria that can cause severe infections as human pathogens. These infections range from mild conjunctivitis, through pneumonia to (potentially lethal) meningitis. However, less virulent strains are frequently found as part of the indigenous skin micro flora on perfectly healthy individuals. Many different strains have been thus isolated and classified according to different criteria. In this study we have used the small Universal LNA array to identify and classify different isolates of *H. influenzae, H. aegyptius* and "Brazilian Purpuric Fever". The latter is a particularly virulent strain that has claimed more than 20 casualties in Brazil. DNA was isolated with the FastDNA Kit (BIO 101, USA) according to the manufacturer's instructions from five strains provided by Prof. Mogens Kilian, from the Institute for Clinical Microbiology and Immunology, University of Aarhus, Denmark. From each strain we amplified a region of about 500 nt from two different household genes:

1) the adenylate kinase, *adk,* gene using the primer sequences:

adkUP: 5'-ggtgcaccgggtgcaggtaa-3' (SEQ ID NO: 13)
adkDN: 5'-cctaagattttatctaactc-3' (SEQ ID NO: 14)

2) *recA,* a gene involved in homologous recombination, using the primers:

recAUP: 5'-atggcaactcaagaagaaaa-3' (SEQ ID NO: 15)
recADN: 5'-ttaccaaacatcacgcctat-3' (SEQ ID NO: 16)

Both amplificates were generated using a hot start PCR protocol with 2.5 mM $MgCl_2$ and an annealing temperature of 50 ˚C. The amplificate was purified with the QIAquick PCR purification kit from QIAgen according to the manufacturer's guidelines. Labelled single-stranded target was generated by a linear PCR with a single Cy3-labelled primer (i.e. Cy3-adkUP and Cy3-recAUP). The linear amplificates were likewise purified with the QIAquick kit before being used for hybridization as described in Example 8B. A target concentration of 1 ng/μl was used in all hybridization rexperiments. Five different arrays containing 280 LNA enhanced capture probes in four replicates were used to generate signatures with the *adk* amplificate and five other arrays to generate signatures with the *recA* amplificates. The hybridization patterns were recorded and analyzed as described in Example 8B. The relatively complex analysis program written in Mathematica is listed below in abbreviated form for reference purposes. It follows the general description

outlined in Example 8B.

```
Off[General : : "spell1"];
filename = "C:/Mathematica/Hybdata/LNAprobes_haemophilus.txt";
inData = ReadList[filename, Word, RecordLists -> True];
inDataN =
 Table[ToExpression[inData[[i, j]]], {i, 2, Length[inData]}, {j, 1,
    Length[inData[[1]]]}];
inDataT = Transpose[inDataN];
LNAsample = Partition[inDataT, 6];
columns = Length[inDataT];
rows = Length[inDataT[[l]]];
samples = Length[LNAsample];
Print["INPUT LNA DATA MATRIX"];
Print["Number of columns ", columns];
Print["Number of rows ", rows];
Print["Number of samples ", samples]
Print["Number of LNA probes ", Length[LNAsample[[1, 1]]]]
titles = Partition[inData[[1]], 6];
sampleNames = Table[titles[[i, 6]], {i, 1, samples}]
Print["BEFORE CALIBRATION"];
calLNAsample = Flatten[LNAsample, 1];
p0 = ListDensityPlot[calLNAsample,
   ColorFunction -> (GrayLevel[1 - #] &),
   DisplayFunction -> Identity,
   PlotLabel -> "Data matrix before calibration",
   Mesh -> False,
   AspectRatio -> 120/280];
Show[p0, DisplayFunction -> $DisplayFunction, ImageSize -> {1000, 500}];
(* PARAMETERS *)
factor = 3;
Print["AFTER MEDIAN FILTERING"];
<< Statistics DescriptiveStatistics`
Do[
  median =
   Median[{calLNAsample[[j*6 + 1, i]], calLNAsample[[j*6 + 2, i]],
      calLNAsample[[j*6 + 3, i]], ca[LNAsample[[j*6 + 4, i]]}];
  If[calLNAsample[[j*6 + 1, i]] > factor*median ||
    calLNAsample[[j*6 + 1, i]] < median/factor,
   calLNAsample[[j*6 + 1, i]] = median,];
  If[calLNAsample[[j*6 + 2, i]] > factor*median ||
    calLNAsample[[j*6 + 2, i]] < median/factor,
   calLNAsample[[j*6 + 2, i]] = median,];
  If[calLNAsample[[j*6 + 3, i]] > factor*median ||
    calLNAsample[[j*6 + 3, i]] < median/factor,
   calLNAsample[[j*6 + 3, i]] = median,];
  If[calLNAsample[[j*6 + 4, i]] > factor*median ||
    calLNAsample[[j*6 + 4, i]] < median/factor,
   calLNAsample[[j*6 + 4, i]] = median,];
  calLNAsample[[j*6 + 5,
      i]] = (calLNAsample[[j*6 + 1, i]] + calLNAsample[[j*6 + 2, i]] +
       calLNAsample[[j*6 + 3, i]] + calLNAsample[[j*6 + 4, i]])/4;
  calLNAsample[[j*6 + 6, i]] =
   Median[{calLNAsample[[j*6 + 1, i]], ca[LNAsample[[j*6 + 2, i]],
     calLNAsample[[j*6 + 3, i]], calLNAsample[[j*6 + 4, i]]}]
  , {i, 1, Length[calLNAsample[[1]]]}, {j, 0, Length[calLNAsample]/6 - 1}];
p0 = ListDensityPlot[calLNAsample,
   ColorFunction -> (GrayLevel[1- #] &),
   DisplayFunction -> Identity,
   PlotLabel -> "Data matrix after median filtering",
   Mesh -> False,
   AspectRatio -> 120/280];
Show[p0, DisplayFunction -> $DisplayFunction, ImageSize -> {1000, 500}];
```

```
Print["AFTER CALIBRATION"];
calLength =
  Table[Sqrt[caILNAsample[[i]].calLNAsample[[i]]], {i, 1,
    Length[calLNAsample]}];
Do[calLNAsample[[i]] = calLNAsample[[i]]/calLength[[i]], {i, 1,
   Length[calLNAsample]}];
p0 = ListDensityPlot[calLNAsample,
   ColorFunction -> (GrayLevel[1 - #] &),
   Displayfunction -> Identity,
   PlotLabel -> "Data matrix after calibration",
   Mesh -> False,
   AspectRatio -> 120/280];
Show[p0, DisplayFunction -> $DisplayFunction, ImageSize -> {1000, 500}];
medianLNAdata = Table[calLNAsample[[i*6]], {i, 1, Length[calLNAsample]/6}];
allLNAdata =
  Partition[
   Flatten[
    Table[{calLNAsample[[I*6 + 1]], calLNAsample[[i*6 + 2]],
       calLNAsample[[i*6 + 3]], calLNAsample[[i*6 + 4]]}, {i, 0,
       Length[calLNAsample]/6 - 1}]], 4*Length[calLNAsample[[1]]]];
(* PARAMETERS *)
min = 0.001;   (*
 min is added to both numerator and denominator before determining ratio to \
avoid division by 0 and reduce influence of low intensity probes *)
signature = calLNAsample;
distanceMatrix =
 Table[0, {i, 1, Length[signature]}, {j, 1, Length[signature]}];
Do[distanceMatrix[[i, j]] =
  Sqrt[Sum[(signature[[i, k]] - signature[[j, k]])^2, {k, 1,
      Length [signature[[1]]]}]]
 , {i, 1, Length[signature]}, {j, 1, Length[signature]}];
plotmatrix = Transpose[distanceMatrix];
p1 = ListDensityPlot[plotMatrix,
  ColorFunction -> (GrayLevel[1 - #] &),
   DisplayFunction -> Identity,
   PlotLabel -> "All dataset (R1+R2+R3+R4+mean+median)",
   Mesh -> False,
  AspectRatio -> 1];
signature = medianLNAdata;
distanceMatrix =
 Table[0, {i, 1, Length[signature] + 1}, {j, 1, Length[signature]}];
Do[distanceMatrix[[i + 1, j]] =
   Sqrt[Sum[(signature[[i, k]] - signature[[j, k]])^ 2, {k 1,
      Length[signature[[1]]]}]]
 , {i, 1, Length[signature]}, {j, 1, Length [signature]}];
distanceMatrix[[1]] = sampleNames;
p2a = TableForm[distanceMatrix];
plotmatrix = Transpose[Delete[distanceMatrix, 1]];
p2 = ListDensityPlot[plotMatrix,
   ColorFunction -> (GrayLevel[1- #] &),
   DisplayFunction -> Identity,
   PlotLabel -> "Median of replicates",
   Mesh -> False,
   AspectRatio -> 1];
signature = allLNAdata;
distanceMatrix =
  Table[0, {i, 1, Length[signature] + 1}, {j, 1, Length[signature]}];
Do[distanceMatrix[[i + 1, j]] =
   Sqrt[Sum[(signature[[i, k]] - signature[[j, k]])^2, {k, 1,
       Length[signature[[1]]]}]]
  , {i, 1, Length[signature]}, {j, 1, Length[signature]}];
distanceMatrix[[1]] = sampleNames;
p3a = TabIeForm[distanceMatrix];
```

```
plotMatrix = Transpose[Delete[distanceMatrix, 1]];
p3 = ListDensityPlot[plotMatrix,
   ColorFunction -> (GrayLevel[1 - #] &),
   DisplayFunction -> Identity,
   PlotLabel -> "All dataset combined",
   Mesh -> False,
   AspectRatio -> 1];
Show[GraphicsArray[{p1, p2, p3}], ImageSize -> {900, 300}];
Print["Distance matrix: Median of replicates", p2a];
Print[" "];
Print["Distance matrix: All dataset combined", p3a];
```

A barcode representation of the ten resulting signatures is shown in Figure 39. In this representation, the hybridization pattern of each slide is represented by six rows, one for each of the four replicates of the 280 LNA probes, one row representing the mean value and the last row the median value. A distinctly different hybridization pattern is observed for the five slides with adk amplificate (row 1 to 30) as opposed to slides with the recA amplificate (row 31 to 60). The barcode of each slide can be compared quantitatively to the barcode of another slide to obtain a pairwise similarity matrix (Figure 40). This matrix depicts the relatedness of each sample to each of the other samples using the sum of squared intensity difference as similarity criteria. The calculated similarity matrix show a very low degree of similarity between signatures for the two different genes (black square corners caused by a comparing an *adk* signature from one organism with a *recA* signature from another, (i.e. comparing apples and oranges)). However, the similarity matrix of the two genes (obtained when comparing the same gene signature from different organisms) resembles each other as is expected for two household genes that mutate at the same and constant frequency.

We can further analyze the similarity matrix by depicting is as a similarity tree, again according to a minimal least squares criteria (Figure 41). The similarity tree based on the similarity matrix computed in Mathematica was generated with the FITCH algorithm in the PHYLIP package. The "similarity tree" reflects a quantification of the difference between each of the signatures so that signatures which are similar are placed close together in the tree topology, whereas dissimilar signatures are more distant. As similarity of hybridization pattern is likely to reflect sequence similarity, the derived tree should represent the sequence similarity between genes from different strains. The tree could thus ultimately be related to the phylogenetic distances between the strains as they are reflected in sequence variation for common household genes.

It is remarkable how the generated tree topography for the two genes resemble each other (Figure 41) as may be expected for two household genes. Unfortunately we do not yet know the sequences of both the genes from all the strains we have used here so we do not know if the produced tree resembles the correct sequence based phylogeny for the genes and strains in question.

<u>Example 8F: Classification of RNA samples from yeast before and after heat shock</u>

The simple test array described in Example 8B was further used to classify complex RNA samples from Yeast containing different gene expression patterns before and after a heat shock treatment (Figure 43). This experiment was designed to demonstrate the potential of a universal LNA heptamer array to classify expression patterns from different tissue samples or cell lines based on the observed hybridization pattern with labeled RNA from the sample.

**Yeast cultures** *Saccharomyces cerevisiae* wild type (BY4741, MATa; his3Δ1; leu2Δ0; met15Δ0; ura3Δ0) and (EUROSCARF) was grown in YPD medium at 30 °C until the $A_{600}$ density of the cultures reached 0.8. Half of the cultures were collected by centrifugation and resuspended in 1 vol. of 40 °C preheated YPD. Incubation was continued for an additional 30 min at 30 °C or 40 °C for the standard and heat-shocked cultures, respectively. Cells were harvested by centrifugation and stored at -80 °C.

*RNA extraction and synthesis of fluorochrome-labelled yeast cDNA target*

Yeast total RNA was extracted using the FastRNA Kit-RED (BIO 101, USA) according to the manufacturer's instructions. The quantity and quality of the total RNA preparations were assessed by standard spectrophotometry using a NanoDrop ND-1000 (NanoDrop, USA) combined and by agarose gel electrophoresis. Two replicate samples of total RNA from both wild type and heat shocked wild type yeast cells, were labeled with the Cy3-ULS labeling kit according to the manufacturer's instructions (Amersham Biosciences, USA). The four samples were subsequently purified with a ProbeQuant 650 spin down column, to produce about 500 ng labeled total RNA in about 50 μl.

Each of the four samples were hybridized with a different slide at 1 ng/μl target concentration as described in Example 8B. However the slides were scanned twice first after a standard 5 min wash in 5xSSCT and 2.5 mM $MgCl_2$

at 4 °C (labeled the "A" samples) then again after a stringent 30 min wash in the same solution at 25 °C. (Labelled the "B" samples).

The resulting hybridization patterns after the first were quite complex as expected for the highly complex targets (Figure 44). A representative hybridization pattern shown in panel A, clearly reveals the large performance difference between LNA and DNA heptamer capture probes as well as the high degree of reproducibility for the four different replicates of the 384 probe set, even at low stringency. As the applied target mixture is a complex mixture of hundreds of different mRNAs and rRNAs, most capture probes produce a signal and the resulting barcode (Figure 44B) is less "ON/OF" with more gray values than previous versions.

Still a distinct and reproducible pattern is clearly discernable and a similarity analysis (as in example 8B and 8E enables us to correctly classify the eight scan as containing either a heat shocked yeast sample or a non shocked control sample. The distinction is evident from the similarity tree (Figure 45) where all heat shock samples branch to the same side of the tree and all non-shocked samples to the opposite side. This distinction was possible even when: 1) using a total RNA preparation that in all cases is known to be dominated by invariable rRNA sequences; 2) the samples were scanned twice at very different stringencies (20 °C different washing temperatures); and 3) using a very simple LNA array with only 280 heptamer probes evaluated in four replicates

We clearly believe that the distinction between the different mRNA pools will become even more evident with a higher resolution Universal LNA Array and that the independence of hybridization stringency is highly promising for the general robustness of assay based on the universal array platform.

Example 8G: LNA enhanced heptamer array in a microfluidic device

A universal LNA array consisting of all possible oligonucleotides of a given length can be used as a general purpose tool to obtain temperature dependent hybridization patterns (= DNA signatures). These detailed signatures may, in turn, be classified by comparison to a large set of standard signatures. As each signature contain many thousands of data points, a numerical deconvolution of a complex sample signature into a large number of constituents may be possible (i.e. due to a highly overdetermined equation system). Furthermore, it is possible to compare a sample signature to the best possible combination of standards to determine the goodness of fit, i.e. if a linear combination of the known standards adequately describes the sample of interest. This feature is essential for medical applications, where it will be possible to identify samples that cannot be resolved reliably with this technique.

The prime advantage of a universal chip approach is the flexibility. The vision: that a low-cost universal LNA array can generate sequence specific hybridization patterns = a detailed genetic signature that can be used to classify samples is attractive. The universal array can be used in many different assays by comparing the signature after any given pretreatment (e.g. PCR amplification with context specific primers) to similarly treated standards that are relevant for the given assay. By developing many different assays that all make use of the same array, it will be possible to produce the array in large quantities, which will greatly reduce the cost of individual arrays. A mass-produced array and subsequent robust analysis procedure may eventually be used as a low cost generic nucleic acid characterization tool like we use gel-electrophoresis today.

The reduced complexity of an LNA enhanced heptamer array containing only 1200 capture probes spotted in triplicates, makes it feasible to synthesize and spot a universal LNA array in an easy-to-use, self-contained microfluidic device, such as a prototype being developed by Exiqon in collaboration with STEAG MicroParts, Germany (Figure 30). The hybridization chamber is covered with a foil after spotting to form a protected hybridization channel with a total volume of less than 10 μl. The slide also contains an inlet that fits standard micropipettes and an integrated waste chamber. The slide has the same footprint as conventional microscope slides (75 x 25 x 1 $mm^3$) and is compatible with standard array scanners.

Example 9: General Reaction Conditions for Synthesis of Some Compounds of the Invention

Reactions were conducted under an atmosphere of nitrogen when anhydrous solvents were used. All reactions were monitored by thin-layer chromatography (TLC) using EM reagent plates with florescence indicator ($SiO_2$-60, F-254). The compounds were visualized under UV light and by spraying with a mixture of 5% aqueous sulfuric acid and ethanol followed by heating. Silica gel 60 (particle size 0.040-0.063 mm, Merck) was used for flash column chromatography. NMR spectra were recorded at 300 MHz for $^1$H NMR, 75.5 MHz for $^{13}$C NMR and 121.5 MHz for $^{31}$P NMR on a Varian Unity 300 spectrometer. δ-Values are in ppm relative to tetramethyl silane as internal standard ($^1$H and $^{13}$C NMR) and relative to 85% $H_3PO_4$ as external standard ($^{31}$P NMR). Coupling constants are given in Hertz. The assignments, when given, are tentative, and the assignments of methylene protons, when given, may be interchanged. Bicyclic compounds are named according to the Von Bayer nomenclature. Fast atom bombardment mass spectra (FAB-MS) were recorded in positive ion mode on a Kratos MS50TC spectrometer. The composition of the oligonucleotides were verified by MALDI-MS on a Micromass Tof Spec E mass spectrometer using a matrix of diammonium citrate and 2,6-dihydroxyacetophenone.

Example 10: Selective Binding Complementary (SBC) nucleotides

Self-complementarity is an important issue in nucleic acid technologies as reported for DNA, PNA and LNA, and in different biological applications especially in the field of homogeneous assays. LNA: LNA duplexes are the most thermally stable nucleic acid type duplex system known, making the reduction of self-complementarity even more important. Selective Binding Complementary (SBC) nucleotides are able to form stable, sequence-specific hybrids with complementary unmodified strands of nucleic acids, yet they form less stable hybrids with each other. Thus, the reduced ability of SBC oligonucleotides to form intramolecular hydrogen bond base-pairs between regions of substantially complementary sequence causes a reduced level of secondary structure.

The use of a matched pair of oligonucleotides where each member of the pair is complementary or substantially complementary in the Watson-Crick sense to a target sequence of duplex nucleic acid where the two strands of the target sequence are themselves complementary to one another has been reported. The oligonucleotides include modified nucleobases called SBC monomers of such nature that the SBC modified nucleobase forms a stable Watson-Crick hydrogen bonded base pair with the natural partner base but forms a less stable Watson-Crick hydrogen bonded base pair with its modified partner.

Exemplary SBC oligonucleotides contain 2,6-diaminopurine or 2-amino-A (D) and $^{2S}$T incorporated in the same oligonucleotide as replacements of at least one pair of A and T, respectively. The SBC name refers to the fact that D and $^{2S}$T form a destabilised base-pair with only 1 Watson-Crick hydrogen bond , see Figure 4, compared to the A-T base pair with 2 Watson-Crick hydrogen bonds, but D-T and $^{2S}$T-A base pairs are more stable - with 3 and 2 Watson-Crick hydrogen bonds, respectively - than the original A-T base pair. Exemplary SBC C:G base pairs include PyrroloPyr and hypoxanthine and 2-thio-C and G (Figure 9). Other exemplary SBC nucleotide derivatives are shown in Figures 10-12.

Generally speaking, the SBC nucleobases described may also include some other modified nucleobases as long as they retain the ability to reduce the number of intramolecular Watson-Crick hydrogen bonds as described above. The phosphate backbone of the oligonucleotides containing SBC nucleobases may include phosphorthioate linkages as well.

A general structure of a preferred class of A'-T' SBC nucleobases is shown in Figure 10 where the sugar is of the LNA type or 2-deoxy-D-ribose (DNA type). A preferred embodiment of the SBC nucleobase T' has 2-thio-uracil as SBC nucleobase as shown in formula (i) in Figure 10 where $R_2$ = H. Another preferred embodiment of the SBC nucleobase T' has 2-thio-thymine as SBC nucleobase as shown in formula (i) in Figure 10 where $R_2 = CH_3$. A preferred embodiment of the SBC nucleobase A' has 2,6-diaminopurine as SBC nucleobase as shown in formula (ii) in Figure 10 where X = N and $R_1 = NH_2$. In yet another preferred embodiment, both sugars are of the LNA type and the SBC nucleobase A' has 2,6-diaminopurine as SBC nucleobase and the SBC nucleobase T' has 2-thio-uracil or 2-thio-thymine as SBC nucleobase.

A general structure of a preferred class of C'-G' SBC nucleobases are shown in Figure 11 where the sugar is of the LNA type or 2-deoxy-D-ribose (DNA type). A preferred embodiment of the SBC nucleobase C' has pyrrolo-[2,3-d]pyrimidine-2(3H)-one as SBC nucleobase as shown in formula (iii) in Figure 11 where $R_4$ = H. A preferred embodiment of the SBC nucleobase G' has hypoxanthine as SBC nucleobase as shown in formula (iv) in Figure 11 where $R_5$ = H. In yet another preferred embodiment, both sugars are of the LNA type and the SBC nucleobase C' has pyrrolo-[2,3-d] pyrimidine-2(3H)-one as SBC nucleobase and the SBC nucleobase G' has hypoxanthine as SBC nucleobase.

A general structure of another preferred class of C'-G' SBC base pair are shown in Figure 12 where the sugar is of the LNA type or 2-deoxy-D-ribose (DNA type). A preferred embodiment of the SBC nucleobase C' has 2-thio-cytosine as SBC nucleobase as shown in formula (v) in Figure 12 where $R_1$ = H. A preferred embodiment of the SBC nucleobase G' has guanine as SBC nucleobase as shown in formula (vi) in Figure 12. In yet another preferred embodiment both sugars are of the LNA type and the SBC nucleobase C' has 2-thio-cytosine as SBC nucleobase and the SBC nucleobase G' has hypoxanthine as SBC nucleobase.

If desired, SBC monomers may be incorporated into the nucleic acids and arrays of the invention, using standard methods.

Table 7 shows 3 isosequential sequences (entry 1-3) where A and T have been partly replaced with the SBC LNA monomers D and $^{2S}$U. For example, when LNA-A and LNA-T are replaced with the SBC LNA monomers LNA-D and LNA 2-thio-U, respectively, see Table 7, in self complementary oligonucleotides, the $T_m$ is radically decreased e.g. from 90˚C (entry 1) to 53.5˚C (entry 2) thus verifying the reduced strength of the intramolecular hydrogen bonds of the self complementary oligonucleotide. At the same time the oligonucleotides containing the SBC LNA monomers are able to hybridize to complementary DNA due to the increased binding efficiency of LNA-D and LNA-$^{2S}$U. Similarly as exemplified in Table 8 (see below) the $T_m$ of a duplex between 2 complementary oligonucleotides containing e.g. 3 SBC LNA pairs (entry 3) is reduced to 59˚C from the corresponding non-SBC LNA duplex (82˚C - entry 1) while the single-stranded SBC LNA oligonucleotides still are capable to hybridize to complementary non-modified LNA oligonucleotides as well

as DNA oligonucleotides with increased $T_m$.

Table 7: $T_m$s of self complementary duplexes of dual labeled SBC-LNA probes. Modified monomers (LNA monomers are in CAPITAL): **$^{2S}$U** = LNA 2-thiouracil; **D** = LNA 2,6-diaminopurine. Fitc = Fluorescein; EQL = Eclipse quencher.

| Entry | Sequences | $T_m$ [a](˚C) | $T_m$ [b](˚C) | $T_m$ [c](˚C) |
|---|---|---|---|---|
| 1 | 5'-Fitc-tAAATTTTA-EQL | >90 (94-96) | 47 | - |
| 2 | 5'-Fitc-ta**DD**T$^{2S}$**U**$^{2S}$**U**ta-EQL | 53.5 | 37 | 53 |
| 3 | 5'-Fitc-t**DDD**t$^{2S}$**U**$^{2S}$**U**$^{2S}$**U**a-EQL | 64.5 | 44 | 68 |

[a]The melting temperatures ($T_m$ values) were obtained as a maxima of the first derivative of the corresponding melting curves (optical density at 260 nm versus temperature). Concentration of the duplexes: 2 μM. Buffer: 0.1 M NaCl; 10 mM Na-phosphate (pH 7.0); 1 mM EDTA.
[b]$T_m$ against complementary DNA predicted using Exiqon's $T_m$-prediction tool (www.exiqon.com) where LNA-D = LNA-A and LNA-$^{2S}$U = LNA-T.
[c]$T_m$ against complementary DNA predicted using the data against DNA (see column to the left) predicted using Exiqon's $T_m$-prediction tool (www.exiqon.com) and adding 6˚C per modification for LNA-D and 2˚C per modification for LNA-$^{2S}$U.

Table 8: $T_m$s[a] of the duplexes containing SBC-LNA 6-mers and their DNA-LNA controls. Modified monomers (LNA monomers are in CAPITAL): **$^{2S}$U** = LNA 2-thiouracil; **D** = LNA 2,6-diaminopurine; C = LNA methyl-C.

| E | | 3'-TGTATC | 3'-TG **$^{2S}$U**ATC | 3'-**$^{2S}$U**G**$^{2S}$U**A**$^{2S}$U**C | 3'-ctgtatcc |
|---|---|---|---|---|---|
| 1 | 5'-ACATAG | 82 | 85 | 83 | 27 |
| 2 | 5'-AC**D**TAG | 93 | 75 | 73 | 37 |
| 3 | 5'-**D**C**D**T**D**G | > 97 | 95 | 59 | 55 |
| 4 | 5'-gacatagg | 28 | 33 | 37 | 24 |

[a]The melting temperatures ($T_m$ values) were obtained as a maxima of the first derivative of the corresponding melting curves (optical density at 260 nm versus temperature). Concentration of the duplexes: 2.5 μM. Buffer: 0.1 M NaCl; 10 mM Na-phosphate (pH 7.0); 10 mM EDTA.

Additionally, SBC LNA monomers can be used in combination with SBC DNA monomers to reduce the strength of intramolecular hydrogen bonds. For example, LNA-D can be used in combination with DNA 2-thio-thymidine as verified in the example shown in Table 6 where the $T_m$ of a duplex between an oligonucleotide containing LNA-D and the complementary oligonucleotide where the nucleotide opposite the LNA-D nucleotide is a DNA 2-thio-T nucleotide (s) is reduced to 59.4˚C compared to the $T_m$ of 67.8˚C of the reference duplex. Likewise, LNA 2-thio-U/T can be used in combination with DNA 2,6-diaminopurine (d) as verified in the example shown in Table 9 where the $T_m$ of a duplex between an oligonucleotide containing DNA-d and the complementary oligonucleotide where the nucleotide opposite the DNA-d nucleotide is a LNA 2-thiouracil ($^{2S}$U) nucleotide is reduced to 47.3˚C compared to the $T_m$ of 58.4˚C of the reference duplex.

Table 9: $T_m$s[a] of the duplexes between SBC-LNA 8-mers and DNA. Modified monomers (LNA monomers are in CAPITAL): **$^{2S}$U** = LNA 2-thiouracil; **d** = DNA 2,6-diaminopurine; C = LNA methyl-C.

| Oligo | $T_m$ (˚C) of complementary duplexes[a] | | |
|---|---|---|---|
| | 3'-CTGTATCC | 3'-CTG$^{2S}$**U**ATCC | 3'-C$^{2S}$**U**G$^{2S}$**U**A$^{2S}$**U**CC |
| 5'-gacatagg | 54.6 | 58.4 | 62.0 |
| 5'-gacdtagg | 60.5 | 47.3 | 51.6 |

(continued)

| Oligo | $T_m$ (˚C) of complementary duplexes[a] | | |
|---|---|---|---|
| | 3'-CTGTATCC | 3'-CTG[2S]**U**ATCC | 3'-C[2S]**U**G[2S]**U**A[2S]**U**CC |
| 5'-g**d**c**dt****d**gg | Not determined | 56.9 | nc[b] |

[a]The melting temperatures ($T_m$ values) were obtained as a maxima of the first derivative of the corresponding melting curves (optical density at 260 nm versus temperature). Concentration of the duplexes: 2.5 μM. Buffer: 0.1 M NaCl; 10 mM Na-phosphate (pH 7.0); 1 mM EDTA.
[b]No cooperative transition observed

Kutyavin et al. (Biochemistry, (1996), 35, 11170) reported on the use of a pair of oligonucleotides containing the SBC monomers 2-aminoadenine and 2-thiothymine for strand invading the ends of a double-stranded DNA. Compagno et al (J. Biol. Chem., 1999, 274, 8191) likewise reported on the use of the same type of SBC oligonucleotides as antisense agent targeting a hairpin in the mini-exon RNA of *Leischmania amazonensis.* Double duplex strand invasion inhibiting transcription of the T7 phage RNA polymerase was also demonstrated with Peptide Nucleic Acid (PNA) using the PNA version of the SBC monomers 2-aminoadenine and 2-thiouracil (Lohse et al., Proc Nat Sci USA, (PNAS), (1999), 96, 11804. Izvolsky et al, Biochemistry, (2000), 39, 10908). Woo et al. (Nucleic Acid res, (1996) 24, 2470) reported on the use the SBC monomers Inosine and PyrroloPyr in a pair of self-complementary oligonucleotides for strand invading the end of a duplex DNA.

Example 11: Exemplary Methods for Synthesizing LNA-2-thiopyrimidine Nucleosides and Nucleotides

2-Thiopyrimidine nucleosides can be prepared in several ways (see Figure 6). For example, the 2-thiouridine-nucleosides (**IV**) can be synthesized from a substituted uridine nucleoside (**VIII**). By protection of the O4-position (**IX**) on the nucleobase, thionation can be performed, O2 position, which results in the 2-thio-uridine nucleoside (**IV**). Performing sulphurisation on both O2 and O4 results in 2,4-dithio-uridine nucleoside (**X**) which may be transformed into the 2-thio-uridine nucleoside (**IV**) (Saladino, et. al., Tetrahedron, 1996, 52, 6759). Another way is to generate a cyclic ether (**XI**) through reaction with the 5' position. This product can then be transformed to the 2-thio-uridine nucleoside (**IV**) or the 2-0-alkyl-uridine nucleoside (**XII**). The 2-O-alkyl-uridine nucleoside (**XII**) can also be generated by direct alkylation of the uridine nucleoside (**VIII**). Treatment of the 2-O-alkyl-uridine nucleoside (**XII**) can also be transformed into the 2-thio-uridine nucleoside (Brown et. al., J. Chem. Soc. 1957, 868; Singer, et. al., Proc. Natl. Acad. Sci. USA, 1983, 80, 4884; Rajur and McLaughlin, Tetrahedron Lett., 1992, 33, 6081).
In another method (see Figure 7), lewis acid-catalyzed condensation of a properly substituted sugar (**I**) and a substituted 2-thio-uracil (**II**) can result in a substituted 2-thio-uridine nucleoside of the structure (**III**) which by further synthetic manipulations can be transformed into the LNA 2-thiouridine nucleoside (**IV**) (Hamamura et. al., Moffatt, J. Med. Chem., 1972, 15, 1061; Bretner et. al., J. Med. Chem., 1993, 36, 3611), see Figure 7.
Using a properly substituted amino-sugar (**V**) (see Figure 8), a 2-thio-uridine nucleoside can be synthesized through ring-synthesis of the nucleobase by reaction of the amino sugar (**V**) and an substituted isothiocyanate (**VI**), yielding the substituted LNA 2-thio-uracil nucleoside (**VI**) (Shaw and Warrener, J. Chem. Soc. 1957, 153; Cusack et al., J. Chem. Soc. Perkin 1, 1973, 1721), see Figure 8.

Example 12: Exemplary Methods for Synthesizing [2S]T-LNA

Three different strategies for synthesis of [2S]T-LNA are outlined in Figure 5.
Strategy A involves coupling a glycosyl-donor and a nucleobase, using standard methodology for synthesis of existing LNA monomers. Strategy B involves ring synthesis of the nucleobase. This strategy is desirable because the availability of 1-amino-LNA enables introduction of a variety of new nucleobases. Strategy C includes modification of T-LNA; the easy synthesis of LNA-T diol makes this an attractive pathway.
In a desirable embodiment, [2S]T-LNA is synthesized as illustrated in Figure 13:

In particular, the known coupling sugar 1,2-di-*O*-acetyl-3, 5 di-O-benzyl, 4-C-mesyloxymethyl, α,β-D-ribofuranose **1** (Figure 13) was coupled with the nucleobase 2-thio-thymidine in a Vorbrüggen type reaction. Thus, the nucleobase was silylated and condensed with the sugar using $SnCl_4$ as catalyst to promote the reaction affording nucleoside **2**. Mass spectrometry and NMR subsequently identified the isolated product as the desired one. NMR data were compared with published data of a 2-thio-thymidine derivative (Kuimelis and Nambiar, Nucleic Acid Res., 1994, 22, 1429-1436) in order to validate the correct attachment point of the nucleobase.

Subsequently, a base mediated ring-closing reaction afforded the di-benzylated LNA derivative **3** in 77% yield. The signals in the $^1$H-NMR spectrum of the compound appeared as singlets, thus proving that the cyclization had occurred to give the LNA skeleton, in which the 1'-H and 2'-H are perpendicular to each other causing the $^3]_{1',2'}$ to be 0 Hz. MALDI mass spectrometry was likewise used for the identification of the compound.

The LNA derivative was protected at the nucleobase with the toluoyl protective group to give **4**. This group is well known for the protection of 2-thio-thymidine derivatives, (Kuimelis and Nambiar, Nucleic Acid Res., 1994, 22, 1429-1436). The protection of the nucleobase occurs at both the N-3 and the O-4 position and hence the compound is isolated as a mixture of two compounds. NMR shows that the ratio of the two isomers in the isolated mixture is 2: 1.

These methods are described further below.

<u>1-(2-*O*-acetyl-3-O, 5-*O*-dibenzyl, 4-*C*-mesyloxymethyl-β-D-ribofuranosyl)-2-thio-thymine (2)</u>

1, 2-di-*O*-acetyl-3, 5 di-*O*-dibenzyl, 4-*C*-mesyloxymethyl, α,β-D-ribofuranose (**1**, 2.0g, 3.83 mmol) and 2-thio-thymine (552mg, 3.89mmol) were co-evaporated with anhydrous acetonitrile (100 ml) and redissolved in anhydrous acetonitrile (80ml), *N,O*-bistrimethylsilylacetamide (1.5, 5.85mmol) was added, and the reaction was stirred at 80°C for one hour. The mixture was cooled to 0°C, $SnCl_4$ (0.9 ml, 7.66mmol) was added, and the reaction was left to stir for 24 hours. The reaction mixture was diluted with EtOAc and washed with $NaHCO_3$ and subsequently with water. The organic phase was dried ($Na_2SO_4$) and evaporated to dryness. The product was purified using column chromatography, giving the thio-thymidine derivative **2** (1.1g, 1.82mmol, 40%) as a white foam. $R_f$ (10% THF/dichloromethane): 0.75.

MALDI-MS: 627 (M+Na) $^{13}$C-NMR ($CDCl_3$): δ= 174.40, 169.29, 159.89, 136.13, 136.51, 136.05, 128.62, 128.56, 128.41, 128.29, 128.07, 127.89, 12767, 116.18, 91.41, 86.21, 75,59, 75.31, 74.46, 74.22, 73.61, 69.25, 69.04, 37.52, 20.62, 11.91

<u>(*1R*,*3R*,*4R*,*7S*)-(benzyloxy)-1-(benzyloxymethyl)-3-(2-thiothymidine)-2,5-dioxabicyclo[2.2.1]heptane (3)</u>

1-(2-*O*-acetyl-3-O, 5-*O*-dibenzyl, 4-*C*-mesyloxymethyl-β-D-ribofuranosyl)-2-thio-thymine (**2**, 630mg, 1.04mmol) was dissolved in dioxane (15ml) and water (8ml), and aqueous NaOH (2M, 5ml) was added, and the reaction was left to stir at room temperature for one hour. The yellow solution was neutralized with HCl (1M, 6ml) affording a precipitation. The mixture was diluted with dichloromethane and ethyl acetate causing an emulsion. After separation, the aqueous phase extracted with ethyl acetate, and the combined organic phase was dried ($Na_2SO_4$) and evaporated to dryness. The compound was purified by column chromatography (0-2, then 5% THF/dichloromethane), giving the ring closed compound **3** as a white foam (370mg, 0.79mmol, 77%). $R_f$ (2% MeOH/dichloromethane): 0.23.

MALDI-MS: 488 (M+Na) $^{13}$C-NMR ($CDCl_3$): δ= 173.14, 160.39, 137.20, 136.63, 136.00, 128.46, 128.34, 128.02, 127.66, 115.52, 90.29, 87.77, 77.39,75.26, 73.77, 72.07, 71.70, 64.15, 30.17, 12.33

$^1$H-NMR ($CDCl_3$): δ= 9.87 (s, 1H), 7.69 (d, 1.1Hz, 1H), 7.26-7.37 (m, 10H), 6.13 (s, 1H), 4.84 (s, 1H), 4.66 (d, J= 11.3 Hz, 1H), 4.61 (s, 2H), 4.52 (d, 11.5Hz, 1H), 4.04 (d, J=7.7Hz, 1H), 3.93 (s, 1H), 3.88 (d, J= 11.0Hz, 1H), 3.82 (d, J= 7.7Hz, 1H), 3.82 (d, J= 10.8 Hz, 1H), 1.59 (d, J= 1.1 Hz, 3H)

<u>(*1R,3R,4R,7S*)-7-(benzyloxy)-1-(benzyloxymethyl)-3-(2-thio-N3/O4-toluoyl- thymidine)-2,5-dioxabicyclo[2.2.1]heptane (4)</u>

(1R,3R,4R,7S)-7-(benzyloxy)-1-(benzyloxymethyl)-3-(2-thiothymidine)-2,5-dioxabicyclo[2.2.1]heptane (**3**, 290mg, 0.62mmol) was dissolved in anhydrous pyridine and diisopropylethylamine (0.2ml, 1.15mmol), toluoyl chloride (0.25ml, 1.89 mmol) was added, and the reaction mixture was stirred at room temperature for three hours. After completion, the reaction mixture was diluted with dichloromethane, and the reaction was quenched by addition of water. The phases were separated, and the organic phase was dried ($Na_2SO_4$) and evaporated to dryness. The residue was co-evaporated with toluene. The product was purified by column chromatography (0-1% MeOH/dichloromethane) to give nucleoside **4** as a white foam (320 mg, 0.55mmol, 89%). $R_f$ (2%MeOH/dichloromethane): 0.78.

MALDI-MS: 606 (M+Na) $^{13}$C-NMR ($CDCl_3$): δ=171.98, 168.30, 160.30, 145.92, 145.82, 137.22, 136.65, 135.98, 130.39, 130.27, 129.85, 129.50, 128.51, 128.41, 128.08, 127.73, 115.11, 90.10, 87.81, 76.01, 75.80, 75.39, 75.01, 73.83, 72.19, 72.09, 71.74, 64.15, 21.75, 12.40.

In another desirable embodiment, $^{2S}$U-LNA phosphoramidite **45** is synthesized as illustrated in Figure 19.

**5-*O*-Benzoyl-3-*O*-benzyl-4-*C*-methanesulfonoxymethyl-1,2-*O*-isopropylidene-α-D-ribofuranose (33).** To a solution of 3-*O*-benzyl-4-*C*-methanesulfonoxymethyl-5-methanesulfonyl-1,2-*O*-isopropylidene-α-D-*erythro*-pentofuranose **32** (10 g, 21.44 mmol) in anhydrous DMSO (50 mL) was added NaOBz (6.17 g, 42.87 mmol) and the mixture was stirred for 24 h at 140 °C. The mixture was cooled to rt and $H_2O$ (400 mL) was added under intensive stirring. After

cooling overnight at 4 ˚C the formed precipitate was filtered off and washed with $H_2O$- Crystallization from EtOH gave compound **33** (8.9 g, 84%) as a white solid material. $^1$H NMR ($CDCl_3$) δ 7.94-7.90 (m, 2H), 7.60-7.53 (m, 1H), 7.43-7.36 (m, 2H), 7.28-7.22 (m, 5H), 5.83 (d, *J* = 3.9 Hz, 1H), 4.96 (d, *J* = 11.9 Hz, 1H), 4.76 (d, *J* = 11.6 Hz, 1H), 4.71 (dd, *J* = 5.1 and 3.9 Hz, 1H), 4.59 (s, 1H), 4.54 (s, 1H), 4.50 (d, *J* = 11.6 Hz, 1H), 4.28 (d, *J* = 11.7 Hz, 1H), 4.20 (d, *J* = 5.1 Hz, 1H), 3.11 (s, 3H), 1.72 (s, 3H), 1.36 (s, 3H). $^{13}$C NMR ($CDCl_3$) δ 165.1, 136.0, 132.6, 129.0, 128.9, 127.8, 127.5, 127.3, 113.2, 103.9, 83.0, 78.9, 77.2, 71.9, 69.1, 63.6, 37.5, 25.5, 25.0. MALDI-MS *m/z* 515.0 $[M+Na]^+$.

**3-*O*-Benzyl-5-hydroxy-4-*C*-methanesulfonoxymethyl-1,2-*O*-isopropylidene-α-D-ribofuranose (34).** To a solution of compound **33** (8.9 g, 18.1 mmol) in THF/MeOH (100 mL, 1/1 v/v) was added 2M NaOH (20 mL) and the mixture was stirred for 1 h, followed by addition of EtOAc and saturated NaHCO3 (100 mL each). The organic phase was separated, washed with saturated NaHCO3 and brine, dried over Na2SO4, and concentrated to an oily residue. The residue was dried *in vacuo* to give **34** (6.95 g, 98%) as a white crystalline material, which was used without additional purification. $^1$H NMR ($CDCl_3$) δ 7.37-7.34 (m, 5H), 5.78 (d, J = 3.8 Hz, 1H), 4.85 (d, *J* = 11.7 Hz, 1H), 4.77 (d, *J* = 11.7 Hz, 1H), 4.65 (dd, *J* = 5.0 and 3.9 Hz, 1H), 4.57 (d, *J* = 11.7 Hz, 1H), 4.39 (d, *J* = 11.9 Hz, 1H), 4.27 (d, *J* = 5.1 Hz, 1H), 3.81 (dd, *J* = 12.1 and 1.6 Hz, 1H), 3.48 (dd, *J* = 12.1 and 8.8 Hz, 1H), 3.05 (s, 3H), 1.91 (dd, *J* = 8.8 and 1.6 Hz, 1H), 1.68 (s, 3H), 1.34 (s, 3H). $^{13}$C NMR ($CDCl_3$) δ 136.9, 128.4, 128.0, 127.8, 113.4, 104.4, 85.0, 78.0, 77.6, 72.6, 69.9, 62.1, 37.7, 26.1, 25.5. MALDI-MS *m/z* 411.2 $[M+Na]^+$.

**Di-3,5-hydroxy-4-*C*-methanesufonoxymethyl-1,2-*O*-isopropylidene- α- D- ribofuranose (35).** $Pd(OH)_2/C$ (20%, 1.2 g) and $HCO_2NH_4$ (2g) were added to a solution of compound **34** (6.12 g, 15.8 mmol) and the mixture was stirred under refluxing. Additional amounts of $HCO_2NH_4$ were added by portions of 1g to the reaction mixture at intervals of an hour (4 times). After reaction completed, the catalyst was filtered off and washed with MeOH. The combined filtrates were concentrated under reduced pressure to give a low-melting solid residue. Crystallization from EtOAc gave compound **35** (4.32 g, 92%) as a white solid material. mp 109-110 ˚C. $^1$H NMR (DMSO-$d_6$) δ 5.69 (d, *J* = 3.7 Hz, 1H), 5.41 (d, *J* = 5.6 Hz, 1H), 4.86 (dd, *J* = 6.7 and 5.0 Hz, 1H), 4.64-4.59 (m, 2H), 4.35 (t, *J* = 5.6 Hz, 1H), 4.21 (d, J = 11.2 Hz, 1H), 3.50 (dd, *J* = 11.6 and 4.7 Hz, 1H), 3.35 (dd, *J* = 11.6 and 6.8 Hz, 1H), 3.16 (s, 3H), 1.53 (s, 3H), 1.27 (s, 3H). $^{13}$C NMR (DMSO-$d_6$) δ 112.2, 103.6, 85.0, 80.2, 71.1, 70.8, 61.2, 37.1, 26.3, 25.8. MALDI-MS m/z 321.2 $[M+Na]^+$. Anal. Calcd for $C_{10}H_{18}O_8S$: C, 40.26; H, 6.08. Found: C, 40.30; H, 6.06.

**3,5-Di-*O*-acetyl-4-*C*-methanesulfonoxymethyl-1,2-*O*-isopropylidene-α-D-ribofuranose (36).**

A solution of compound **35** (10.5 g, 35 mmol) in anhydrous pyridine (80 mL) was treated with Ac20 (11 mL) overnight. The mixture was diluted with EtOAc (50 mL), washed with saturated NaHCO3 (2 x 100 mL) and brine (100 mL), dried (NaSO4), and concentrated to an oily residue. The residue was co-evaporated with toluene (2 x 30 mL) to give white crystalline material that was dried *in vacuo* to yield 13.5 g (99%) of compound 36. mp 114-115 ˚C. $^1$H NMR ($CDCl_3$) δ 5.86 (d,*J* = 4.0 Hz, 1H), 5.12 (d, *J* = 5.7 Hz, 1H), 4.90 (dd, *J* = 5.6 and 3.9 Hz, 1H), 4.75 (d,*J* = 11.4 Hz, 1H), 4.43 (d, *J* = 11.4 Hz, 1H), 4.28 (d, *J* = 11.9 Hz, 1H), 4.12 (d,*J* = 11.9 Hz, 1H), 2.16 (s, 3H), 2.08 (s, 3H), 1.64 s, 3H), 1.33 (s, 3H). $^{13}$C NMR ($CDCl_3$) δ 169.8, 169.4, 113.8, 104.47, 82.7, 77.9, 73.3, 68.3, 63.9, 38.0, 26.0, 25.7, 20.6, 20.4. MALDI-MS m/z 405.1 $[M+Na]^+$. Anal. Calcd for $C_{14}H_{22}O_{10}S$: C, 43.98; H, 5.80. Found: C, 44.02; H, 5.74.

**1,2,3,5-Tetra-*O*-acetyl-4-*C*-methanesulfonoxymethyl-D-ribofuranose (37).** To a solution of compound **36** (15.6 g, 40.8 mmol) in AcOH (180 mL) were added Ac20 (20 mL) and cH2SO4 (0.2 mL). The mixture was stirred overnight and 2M NaOH (150 mL) was added slowly under intensive stirring. The mixture was washed with CH2Cl2 (3 x 100 mL). The combined organic phases were washed with 1M Na2HPO4 (150 mL), saturated NaHCO3 (2 x 150 mL), dried (NaSO4), and concentrated under reduced pressure to give compound **37** (17.3g, 99%) as a clear oily material consisted of two isomers (ratio α:β = 5:9). MALDI-MS m/z 449.1 $[M+Na]^+$.

**1-(2,3,5-Tri-hydroxy-4-*C*-methanesulfonoxymethyl-β-D-ribofuranosyl)-2-thiouracil (39).** A mixture of furanose **37** (11.7 g, 27.4 mmol) and 2-thiouracil (10.55 g, 82.3 mmol) was suspended in anhydrous MeCN (150 mL). To the mixture were added BSA (20.3 mL) and SnC14 (12.8 mL). After intensive stirring for 2 h more BSA (25 mL) and SnCl4 (12.8 mL) were added resulted in formation of a clear slightly yellow solution. After further stirring for 4 h the reaction mixture was diluted with $H_2O$ (200 mL) and stirred for another ½ h. The formed precipitate was filtered off and washed with $CH_2Cl_2$ (200 mL). The combined filtrates were divided in separation funnel, and water layer was washed with EtOAc (150 mL). The combined organic phases were dried ($Na_2SO_4$) and concentrated under reduced pressure. The residue was applied to silica gel column chromatography (20-70 % v/v EtOAc/CH2Cl2) to give crude compound **38** (9.8 g, slightly admixed with 2-thiouracil) as a mixture of two structural isomers (ratio *N1*/*N3* = 3/1). All amounts of **38** were dissolved in 1M methanolic HCl and stirred overnight. The solvents were removed under reduced pressure and the residue was twice crystallized from MeCN to give compound **39** (3.83 g, 38% from **38**). $^1$H NMR (DMSO-$d_6$) 12.64 (br s, 1H), 8.04 (d,*J* = 8.1 Hz, 1H), 6.85 (d,*J* = 6.8 Hz, 1H), 6.03 (d, *J* = 8.1 Hz, 1H), 5.51 (d,*J* = 6.2 Hz, 1H), 5.49 (d,J = 5.0 Hz, 1H), 5.46 (t, J = 5.1 Hz, 1H), 4.36 (d,*J* = 10.8 Hz, 1H), 4.28 (d,*J* = 11.0 Hz, 1H), 4.24 (t, *J* = 5.9 Hz, 1H), 4.16 (t, *J* = 5.0 Hz, 1H), 3.59 (m, 2H), 3.17 (s, 3H), 2.06 (s, 3H). δ $^{13}$C NMR (DMSO-$d_6$) δ 177.5, 159.5, 141.1, 107.1, 91.0, 86.1, 74.0, 71.0, 70.2, 61.9, 36.8. MALDI -MS *m/z* 390.6 $[M+Na]^+$.

**Compound 40. 1-(2-hydroxy-4-*C*-methanesulfonoxymethyl-3,5-(1,1,3,3-tetraisopropyldisiloxan-1,3-diyl)-β-D- ribofuranosyl)- 2- thiouracil.** To a solution of **39** (1.75 g, 4.82 mmol) in anhydrous pyridine (15 mL) was added 1,3-dichloro-1,1,3,3-tetraisopropyldisiloxane (1.70 mL, 5.31 mmol). The mixture was stirred overnight, diluted with EtOAc (50 mL), washed with saturated NaHCO3 (2 x 50 mL), dried (NaSO4), and concentrated to a solid residue. Silica gel column chromatography (20-60 % v/v EtOAc/CH2Cl2) afforded compound **40** (1.08 g, 36%) as a white solid material. $^{1}H$ NMR (DMSO-$d_6$) δ 12.71 (br s, 1H), 7.78 (d, *J* = 8.2 Hz, 1H), 6.52 (s, 1H), 5.93 (d, *J* = 8.1 Hz, 1H), 5.90 (d, *J* = 5.0 Hz, 1H), 4.83 (d, *J* = 11.7 Hz, 1H), 4.40 (d, *J* = 5.7 Hz, 1H), 4.37 (d, *J* = 11.6 Hz, 1H), 4.27 (t, *J* = 5.5 Hz, 1H), 4.06 (d, *J* = 12.3 Hz, 1H), 3.91 (d, *J* = 12.3 Hz, 1H), 3.14 (s, 3H), 1.09-0.95 (m, 28H). $^{13}C$ NMR (DMSO-$d_6$) δ 175.8, 159.7, 140.4, 106.5, 94.0, 85.3, 74.3, 71.9, 69.7, 62.9, 37.2, 17.4, 17.3, 17.2, 17.1, 17.0, 16.9, 13.1, 13.0, 12.5, 12.3.

**Compound 41. (*1S*,*3R*,*4R*,*7S*)-1,7-(1,1,3,3-tetraisopropyldisiloxan-1,3-diyl)-3-(2-thio-(3-N/4-*O*)-toluoyl-uracil-1-yl)-2,5-dioxabicyclo[2.2.1]heptane.** To a solution of compound 40 (900 mg, 1.44 mmol) in anhydrous THF (8 mL) was added NaH (60% suspension in mineral oil; 100 mg, 2.50 mmol). The mixture was stirred for lh, diluted with EtOAc (50 mL), washed with saturated $NaHCO_3$ (2 x 50 mL), dried ($Na_2S0_4$), and concentrated under reduced pressure. Purification by silica gel column chromatography (0-12.5% v/v EtOAc/$CH_2Cl_2$) gave compound **41** (410 mg, 54%) as a white solid material. $^{1}H$ NMR (DMSO-$d_6$) δ 12.80 (br s, 1H), 7.75 (d, *J* = 8.2 Hz, 1H), 5.98 (s, 1H), 5.89 (d, *J* = 8.2 Hz, 1H), 4.53 (s, 1H), 4.12 (d, *J* = 13.7 Hz, 1H), 4.06 (s, 1H), 3.91 (d, *J* = 13.7 Hz, 1H), 3.85 (d, *J* = 8.4 Hz, 1H), 3.72 (d, *J* = 18.3 Hz, 1H), 1.07-0.94 (m, 28H). $^{13}C$ NMR (DMSO-$d_6$) δ 175.0, 159.8, 148.9, 106.1, 89.8, 89.5, 77.9, 70.6, 70.0, 56.7, 17.3, 17.1, 17.0, 16.9, 16.8, 13.2, 12.6, 12.4, 11.8.

**(*1S,3R,4R,7S*)-7-Hydroxy-1-hydroxymethyl-3-(2-thio-(3-*N*/4-*O*)-toluoyl-uracil-1-yl)-2,5-dioxabicyclo [2.2.1]heptane (43).** Toluoyl chloride (0.26 mL, 1.90 mmol) and diisopropylethylamine (0.17 mL, 1.0 mmol) were added to a solution of **41** (0.40 g, 0.75 mmol) in anhydrous pyridine (10 mL). The mixture was stirred for 3h, diluted with CH2Cl2 (40 mL), washed with saturated NaHCO3 (40 mL), dried (Na2SO4), and concentrated to a solid residue. The residue was purification by silica gel column chromatography (0-20% v/v EtOAc/CH2Cl2) to give intermediate **42** (0.43 g) as a white solid material. Compound **42** was dissolved in anhydrous THF (10 mL) and AcOH (0.2 mL) and Et3N.3HF (0.3 mL) were added. The mixture was stirred overnight and concentrated to an oily residue. The residue was co-evaporated with EtOAc (20 mL) and purified by silica gel column chromatography (3-7% v/v MeOH/CH2Cl2) to give compound **43** (0.25 g, 85% from **41**) consisted of two isomers (ratio ca.1:1 by $^{1}H$ NMR).

**(*1R,3R,4R,7S*)-1-(4,4'-dimethoxytrityloxymethyl)-7-hydroxy-3-(2-thio-(3-*N*/4-*O*)-toluoyluracil-1-yl)- 2,5-dioxabicyclo[2.2.1]heptane (44).** A mixture of **43** (25 g, 0.64 mmol) and DMT-Cl (0.22 g, 0.70 mmol) was suspended in anhydrous pyridine and stirred overnight. Toluene (50 mL) was added and the solution was washed with saturated NaHCO3 (2 x 40 mL) and concentrated to an oily residue. The residue was co-evaporated with toluene (2 x 20 mL) and purified by silica gel column chromatography (0-10% v/v EtOAc/CH2Cl2 containing 0.5% of Et3N) to give **44** (0.35 g, 79%) as a white solid material. MALDI-MS m/z 713 $[M+Na]^+$.

**(1R,3R,4R,7S)-7-(2-Cyanoethoxy(diisopropylamino)phosphinoxy)-1-(4,4'-dimethoxytrityloxymethyl)-3-(2-thio-(3-N/4-O)-toluoyluracil-1-yl)- 2,5-dioxabicyclo[2.2.1]heptane (45).** To a solution of compound **44** (0.35 g, 0.51 mmol) in anhydrous CH2Cl2 (3 mL) were added 2-cyanoethyl-*N*,*N*,*N'*,*N'*-tetraisopropyl phosphoradiamidite (0.19 g, 0.63 mmol) and 0.75 M solution of DCI in EtOAc (0.63 mL, 0.47 mmol). The mixture was stirred for 2 h, diluted with toluene (50 mL) and applied to a silica gel column. Phosphoramidite **45** (0.41 g, 91%) was obtained after chromatography (0-7.5% v/v EtOAc/$CH_2Cl_2$, containing 1% of Et3N) as a white solid material. $^{31}P$ NMR (DMSO-$d_6$) δ 149.20, 148.85, 148.67.

*Synthesis of Oligomers*

Along with previously described LNA phosphoramidites (Koshkin *et al., supra;* and Pedersen et al., Synthesis p. 802, 2002), the phosphoramidite monomers **31,** and **45** were successfully applied for automated oligonucleotide synthesis (Caruthers, Acc. Chem. Res. 24:278, 1991) to produce the LNA oligomers depicted in Table 7, B, and C. Oligonucleotide syntheses were performed on a 0.2 μmol scale using an Expedite synthesizer (Applied Biosystems) with the recommended commercial reagents. Standard protocols for DNA synthesis were used, except that the coupling time was extended to 5 minutes and the oxidation time was extended to 30 second cycles. Deprotection of the oligonucleotides were performed by treatment with concentrated ammonium hydroxide for five hours at 60 °C. All the synthesized oligonucleotides were purified by RP-HPLC, and their structures were verified by MALDI-TOF mass spectra.

<u>Example 13: Exemplary Methods for Synthesizing LNA-I, LNA-D, and LNA-2AP</u>

2'-*O*, 4'-*C*-methylene linked (LNA) nucleosides containing hypoxanthine (or inosine) (LNA-I), 2,6-diaminopurine (LNA-D), and 2-aminopurine (LNA-2AP) nucleobases were efficiently prepared via convergent syntheses. The nucleosides were converted into phosphoramidite monomers and incorporated into LNA oligonucleotides using an automated phosphoramidite method. The complexing properties of oligonucleotides containing these LNA nucleosides were assessed against perfect and singly mismatch DNA.

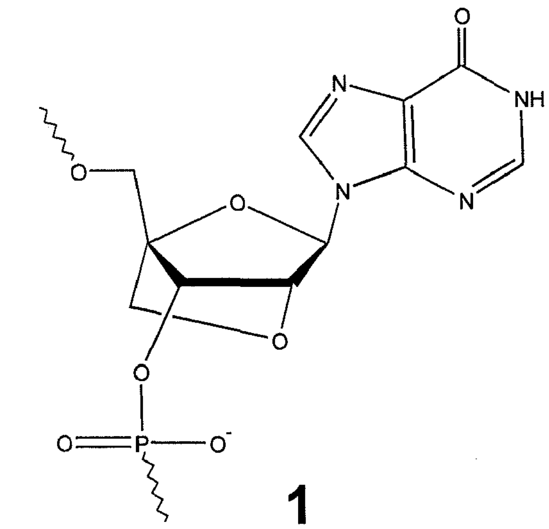

1

LNA-I

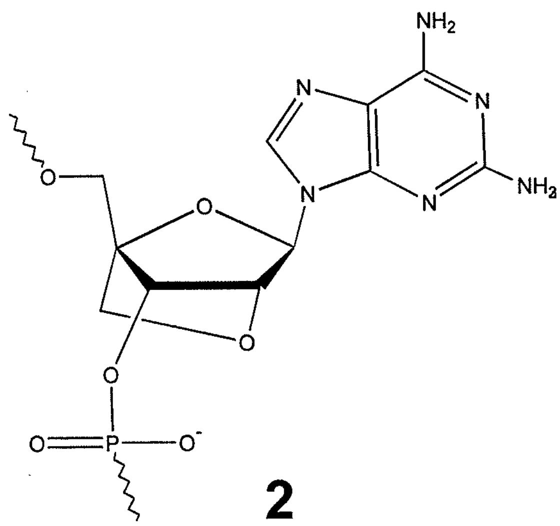

2

LNA-D

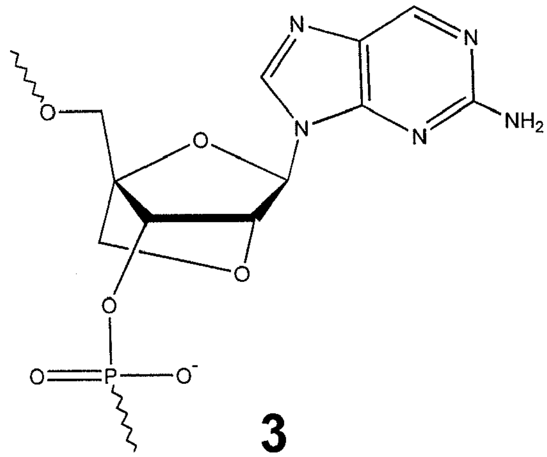

**3**

LNA-2AP

Hypoxantine, the nucleobase found in the nucleotides inosine and deoxyinosine, is considered a guanine analogue in nucleic acids.

Oligonucleotides containing 2,6-diaminopurine replacements for adenines are expected to bind more strongly to their complementary sequences especially as part of A-type helixes due to the potential formation of three hydrogen bounds with thymine or uracil. The reported effect of 2,6-diaminopurine deoxyriboside (D) on the stability of polynucleotide duplexes reaches, on average, about 1.5˚C per modification. Higher stabilisation effects for mismatches were observed for D nucleosides involved in formation of duplexes prone to form A-type helixes. LNA D and LNA 2'-OMe-D are expected to have increased stabilization and mismatch discrimination. LNA can be used in combination with 2-thio-T for construction of selectively binding complementary oligonucleotides. Taking into consideration the extremely high stability of LNA: LNA duplexes, this approach might be very useful for constructing of LNA containing capture probes and antisense reagents.

2-Aminopurine (2-AP) is a fluorescent nucleobase (emission at 363 mn), which is useful for probing nucleic acids structure and dynamics and for hybridizing with thymine in Watson-crick geometry. LNA-I, LNA-D, and/or LNA-2AP may be used in the nucleic acids of the present invention, e.g., to increase the priming efficiency of DNA oligonucleotides in PCR experiments and to construct selectively binding complementary agents.

*Synthesis of LNA-I (Figure 15)*

The synthetic route to LNA-I phosphoramidite **11** is depicted in Figure 15. The previously described 4-*C*-branched furanose **4** (Koshkin *et al., supra*) was used as a glycosyl donor in coupling reaction with silylated hypoxantine by the method of Vorbrüggen *et al.* (Vorbrüggen et al., Chem. Ber. 114:1234, 1981; Vorbrüggen et al., Chem. Ber. 114:1256, 1981; and Vorbrüggen, Acta Biochim. Pol., 43:25, 1996). The reaction resulted in high yield formation of desired β-configurated nucleoside derivative **5**. However, analogous to the coupling reaction of **4** with protected guanines, the formation of undesired *N-7* isomer (ratio of *N-9*/*N*-7 = 4: 1) was also detected. The mixture of the isomers was used for the ring closing reaction and protected LNA nucleoside **6** was isolated in 68 % yield as a crystalline compound. The correct structure of the isolated isomer was confirmed later by chemical conversion of LNA-I into LNA-A nucleoside (*vide infra*). Deprotection of the 5'-hydroxy group of **6** was accomplished via two-step procedure developed for the syntheses of other LNA nucleosides (Koshkin *et al., supra*). First, 5'-O-mesyl group was displaced by sodium benzoate to produce nucleoside **7**. The latter was converted into 5'-hydroxy derivative **8** after saponification of the 5'-benzoate. Direct removal of the 3'-O-benzyl group from compound **8** was unsuccessful under the conditions tested due to a solubility problem. Therefore, compound **8** was converted to DMT-protected nucleoside **9** prior to catalytic debenzylation of the 3'-*O*-hydroxy group. The phosphoramidite **11** was finally afforded via standard phosphitylation (McBride et al., Tetrahedron Lett. 24:245, 1983; Sinha et al., Tetrahedron Lett. 24:5843, 1983; and Sinha et al., Nucleic Acids Res. 12:4539, 1984) of the nucleoside **10.** In order to verify the correct orientation of the glycoside bond (N-9 isomer) in synthesized LNA-I nucleoside, compound **7** was successfully converted into the known LNA-A derivative **13** (Koshkin *et al., supra*) (Scheme 2). Thus, a treatment of 7 with phosphoryl chloride according to the procedure reported by Martin (Helv. Chim. Acta 78:486, 1995) resulted in a high yield formation of 6-chloropurine derivative **12**. The adenosine derivative **13** was derived from 1**2** after reaction with ammonia.

*Exemplary Analytical Data*

Data for compound **8** includes the following: mp 302-305°C (dec). $^1$H NMR (DMSO-$d_6$): δ 8.16, (s, 1H), 8.06 (s, 1H), 7.30-7.20 (m, 5H), 5.95 (s, 1H), 4.69 (s, 1H), 4.63 (s, 2H), 4.28 (s, 1H), 3.95 (d, *J* = 7.7, 1H), 3.83 (m, 3H). $^{13}$C NMR (DMSO-$d_6$): δ 156.6, 147.3, 146.1, 137.9, 137.3, 128.3, 127.6, 127.5, 124.5, 88.2, 85.4, 77.0, 72.1, 71.3, 56.7. MALDI-MS *m/z:* (M+H)$^+$. Anal. Calcd for $C_{18}H_{18}N_4O_5$·5/12 $H_2O$: C, 57,21; H, 5.02; N, 14.82. Found: C, 57,47; H, 4.95; N, 14.17.

*Exemplary Experimental Conditions*

(*1R,3R,4R,7S*)-7-(2-Cyanoethoxy(diisopropylamino)phosphinoxy)-1-(4,4'-dimethoxytrityloxymethyl)-3-(hyroxanthin-9-yl)-dioxabicyclor[2.2.1]heptane (**11**)

Compound **10** (530 mg, 0.90 mmol, described previously, (see for example, WO 00/56746) was dissolved in anhydrous EtOAc (5 mL) and cooled in an ice-bath. DIPEA (0.47 mL, 2.7 mmol) and (250 μL, 1.1 mmol) were added under intensive stirring. Formation of insoluble material was observed, and $CH_2Cl_2$ (3 mL) was added to produce a clear solution. More 2-cyanoethyl-N,N-diisopropylphosphoramidochloridite (200 μL, 0.88 mmol) was added after one hour, and the mixture was stirred overnight. EtOAc (30 mL) was added, the mixture was washed with sat. $NaHCO_3$ (2 x 50 mL), brine (50 mL), dried ($Na_2SO_4$), and concentrated to a solid residue. Purification by silica gel HPLC (1-5 %Me-OH/$CH_2Cl_2$ v/v, containing 0.1% of pyridine) gave compound **11** (495 mg, 75%) as a white solid material. $^{31}$P NMR (DMSO-$d_6$): δ 148.90.

*Synthesis of LNA-D*

Taking advantage of a high availability of the natural deoxy- and riboguanosines, a number of effective methods were developed for their conversion into 2,6-diaminopurine (D) nucleosides (Fathi et al., Tetrahedron Lett. 31:319, 1990; Gryaznov et al., Tetrahedron Lett., 35:2489, 1994; and Lakshman et al., Org. Lett., 2:927, 2000). However, the production of LNA-G nucleoside is a multi-step synthetic procedure.

Scheme for Synthesis of LNA-G

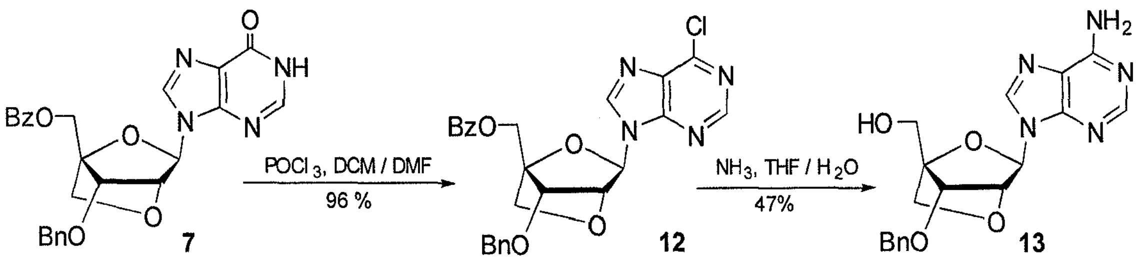


For the synthesis of LNA-D nucleoside, a novel synthesis method was developed that employed a common convergent scheme, related to the strategy used earlier for the synthesis of its anhydrohexitol counterpart (Boudou et al., Nucleic Acids Res. 27:1450, 1999). In particular, a properly protected carbohydride unit was conjugated with 6-chloro-2-aminopurine to give a stable 6-chloro intermediate derivative (Figure 16) which was further converted into desired diaminopurine nucleoside.

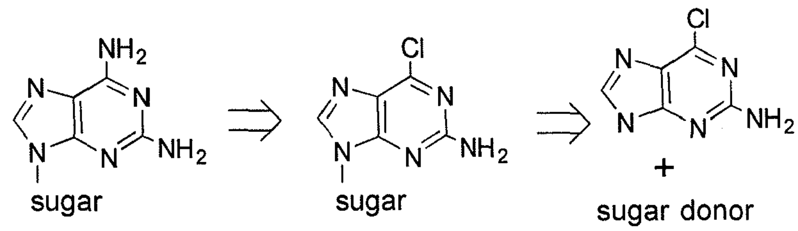

Thus, it was shown that glycosylation of 2-chloro-6-aminopurine with compound 4 resulted in highly stereoselective formation of the nucleoside derivative **14**. To promote the ring closing reaction, a solution of **14** in aqueous 1,4-dioxane was treated with 10-fold excess of sodium hydroxide to give bicyclic compound **15** in 87% yield. The standard reaction with sodium benzoate in hot DMF was then successfully applied for displacement of 5'-mesylate of **15**. Notably, this reaction proceeded in very selective manner and no side products originating from the modification of the nucleobase were detected. The desired compound **16** was precipitated from the reaction mixture after addition of water. In order to introduce the 6-amino group into nucleobase structure, intermediate 6-azido derivative **17** was synthesized via reaction of **16** with sodium azide. The nucleoside derivative **18** was isolated as a crystalline compound after saponification of the 5'-benzoate of **17.** Subsequent catalytic hydrogenation of **18** on palladium hydroxide resulted in simultaneous reduction of 6-azido and 3'-benzyl groups to give LNA-D diol **19** after crystallization from water. By the use of peracelation method, 2- and 6-amino groups of **19** were benzoylated at the next step to give the nucleobase protected derivative **20**, which was in the standard way further converted into phosphoramidite monomer **21**.

This phosphoramidite has been produced in a quantity of 0.5 grams.

*Exemplary Analytical Data*

Data for compound **19** includes the following: $^1$H NMR (DMSO-$d_6$): δ 7.81 (s, 1H), 6.78 (br s, 2H), 5.91 (br s, 2H), 5.71 (s, 1H), 5.66 (br s, 1H), 5.04 (br s, 1H), 4.31 (s, 1H), 4.20 (s, 1H), 3.90 (d, *J* = 7.7 Hz, 1H), 3.77 (m, 2H), 3.73 (d, *J* = 7.7 Hz, 1H). $^{13}$C NMR(DMSO-*$d_6$*): δ 160.5, 156.2, 150.9, 134.2, 113.4, 88.3, 85.0, 79.3, 71.5, 70.0, 56.8. MALDI-MS m/z: 295.0 $(M+H)^+$. Anal. Calcd for $C_{11}H_{14}N_6O_4 \cdot 1.5\ H_2O$: C, 41,12; H, 5.33; N, 26.15. Found: C, 41.24; H, 5.19; N, 25.80.

The $^{31}$P NMR (DMSO-*$d_6$*) spectrum for compound 24 contained signals at δ 149.19 and 148.98.

Data for compound **23** includes the following: crystallized from MeOH. mp. 227.5-229˚C (dec). $^1$H NMR (DMSO-$d_6$): δ 8.60 (s, 1H), 8.15 (s, 1H), 6.64 (br s, 2H), 5.82 (s, 1H), 5.71 (br s, 1H), 5.04 (br s, 1H), 4.40 (s, 1H), 4.21 (s, 1H), 3.92 (d, *J* = 7.7 Hz, 1H), 3.79 (m, 2H), 3.75 (d, *J* = 7.7 Hz, 1H). $^{13}$C NMR(DMSO-*$d_6$*): δ 160.6, 152.0, 149.4, 139.3, 127.1, 88.6, 84.8, 79.1, 71.6, 70.2, 56.8. MALDI-MS m/z: 334.7 $(M+H)^+$.

For protected compound **23,** the $^{31}$P NMR (DMSO-$d_6$) spectrum has a signal at 148.93 and 148.85.

*Exemplary Experimental Conditions*

<u>(*1S,3R,4R,7S*)-3-(2-amino-6-chloropurin-9-yl)-7-benzyloxy-1-methanesulfonoxymethxl-2,5-dioxabicyclo[2.2.1]heptane (15)</u>

To a solution of compound **14** (40 g, 64.5 mmol) in 1,4-dioxane (300 mL) was added 1 M NaOH (350 mL). The mixture was stirred for one hour at 0 ˚C, neutralized with AcOH (40 mL), and washed with $CH_2Cl_2$ (2 x 200 mL). The combined organic layers were dried ($Na_2SO_4$) and concentrated under reduced pressure. The solid residue was purified by silica gel flash chromatography to give compound **15** (27.1 g, 87%) as a white solid material. $^1$H NMR ($CDCl_3$): δ 7.84 (s, 1H), 7.32-7.26 (m, 5H), 5.91 (s, 1H), 4.73 (s, 1H), 4.66 (d, *J* = 11.7 Hz, 1H), 4.61 (d, *J* = 11.7 Hz, 1H), 4.59 (s, 2H), 4.31 (s, 1H), 4.18 (d, *J* = 8.0 Hz, 2H), 3.99 (d, J = 7.9 Hz, 1H), 3.05 (s, 3H). $^{13}$C NMR ($CDCl_3$) δ 158.9, 152.2, 151.4, 139.1, 136.4, 128.4, 128.2, 127.7, 125.3, 86.5, 85.2, 77.2, 76.8, 72.4, 72.1, 64.0, 37.7. MALDI-MS m/z 482.1$[M+H]^+$.

<u>(*1S,3R,4R,7S*)-3-(2-amino-6-chloropurin-9-yl)-1-benzoyloxymethyl-7-benzyloxy-2,5-dioxabicyclo[2.2.1]heptane (16)</u>

A mixture of sodium benzoate (7.78 g, 54 mmol) and compound **15** 13 g, 27 mmol) was suspended in anhydrous DMF (150 mL) and stirred for two hours at 105 ˚C. Ice-cold water (500 mL) was added to the solution under intensive stirring. The precipitate was filtered off, washed with water, and dried *in vacuo.* The intermediate product **16** (8 g) was used for ext step without further purification. Analytical sample was additionally purified by silica gel HPLC (0-2% MeOH/$CH_2Cl_2$v/v). $^1$H NMR ($CDCl_3$) δ 7.98-7.95 (m, 2H), 7.79 (s, 1H), 7.62-7.58 (m, 1H), 7.48-7.44 (m, 2H), 7.24 (m,

5H), 5.93 (s, 1H), 4.80 (d, *J* = 12.6 Hz, 1H), 4.77 (s, 1H), 4.67 (d, *J* = 11.9 Hz, 1H), 4.65 (d, *J* = 12.6 Hz, 1H), 4.56 (d, *J* = 11.9 Hz, 1H), 4.27 (d, *J* = 8.0 Hz, 1H), 4.25 (s, 1H), 4.08 (d, *J* = 7.9 Hz, 1H). $^{13}C$ NMR ($CDCl_3$) δ 165.7, 158.8, 152.1, 151.3, 138.9, 136.4, 133.4, 129.4, 129.0, 128.5, 128.4, 128.2, 127.6, 125.4, 86.4, 85.7, 77.2, 76.7, 72.5, 72.3, 59.5. MALDI-MS *m*/*z* 508.0 $[M+H]^+$.

(*1S,3R,4R,7S*)-3-(2-amino-6-azidopurin-9-yl)-7-benzyloxy-1-hydroxymethyl-2,5-dioxabicyclo[2.2.1]heptane (18)

All the amount of compound **16** from the previous experiment was dissolved in anhydrous DMSO (100 mL) and $NaN_3$ (5.4 g, 83 mmol) was added. The mixture was stirred for two hours at 100 ˚C and cooled to room temperature. Water (400 ml) was added, and the mixture was stirred for 30 minutes at 0 ˚C (ice-bath) to give a yellowish precipitate **17**. The precipitate was filtered off, washed with water, and dissolved in THF (25 mL). 2M NaOH (30 mL) was then added to the solution, and after 15 minutes of stirring the mixture was neutralized with AcOH (4 mL). The mixture was concentrated to approximately 1/2 of its volume and cooled in an ice-bath. The titel compound was collected by filtration, washed with cold water, and dried *in vacuo.* Yield: 8.8 g (79% from **15**). $^1H$ NMR (DMSO-$d_6$) δ 8.53 (br s, 2H), 8.23 (s, 1H), 7.31-7.26 (m, 5H), 6.00 (s, 1H), 5.26 (t, *J* = 5.7 Hz, 1H), 4.76 (s, 1H), 4.64 (s, 1H), 4.31 (s, 1H), 3.99 (d, *J* = 7.9 Hz, 1H), 3.88-3.85 (m, 3H). $^{13}C$ NMR (DMSO-$d_6$) δ 146.0, 144.0, 143.8, 137.9, 137.0, 128.3, 127.7, 127.6, 112.3, 88.3, 85.6, 77.1, 77.0, 72.2, 71.4, 56.8. MALDI-MS m/z 384.7 $[M+H]^+$ for 2,6-diaminopurine product, 410.5 $[M+H]^+$. Anal. Calcd for $C_{18}H_{18}N_8O_4$: C, 52.68; H, 4.42; N, 27.30. Found: C, 52.62; H, 4.36; N, 26.94.

(*1S*,*3R*,*4R*,*7S*)-3-(2,6-Diaminopurin-9-yl)-7-hydroxy- 1-hydroxymethyl-2,5-dioxabicyclo[2.2.1]heptane (19)

To a suspension of compound **18** (8 g, 19.5 mmol) in MeOH (100 mL) were added $Pd(OH)_2$/C (20%, 5.5 g) and $HCO_2NH_4$ (3g). The mixture was refluxed for 30 minutes and more $HCO_2NH_4$ (3g) was added. After refluxing for further 30 minutes, the catalyst was filtered off and washed with boiling MeOH/$H_2O$ (1/1 v/v, 200 mL). The combined filtrates were concentrated to approximately 100 mL and cooled in an ice-bath. The precipitate was filtered off, washed with ice-cold $H_2O$ and dried *in vacuo* to give compound **19** (5.4 g, 94 %) as a white solid material. $^1H$ NMR (DMSO-$d_6$): δ 7.81 (s, 1H), 6.78 (br s, 2H), 5.91 (br s, 2H), 5.71 (s, 1H), 5.66 (br s, 1H), 5.04 (br s, 1H), 4.31 (s, 1H), 4.20 (s, 1H), 3.90 (d, *J* = 7.7 Hz, 1H), 3.77 (m, 2H), 3.73 (d, *J* = 7.7 Hz, 1H). $^{13}C$ NMR (DMSO-$d_6$) δ 160.5, 156.2, 150.9, 134.2, 113.4, 88.3, 85.0, 79.3, 71.5, 70.0, 56.8. MALDI-MS m/z: 295.0 $(M+H)^+$. Anal. Calcd for $C_{11}H_{14}N_6O_4{\cdot}1.5\ H_2O$ C, 41,12; H, 5.33; N, 26.15. Found: C, 41.24; H, 5.19; N, 25.80.

(*1S*,*3R*,*4R*,*7S*)-3-(2,6-Di-(*N*-benzoylamino)purin-9-yl)-7-hydroxy-1-hydroxymethyl-2,5-dioxabicyclo[2.2.1]heptane (20)

A solution of compound **19** (0.5 g, 1.7 mmol) in anhydrous pyridine (20 mL) was cooled in an ice-bath and benzoyl chloride (1.5 mL, 12.9 mmol) was added under intensive stirring. The mixture was allowed to warm to room temperature and was stirred overnight. Ethanol (20 mL) and 2 M NaOH (20 mL) were added, and the mixture was stirred for an additional hour. EtOAc (75 mL) was added and the solution was washed with water (2 x 50 mL). The combined aqueous layers were washed with $CH_2Cl_2$ (2 x 50 mL). The combined organic phases were dried ($Na_2SO_4$) and concentrated under reduced pressure to a solid residue. The residue was suspended in $Et_2O$ (75 mL, under refluxing for 30 minutes) and cooled in an ice-bath. The product was collected by filtration, washed with cold $Et_2O$, and dried *in vacuo* to give compound **20** (530 mg, 62 %) as a slightly yellow solid material.

(*1R*,*3R*,*4R*,*7S*)-3-(2,6-Di-(N-benzoylamino)purin-9-yl)-1-(4,4'-dimethoxytrityloxymethyl)-7-hydroxy-2,5-dioxabicyclo [2.2.1]heptane (21)

Compound **20** (530 mg, 1.06 mmol) was co-evaporated with anhydrous pyridine (2 x 20 mL) and dissolved in anhydrous piridine (10 mL). DMT-Cl (600 mg, 1.77 mmol) was added, and the solution was stirred overnight at rt. The mixture was diluted with EtOAc (100 mL), washed with saturated $NaHCO_3$ (100 mL) and brine (50 mL). Organic layer was dried over $Na_2SO_4$ and concentrated under reduced pressure. Purification by silica gel HPLC (20-100% EtOAc/hexane v/v, containing 0.1 % of pyridine) gave compound **21** (670 mg, 79%) as a white solid material. **$^1H$** NMR ($CD_3OD$): δ 8.41 (s, 1H), 8.15-8.03 (m, 4H), 7.71-7.22 (m, 15H), 6.92-6.86 (m, 4H), 6.23 (s, 1H), 4.77 (s, 1H), 4.62 (s, 1H), 4.03 (d, J = 7.9 Hz, 1H), 3.99 (d, J = 7.9 Hz, 1H), 3.79 (s, 6H), 3.67 (d, J = 10.9 Hz, 1H), 3.54 (d, J = 10.8 Hz, 1H),. MALDI-MS m/z: 826 $(M+Na)^+$. Anal. Calcd for $C_{46}H_{40}N_6O_8{\cdot}H_2O$: C, 67.14; H, 5.14; N, 10.21. Found: C, 67.24; H, 4.97; N, 10.11.

*(1R,3R,4R,7S)*-7-(2-Cyanoethoxy(diisopropylamino)phosphinoxy)-3-(2 6-di-(*N*-benzoylamino)purin-9-yl)-1-(4,4'-dimethoxytrityloxymethyl)-2,5-dioxabicyclo[2.2.1]heptane (21)

To a stirred solution of compound **2**0 (640 mg, 0.8 mmol) in anhydrous DMF (5 mL) were added DIPEA (420 L, 2.4 mmol) and 2-cyanoethyl-N,N-diisopropylphosphoramidochloridite (300 μL, 1.2 mmol). The mixture was stirred for 1.5 hours at room temperature, diluted with EtOAc (100 mL), and washed with saturated $NaHCO_3$ (2 x 100 mL) and brine (50 mL). Organic layer was dried ($Na_2SO_4$) and concentrated under reduced pressure to give a yellow solid residue. Purification by silica gel HPLC (20-100 % EtOAc/hexene containing 0.1 % of pyridine) gave compound **21** (590 mg, 74%) as a white solid material. $^{31}P$ NMR (DMSO-$d_6$) δ 149.19, 148.98.

*Synthesis of Pac-protected LNA-D amidite*

Figure 17 illustrates a method for synthesizing a Pac-protected version of LNA-D amidite.

Compound 27

Compound **26** (1g, 3.39 mmol) was co-evaporated with anhydrous DMF (2 x 10 mL) and dissolved in DMF (10 mL). Imidazole (0.69 g, 10.17 mmol) and 1,3-dichloro-1,1,3,3-tetraisopropyldisiloxane (1.4 mL, 4.37 mmol) were added, and the mixture was stirred overnight. $H_2O$ (100 mL) was added under intensive stirring to precipitate nucleoside material. The precipitate was filtered off, washed with $H_2O$, and dried *in vacuo.* Crystallization from ethanol gave compound **27** (1.15 g, 63%) as a white solid material. MALDI-MS: *m*/*z* 537.3 $(M+H)^+$.

Compound 28

To a solution of compound **27** (1.15 g, 2.14 mmol) in anhydrous pyridine (5 mL) was added phenoxyacetic anhydride (2 g, 7.0 mmol) and the mixture was stirred for four hours. EtOAc (100 mL) was added, and the solution was washed with sat. $NaHCO_3$ (2 x 100 mL), brine (50 mL), dried ($Na_2SO_4$), and concentrated to a solid residue. Purification by silica gel HPLC (50-100% v/v EtOAc/hexane) gave compound **28** (1.65 g, 95%) as a white solid material. MALDI-MS: m/z 827.3 $(M+Na)^+$.

(1S,3R,4R,7S)-3-(2,6-Di-(*N*-phenoxyacetylamino)purin-9-yl)-7-hydroxy-1-hydroxymethyl-2,5-dioxabicyclor[2.2.1]heptane (29)

To a solution of compound **28** (0.96 g, 1.19 mmol) in anhydrous THF (10 mL) was added $Et_3N{\cdot}3HF$ (0.2 mL) and the mixture was stirred overnight at room temperature. The formed precipitate was collected by filtration and washed with THF (5 mL) and pentane (5 mL) to give after drying compound **29** (650 mg, 97%) as a white solid material. MALDI-MS: *m*/*z* 563.0 $(M+H)^+$.

*(1R,3R,4R,7S)-3-(*2,6-Di-(*N*-phenoxyacetylamino)-purin-9-yl)-1-(4,4'-dimethoxytrityloxymethyl)-7-hydroxy-2,5-dioxabicyclo[2.2.1]heptane (20)

To a solution of compound **29** (650 mg, 1.15 mmol) was added DMT-Cl (500 mg, 1.48 mmol). The mixture was stirred for five hours, diluted with EtOAc (100 mL), and washed with sat. $NaHCO_3$ (2 x 100 mL). The organic layer was dried and concentrated to a solid residue. Crystallization from EtOAc gave compound **30** (810 mg, 81%) as a white solid material.

(*1R,3R,4R,7S*)-7-(2-Cyanoethoxy(diisopropylamino)phosphinoxy)-3-(2.6-di-(*N*-phenoxyacetylamino)-purin-9-yl)-1-(4,4'-dimethoxytrityloxymethyl)-2,5-dioxabicyclo[2.2.1]heptane (21)

To a solution of compound **30** (800 mg, 0.92 mmol) in anhydrous DMF (10 mL) were added 0.75 M solution of DCI in EtOAc (0.7 mL) and 2-cyanoethyl tetraisopropylphosphorodiamidite (0.32 mL, 1.01 mmol). The mixture was stirred at room temperature overnight and EtOAc (75 mL) was added. The resulting solution was washed with sat. $NaHCO_3$ and brine, dried and concentrated to a solid residue. Purification by silica gel HPLC (30-100% v/v EtOAc/hexane, containing 0.1% of pyridine) gave phosphoramidite **31** (550 mg, 56%) as a white solid material.

$^{31}P$ NMR (DMSO-$d_6$): δ 149.08, 148.8.

*Synthesis of LNA-2AP*

The intermediate derivative **16** was also used for the synthesis of LNA-2AP nucleoside. First, the 5'-O-benzoyl group of **16** was hydrolyzed by aqueous sodium hydroxide to give the nucleoside derivative **22** in 72% yield (see Figure 18). The conditions of catalytic transfer hydrogenation usually used for removal of the 3'-O-benzyl group turned out to be suitable for complete dechlorination of the nucleobase of **22**. Thus, totally deprotected LNA-2AP nucleoside **23** was afforded in high yield after refluxing of the methanolic solution of **22** in the presence of paladium hydroxide and ammonium formate. The 2-amine of **23** was selectively protected with an amidine group after treatment with *N,N*-dimethylformamide dimethyl acetal. The resulting diol **24** was then 5'-O-DMT protected and 3'-O-phosphitylated to yield the desired phosphoramidite LNA-2AP monomer **25** (McBride et al., J. Am. Chem. Soc. 108:2040, 1986).

*Exemplary Experimental Conditions*

(*1S*,*3R*,*4R*,*7S*)-3-(2-amino-6-chloropurin-9-yl)-7-benzyloxy-1-hydroxymethyl-2,5-dioxabicyclo[2.2.1]heptane (22)

To a solution of compound **16** (3 g, 5.92 mmol) in 1,4-dioxane (20 mL) was added 2 M NaOH (20 mL) and the mixture was stirred for one hour. AcOH (3 mL) was added, and the solvents were removed under reduced pressure. The solid residue was re-dissolved in 20% MeOH/EtAc (50 mL), washed with $NaHCO_3$ (2 x 50 mL), dried ($Na_2SO_4$) and concentrated to a solid residue. The residue was purified by silica gel column chromatography (1-2% MeOH/EtAc v/v) to give compound **22** (1.72 g, 72%) as a white solid material.

(*1S*,*3R*,*4R*,*7S*)-3-(2-aminopurin-9-yl)-7-hydroxy-1-hydroxymethyl-2,5-dioxabicyclo[2.2.1]heptane (23)

To a solution of compound **22** (0.72 g, 1.79 mmol) in MeOH/dioxane (1/1 v/v) were added $Pd(OH)_2$/C (20%, 0.5 g) and $HCO_2NH_4$ (1.5 g, 23.8 mmol). The mixture was stirred under refluxing for 30 minutes and cooled to room temperature. The catalyst was filtered off and washed with MeOH. The combined filtrates were concentrated under reduced pressure to yield compound **23** (0.44 g, 89 %) as a white solid material. Analytical sample was crystallized from MeOH. mp. 227.5-229°C (dec). $^1$H NMR (DMSO-$d_6$): δ 8.60 (s, 1H), 8.15 (s, 1H), 6.64 (br s, 2H), 5.82 (s, 1H), 5.71 (br s, 1H), 5.04 (br s, 1H), 4.40 (s, 1H), 4.21 (s, 1H), 3.92 (d, *J* = 7.7 Hz, 1H), 3.79 (m, 2H), 3.75 (d, *J* = 7.7 Hz, 1H). $^{13}$C NMR(DMSO-*$d_6$*): δ 160.6, 152.0, 149.4, 139.3, 127.1, 88.6, 84.8, 79.1, 71.6, 70.2, 56.8.

(*1R*,*3R*,*4R*,*7S*)-1-(4,4'-dimethoxytrityloxymethyl)-3-(2-N-. (dimethylaminomethylidene)aminopurin-9-yl)-7-hydroxy-2,5-dioxabicyclo [2.2.1]heptane (5' DMT protected version of 24)

Compound **23** (0.4 g, 1.43 mmol) was co-evaporated with anhydrous DMF (10 mL) and dissolved in DMF (15 mL). *N*,*N*-Dimethylformamide dimethylacetal (0.8 mL) was added and the solution was stirred for three days at room temperature. Water (5 mL) was added, and the solvents were removed under reduced pressure. The solid residue was co-evaporated with anhydrous pyridine (2 x 10 mL) and dissolved in anhydrous pyridine (5 mL). DMT-Cl (0.7 g, 2.1 mmol) was added, the solution was stirred for four hours, diluted with EtOAc (50 mL), and washed with $NaHCO_3$ (2 x 50 mL) and brine (50 mL). Organic layer was dried ($Na_2SO_4$) and concentrated to a yellow solid residue. Purification by silica gel HPLC (1-6% MeOH/$CH_2Cl_2$ v/v, containing 0.1% of pyridine) gave the 5' DMT protected version of compound **24** (0.87 g, 87%) as a white solid material.

(*1R*,*3R*,*4R*,*7S*)-7-(2-Cyanoethoxy(diisopropylamino)phosphinoxyl-1-(4,4'-dimethoxytrityloxymethyl)-3-(2-N-(dimethylaminomethylidene)aminopurin-9-yl)-2,5-dioxabicvclor2.2.1)heptane (25)

The 5' DMT protected version of compound **24** (0.5 g, 0.79 mmol) was dissolved in anhydrous DMF (10 mL) and DIPEA (350 μL) and 2-cyanoethyl-*N,N*-diisopropylphosphoramidochloridite (250 μL) were added. The mixture was stirred for one hour, diluted with EtOAc (50 mL), washed with saturated $NaHCO_3$ (2 x 100 mL) and brine (50 mL), dried ($Na_2SO_4$), and concentrated to a solid residue. Purification by silica gel HPLC (0-3% MeOH/$CH_2Cl_2$ v/v, containing 0.1% of pyridine) gave compound **25** (0.42 g, 64%) as a white solid material. $^{31}$P NMR (DMSO-$d_6$) δ 148.93, 148.85.

*Synthesis of Oligomers*

Along with previously described LNA phosphoramidites (Koshkin *et al., supra;* and Pedersen et al., Synthesis p. 802, 2002), the phosphoramidite monomers **11**, **21**, and **25** were successfully applied for automated oligonucleotide synthesis (Caruthers, Acc. Chem. Res. 24:278, 1991) to produce the LNA oligomers depicted in Table 4. Oligonucleotide syntheses were performed on a 0.2 μmol scale using an Expedite synthesizer (Applied Biosystems) with the recommended commercial reagents. Standard protocols for DNA synthesis were used, except that the coupling time was extended to 5 minutes and the oxidation time was extended to 30 second cycles. Deprotection of the oligonucleotides were performed by treatment with concentrated ammonium hydroxide for five hours at 60 ˚C. After that, the LNA-D containing oligonucleotides were additionally treated with AMA (concentrated ammonium hydroxide / 40% aqueous $MeNH_2$; 1/1 v/v) for one hour at 60˚C. All the synthesized oligonucleotides were purified by RP-HPLC, and their structures were verified by MALDI-TOF mass spectra.

The complexing properties of oligonucleotides containing new LNA monomers **1-3** were assessed. Comparative binding data from an 8-mer LNA sequence is shown in Table 4 as the melting temperatures against complementary single-stranded DNA. An exemplary sequence for this comparison is GACATAGG, which is the central part of a capture probe used for SNP detection in GludVS7-7asA (A:a mismatch position). The thermal stabilities of reference DNA duplexes (entries 1-7, Table 4) can be directly compared with their LNA counterparts (entries 8-14). The hybridizing ability of all LNA 8-mers is superior to that of isosequencial DNA oligonucleotides. The average melting temperatures of DNA and LNA 8-mers against complementary DNAs typically differ by about 40˚C. The replacement of one internal LNA-A nucleotide by LNA-D resulted in the further stabilization of the complementary duplex (i.e., compare entries 8 and 11) by 6.2˚C. Interestingly, the analogous replacement made in an DNA octamer destabilized the corresponding duplex by 0.5˚C (i.e., entries 1 and 4). D-nucleosides may facilitate a B to A helix transition, because the A-type structure of an LNA: DNA duplex is more suitable for effective D:t pairing. This stabilizing effect is expected to be even more pronounced for LNA: RNA duplexes, which can be very useful for construction of antisense or other gene-silencing reagents. The mismatch discrimination ability of the D-nucleoside was also studied (entry 11). In comparison to LNA-A (entry 8) D-nucleoside demonstrated remarkable increased mismatch discrimination against DNA-g nucleoside.

Table 4. Melting temperatures ($T_m$) of the complementary DNA-DNA and LNA-DNA duplexes.[a] Modified monomers (LNA are in CAPITALS): **I** = inosine; **D** = 2,6-diaminopurine; **X** = 2-aminopurine.

| Entry | Oligonucleotide structure | $T_m$ (± 0.5˚C) of the duplexes with complementary deoxynucleotide | | | |
|---|---|---|---|---|---|
| | | 3'-ctg**t**atcc | 3'-ctg**a**atcc | 3'-ctg**g**atcc | 3'-ctg**c**atcc |
| 1 | 5'-gac**a**tagg | **23.8** | <10 | <10 | <10 |
| 2 | 5'-gac**t**tagg | <10 | **22.6** | <10 | <10 |
| 3 | 5'-gac**g**tagg | <10 | <10 | <10 | **25.0** |
| 4 | 5'-gac**d**tagg | **23.3** | <10 ‘ | <10 | <10 |
| 5 | 5'-g**d**c**d**t**d**gg | **33.4** | <10 | <10 | 17.7 |
| 6 | 5'-gac**i**tagg | <10 | <10 | <10 | 20.9 |
| 7 | 5'-gac**x**tagg | <10 | <10 | <10 | <10 |
| 8 | 5'-GAC**A**TAGG | **61.6** | 38.2 | 43.4 | 40.6 |
| 9 | 5'-GAC**T**TAGG | 28.0 | **60.7** | 36.4 | 23.5 |
| 10 | 5'-GAC**G**TAGG | 55.0 | 32[b] | 41[b] | **70.9** |
| 11 | 5'-GAC**D**TAGG | **67.8** | 42.2 | 41.4 | 52.4 |
| 12 | 5'-G**D**C**D**T**D**GG | **78.3** | 55.9 | 54.7 | 63.8 |
| 13 | 5'-GAC**I**TAGG | 53.1 | 48.2 | 43.0 | **59.9** |
| 14 | 5'-GAC**X**TAGG | **60.8** | 45.5 | 44.0 | 53.9 |

[a] The melting temperatures ($T_m$ values) were obtained as a maxima of the first derivative of the corresponding melting curves (optical density at 260 nm versus temperature).
Concentration of the duplexes: 2.5 μM. Buffer: 0.1 M NaCl; 10 mM Na-phosphate (pH 7.0); 1 mM EDTA.
[b] Low cooperativity of transitions (accuracy ± 1˚C).

Table 5. The mismatch discrimination effect of the chimeric LNA-DNA 12-mers containing LNA-A or LNA-D nucleosides against the point of mutation

| The structure of LNA-DNA oligonucleotide | $T_m$ (± 0.5˚C) of the complementary duplexes with DNA oligonucleotides (Δ $T_m$ between singly mismatched and perfect duplexes) | |
|---|---|---|
| HNFas128A-2 | | |
| | caaca**t**cccaca (SEQ ID NO: 19) | caaca**a**cccaca (SEQ ID NO: 20) |
| tGtggG**A**TGttg | 61.0 | 45.9 (-15.1) |
| tGtggG**D**TGttg | 65.5 | 49.7 (-15.8) |
| Gluc53as-A | | |
| | aagag**t**ccagtg (SEQ ID NO: 23) | aagag**g**ccagtg (SEQ ID NO: 24) |
| cAmCtgG**A**mCtctt | 61.5 | 50.6 (-10.9) |
| cAmCtgG**D**mCtctt | 65.3 | 45.4 (-19.9) |
| [a] Concentration of duplexes: 2 μM; Buffer: see Table 4. | | |

Table 6. Melting temperatures of the LNA and DNA duplexes (LNAs are CAPITALIZED) containing 2-thio-deoxythymidine (**s**) and diaminopurineriboside (**d**). See Table 4 for experimental conditions.

| oligo structure | $T_m$ (± 0.5˚C) of complementary duplexes with | | | | |
|---|---|---|---|---|---|
| | 3'-ctg**t**atcc | 3'-ctg**s**atcc | 3'-CTG**s**ATCC | 3'-CTG**t**ATCC | 3'-CTG**T**ATCC |
| 5'-gac**a**tagg | **23.8** | **27** | **54.4** | **49.4** | **54.6** |
| 5'-gac**d**tagg | **23.3** | **<6** | **45.4** | **55.2** | **60.5** |
| 5'-GAC**A**TAGG | **61.6** | **64.6** | **87*** | **88** | **88** |
| 5'-GAC**D**TAGG | **67.8** | **59.4** | **80** | **>90** | **>90** |
| * $T_m$ values in the shaded cells were measured in low salt buffers (1 mM Na-phosphate, pH 7.0). Low cooperativity of the transitions was observed (accuracy ±1.5˚C). | | | | | |

Likewise, oligonucleotides containing LNA-D were evaluated against RNA, see Table 10. Thus the incorporation of LNA-D instead of LNA-A gave a general increase in $T_m$ of 5˚C per modification while retaining discrimination abilities.

Table 10: $T_m$s[a] of the duplexes containing SBC-LNA 8-mers and their RNA-LNA controls. Modified monomers (LNA monomers are in CAPITAL): **D** = LNA 2,6-diaminopurine; C = LNA methyl-C

| Entry | Oligonucleotide structure | $T_m$ (± 0.5˚C) of the duplexes with complementary **RNA** | | | |
|---|---|---|---|---|---|
| | | 3'-ctg**t**atcc | 3'-ctg**a**atcc | 3'-ctg**g**atcc | 3'-ctg**c**atcc |
| 1 | 5'-GAC**T**TAGG | 48.1 | **75.6** | 65.2 | 49.5 |
| 2 | 5'-GAC**G**TAGG | 66.7 | 48[b] | 58.2 | **80.8** |
| 3 | 5'-GAC**A**TAGG | **70.3** | 53.7 | 55.2 | 55.8 |
| 4 | 5'-GAC**D**TAGG | **75.6** | 54.7 | 55.0 | 65.6 |
| 5 | 5'-G**D**C**D**T**D**GG | **86.0** | 66.2 | 66.4 | 75.6 |
| [a] The melting temperatures ($T_m$ values) were obtained as a maxima of the first derivatives of the corresponding melting curves (optical density at 260 nm vesus temperature).<br>Concentration of the duplexes: 2.5 μM. Buffer: 0.1 M NaCl; 10 mM Na-phosphate (pH 7.4); 1 mM EDTA.<br>[b]Low cooperativity of transition (accuracy ± 1 ˚C). | | | | | |

Example 14: Exemplary Methods for Synthesizing LNA-PyrroloPyr-SBC-C

The furanopyrimidine phosphoramidite **6pC** used for incorporation of the pyrroloC analogue can be synthesized from LNA-U through a series of reactions as illustrated below and in Figure 14. Starting from LNA-U **1pC** iodine can be introduced on the 5 position on the nucleobase (Chang and Welch, J. Med. Chem. 1963, 6, 428). This compound can be used in a Sonogashira type palladium coupling reaction (Sonogashira, Tohda and Hagihara, Tetrahedron Lett. 1975, 4467) resulting in the 5-ethynyl-LNA-U **3pC.** The 5-ethynyl-LNA-U **3pC** can be transformed to the furanopyrimidie LNA analogue **4pC** when reacted with CuI, and then transformed into the DMT-protected phosphoramidite **6pC** (Woo, Meyer, and Gamper, Nucleic Acids Res., 1996, 24, 2470). LNA-PyrroloPyr-SBC-C is formed when **6pC** or an oligonucleotide containing **6pC** is deprotected with ammonia.

Example 15: Thermal denaturation studies

The thermal denaturation experiments were performed on a Perkin-Elmer UV/VIS spectrometer fitted with a PTP-6 Peltier temperature-programming element using a medium salt buffer solution (10 mM sodium phosphate, 100 mM sodium chloride, 0.1 mM EDTA, pH 7.0). Concentrations of 1.5 mM of the two complementary strands were used assuming identical extinction coefficients for modified and unmodified oligonucleotides. The absorbance was monitored at 260 nm while raising the temperature at a rate of 1 ˚C per min. The melting temperatures ($T_m$ values) of the duplexes were determined as the maximum of the first derivatives of the melting curves obtained.

Other Embodiments

From the foregoing description, it will be apparent that variations and modifications may be made to the invention described herein to adopt it to various usages and conditions. The foregoing description of the invention is merely illustrative thereof, and it understood that variations and modifications can be effected without departing from the scope or spirit of the invention.

All publications, patent applications, and patents mentioned in this specification are herein incorporated by reference to the same extent as if each independent publication, patent, or patent application was specifically and individually indicated to be incorporated by reference.

Particular embodiments

The invention can also be presented by means of the following embodiments represented by the following items:

Item 1. A population of nucleic acids bonded to a solid support, said population comprising a first population of nucleic acids of the same length, said length being in the range of 5-15 nucleotides or units, said first population representing at least 1% of the possible different nucleic acid sequences for nucleic acids of said length, at least one nucleic acid in the first population being an LNA oligomer.

Item 2. The population of item 1, wherein at least 90% of the nucleic acids in the first population are LNA oligomers.

Item 3. The population of any of the preceding items, wherein the variance in the melting temperature of the first population is at least 50% less than the variance in the melting temperature of the corresponding control population of nucleic acids.

Item 4. The population of any of the preceding items, wherein the variance in the melting temperature of the first population is less than 25˚C.

Item 5. The population of any of the preceding items, wherein at least one LNA oligomer of the first population has a melting temperature that is at least 20˚C higher than that of the corresponding control nucleic acid.

Item 6. The population of item 5, wherein at least 90% of the nucleic acids in the first population are LNA oligomers with a melting temperature that is at least 20˚C higher than that of the corresponding control nucleic acid.

Item 7. The population of any of the preceding items, wherein the first population has at least one LNA oligomer with a capture efficiency that is at least 500% greater than that of the corresponding control nucleic acid at the temperature equal to the melting temperature of the nucleic acid of the first population.

Item 8. The population of item 7, wherein at least 90% of the nucleic acids in the first population are LNA oligomers with a capture efficiency that is at least 500% greater than that of the corresponding control nucleic acid at the temperature equal to the melting temperature of the nucleic acid of the first population.

Item 9. The population of any of the preceding items, wherein at least 90% of the nucleic acids in the first population are LNA oligomers with a melting temperature that is at least 25˚C higher than that of the corresponding control nucleic acid and with a capture efficiency at least 800% greater than that of the corresponding control nucleic acid at the temperature equal to the melting temperature of the nucleic acid of the first population.

Item 10. The population of any of the preceding items, wherein the length of the nucleic acids in the first population is 5, 6, 7, 8, 9, or 10 nucleotides or units.

Item 11. The population of any of the preceding items, wherein the first population includes 1-9% of the possible different nucleic acid sequences for nucleic acids of that length.

Item 12. The population of any of the preceding items, wherein the first population of nucleic acids has at least 100 different nucleic acids.

Item 13. The population of any of the preceding items, wherein at least one LNA oligomer has at least one LNA unit selected from the group consisting of LNA C, LNA G, LNA U, LNA A and LNA T.

Item 14. The population of item 13, wherein at least one LNA oligomer has at least one LNA unit selected from the group consisting of LNA A and LNA T.

Item 15. The population of item 14, wherein each LNA oligomer has at least one LNA unit selected from the group consisting of LNA A and LNA T.

Item 16. The population of any of the preceding items, wherein all of the adenine and thymine-containing nucleotides in the LNA oligomers are LNA A and LNA T, respectively.

Item 17. The population of any of the preceding items, wherein all of the adenine and cytosine-containing nucleotides in the LNA oligomers are LNA A and LNA C, respectively.

Item 18. The population of any of the preceding items, wherein the first population only has nucleic acids and LNA oligomers with naturally-occurring nucleobases.

Item 19. The population of any of the preceding items, wherein the position of LNA units in the LNA oligomers has been chosen to reduce their propensity to form hairpins, dimer duplexes or other secondary structures that would otherwise inhibit or prevent their binding to a target nucleic acid.

Item 20. The population of item 19, wherein the position of LNA units in each LNA oligomer has been chosen by an algorithm substantially as described in Example 6 to reduce their propensity to form hairpins dimer duplexes or other secondary structures.

Item 21. The population of any of the preceding items, wherein opposing nucleotides in a palindrome pair or opposing nucleotides in inverted repeats are not both LNA units.

Item 22. The population of any of the preceding items, wherein the nucleic acids in the first population form less than 3 intramolecular base-pairs.

Item 23. The population of any of the preceding items, wherein the first population comprises nucleic acids wherein at least one nucleotide or unit includes an SBC nucleobase.

Item 24. The population of item 23, wherein the SBC nucleobase is selected from the group consisting of 2,6-diaminopurine, 2-thio-thymine and 2-thio-uracil.

Item 25. The population of item 24, wherein at least one LNA oligomer has at least one LNA unit with a nucleobase selected from the group consisting of 2,6,-diaminopurine, 2-thio-thymine and 2-thio-uracil.

Item 26. The population of any of the preceding items, wherein the first population comprises an LNA oligomer as defined in any of the items 51, 57 and 60.

Item 27. The population of any of the preceding items, wherein the first population comprises nucleic acids wherein at least one nucleotide or unit includes a universal nucleobase.

Item 28. The population of item 27, wherein one or more nucleic acids of the first population have a nucleotide or unit that includes a universal nucleobase located at the 5' or 3' terminus of the nucleic acid.

Item 29. The population of item 28, wherein one or more nucleic acids of the first population have one or more nucleotides or units that include a universal bases located at the 5' and 3' termini of the nucleic acid.

Item 30. The population of item 29, wherein all nucleic acids of the first population have at least one nucleotide or unit that includes a universal nucleobase.

Item 31. The population of any of items 27-30, wherein said universal nucleobases are selected from the group consisting of hypoxanthine, pyrene, 3-nitropyrrole and 5-nitroindole.

Item 32. The population of any of item 27-31, wherein the first population comprises an LNA oligomer as defined in item 45.

Item 33. The population of any of the preceding items, wherein LNA units of the LNA oligomer(s) have the formula

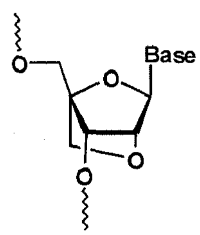

wherein "Base" designates a nucleobase.

Item 34. The population of any of the preceding items, wherein the nucleic acids of the first population are bonded to the solid support in a predefined arrangement.

Item 35. A method for detecting the presence of one or more target nucleic acids in a sample, said method comprising (a) incubating said sample comprising said one or more target nucleic acids with the population of nucleic acids defined in any of items 1-40, under conditions that allow at least one of said target nucleic acids to hybridize to at least one of the nucleic acids in said population of nucleic acids.

Item 36. The method of item 35, wherein the hybridization is detected between at least 10 target nucleic acids and the nucleic acids of the first population.

Item 37. The method of any of items 35-36, wherein the one or more target nucleic acids include(s) a nucleic acid of a pathogen (e.g. a nucleic acid in a sample such as a blood or urine sample from a mammal).

Item 38. The method defined in any of the items 35-37, further comprising the step of (b) detecting the hybridization.

Item 39. The method of any of items 35-38, wherein at least 10 target nucleic acids hybridize to the nucleic acids of the first population.

Item 40. A method for classifying a test nucleic acid sample comprising target nucleic acids, said method comprising the steps of:

(a) incubating a test nucleic acid sample with the population of nucleic acids as defined in any of items 1-40 under conditions that allow at least one of the nucleic acids in said test sample to hybridize to at least one nucleic acid in said population;

(b) detecting the hybridization pattern of said test nucleic acid sample; and

(c) comparing said hybridization pattern to the hybridization pattern of a first nucleic acid standard.

Item 41. The method of item 40, wherein said comparison indicates whether or not said test sample has the same classification as said first standard.

Item 42. The method of any of items 40-41, further comprising the step of (d) comparing the hybridization pattern of said test nucleic acid sample to the hybridization pattern of a second standard.

Item 43. The method of any of items 40-42, wherein the identification of the target nucleic acid is performed by comparing the hybridization pattern thereof to the hybridization pattern of said standard.

Item 44. The method of any of items 40-43, wherein the hybridization pattern of the test nucleic acid sample is compared to at least 10 standards and deconvolved to determine the abundance of each standard in said sample.

Item 45. A complex of one or more target nucleic acids and the population of nucleic acids defined in any of the items 1-34, wherein one or more target nucleic acids are hybridized to the population of nucleic acids.

Item 46. The complex of item 45, wherein at least 10 different target nucleic acids are hybridized.

Item 47. The complex of any of items 45-46, wherein the target nucleic acids are cDNA molecules reverse transcribed from a patient sample.

Item 48. An LNA monomer being LNA-hypoxanthine (LNA-I) of the formula

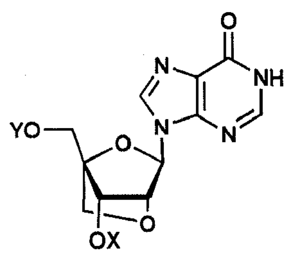

wherein X is a phosphoamidite group and Y is an oligonucleotide compatible hydroxyl-protection group such as DMT.

Item 49. A method of synthesizing the LNA-hypoxanthine (LNA-I) monomer defined in item 48, essentially comprising the steps described in Example 13 herein.

Item 50. An LNA oligomer comprising a LNA-hypoxanthine (LNA-I) unit as shown in formula 1 below

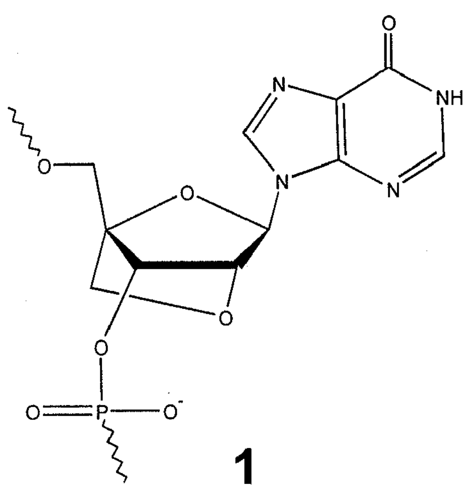

**1** .

Item 51. An LNA monomer being LNA-2,6-diaminopurine (LNA-D) of the formula

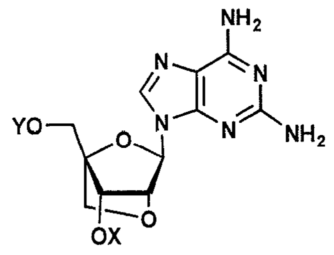

wherein X is a phosphoamidite group and Y is an oligonucleotide compatible hydroxyl-protection group such as DMT.

Item 52. A method of synthesizing the LNA-2,6-diaminopurine (LNA-D) monomer defined in item 51, essentially comprising the steps described in Example 13 herein.

Item 53. An LNA oligomer comprising an LNA-2,6-diaminopurine (LNA-D) unit as shown in formula 2 below

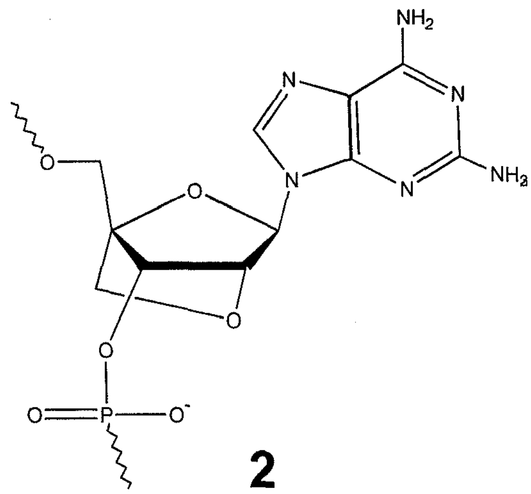

**2**

Item 54. An LNA monomer being LNA-2-aminopurine (LNA-2AP) of the formula

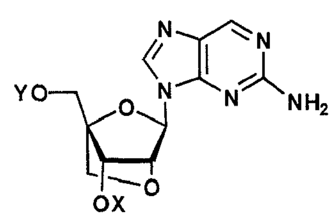

wherein X is a phosphoamidite group and Y is an oligonucleotide compatible hydroxyl-protection group such as DMT.

Item 55. A method of synthesizing the LNA-2-aminopurine (LNA-2AP) monomer defined in item 54, essentially comprising the steps described in Example 13 herein.

Item 56. An LNA oligomer comprising an LNA-2-aminopurine (LNA-2AP) unit as shown in formula 3 below

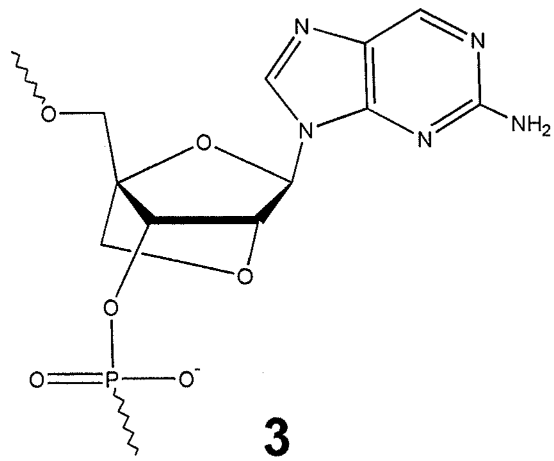

3

.

Item 57. An LNA monomer being LNA-2-thiothymine (LNA-$^{2S}$T) of the formula

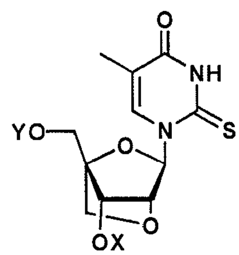

wherein X is a phosphoamidite group and Y is an oligonucleotide compatible hydroxyl-protection group such as DMT.

Item 58. A method of synthesizing the LNA-2-thiothymine (LNA-$^{2S}$T) monomer defined in item 57, essentially comprising the steps described in Example 12 herein.

Item 59. An LNA oligomer comprising an LNA-2-thiothymine (LNA-$^{2S}$T) unit as shown in formula 4 below

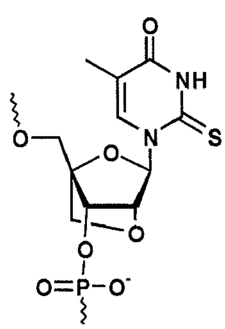

.

Item 60. An LNA monomer being LNA-2-thiouracil (LNA-$^{2S}$U) of the formula

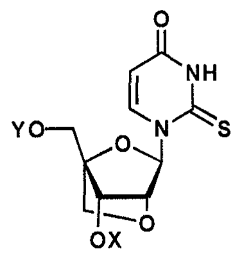

wherein X is a phosphoamidite group and Y is an oligonucleotide compatible hydroxyl-protection group such as DMT.

Item 61. A method of synthesizing the LNA-2-thiouracil (LNA-$^{2S}$U) monomer defined in item 60, essentially comprising the steps described in Example 11 herein.

Item 62. An LNA oligomer comprising an LNA-2-thiouracil (LNA-$^{2S}$U) unit as shown in formula 5 below

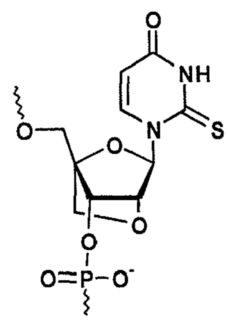

.

Item 63. A pair of substantially complementary oligonucleotides, each comprising, in pairwise opposing positions, one or more SBC nucleotides or units, wherein at least one of the oligonucleotides is an LNA oligomer having SBC LNA units.

Item 64. An array including a solid support and a population of nucleic acids bonded to said solid support, said population comprising a first population of nucleic acids of the same length, said length being in the range of 5-15 nucleotides or units, said first population representing at least 1% of the possible different nucleic acid sequences for nucleic acids of said length, at least 50% of the nucleic acids in the first population being LNA oligomers, and the variance in the melting temperature of the first population is at least 50% less than the variance in the melting temperature of the corresponding control population of nucleic acids.

**Claims**

**1.** An LNA monomer being LNA-2,6-diaminopurine (LNA-D) of the formula

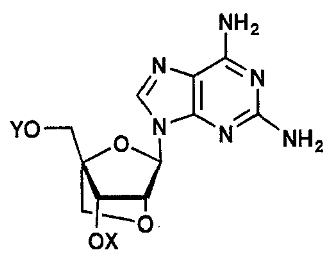

wherein X is a phosphoamidite group and Y is an oligonucleotide compatible hydroxyl-protection group such as DMT.

**2.** A method of synthesizing the LNA-2,6-diaminopurine (LNA-D) monomer defined in claim 1, essentially comprising the steps described in Example 13 herein.

**3.** An LNA oligomer comprising an LNA-2,6-diaminopurine (LNA-D) unit as shown in formula 2 below

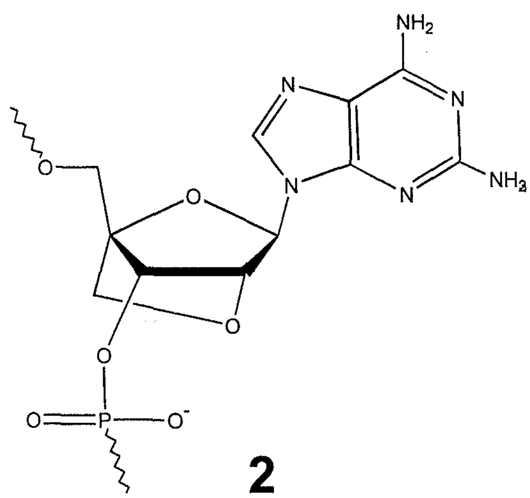

**2**

**4.** An LNA monomer being LNA-2-aminopurine (LNA-2AP) of the formula

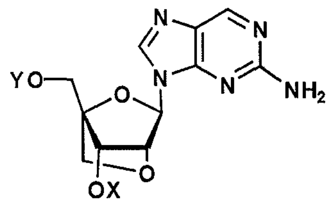

wherein X is a phosphoamidite group and Y is an oligonucleotide compatible hydroxyl-protection group such as DMT.

**5.** A method of synthesizing the LNA-2-aminopurine (LNA-2AP) monomer defined in claim 54, essentially comprising the steps described in Example 13 herein.

**6.** An LNA oligomer comprising an LNA-2-aminopurine (LNA-2AP) unit as shown in formula 3 below

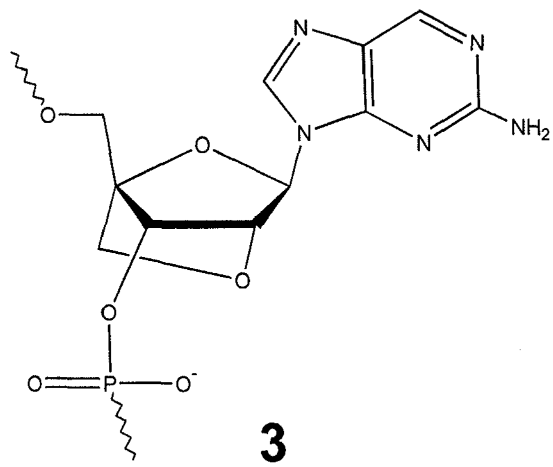

**3**

.

**7.** A pair of substantially complementary oligonucleotides, each comprising, in pairwise opposing positions, one or more SBC nucleotides or units, wherein at least one of the oligonucleotides is an LNA oligomer having SBC LNA units.

Figure 1

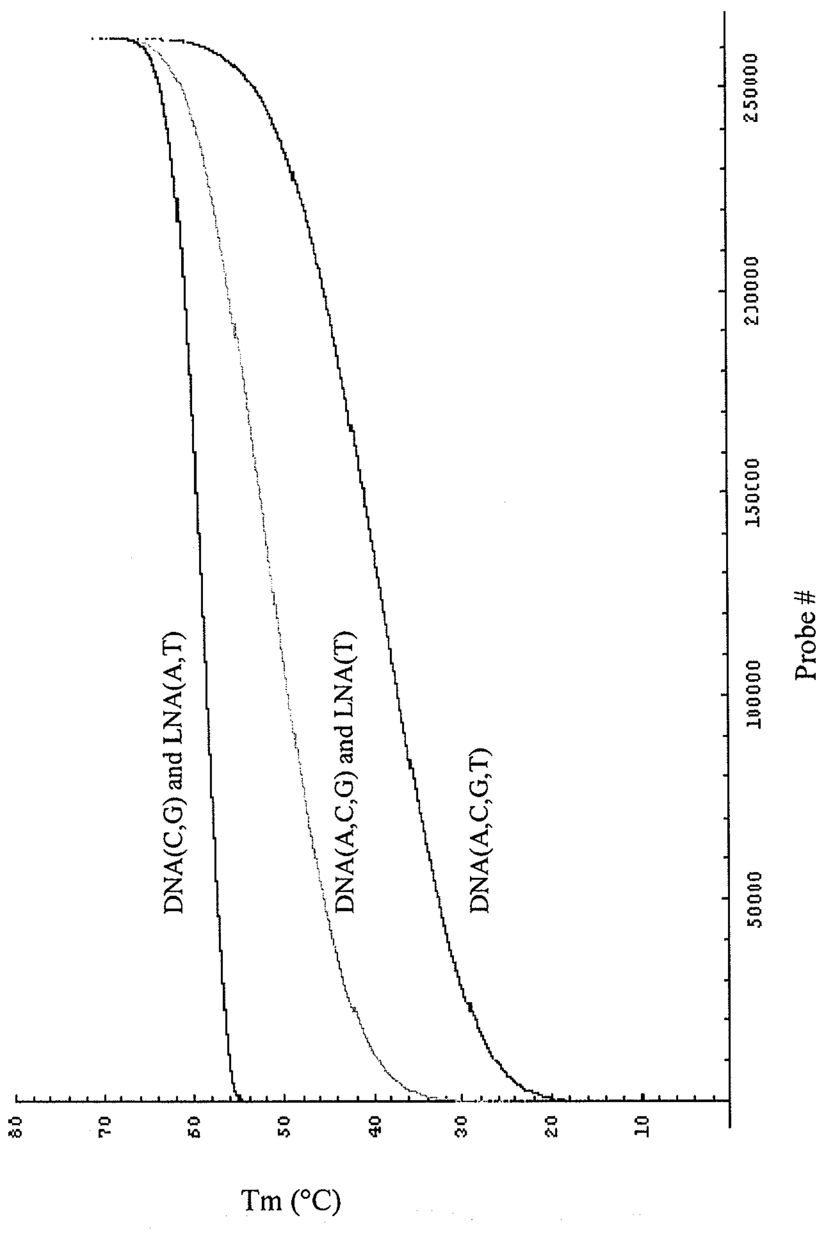
DNA(C,G) and LNA(A,T)
DNA(A,C,G) and LNA(T)
DNA(A,C,G,T)
Probe #
Tm (°C)
80
70
60
50
40
30
20
10
50000
100000
150000
200000
250000

Figure 2

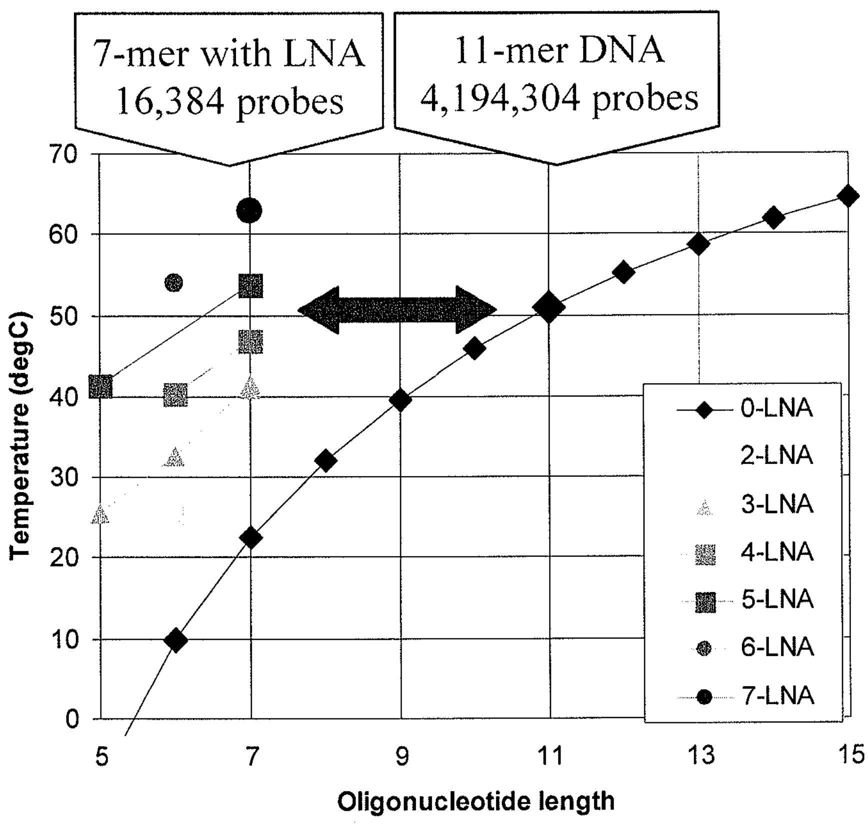
7-mer with LNA
16,384 probes
11-mer DNA
4,194,304 probes
70
60
50
40
30
20
10
0
Temperature (degC)
5
7
9
11
13
15
Oligonucleotide length
0-LNA
2-LNA
3-LNA
4-LNA
5-LNA
6-LNA
7-LNA

Figure 3

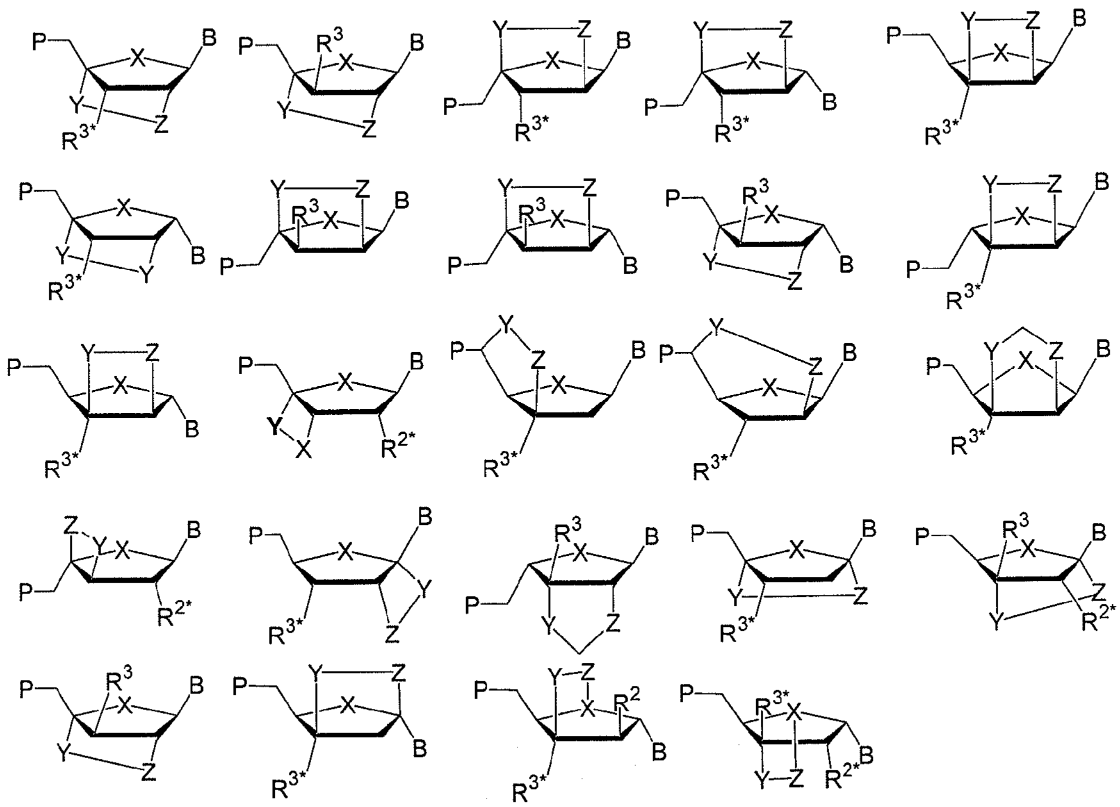

Figure 4

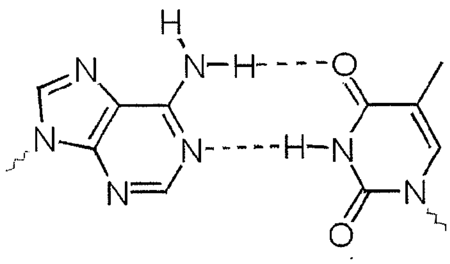

A - T

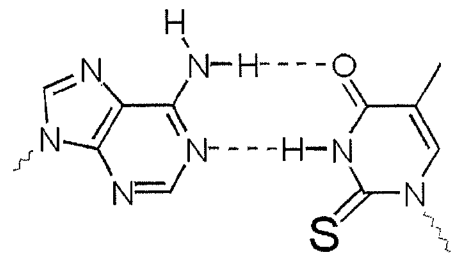

A - $^{2S}$T

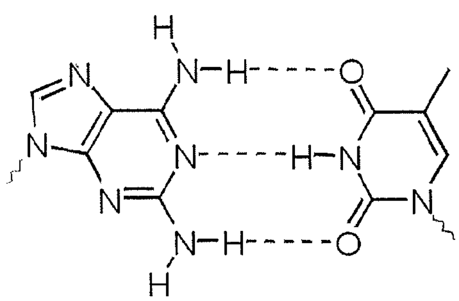

D - T

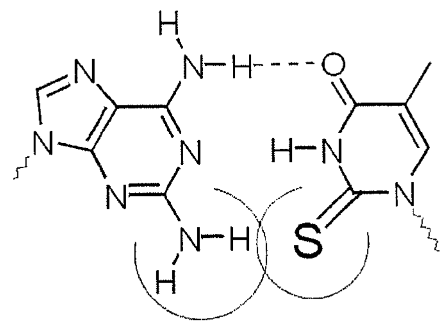

D - $^{2S}$T

Figure 5

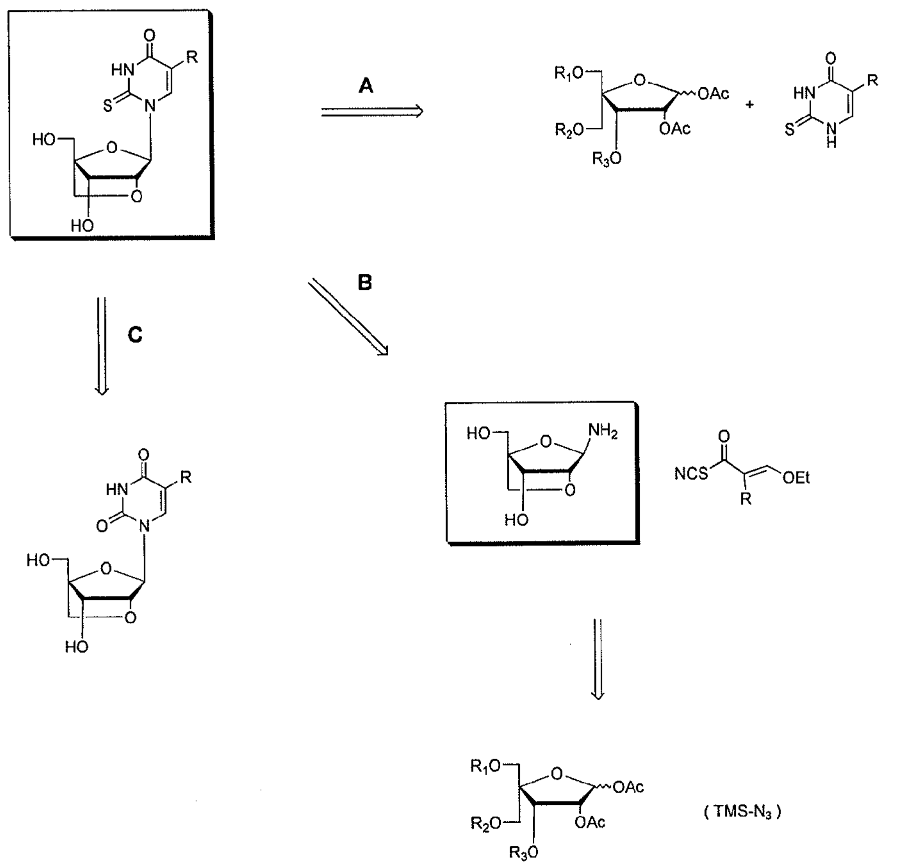

Figure 6

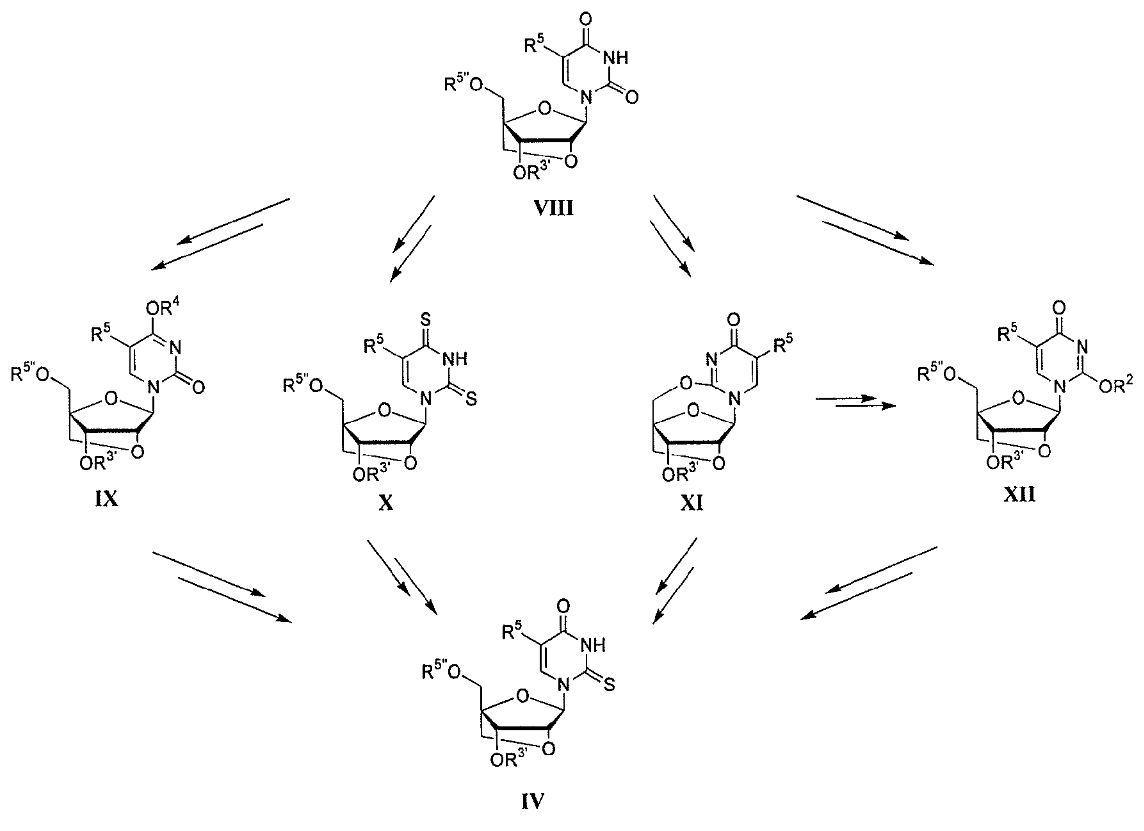

VIII

IX

X

XI

XII

IV

Figure 7

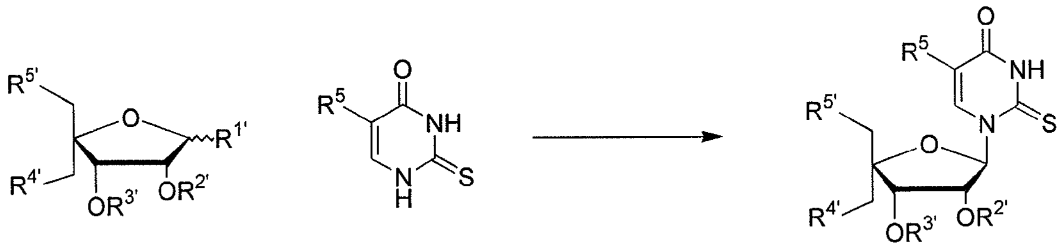

I II III

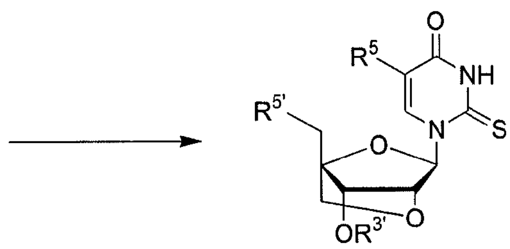

IV

Figure 8

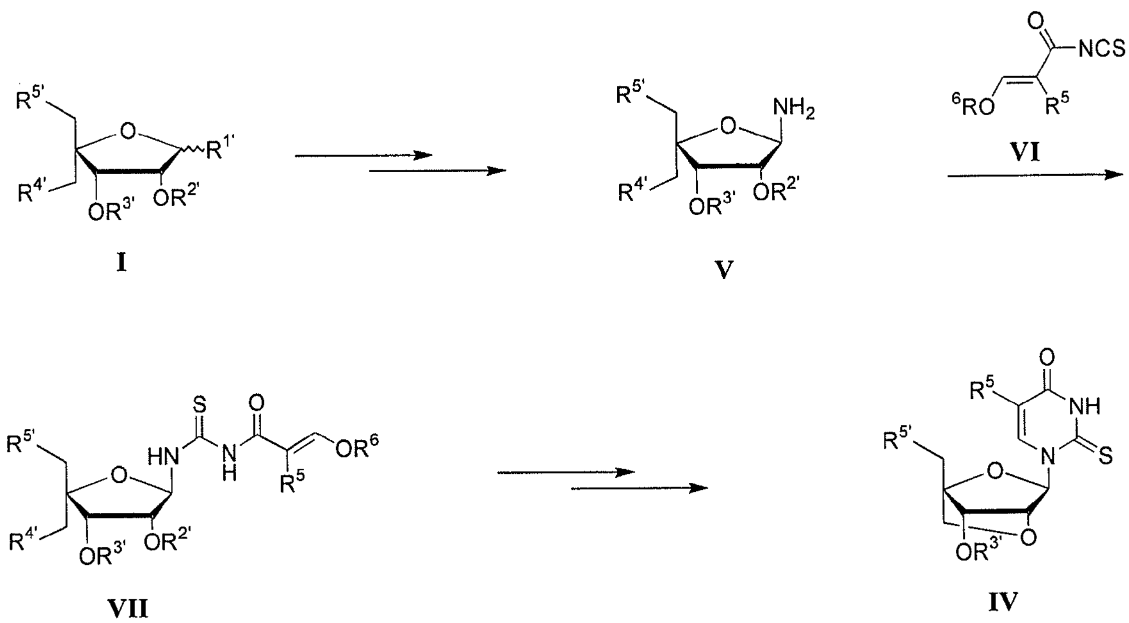

I

V

VI

VII

IV

Figure 9

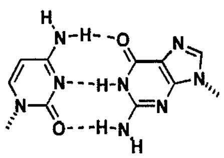

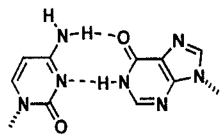

C G          C I

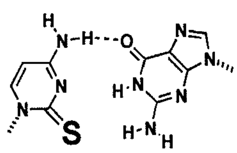

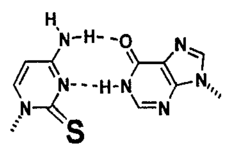

2sC G          2sC I

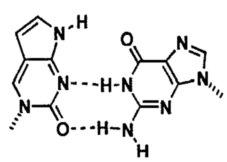

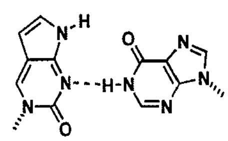

PyrroloPyr G          PyrroloPyr I

Figure 10

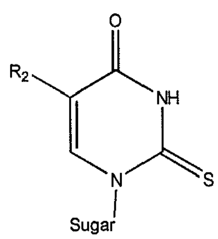

(i)

SBC base T'

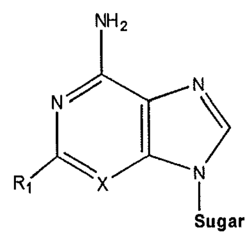

(ii)

SBC base A'

Figure 11

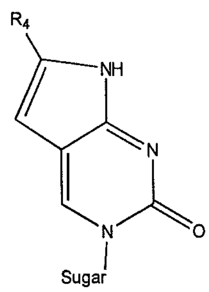

(iii)

SBC base C'

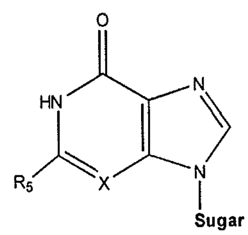

(ii)

SBC base G '

Figure 12

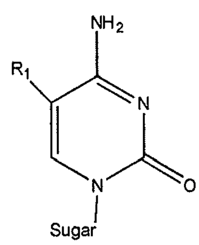

(v)

SBC base C'

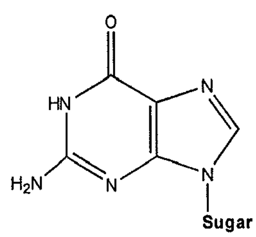

(vi)

SBC base G '

Figure 13

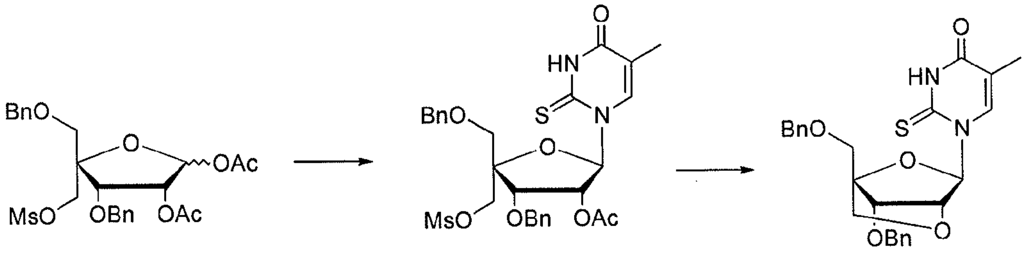

1 2 3

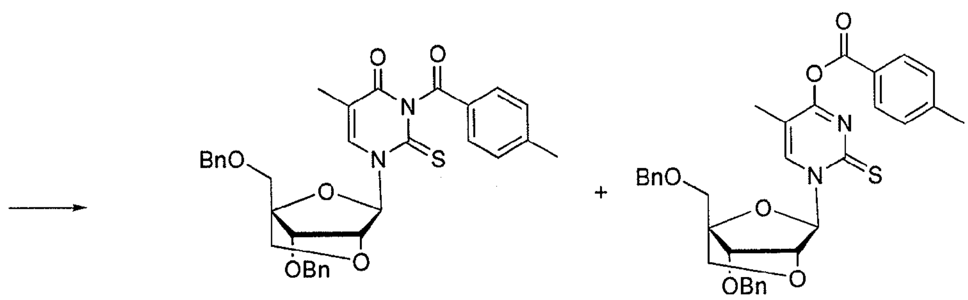

4

Figure 14

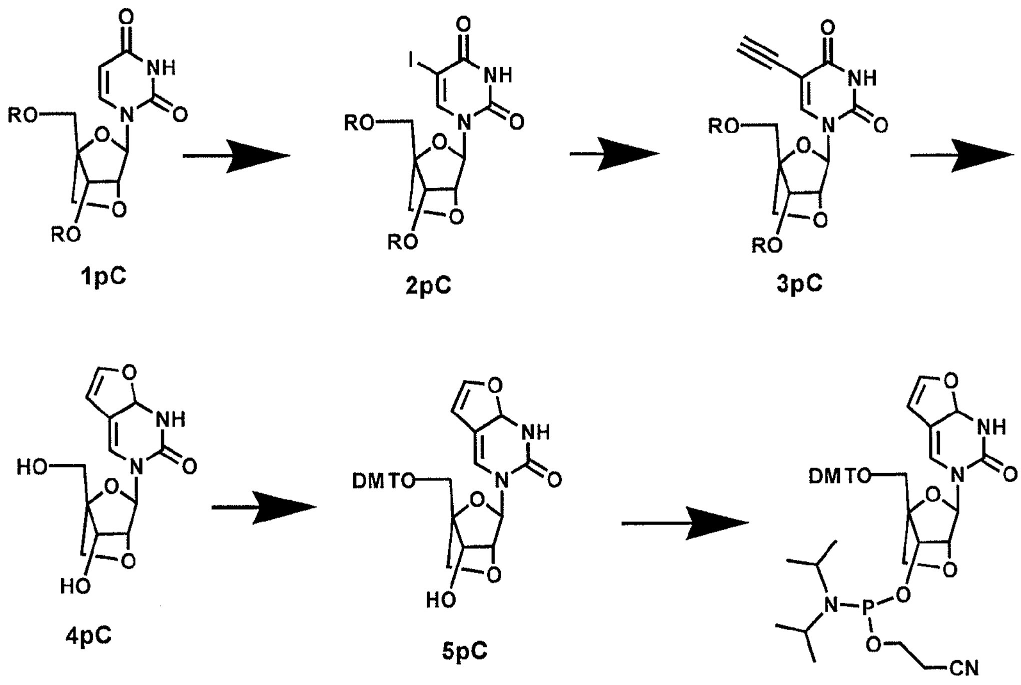

1pC

2pC

3pC

4pC

5pC

6pC

Figure 15

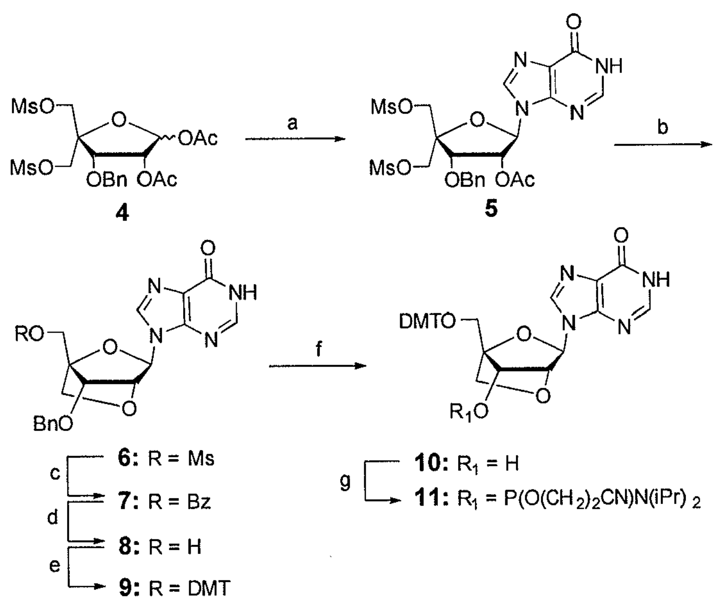


4

5

**6:** R = Ms

**7:** R = Bz

**8:** R = H

**9:** R = DMT

**10:** $R_1$ = H

**11:** $R_1$ = $P(O(CH_2)_2CN)N(iPr)_2$

Figure 16

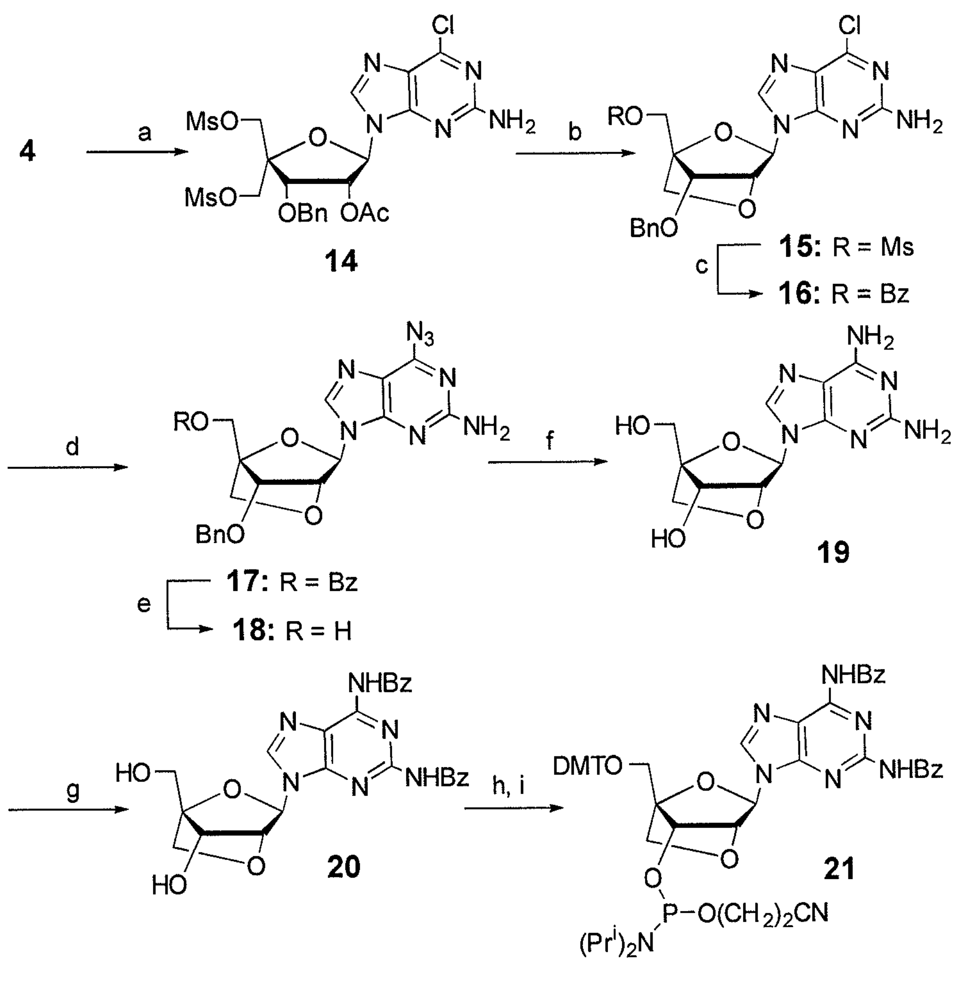


4

14

15: R = Ms

16: R = Bz

17: R = Bz

18: R = H

19

20

21

Figure 17

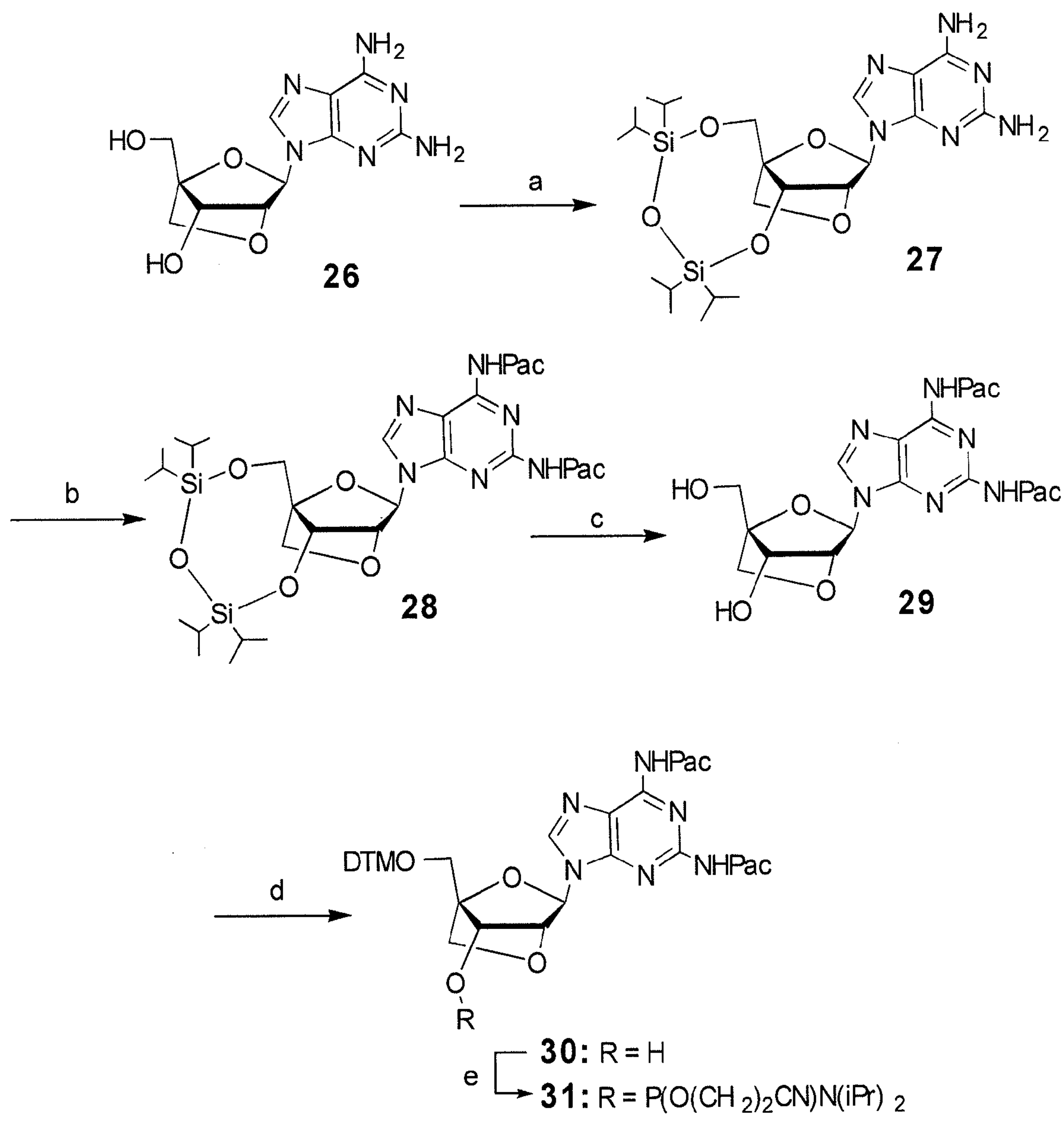


26 27 28 29

**30:** R = H

**31:** R = $P(O(CH_2)_2CN)N(iPr)_2$

Figure 18

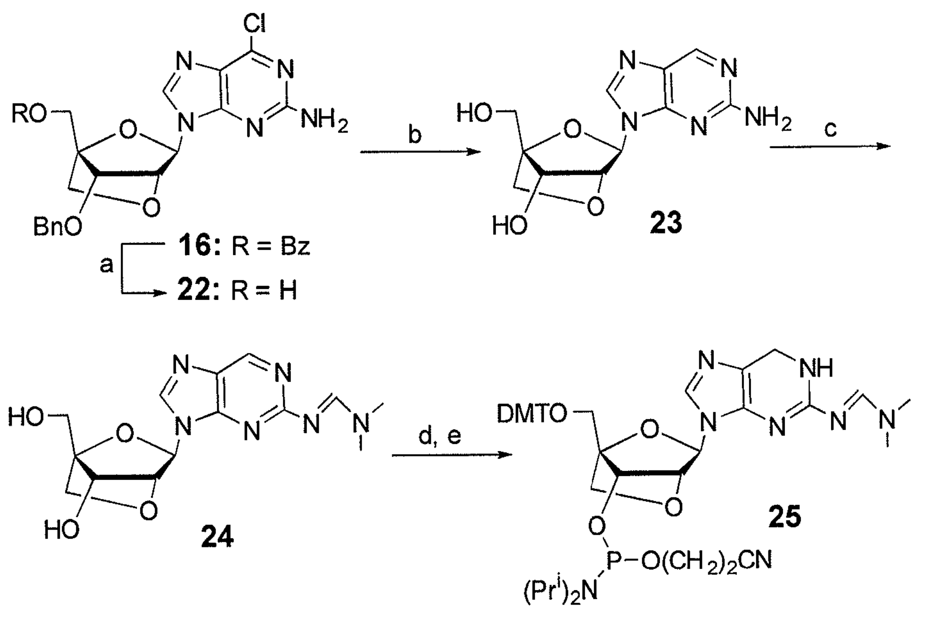


16: R = Bz

22: R = H

23

24

25

Figure 19

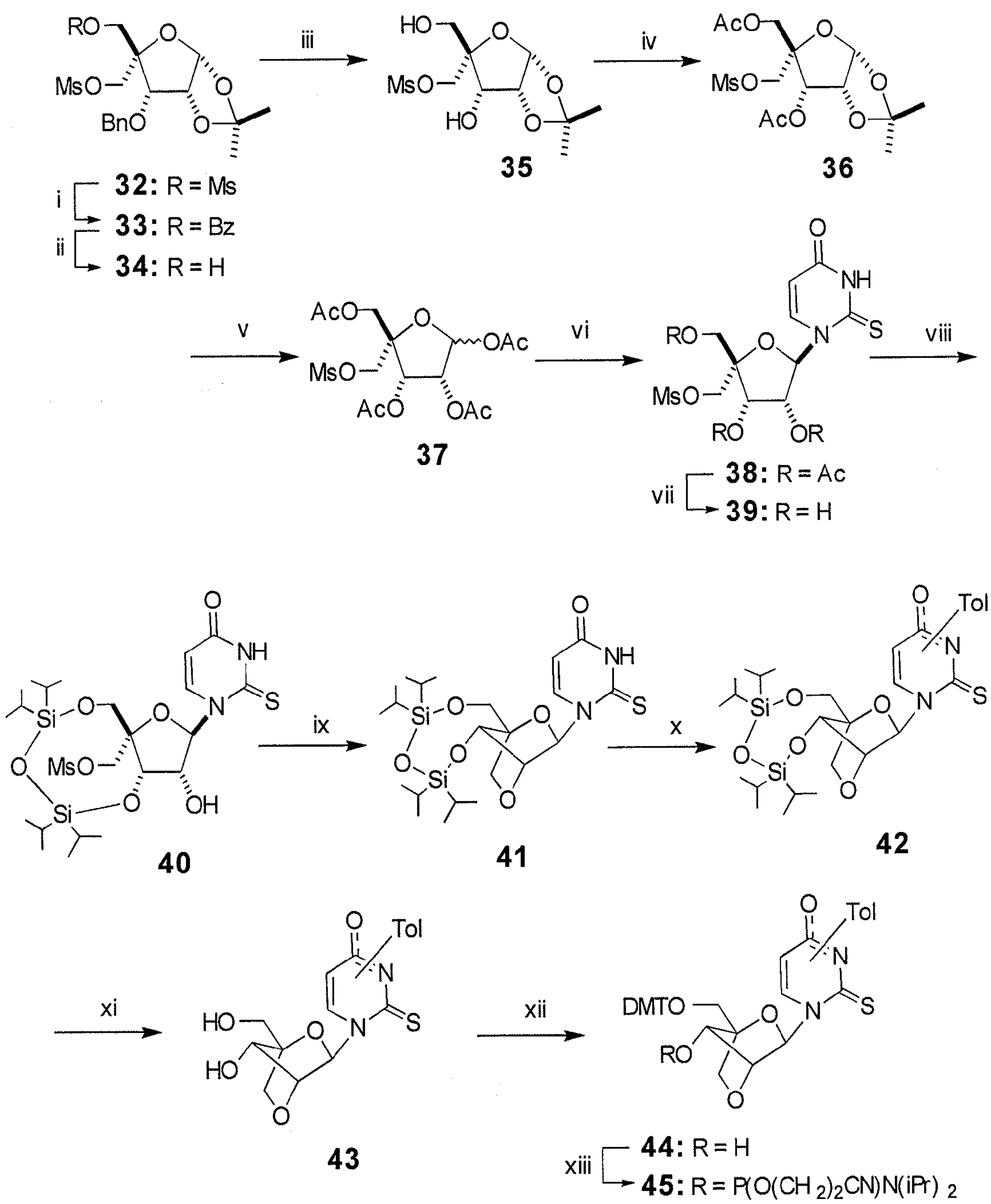


32: R = Ms
33: R = Bz
34: R = H
35
36
37
38: R = Ac
39: R = H
40
41
42
43
44: R = H
45: R = $P(O(CH_2)_2CN)N(iPr)_2$

Figure 20

# – *Mathematica*™ modelling

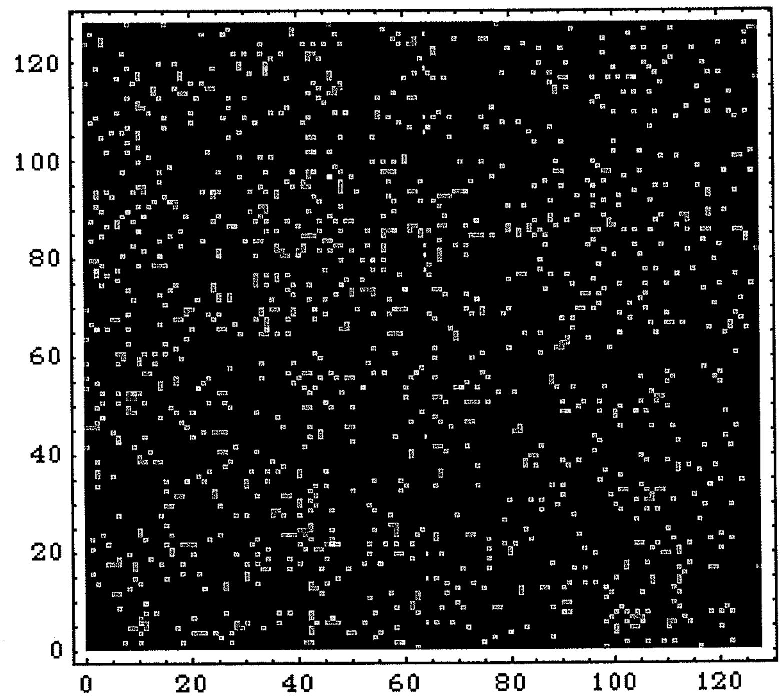


*Pseudomonas fluorescens* 16S rRNA

Figure 21

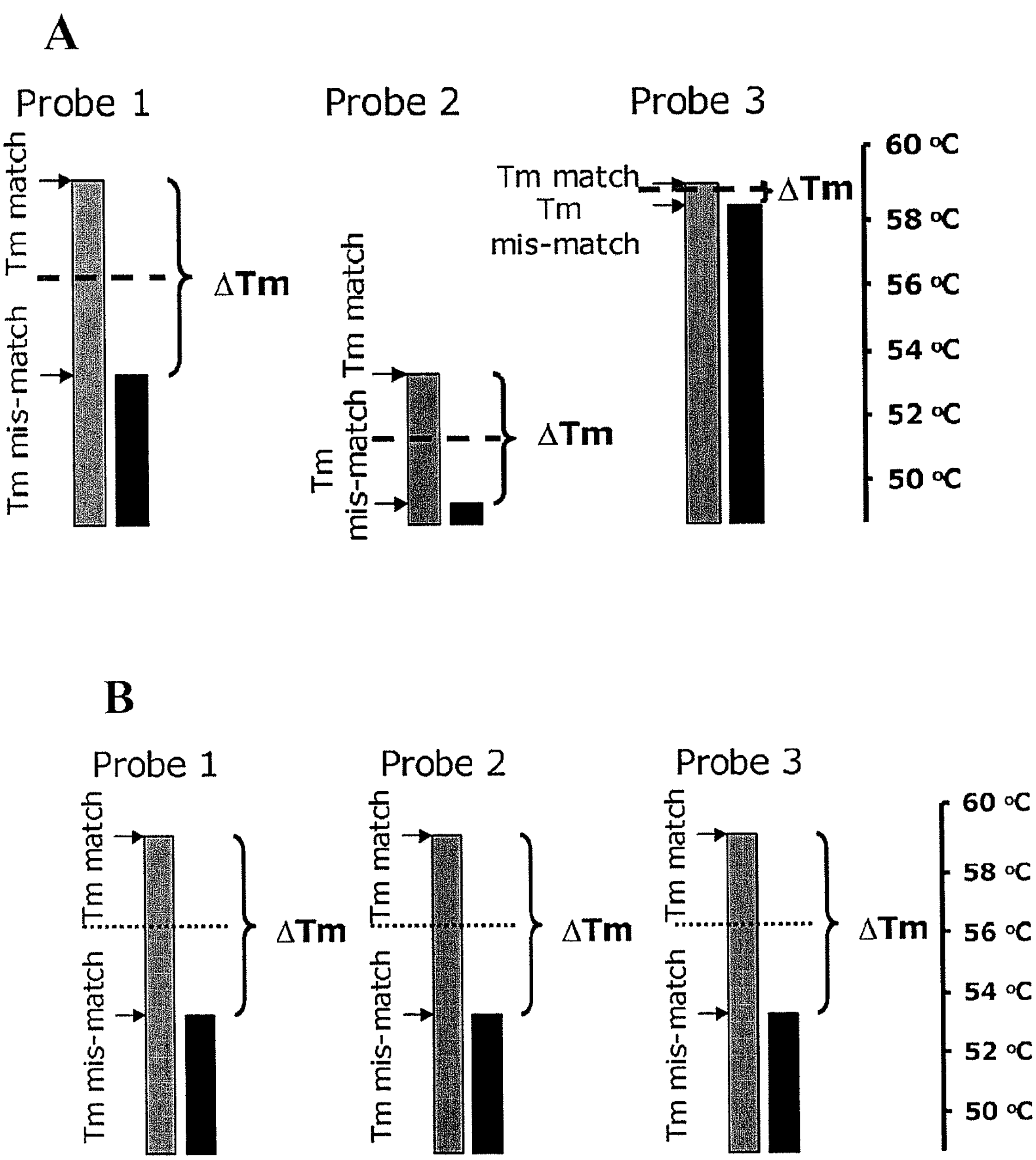
A
Probe 1
Tm match
Tm mis-match
ΔTm
Probe 2
Tm match
Tm
mis-match
ΔTm
Probe 3
Tm match
Tm
mis-match
ΔTm
60 °C
58 °C
56 °C
54 °C
52 °C
50 °C
B
Probe 1
Tm match
Tm mis-match
ΔTm
Probe 2
Tm match
Tm mis-match
ΔTm
Probe 3
Tm match
Tm mis-match
ΔTm
60 °C
58 °C
56 °C
54 °C
52 °C
50 °C

Figure 22

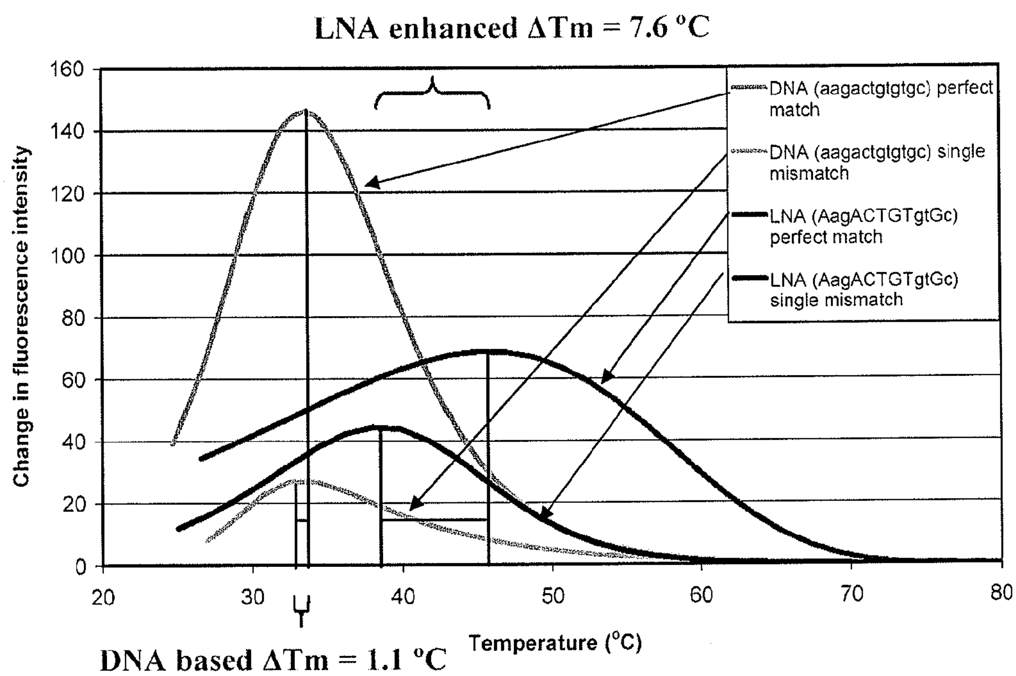
LNA enhanced ΔTm = 7.6 °C
160
140
120
100
80
60
40
20
0
Change in fluorescence intensity
20
30
40
50
60
70
80
Temperature (°C)
DNA based ΔTm = 1.1 °C
DNA (aagactgtgtgc) perfect match
DNA (aagactgtgtgc) single mismatch
LNA (AagACTGTgtGc) perfect match
LNA (AagACTGTgtGc) single mismatch

Figure 23

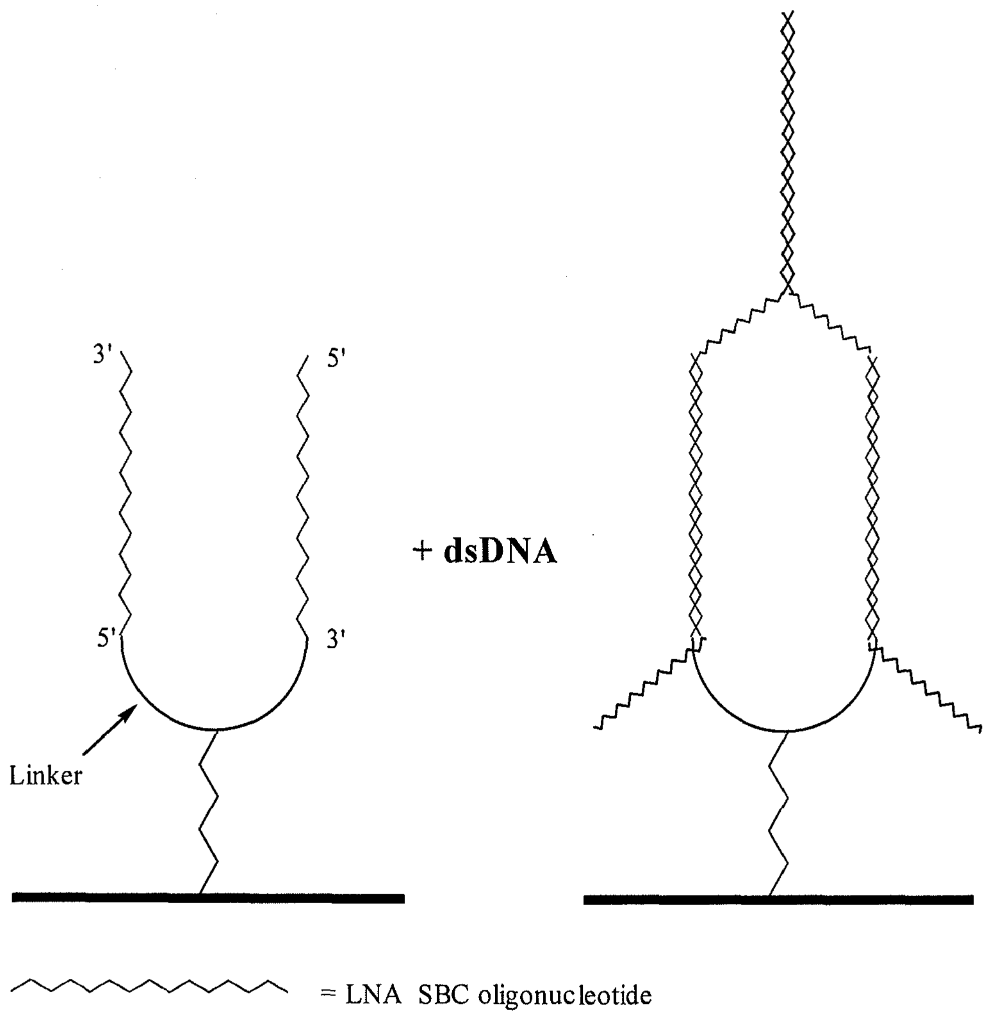

Figure 24

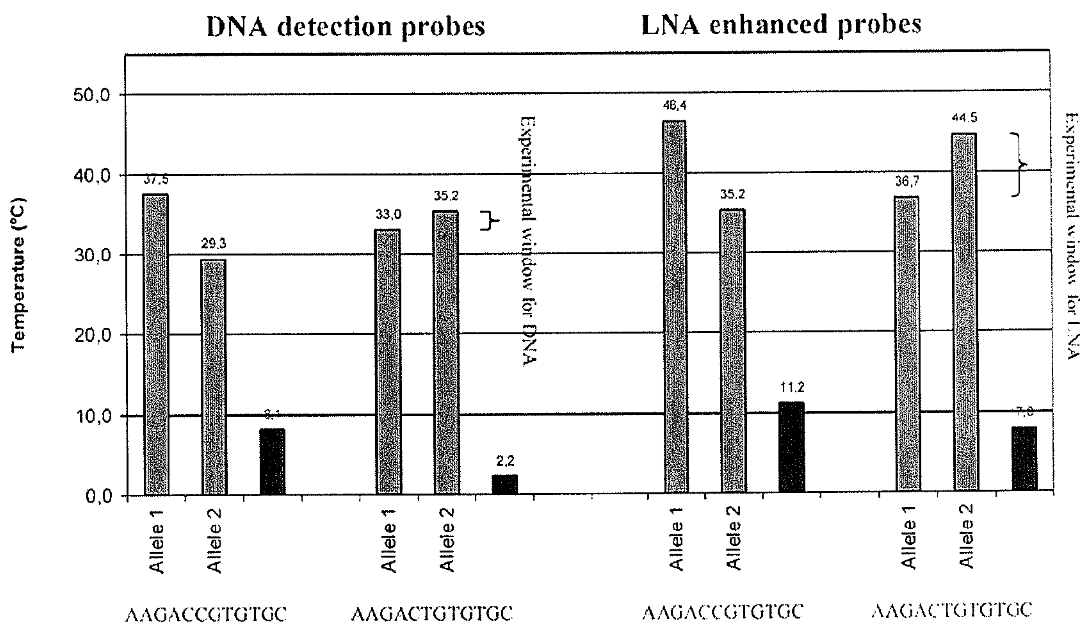
DNA detection probes
LNA enhanced probes
Temperature (°C)
50,0
40,0
30,0
20,0
10,0
0,0
37,5
29,3
33,0
35,2
2,2
46,4
35,2
11,2
36,7
44,5
Experimental window for DNA
Experimental window for LNA
Allele 1
Allele 2
Allele 1
Allele 2
Allele 1
Allele 2
Allele 1
Allele 2
AAGACCGTGTGC
AAGACTGTGTGC
AAGACCGTGTGC
AAGACTGTGTGC

Figure 25

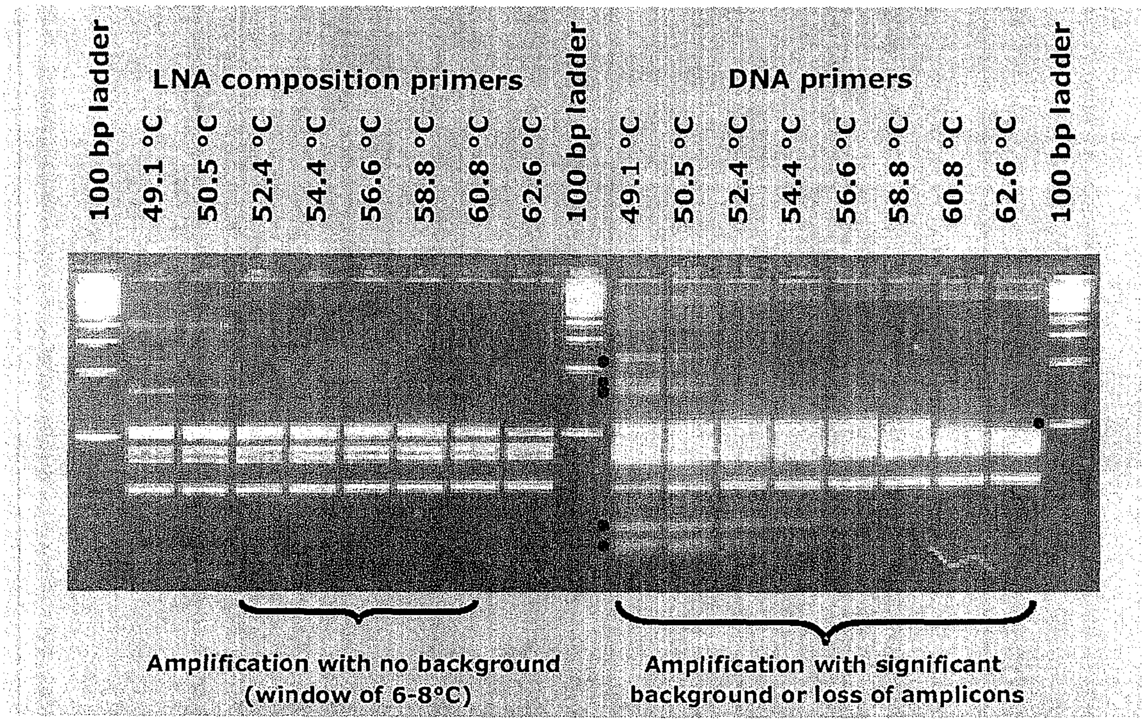
100 bp ladder
LNA composition primers
49.1 °C
50.5 °C
52.4 °C
54.4 °C
56.6 °C
58.8 °C
60.8 °C
62.6 °C
100 bp ladder
DNA primers
49.1 °C
50.5 °C
52.4 °C
54.4 °C
56.6 °C
58.8 °C
60.8 °C
62.6 °C
100 bp ladder
Amplification with no background
(window of 6-8°C)
Amplification with significant
background or loss of amplicons

Figure 26

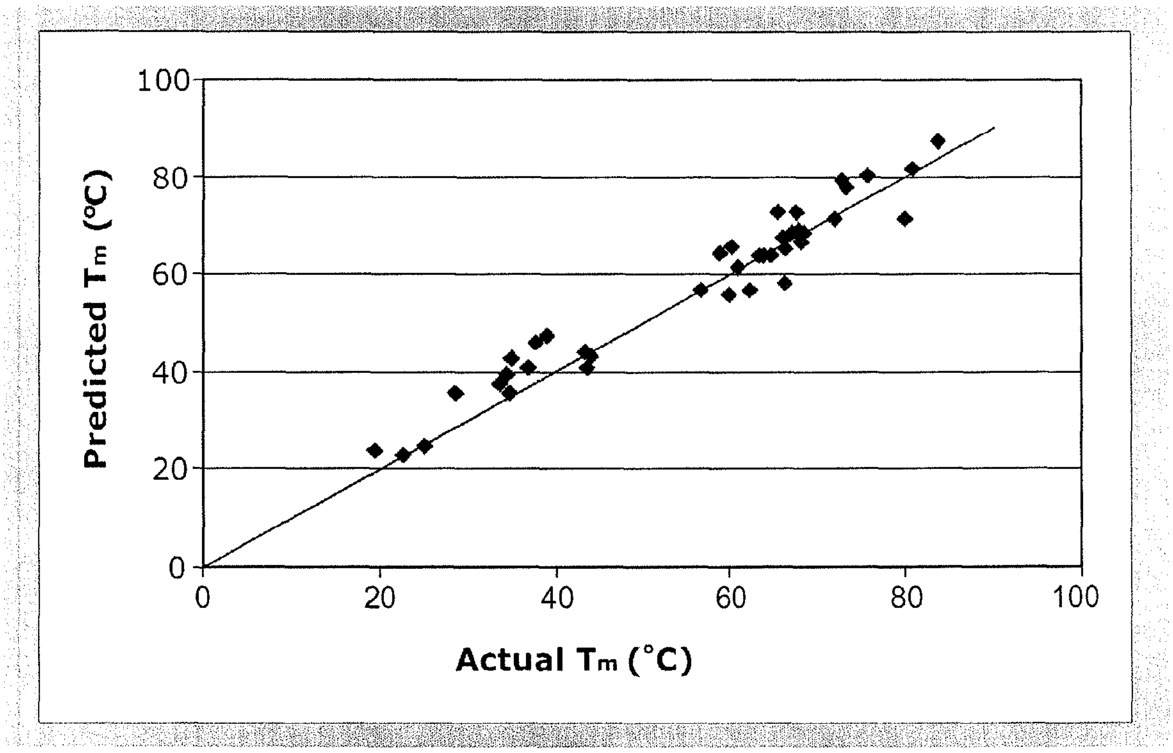

Figure 27

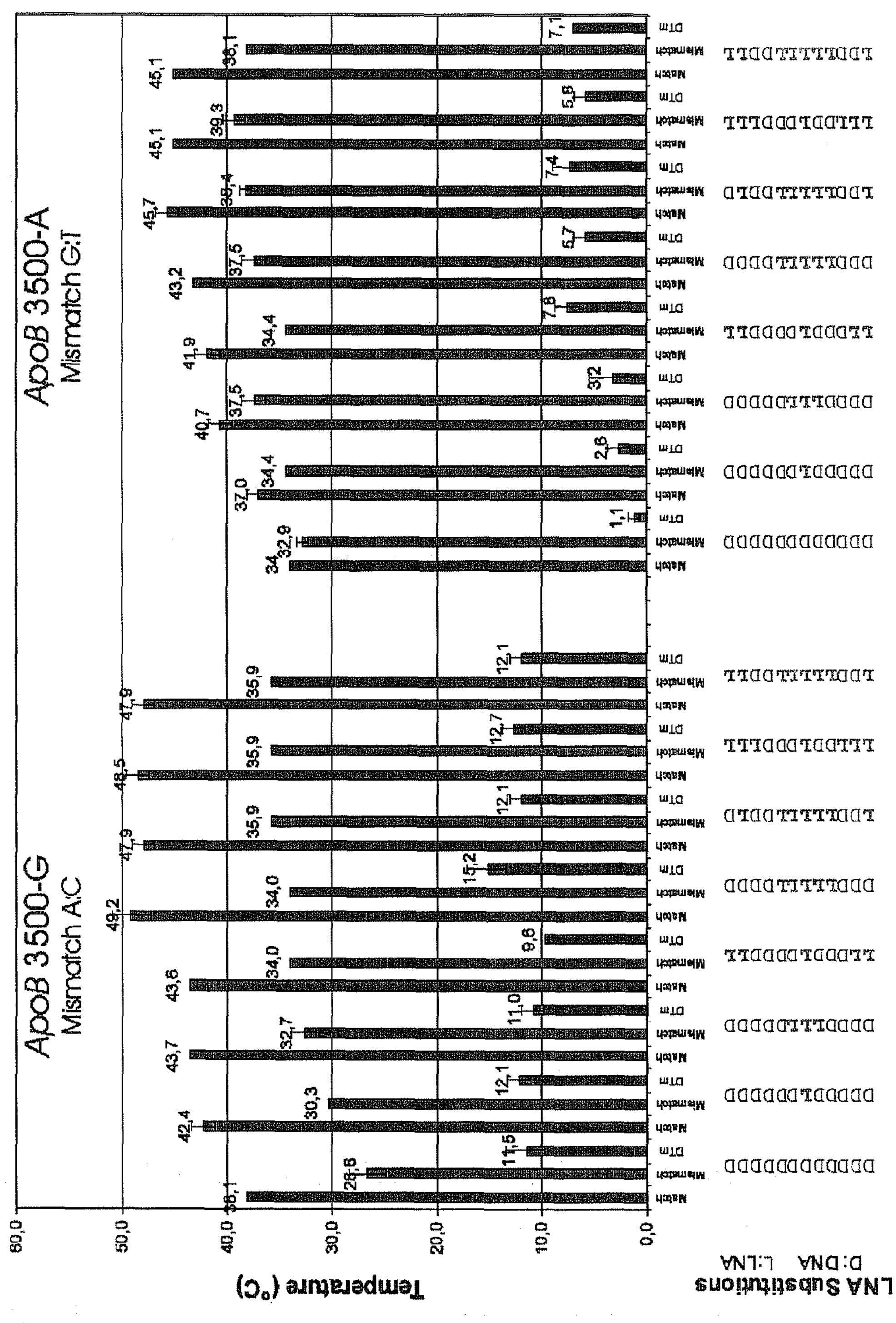
ApoB 3500-G
Mismatch A:C
ApoB 3500-A
Mismatch G:T
Temperature (°C)
LNA Substitutions
D:DNA L:LNA

Figure 28

| | A | B | C | D | E | F | G | H | I | J | K | L | M | N | O | P |
|---|---|---|---|---|---|---|---|---|---|---|---|---|---|---|---|---|
| 1 | Reference | AGGTAmCT | GGTAmCTG | GTAmCTGG | Reference | Reference | Reference | Reference | Reference | GGTAmC | Reference | Reference | Reference | Reference | Reference | Reference |
| 2 | Reference | AgGTAcT | GgTAmCtG | GtAmCTgG | iAgGTAcTi | iGgTAmCtGi | iGtAmCTgGi | Reference | nGgTAmCtGn | GGTAmCT | iGGTAmCTi | iiGGTAmCTii | Reference | Reference | GgAAGcmC | Reference |
| 3 | Reference | AgGtAcT | GgTamCtG | GtAcTgG | Reference | iGgTamCtGi | Reference | .iGgTamCtGi | nGgTamCtGn | GgTAcT | iGgTAcTi | Reference | XgGtAcTgX | zGgTAmCtGz | GgAaGcmC | Reference |
| 4 | Reference | AggTacT | GgtActG | GtamCtgG | iAggTacTi | iGgtActGi | iGtamCtgGi | iiGgtActGii | nGgtActGn | GgTamCt | Reference | iiGgTamCtii | IGGTAmCTI | zgGtAcTgz | GgaAgcmC | Reference |
| 5 | Reference | aGgTamCt | gGtAcTg | gTamCtGg | iaGgTamCti | igGtAcTgi | igTamCtGgi | iigGtAcTgii | ngGtAcTgn | gGtamCt | Reference | iigGtamCtii | IGgTAmCtGI | zAgGTAcTz | gGaAgmCc | Reference |
| 6 | Reference | aggtact | ggtactg | gtactgg | Reference | iggtactgi | igtactggi | iiggtactgii | nggtactgn | ggtact | iggtacti | Reference | IgGtAcTgI | zaGgTamCtz | ggaagcc | Reference |
| 7 | Reference | AGAATmCG | GAATmCGA | AATmCGAT | Reference | Reference | Reference | Reference | Reference | GAATmC | iGAATmCi | iiGAATmCii | XGAATmCGX | zAaTmCGaTz | Reference | Reference |
| 8 | Reference | AgAATcG | GaATmCgA | AaTmCGaT | iAgAATcGi | Reference | iAaTmCGaTi | .iGaATmCgAi | nGaATmCgAn | GAATmCG | iGAATmCGi | iiGAATmCGii | Reference | zaAtmCgAtz | mCtmCTAcmC | Reference |
| 9 | Reference | AgAaTcG | GaAtmCgA | AaTcGaT | iAgAaTcGi | iGaAtmCgAi | iAaTcGaTi | .iGaAtmCgAi | nGaAtmCgAn | GaATcG | iGaATcGi | iiGaATcGii | XgAaTcGaX | zGaATmCgAz | mCtmCtAcmC | Reference |
| 10 | Reference | AgaAtcG | GaaTcgA | AatmCgaT | iAgaAtcGi | iGaaTcgAi | iAatmCgaTi | iiGaaTcgAii | nGaaTcgAn | GaAtmCg | iGaAtmCgi | iiGaAtmCgii | IGAATmCGI | zgAaTcGaz | mCtcTacmC | Reference |
| 11 | Reference | aGaAtmCg | gAaTcGa | aAtmCgAt | iaGaAtmCgi | igAaTcGai | iaAtmCgAti | iigAaTcGaii | ngAaTcGan | gAatmCg | igAatmCgi | iigAatmCgii | IGaATmCgAI | zAgAATcGz | cTcTamCc | Reference |
| 12 | Reference | agaatcg | gaatcga | aatcgat | iagaatcgi | igaatcgai | iaatcgati | iigaatcgaii | ngaatcgan | gaatcg | igaatcgi | iigaatcgii | IgAaTcGaI | zaGaAtmCgz | ctctacc | Reference |
| 13 | Reference | GATGAAT | ATGAATT | TGAATTT | Reference | Reference | Reference | Reference | Reference | ATGAA | iATGAAi | iiATGAAii | XATGAATX | zTgAATtTz | Reference | Reference |
| 14 | Reference | GaTGAaT | AtGAAtT | TgAATtT | iGaTGAaTi | iAtGAAtTi | iTgAATtTi | iiAtGAAtTii | nAtGAAtTn | ATGAAT | iATGAATi | iiATGAATii | XAtGAAtTX | ztGaAtTtz | Reference | Reference |
| 15 | Reference | GaTgAaT | AtGaAtT | TgAaTtT | iGaTgAaTi | iAtGaAtTi | iTgAaTtTi | iiAtGaAtTii | nAtGaAtTn | AtGAaT | iAtGAaTi | iiAtGAaTii | XaTgAaTtX | Reference | Reference | Reference |
| 16 | Reference | GatGaaT | AtgAatT | TgaAttT | iGatGaaTi | iAtgAatTi | iTgaAttTi | iiAtgAatTii | nAtgAatTn | AtGaAt | iAtGaAti | iiAtGaAtii | IATGAATI | zaTgAaTtz | Reference | Reference |
| 17 | Reference | gAtGaAt | aTgAaTt | tGaAtTt | igAtGaAti | iaTgAaTti | itGaAtTti | iiaTgAaTtii | naTgAaTtn | aTgaAt | iaTgaAti | iiaTgaAtii | IAtGAAtTI | zGaTGAaTz | Reference | Reference |
| 18 | Reference | gatgaat | atgaatt | tgaattt | igatgaati | iatgaatti | itgaattti | iiatgaattii | natgaattn | atgaat | iatgaati | iiatgaatii | IaTgAaTtI | zgAtGaAtz | Reference | Reference |

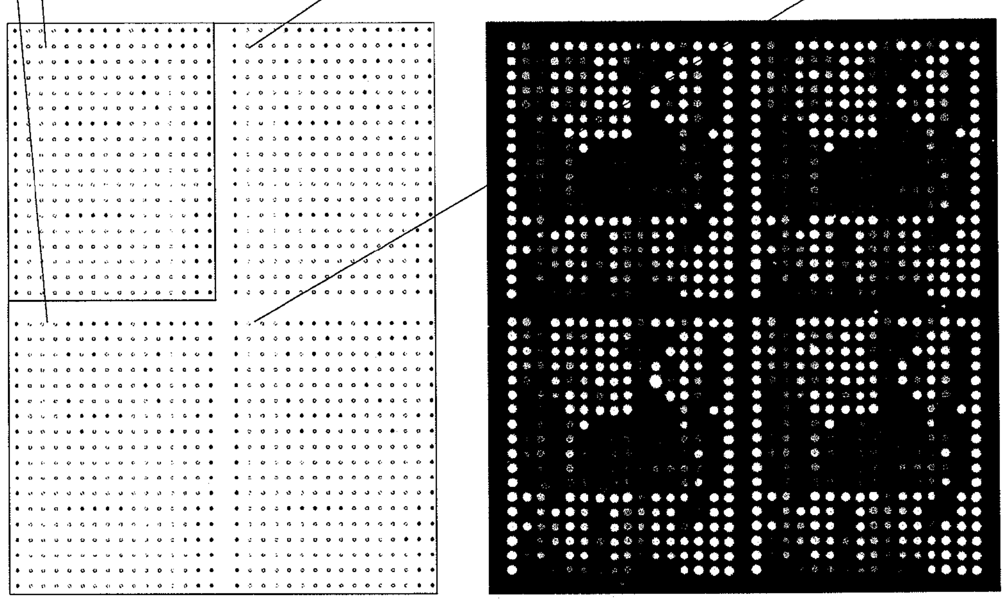

Figure 29

- 320 000 intensity measurements = DNA signature of sample adequate for comparison to a large set of standard signatures

- Assumption:
  *Signature of sample = combination of standard signatures*

- *Example* $$\text{Sample} = 5\%\, S_1 + 80\%\, S_2 + 15\%\, S_3$$

$$\Downarrow$$

$$I_{sample} = 5\%\, I_{S1} + 80\%\, I_{S2} + 15\%\, I_{S3}$$

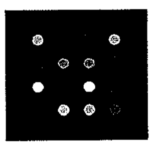
= 5%
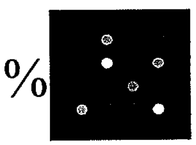
+80%
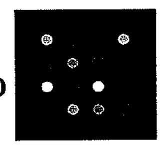
+15%
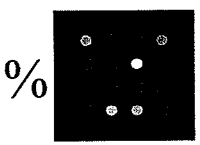

Figure 30

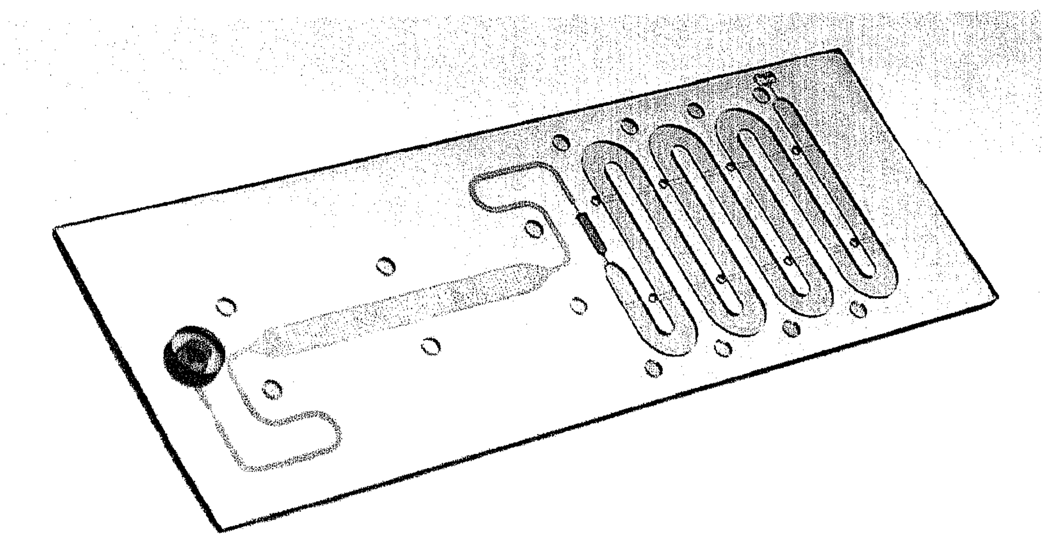

Figure 31

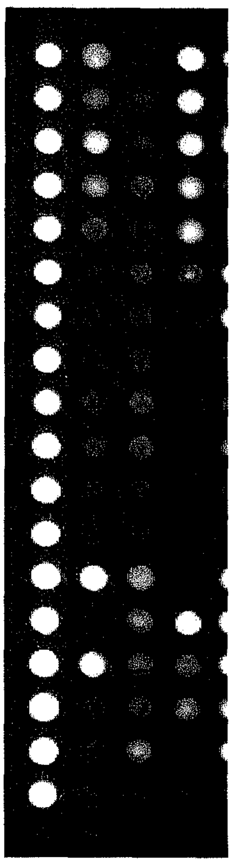

| | | | |
|---|---|---|---|
| Reference | AGGTAmCT | GGTAmCTG | **GTAmCTGG** |
| Reference | AgGTAcT | GgTAmCtG | GtAmCTgG |
| Reference | **AgGtAcT** | GgTamCtG | GtAcTgG |
| Reference | AggTacT | **GgtActG** | GtamCtgG |
| Reference | aGgTamCt | gGtAcTg | gTamCtGg |
| Reference | aggtact | ggtactg | gtactgg |
| Reference | AGAATmCG | GAATmCGA | AATmCGAT |
| Reference | AgAATcG | GaATmCgA | AaTmCGaT |
| Reference | AgAaTcG | **GaAtmCgA** | AaTcGaT |
| Reference | **AgaAtcG** | GaaTcgA | AatmCgaT |
| Reference | aGaAtmCg | gAaTcGa | aAtmCgAt |
| Reference | agaatcg | gaatcga | aatcgat |
| Reference | **GATGAAT** | **ATGAATT** | TGAATTT |
| Reference | GaTGAaT | AtGAAtT | **TgAATtT** |
| Reference | GaTgAaT | AtGaAtT | TgAaTtT |
| Reference | GatGaaT | AtgAatT | TgaAttT |
| Reference | gAtGaAt | aTgAaTt | tGaAtTt |
| Reference | gatgaat | atgaatt | tgaattt |

Figure 32

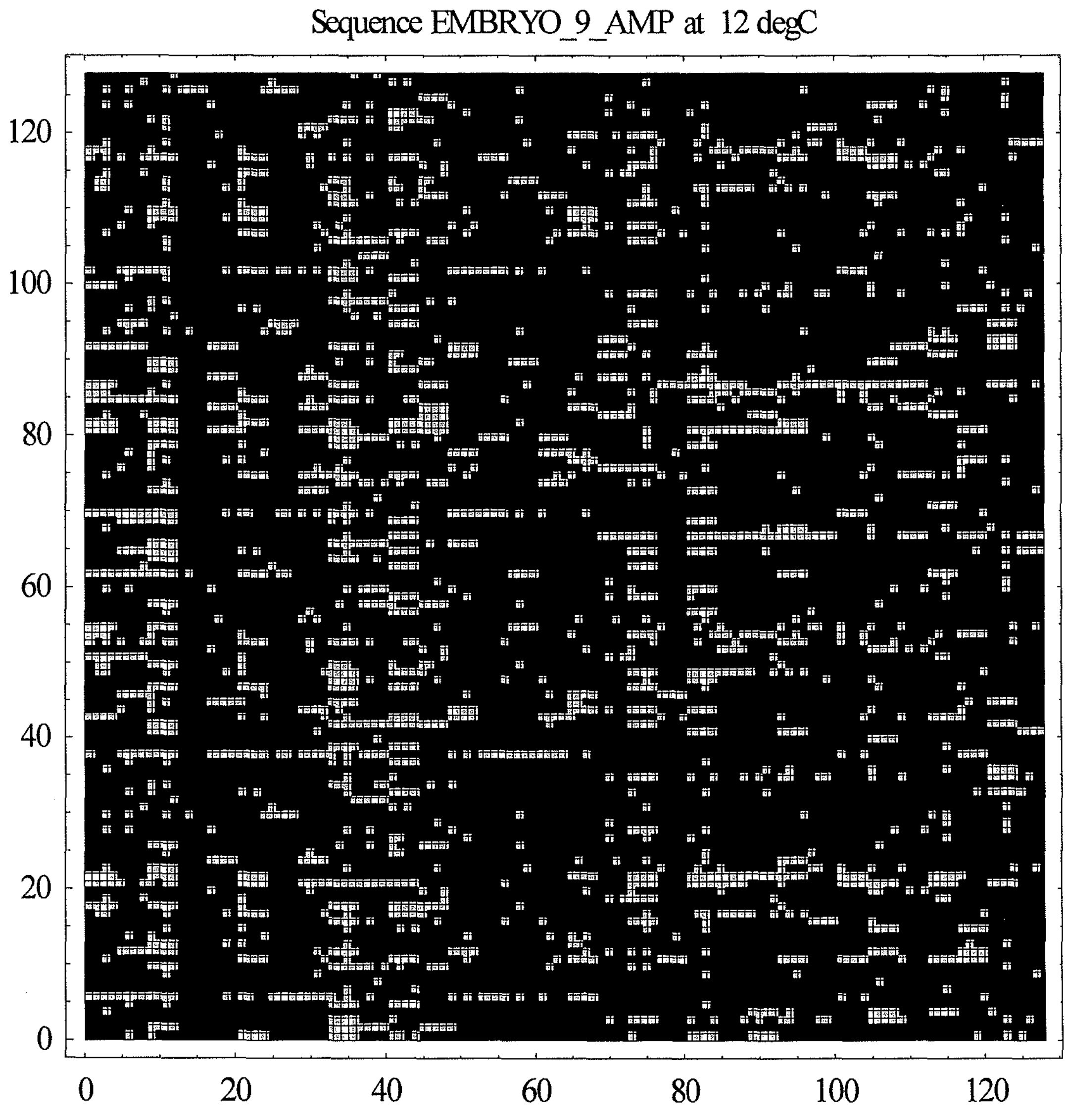
Sequence EMBRYO_9_AMP at 12 degC
120
100
80
60
40
20
0
0
20
40
60
80
100
120

Figure 33

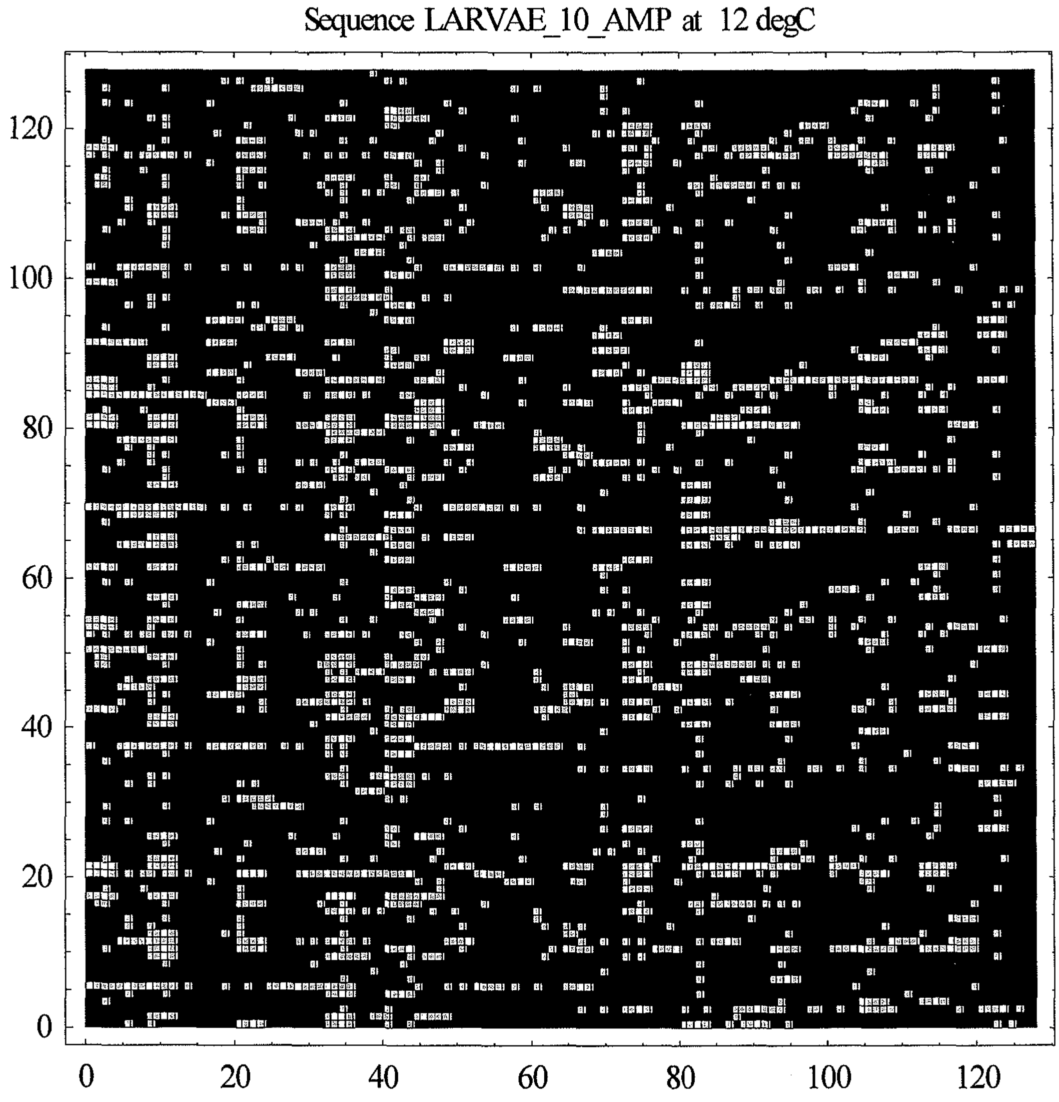
Sequence LARVAE_10_AMP at 12 degC
0
20
40
60
80
100
120
0
20
40
60
80
100
120

Figure 34

Sequence LARVAE_10_MUT at 12 degC

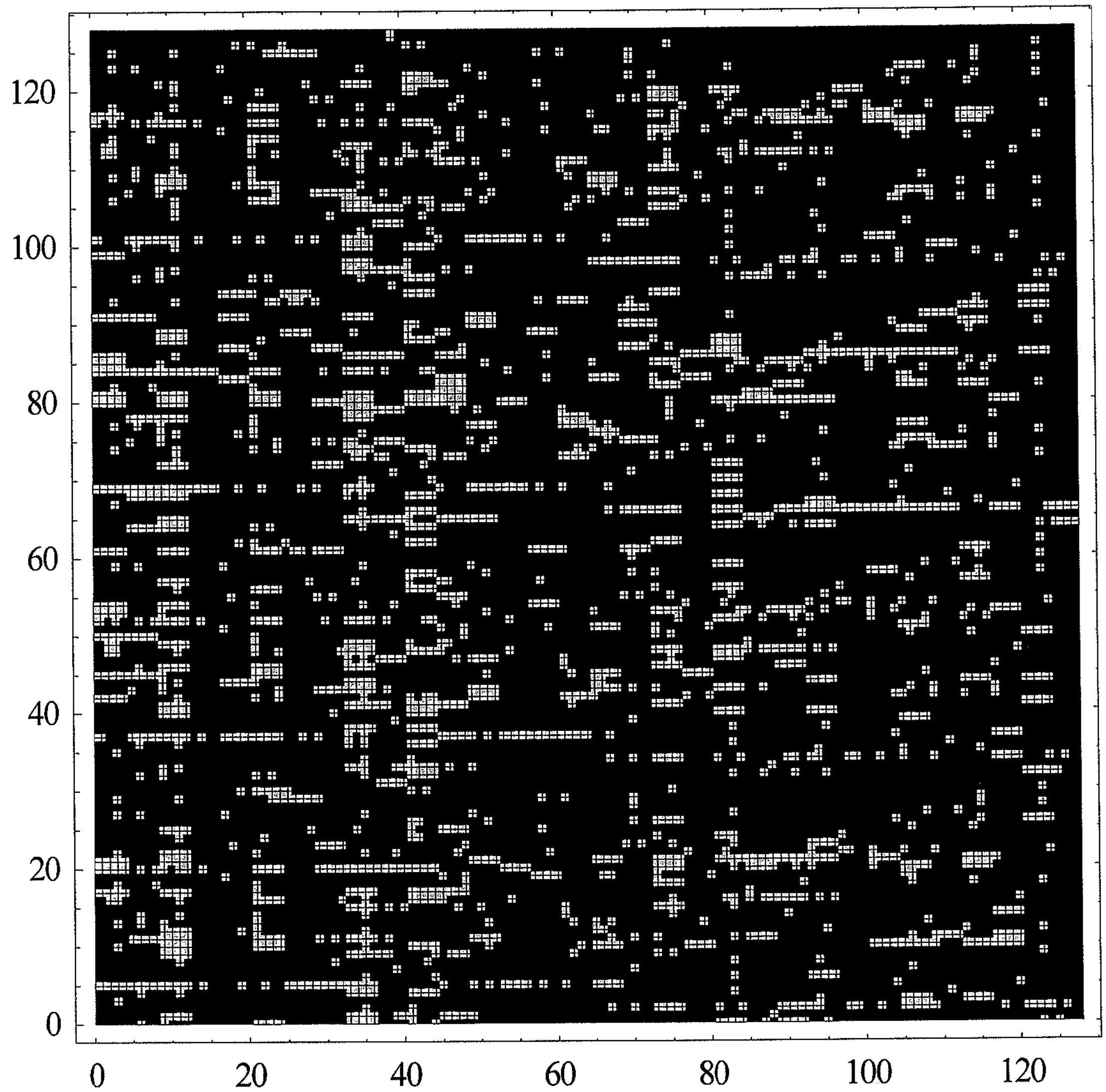

Figure 35

Mixed signal [redacted]=0.3 [redacted]=0.6 z=0.1 [redacted] degC

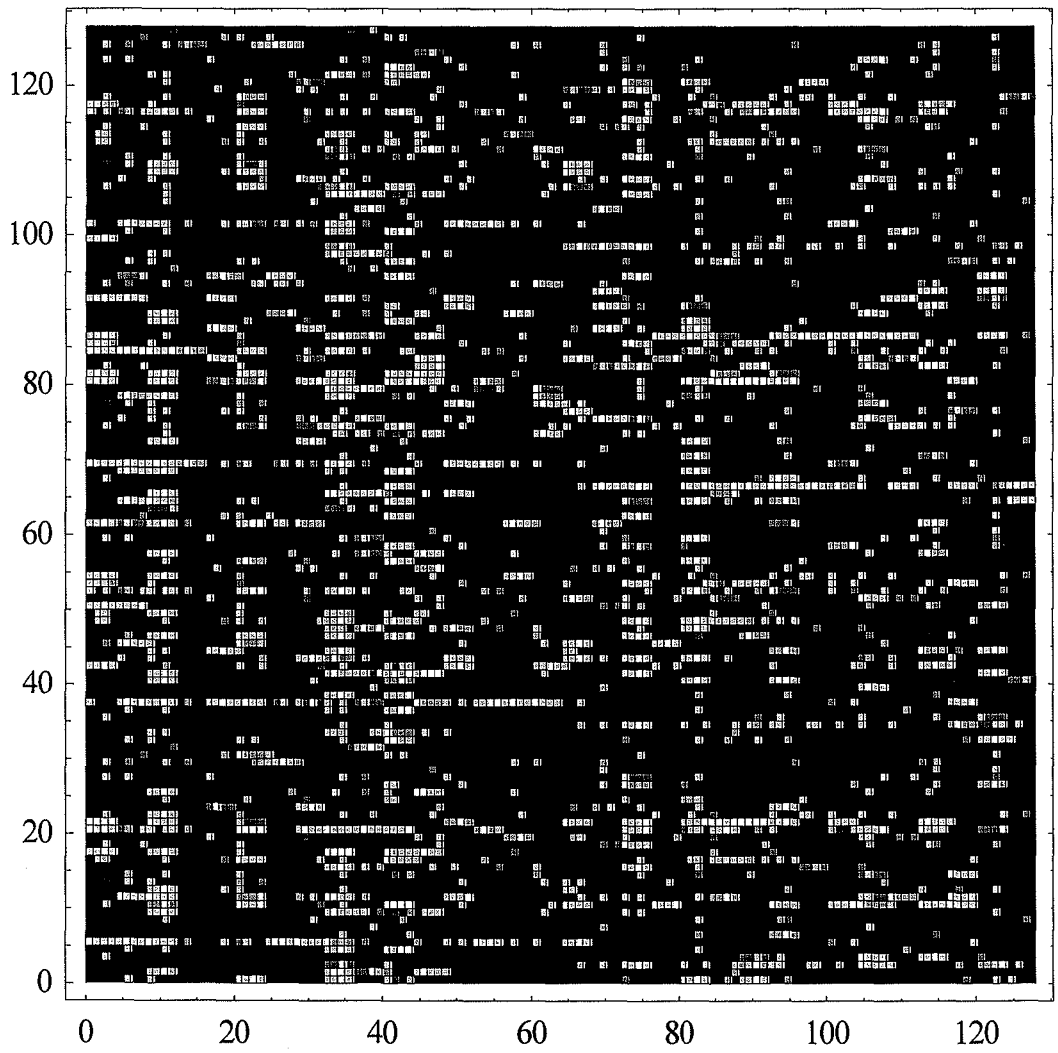

Figure 36

| | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 |
|---|---|---|---|---|---|---|---|---|---|---|---|---|---|---|---|---|---|---|---|---|---|---|---|---|
| 1 | | D15 | D31 | D47 | D63 | D79 | | L15 | L31 | L47 | L63 | L79 | | L109 | L125 | L141 | L157 | L173 | L189 | L205 | L221 | L237 | | |
| 2 | D1 | D16 | D32 | D48 | D64 | D80 | L1 | L16 | L32 | L48 | L64 | L80 | L95 | L110 | L126 | L142 | L158 | L174 | L190 | L206 | L222 | L238 | S1A.1 | S1B.1 |
| 3 | D2 | D17 | D33 | D49 | D65 | D81 | L2 | L17 | L33 | L49 | L65 | L81 | L96 | L111 | L127 | L143 | L159 | L175 | L191 | L207 | L223 | L239 | S1A.2 | S1B.2 |
| 4 | D3 | D18 | D34 | D50 | D66 | D82 | L3 | L18 | L34 | L50 | L66 | L82 | L97 | L112 | L128 | L144 | L160 | L176 | L192 | L208 | L224 | L240 | S1A.3 | S1B.3 |
| 5 | D4 | D19 | D35 | D51 | D67 | D83 | L4 | L19 | L35 | L51 | L67 | L83 | L98 | L113 | L129 | L145 | L161 | L177 | L193 | L209 | L225 | L241 | S1A.4 | S1B.4 |
| 6 | D5 | D20 | D36 | D52 | D68 | D84 | L5 | L20 | L36 | L52 | L68 | L84 | L99 | L114 | L130 | L146 | L162 | L178 | L194 | L210 | L226 | L242 | S1A.5 | S1B.5 |
| 7 | D6 | D21 | D37 | D53 | D69 | D85 | L6 | L21 | L37 | L53 | L69 | L85 | L100 | L115 | L131 | L147 | L163 | L179 | L195 | L211 | L227 | L243 | S1A.6 | S1B.6 |
| 8 | D7 | D22 | D38 | D54 | D70 | D86 | L7 | L22 | L38 | L54 | L70 | L86 | L101 | L116 | L132 | L148 | L164 | L180 | L196 | L212 | L228 | L244 | S1A.7 | S1B.7 |
| 9 | D8 | D23 | D39 | D55 | D71 | D87 | L8 | L23 | L39 | L55 | L71 | L87 | L102 | L117 | L133 | L149 | L165 | L181 | L197 | L213 | L229 | L245 | | S2B.1 |
| 10 | D9 | D24 | D40 | D56 | D72 | D88 | L9 | L24 | L40 | L56 | L72 | L88 | L103 | L118 | L134 | L150 | L166 | L182 | L198 | L214 | L230 | L246 | S2A.1 | S2B.2 |
| 11 | D10 | D25 | D41 | D57 | D73 | D89 | L10 | L25 | L41 | L57 | L73 | L89 | L104 | L119 | L135 | L151 | L167 | L183 | L199 | L215 | L231 | L247 | S2A.2 | S2B.3 |
| 12 | D11 | D26 | D42 | D58 | D74 | D90 | L11 | L26 | L42 | L58 | L74 | L90 | L105 | L120 | L136 | L152 | L168 | L184 | L200 | L216 | L232 | L248 | S2A.3 | S2B.4 |
| 13 | D12 | D27 | D43 | D59 | D75 | D91 | L12 | L27 | L43 | L59 | L75 | L91 | L106 | L121 | L137 | L153 | L169 | L185 | L201 | L217 | L233 | L249 | S2A.4 | S2B.5 |
| 14 | D13 | D28 | D44 | D60 | D76 | D92 | L13 | L28 | L44 | L60 | L76 | L92 | L107 | L122 | L138 | L154 | L170 | L186 | L202 | L218 | L234 | L250 | S2A.5 | S2B.6 |
| 15 | D14 | D29 | D45 | D61 | D77 | D93 | L14 | L29 | L45 | L61 | L77 | L93 | L108 | L123 | L139 | L155 | L171 | L187 | L203 | L219 | L235 | L251 | S2A.6 | S2B.7 |
| 16 | | D30 | D46 | D62 | D78 | D94 | | L30 | L46 | L62 | L78 | L94 | | L124 | L140 | L156 | L172 | L188 | L204 | L220 | L236 | L252 | S2A.7 | |

| 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 |
|---|---|---|---|---|---|---|---|---|---|---|---|---|---|---|---|---|---|---|---|---|---|---|---|
| | D15 | D31 | D47 | D63 | D79 | | L15 | L31 | L47 | L63 | L79 | | L109 | L125 | L141 | L157 | L173 | L189 | L205 | L221 | L237 | | |
| D1 | D16 | D32 | D48 | D64 | D80 | L1 | L16 | L32 | L48 | L64 | L80 | L95 | L110 | L126 | L142 | L158 | L174 | L190 | L206 | L222 | L238 | S1A.1 | S1B.1 |
| D2 | D17 | D33 | D49 | D65 | D81 | L2 | L17 | L33 | L49 | L65 | L81 | L96 | L111 | L127 | L143 | L159 | L175 | L191 | L207 | L223 | L239 | S1A.2 | S1B.2 |
| D3 | D18 | D34 | D50 | D66 | D82 | L3 | L18 | L34 | L50 | L66 | L82 | L97 | L112 | L128 | L144 | L160 | L176 | L192 | L208 | L224 | L240 | S1A.3 | S1B.3 |
| D4 | D19 | D35 | D51 | D67 | D83 | L4 | L19 | L35 | L51 | L67 | L83 | L98 | L113 | L129 | L145 | L161 | L177 | L193 | L209 | L225 | L241 | S1A.4 | S1B.4 |
| D5 | D20 | D36 | D52 | D68 | D84 | L5 | L20 | L36 | L52 | L68 | L84 | L99 | L114 | L130 | L146 | L162 | L178 | L194 | L210 | L226 | L242 | S1A.5 | S1B.5 |
| D6 | D21 | D37 | D53 | D69 | D85 | L6 | L21 | L37 | L53 | L69 | L85 | L100 | L115 | L131 | L147 | L163 | L179 | L195 | L211 | L227 | L243 | S1A.6 | S1B.6 |
| D7 | D22 | D38 | D54 | D70 | D86 | L7 | L22 | L38 | L54 | L70 | L86 | L101 | L116 | L132 | L148 | L164 | L180 | L196 | L212 | L228 | L244 | S1A.7 | S1B.7 |
| D8 | D23 | D39 | D55 | D71 | D87 | L8 | L23 | L39 | L55 | L71 | L87 | L102 | L117 | L133 | L149 | L165 | L181 | L197 | L213 | L229 | L245 | | S2B.1 |
| D9 | D24 | D40 | D56 | D72 | D88 | L9 | L24 | L40 | L56 | L72 | L88 | L103 | L118 | L134 | L150 | L166 | L182 | L198 | L214 | L230 | L246 | S2A.1 | S2B.2 |
| D10 | D25 | D41 | D57 | D73 | D89 | L10 | L25 | L41 | L57 | L73 | L89 | L104 | L119 | L135 | L151 | L167 | L183 | L199 | L215 | L231 | L247 | S2A.2 | S2B.3 |
| D11 | D26 | D42 | D58 | D74 | D90 | L11 | L26 | L42 | L58 | L74 | L90 | L105 | L120 | L136 | L152 | L168 | L184 | L200 | L216 | L232 | L248 | S2A.3 | S2B.4 |
| D12 | D27 | D43 | D59 | D75 | D91 | L12 | L27 | L43 | L59 | L75 | L91 | L106 | L121 | L137 | L153 | L169 | L185 | L201 | L217 | L233 | L249 | S2A.4 | S2B.5 |
| D13 | D28 | D44 | D60 | D76 | D92 | L13 | L28 | L44 | L60 | L76 | L92 | L107 | L122 | L138 | L154 | L170 | L186 | L202 | L218 | L234 | L250 | S2A.5 | S2B.6 |
| D14 | D29 | D45 | D61 | D77 | D93 | L14 | L29 | L45 | L61 | L77 | L93 | L108 | L123 | L139 | L155 | L171 | L187 | L203 | L219 | L235 | L251 | S2A.6 | S2B.7 |
| | D30 | D46 | D62 | D78 | D94 | | L30 | L46 | L62 | L78 | L94 | | L124 | L140 | L156 | L172 | L188 | L204 | L220 | L236 | L252 | S2A.7 | |

| | | | | | | | | | | | | | | | | | | | | | | | | |
|---|---|---|---|---|---|---|---|---|---|---|---|---|---|---|---|---|---|---|---|---|---|---|---|---|
| 1 | | D15 | D31 | D47 | D63 | D79 | | L15 | L31 | L47 | L63 | L79 | | L109 | L125 | L141 | L157 | L173 | L189 | L205 | L221 | L237 | | |
| 2 | D1 | D16 | D32 | D48 | D64 | D80 | L1 | L16 | L32 | L48 | L64 | L80 | L95 | L110 | L126 | L142 | L158 | L174 | L190 | L206 | L222 | L238 | S1A.1 | S1B.1 |
| 3 | D2 | D17 | D33 | D49 | D65 | D81 | L2 | L17 | L33 | L49 | L65 | L81 | L96 | L111 | L127 | L143 | L159 | L175 | L191 | L207 | L223 | L239 | S1A.2 | S1B.2 |
| 4 | D3 | D18 | D34 | D50 | D66 | D82 | L3 | L18 | L34 | L50 | L66 | L82 | L97 | L112 | L128 | L144 | L160 | L176 | L192 | L208 | L224 | L240 | S1A.3 | S1B.3 |
| 5 | D4 | D19 | D35 | D51 | D67 | D83 | L4 | L19 | L35 | L51 | L67 | L83 | L98 | L113 | L129 | L145 | L161 | L177 | L193 | L209 | L225 | L241 | S1A.4 | S1B.4 |
| 6 | D5 | D20 | D36 | D52 | D68 | D84 | L5 | L20 | L36 | L52 | L68 | L84 | L99 | L114 | L130 | L146 | L162 | L178 | L194 | L210 | L226 | L242 | S1A.5 | S1B.5 |
| 7 | D6 | D21 | D37 | D53 | D69 | D85 | L6 | L21 | L37 | L53 | L69 | L85 | L100 | L115 | L131 | L147 | L163 | L179 | L195 | L211 | L227 | L243 | S1A.6 | S1B.6 |
| 8 | D7 | D22 | D38 | D54 | D70 | D86 | L7 | L22 | L38 | L54 | L70 | L86 | L101 | L116 | L132 | L148 | L164 | L180 | L196 | L212 | L228 | L244 | S1A.7 | S1B.7 |
| 9 | D8 | D23 | D39 | D55 | D71 | D87 | L8 | L23 | L39 | L55 | L71 | L87 | L102 | L117 | L133 | L149 | L165 | L181 | L197 | L213 | L229 | L245 | | S2B.1 |
| 10 | D9 | D24 | D40 | D56 | D72 | D88 | L9 | L24 | L40 | L56 | L72 | L88 | L103 | L118 | L134 | L150 | L166 | L182 | L198 | L214 | L230 | L246 | S2A.1 | S2B.2 |
| 11 | D10 | D25 | D41 | D57 | D73 | D89 | L10 | L25 | L41 | L57 | L73 | L89 | L104 | L119 | L135 | L151 | L167 | L183 | L199 | L215 | L231 | L247 | S2A.2 | S2B.3 |
| 12 | D11 | D26 | D42 | D58 | D74 | D90 | L11 | L26 | L42 | L58 | L74 | L90 | L105 | L120 | L136 | L152 | L168 | L184 | L200 | L216 | L232 | L248 | S2A.3 | S2B.4 |
| 13 | D12 | D27 | D43 | D59 | D75 | D91 | L12 | L27 | L43 | L59 | L75 | L91 | L106 | L121 | L137 | L153 | L169 | L185 | L201 | L217 | L233 | L249 | S2A.4 | S2B.5 |
| 14 | D13 | D28 | D44 | D60 | D76 | D92 | L13 | L28 | L44 | L60 | L76 | L92 | L107 | L122 | L138 | L154 | L170 | L186 | L202 | L218 | L234 | L250 | S2A.5 | S2B.6 |
| 15 | D14 | D29 | D45 | D61 | D77 | D93 | L14 | L29 | L45 | L61 | L77 | L93 | L108 | L123 | L139 | L155 | L171 | L187 | L203 | L219 | L235 | L251 | S2A.6 | S2B.7 |
| 16 | | D30 | D46 | D62 | D78 | D94 | | L30 | L46 | L62 | L78 | L94 | | L124 | L140 | L156 | L172 | L188 | L204 | L220 | L236 | L252 | S2A.7 | |

| | | | | | | | | | | | | | | | | | | | | | | | |
|---|---|---|---|---|---|---|---|---|---|---|---|---|---|---|---|---|---|---|---|---|---|---|---|
| | D15 | D31 | D47 | D63 | D79 | | L15 | L31 | L47 | L63 | L79 | | L109 | L125 | L141 | L157 | L173 | L189 | L205 | L221 | L237 | | |
| D1 | D16 | D32 | D48 | D64 | D80 | L1 | L16 | L32 | L48 | L64 | L80 | L95 | L110 | L126 | L142 | L158 | L174 | L190 | L206 | L222 | L238 | S1A.1 | S1B.1 |
| D2 | D17 | D33 | D49 | D65 | D81 | L2 | L17 | L33 | L49 | L65 | L81 | L96 | L111 | L127 | L143 | L159 | L175 | L191 | L207 | L223 | L239 | S1A.2 | S1B.2 |
| D3 | D18 | D34 | D50 | D66 | D82 | L3 | L18 | L34 | L50 | L66 | L82 | L97 | L112 | L128 | L144 | L160 | L176 | L192 | L208 | L224 | L240 | S1A.3 | S1B.3 |
| D4 | D19 | D35 | D51 | D67 | D83 | L4 | L19 | L35 | L51 | L67 | L83 | L98 | L113 | L129 | L145 | L161 | L177 | L193 | L209 | L225 | L241 | S1A.4 | S1B.4 |
| D5 | D20 | D36 | D52 | D68 | D84 | L5 | L20 | L36 | L52 | L68 | L84 | L99 | L114 | L130 | L146 | L162 | L178 | L194 | L210 | L226 | L242 | S1A.5 | S1B.5 |
| D6 | D21 | D37 | D53 | D69 | D85 | L6 | L21 | L37 | L53 | L69 | L85 | L100 | L115 | L131 | L147 | L163 | L179 | L195 | L211 | L227 | L243 | S1A.6 | S1B.6 |
| D7 | D22 | D38 | D54 | D70 | D86 | L7 | L22 | L38 | L54 | L70 | L86 | L101 | L116 | L132 | L148 | L164 | L180 | L196 | L212 | L228 | L244 | S1A.7 | S1B.7 |
| D8 | D23 | D39 | D55 | D71 | D87 | L8 | L23 | L39 | L55 | L71 | L87 | L102 | L117 | L133 | L149 | L165 | L181 | L197 | L213 | L229 | L245 | | S2B.1 |
| D9 | D24 | D40 | D56 | D72 | D88 | L9 | L24 | L40 | L56 | L72 | L88 | L103 | L118 | L134 | L150 | L166 | L182 | L198 | L214 | L230 | L246 | S2A.1 | S2B.2 |
| D10 | D25 | D41 | D57 | D73 | D89 | L10 | L25 | L41 | L57 | L73 | L89 | L104 | L119 | L135 | L151 | L167 | L183 | L199 | L215 | L231 | L247 | S2A.2 | S2B.3 |
| D11 | D26 | D42 | D58 | D74 | D90 | L11 | L26 | L42 | L58 | L74 | L90 | L105 | L120 | L136 | L152 | L168 | L184 | L200 | L216 | L232 | L248 | S2A.3 | S2B.4 |
| D12 | D27 | D43 | D59 | D75 | D91 | L12 | L27 | L43 | L59 | L75 | L91 | L106 | L121 | L137 | L153 | L169 | L185 | L201 | L217 | L233 | L249 | S2A.4 | S2B.5 |
| D13 | D28 | D44 | D60 | D76 | D92 | L13 | L28 | L44 | L60 | L76 | L92 | L107 | L122 | L138 | L154 | L170 | L186 | L202 | L218 | L234 | L250 | S2A.5 | S2B.6 |
| D14 | D29 | D45 | D61 | D77 | D93 | L14 | L29 | L45 | L61 | L77 | L93 | L108 | L123 | L139 | L155 | L171 | L187 | L203 | L219 | L235 | L251 | S2A.6 | S2B.7 |
| | D30 | D46 | D62 | D78 | D94 | | L30 | L46 | L62 | L78 | L94 | | L124 | L140 | L156 | L172 | L188 | L204 | L220 | L236 | L252 | S2A.7 | |

Figure 37

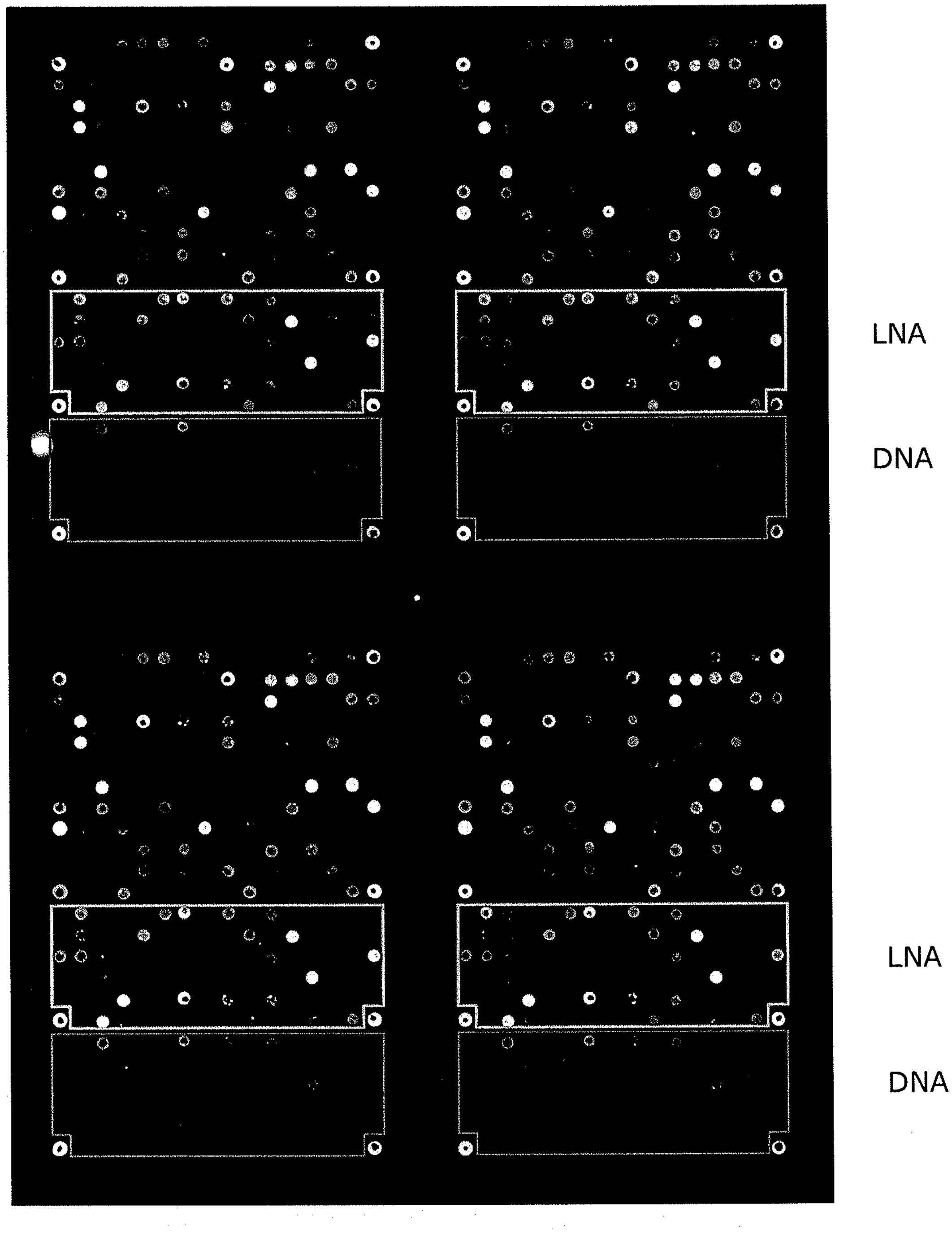

Figure 38

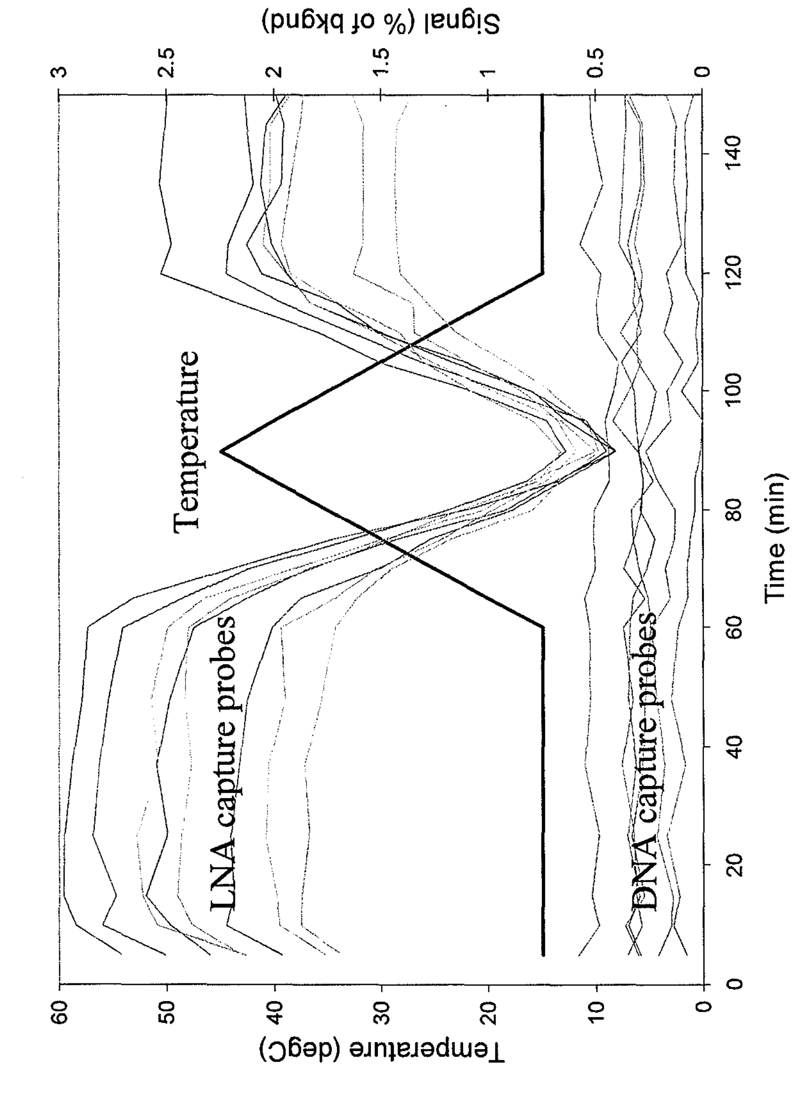
Temperature
LNA capture probes
DNA capture probes
Time (min)
Temperature (degC)
Signal (% of bkgnd)

Figure 39

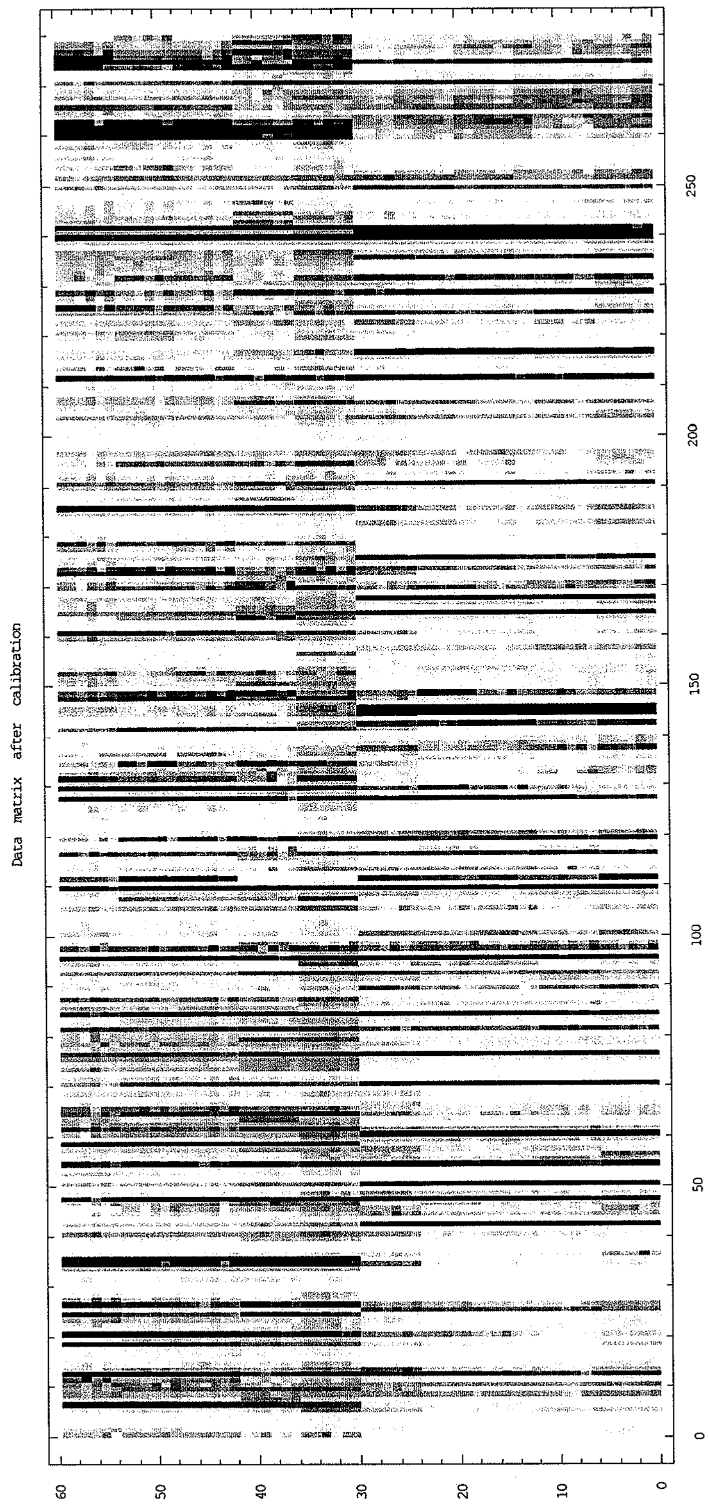
Data matrix after calibration
0
50
100
150
200
250
60
50
40
30
20
10
0

Figure 40

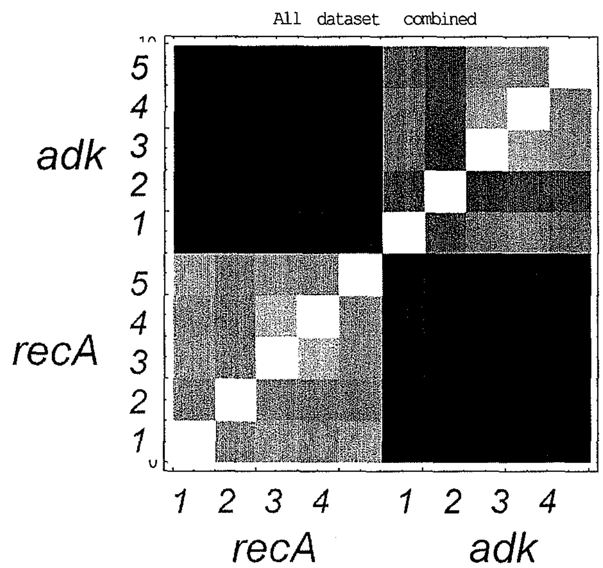
All dataset combined
adk
5
4
3
2
1
recA
5
4
3
2
1
1 2 3 4
recA
1 2 3 4
adk

Figure 41

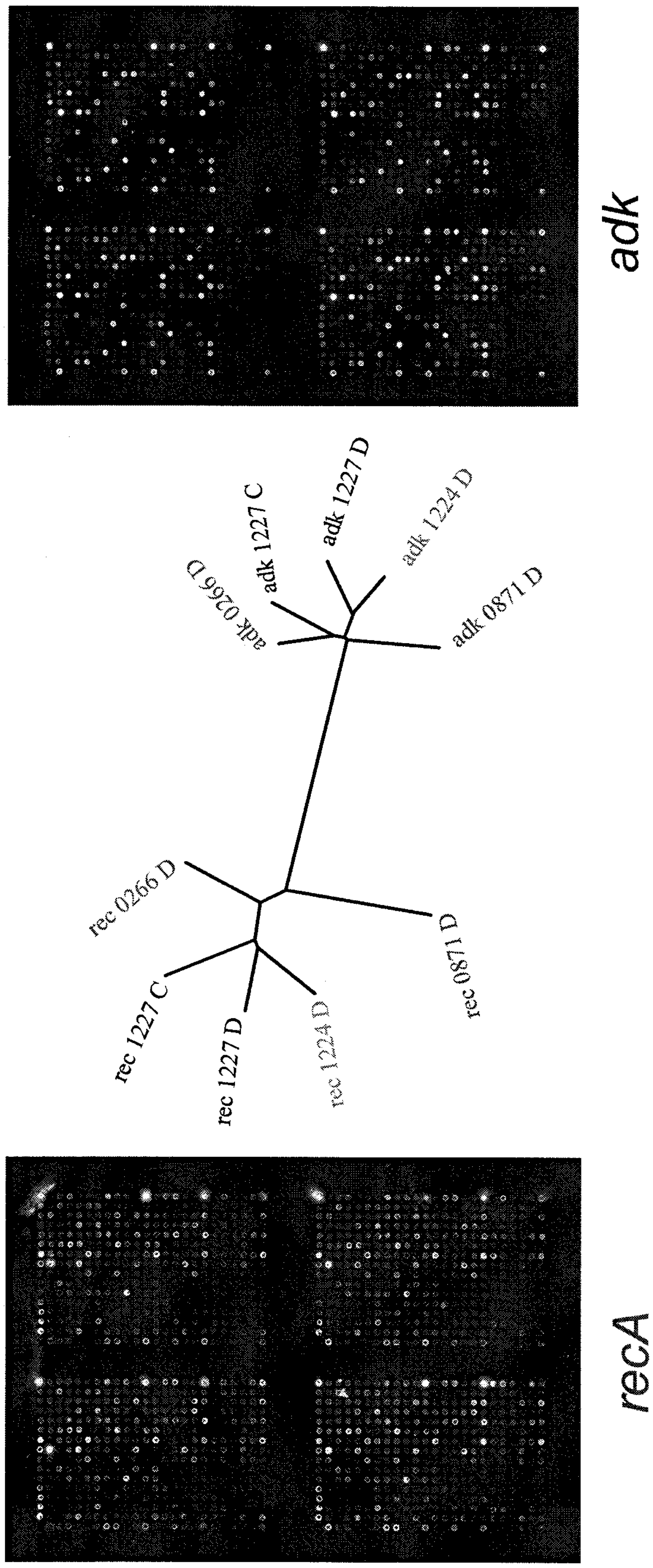
adk
adk 1227 C
adk 1227 D
adk 1224 D
adk 0871 D
adk 0266 D
rec 0266 D
rec 0871 D
rec 1224 D
rec 1227 D
rec 1227 C
recA

Figure 42

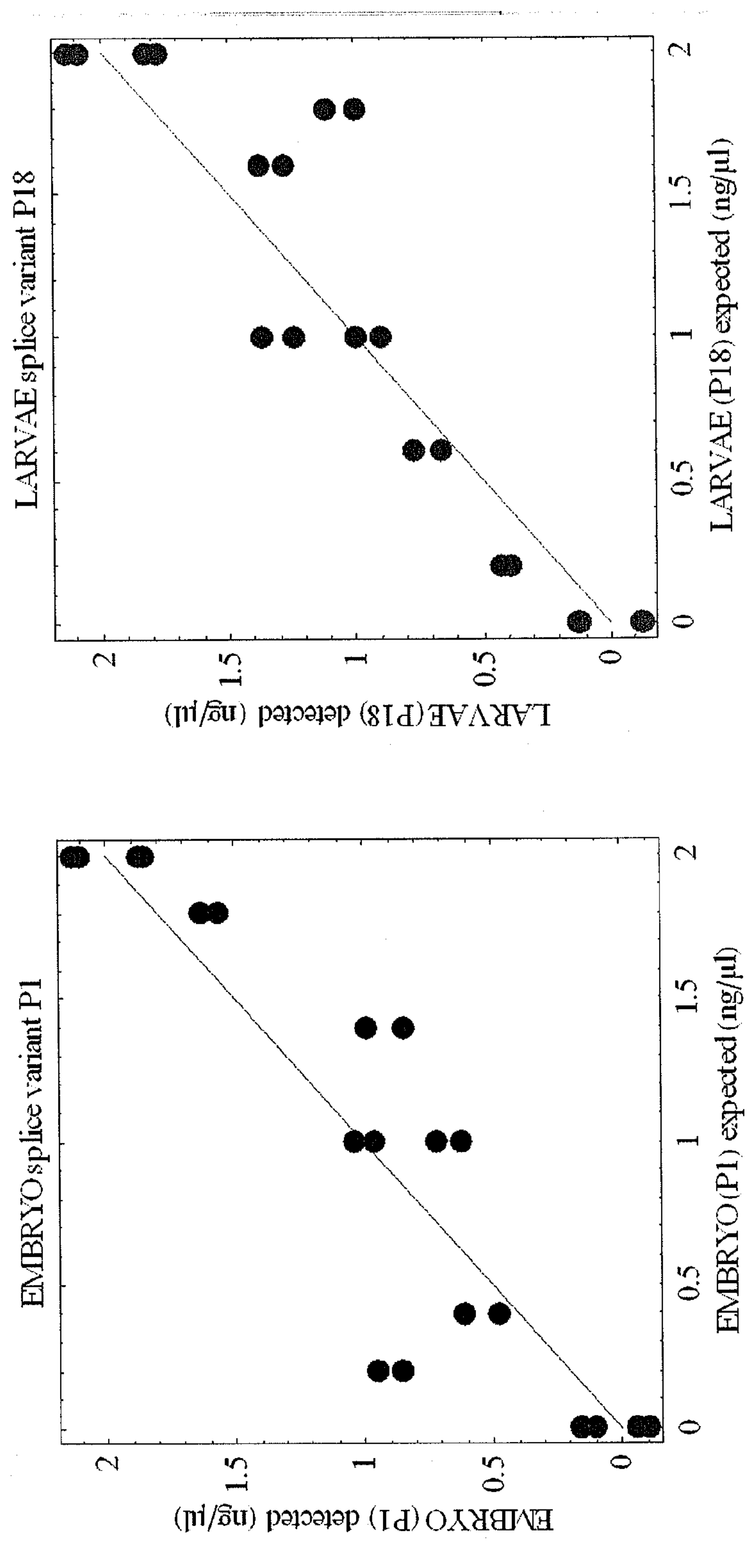
LARVAE splice variant P18
LARVAE (P18) detected (ng/µl)
LARVAE (P18) expected (ng/µl)
EMBRYO splice variant P1
EMBRYO (P1) detected (ng/µl)
EMBRYO (P1) expected (ng/µl)
0
0.5
1
1.5
2

Figure 43

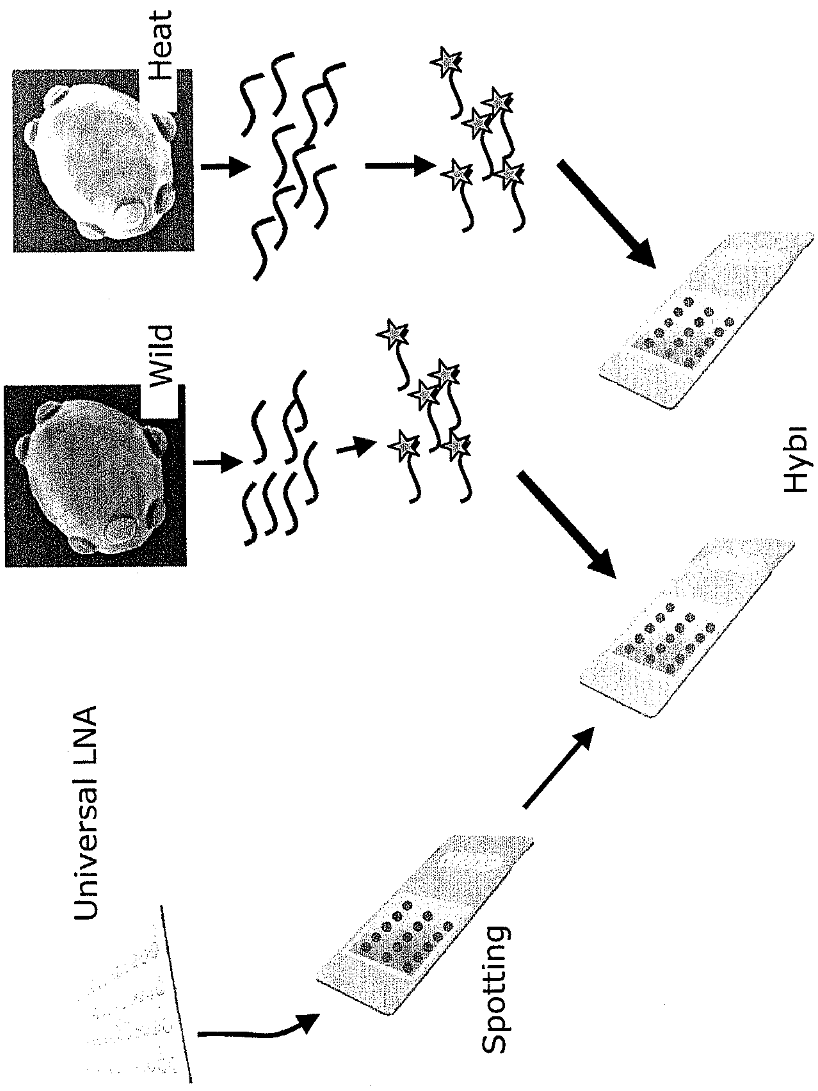

Figure 44

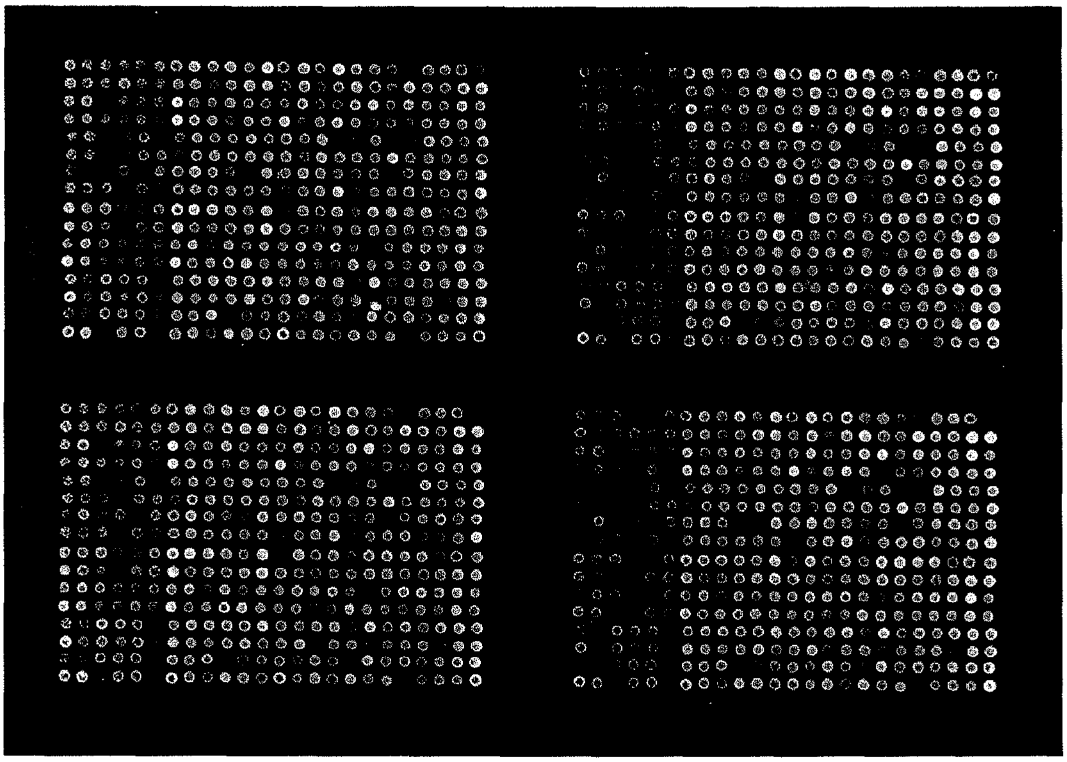

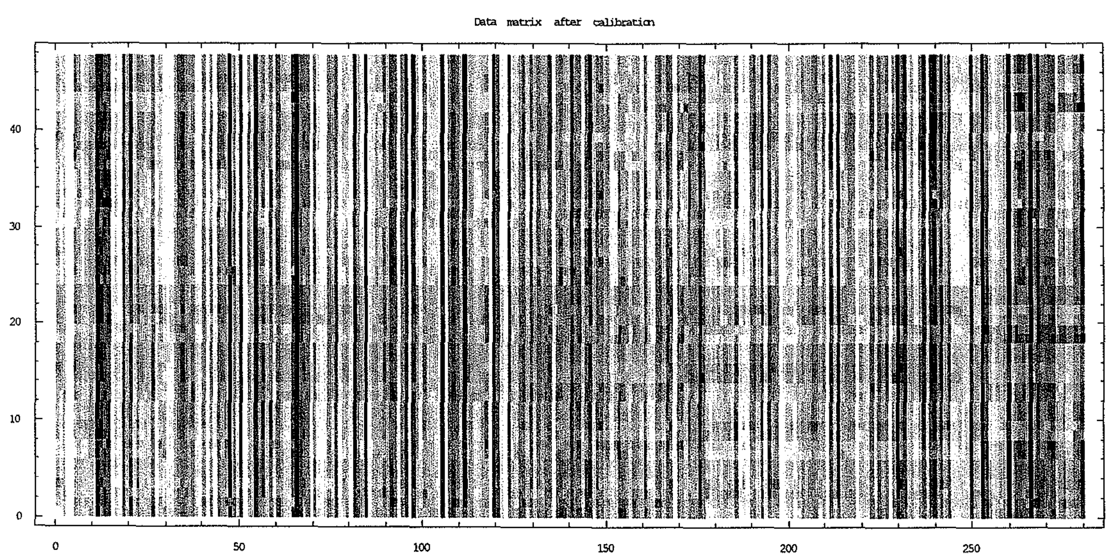
Data matrix after calibration
0
10
20
30
40
0
50
100
150
200
250

Figure 45

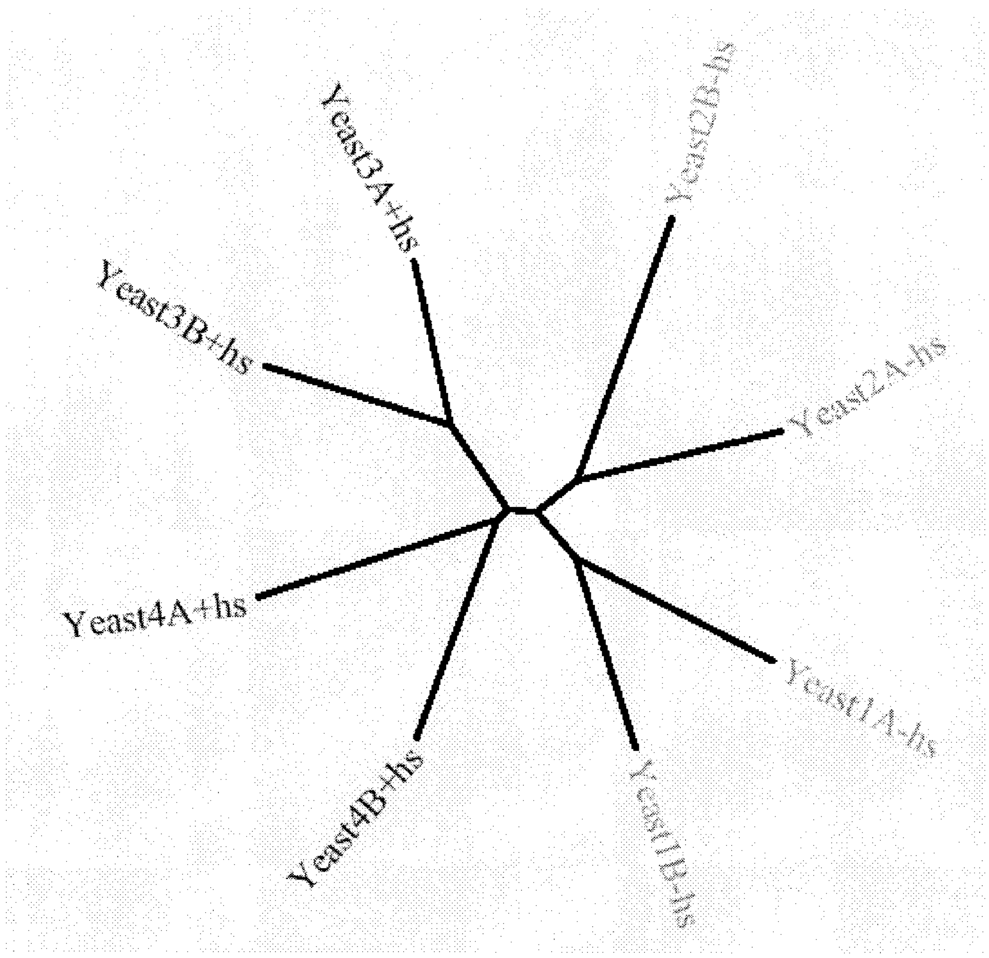
Yeast3A+hs
Yeast2B-hs
Yeast3B+hs
Yeast2A-hs
Yeast4A+hs
Yeast1A-hs
Yeast4B+hs
Yeast1B-hs

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20020197630 A
- WO 03020739 A2
- WO 9712896 A
- WO 9914226 A
- WO 0056746 A
- WO 0056748 A
- WO 0066604 A
- US 5432272 A
- US 3687808 A, Merigan
- US 6316198 B
- US 6303315 B
- WO 9631557 A
- US 6410229 B
- US 6406844 B
- US 6403957 B
- US 6403320 B
- US 6403317 B
- US 6346413 B
- US 6344316 B
- US 6329143 B
- US 6310189 B
- US 6309831 B
- US 6309823 B
- US 6261776 B
- US 6239273 B
- US 6238862 B
- US 6156501 A
- US 5945334 A
- US 5919523 A
- US 5889165 A
- US 5885837 A
- US 5744305 A
- US 5445934 A
- US 58009927 B
- US 5874219 B
- US 6033784 A


### Non-patent literature cited in the description


- Molecular Cloning, A Laboratory Manual. Cold Spring Harbor Laboratory Press, 1989
- Current Protocols In Molecular Biology. Current Publications, 1993
- DNA-protein interactions and The Polymerase Chain Reaction. Current Protocols In Molecular Biology. vol. 2
- **PETERSEN ; WENGEL.** *TIBTECH,* 2003, vol. 21, 74-81
- **HERMANSON.** Immobilized Affinity Ligand Techniques. Academic Press, 1992
- **MORITA et al.** *Bioorg. Med. Chem. Lett.,* 2002, vol. 12 (1), 73-76
- **HAKANSSON et al.** *Bioorg. Med. Chem. Lett.,* 2001, vol. 11 (7), 935-938
- **KOSHKIN et al.** *J. Org. Chem.,* 2001, vol. 66 (25), 8504-8512
- **KVAERNO et al.** *J. Org. Chem.,* 2001, vol. 66 (16), 5498-5503
- **HAKANSSON et al.** *J. Org. Chem.,* 2000, vol. 65 (17), 5161-5166
- **KVAERNO et al.** *J. Org. Chem.,* 2000, vol. 65 (17), 5167-5176
- **PFUNDHELLER et al.** *Nucleosides Nucleotides,* 1999, vol. 18 (9), 2017-2030
- **KUMAR et al.** *Bioorg. Med. Chem. Lett.,* 1998, vol. 8 (16), 2219-2222
- **SUSAN M. FREIER ; KARL-HEINZ ALTMANN.** *Nucleic Acids Research,* 1997, vol. 25, 4429-4443
- **SANGHVI.** Antisense Research and Application. CRC Press, 1993
- **ENGLISCH et al.** Angewandte Chemie. 1991, vol. 30, 613-722
- Concise Encyclopedia of Polymer Science and Engineering. John Wiley & Sons, 1990, 858-859
- **COOK.** *Anti-Cancer Drug Design,* 1991, vol. 6, 585-607
- **MESMAEKER.** *Current Opinion in Structural Biology,* 1995, vol. 5, 343-355
- **NIELSEN et al.** *J. Chem. Soc., Perkin Trans.,* 1997, vol. 1, 3423-33
- **SINGH et al.** *J. Org. Chem.,* 1998, vol. 6, 6078-9
- **BEIER et al.** *Science,* 1999, vol. 283, 699
- **ESCHENMOSER.** *Science,* 1999, vol. 284, 2118
- **C. R. NEWTON et al.** PCR. Springer-Verlag, 1997, 24
- **PEYMAN ; ULMANN.** *Chemical Reviews,* 1990, vol. 90, 543-584
- **CROOKE.** *Ann. Rev. Pharmacol. Toxicol,* 1992, vol. 32, 329-376

- **ZAMECNIK ; STEPHENSON.** *Proc. Natl. Acad. Sci.,* 1974, vol. 75, 280-284
- **MATRAY ; KOOL.** *J. Am. Chem. Soc.,* 1998, vol. 120, 6191
- **LYKKESFELD et al.** *Int. J. Cancer,* 1995, vol. 61, 529-534
- *Nature Genetics,* January 1999, vol. 21, 1-60
- **HARRINGTON ; LIEBER.** *Genes and Develop,* 1994, vol. 3, 1344
- **MURANTE et al.** *J. Biol. Chem.,* 1994, vol. 269, 1191
- **SAMUEL.** Host genetic variability and West Nile virus susceptibility. *Proc. Natl. Acad. Sci. USA,* 21 August 2002
- **BEASLEY.** *Virology,* 2002, vol. 296, 17-23
- **GAO et al.** *Nucleic Acid Research,* 2001, vol. 29, 4744-4750
- **BRESLAUER.** *Meth. Enzymol,* 1995, vol. 259, 221-242
- **SANTALUCIA.** *Proc. Natl. Acad. Sci.,* 1998, vol. 95, 1460-1465
- **ALLAWI et al.** *Biochemistry,* 1997, vol. 36, 10581-10594
- **TØSTESEN et al.** *J. Phys. Chem. B.,* 2001, vol. 105, 1618-1630
- **NIELSEN et al.** *Bioconjug. Chem.,* 2000, vol. 11, 228-238
- **KUTYAVIN et al.** *Biochemistry,* 1996, vol. 35, 11170
- **COMPAGNO et al.** *J. Biol. Chem.,* 1999, vol. 274, 8191
- **LOHSE et al.** *Proc Nat Sci USA, (PNAS,* 1999, vol. 96, 11804
- **IZVOLSKY et al.** *Biochemistry,* 2000, vol. 39, 10908
- **WOO et al.** *Nucleic Acid res,* 1996, vol. 24, 2470
- **SALADINO.** *Tetrahedron,* 1996, vol. 52, 6759
- **BROWN.** *J. Chem. Soc.,* 1957, 868
- **SINGER.** *Proc. Natl. Acad. Sci. USA,* 1983, vol. 80, 4884
- **RAJUR ; MCLAUGHLIN.** *Tetrahedron Lett.,* 1992, vol. 33, 6081
- **HAMAMURA.** *Moffatt, J. Med. Chem.,* 1972, vol. 15, 1061
- **BRETNER.** *J. Med. Chem.,* 1993, vol. 36, 3611
- **SHAW ; WARRENER.** *J. Chem. Soc.,* 1957, 153
- **CUSACK et al.** *J. Chem. Soc. Perkin,* 1973, vol. 1, 1721
- **KUIMELIS ; NAMBIAR.** *Nucleic Acid Res.,* 1994, vol. 22, 1429-1436
- **PEDERSEN et al.** *Synthesis,* 2002, 802
- **CARUTHERS.** *Acc. Chem. Res.,* 1991, vol. 24, 278
- **VORBRÜGGEN et al.** *Chem. Ber.,* 1981, vol. 114, 1234
- **VORBRÜGGEN et al.** *Chem. Ber.,* 1981, vol. 114, 1256
- **VORBRÜGGEN.** *Acta Biochim. Pol,* 1996, vol. 43, 25
- **MCBRIDE et al.** *Tetrahedron Lett.,* 1983, vol. 24, 245
- **SINHA et al.** *Tetrahedron Lett.,* 1983, vol. 24, 5843
- **SINHA et al.** *Nucleic Acids Res.,* 1984, vol. 12, 4539
- **MARTIN.** *Helv. Chim. Acta,* 1995, vol. 78, 486
- **FATHI et al.** *Tetrahedron Lett.,* 1990, vol. 31, 319
- **GRYAZNOV et al.** *Tetrahedron Lett.,* 1994, vol. 35, 2489
- **LAKSHMAN et al.** *Org. Lett,* 2000, vol. 2, 927
- **BOUDOU et al.** *Nucleic Acids Res.,* 1999, vol. 27, 1450
- **MCBRIDE et al.** *J. Am. Chem. Soc.,* 1986, vol. 108, 2040
- **CHANG ; WELCH.** *J. Med. Chem.,* 1963, vol. 6, 428
- **SONOGASHIRA ; TOHDA ; HAGIHARA.** *Tetrahedron Lett.,* 1975, 4467
- **WOO ; MEYER ; GAMPER.** *Nucleic Acids Res.,* 1996, vol. 24, 2470